(19) Europäisches Patentamt
European Patent Office
Office européen des brevets

(11) EP 4 212 177 A1

(12) EUROPEAN PATENT APPLICATION

(43) Date of publication:
**19.07.2023 Bulletin 2023/29**

(21) Application number: **22192363.4**

(22) Date of filing: **29.03.2019**

(51) International Patent Classification (IPC):
***A61K 45/06*** *(2006.01)* ***A61K 31/436*** *(2006.01)*
***A61K 31/138*** *(2006.01)* ***A61K 31/401*** *(2006.01)*
***A61P 37/06*** *(2006.01)*

(52) Cooperative Patent Classification (CPC):
**A01K 67/0275; A01K 67/0276; A01K 67/0278; C12N 15/8509;** A01K 2207/15; A01K 2207/30; A01K 2217/072; A01K 2217/075; A01K 2267/025

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.03.2018 EP 18165107**
**29.03.2018 US 201862650085 P**

(62) Document number(s) of the earlier application(s) in accordance with Art. 76 EPC:
**19713074.3 / 3 773 570**

(71) Applicant: **Reichart, Bruno**
**82319 Starnberg (DE)**

(72) Inventors:
- **Abicht, Jan-Michael**
  **82229 Seefeld (DE)**
- **Mayr, Tanja**
  **86368 Gersthofen (DE)**
- **Längin, Matthias**
  **80336 München (DE)**
- **Brenner, Paolo**
  **82061 Neuried (DE)**
- **Wolf, Eckhard**
  **85256 Vierkirchen (DE)**
- **Reichart, Bruno**
  **82319 Starnberg (DE)**
- **Klymiuk, Nikolai**
  **85411 Hohenkammer (DE)**

(74) Representative: **Hoffmann Eitle**
**Patent- und Rechtsanwälte PartmbB**
**Arabellastraße 30**
**81925 München (DE)**

Remarks:
This application was filed on 26-08-2022 as a divisional application to the application mentioned under INID code 62.

(54) **COMPOSITIONS FOR PROLONGING THE SURVIVAL AFTER ORTHOPIC AND HETEROPIC XENOGENEIC HEART TRANSPLANTATIONS**

(57) The present invention relates to methods for prolonging the survival of a primate that is transplanted a genetically modified heart, kidney, lung or liver from a xenogeneic mammal in a life supporting technique, and to compositions for use in a method of prolonging the survival of a primate that has been transplanted a genetically modified heart, kidney, lung or liver from a xenogeneic mammal in a life supporting technique. The invention is also directed to a living primate, whose heart, kidney, lung or liver is functionally substituted by a transplanted, genetically modified heart, kidney, lung or liver, respectively, from a xenogeneic mammal. Finally, the invention is directed to a genetically modified mammal and a donor organism for xenogeneic organ transplants as well as to methods of producing same.

EP 4 212 177 A1

## Description

### FIELD OF THE INVENTION

The present invention relates to methods for prolonging the survival of a primate that is transplanted a genetically modified heart, kidney, lung or liver from a xenogeneic mammal in a life supporting technique, and to compositions for use in a method of prolonging the survival of a primate that has been transplanted a genetically modified heart, kidney, lung or liver from a xenogeneic mammal in a life supporting technique. The invention is also directed to a living primate, whose heart, kidney, lung or liver is functionally substituted by a transplanted, genetically modified heart, kidney, lung or liver from a xenogeneic mammal. Finally, the invention is directed to a genetically modified mammal and a donor organism for xenogeneic organ transplants as well as to methods of producing same.

### BACKGROUND

Alternatives to allogeneic organ donations are necessary. To introduce genetically modified porcine hearts into the clinic, certain high-level experimental preconditions have been set up in the past, which nobody could meet during the last 25 years. We herein describe methods and compositions for successful life-supporting xenogeneic heart transplantation. These methods and compositions are also applicable to life-supporting xenogeneic kidney, lung and liver transplantation.

Cardiac replacement is still the treatment of choice in terminally ill patients. But the need for organs by far surpasses their supply (cf. references 1, 2, 3). Mechanical assist devices serve as alternative option, either in a bridge-to-transplantation or destination mode (cf. reference 4); severe method related events like bleeding, thrombosis or drive-line infection limit however their long-term success. Recent median survival of 298 days (longest 945 days) in the genetically modified (gm) pig-to-baboon beating, but non-working heterotopic abdominal heart transplant model (non-life supporting) proves the possibility of long-term immunologic acceptance of discordant organs, applying safe immunosuppression (IS; cf. reference 5). Before clinical use however, the guidelines of the ISHLT Advisory Board demand a good graft function for a minimum of 3 months in a life supporting (preferably orthotopic, but also in the intrathoracic heterotopic technique) position in at least two third of consecutive experiments (cf. reference 6), a result which has not been achieved during the last 25 years (cf. reference 7). In fact, it is even thought that orthotopic xenogeneic procedures are not possible in non-human primates.

### DESCRIPTION OF THE INVENTION

The present invention meets the above-described needs and solves the above-mentioned problems in the art by providing the embodiments described below.

The present inventors have found that when a genetically modified heart from a xenogeneic donor organism such as a pig is transplanted into a recipient primate, the transplanted heart overgrows, which ultimately causes the death of the recipient primate. Surprisingly, the inventors have found that the survival of a recipient primate could be significantly prolonged by applying a new transplantation method and/or by administering mTOR inhibitor/antihypertensive agent compositions to the recipient. Specifically, the inventors have surprisingly found that, *inter alia*, the following means improve the survival of the recipient:

- Preserving the heart from the donor organism by non-ischemic preservation until transplantation.
- Administering one or more antihypertensive agent(s) to the recipient after transplantation. Mammals such as pigs have a systolic blood pressure of +/- 80 mmHg, primates such as baboons +/-120 mmHg. The present inventors have found that anti-hypertensive treatment decreases a primate's higher values, which otherwise cause myocardial hypertrophy (overgrowth). Based on these findings, the inventors found that administration of one or more antihypertensive agent(s) prevents the overgrowth of the donor heart in the recipient organism.
- Administering an mTOR inhibitor to the recipient after transplantation. The present inventors surprisingly found that mTOR inhibitors are not only immunosuppressants, but also strong inhibitors of growth hormones in mammals such as pigs. Thus, also administration of an mTOR inhibitor prevents the overgrowth of the donor heart in the recipient organism.
- Decreasing the dosage of a glucocorticoid, which is administered to the recipient as part of an immunosuppressive maintenance therapy, early after transplantation. The inventors surprisingly found that also early weaning of the glucocorticoid prevents the overgrowth of the donor heart in the recipient organism.

The phenomenon of donor organ overgrowth in the recipient following transplantation has also been observed after kidney and lung transplantation (cf. reference 15). The recipients of such transplantations would suffer from the respective terminal diseases. Thus, the methods described in the present invention (in particular administering one or more antihypertensive agent(s) to the recipient after transplantation, administering an mTOR inhibitor to the recipient after transplantation and decreasing the dosage of a glucocorticoid, which is administered to the recipient as part of an immunosuppressive maintenance therapy, early after transplantation) are useful for any xenogeneic organ transplantation, including kidney transplantation, lung transplantation and liver transplantation.

Accordingly, the present invention provides methods for prolonging the survival of a primate that is transplanted a heart, kidney, lung or liver from a xenogeneic mammal and compositions for use in methods of prolonging the survival of a primate that has been transplanted a heart, kidney, lung or liver from a xenogeneic mammal by providing the preferred embodiments described in the following items. The invention also provides a living primate, whose heart, kidney, lung or liver is functionally substituted by a transplanted, genetically modified heart, kidney, lung or liver, respectively, from a xenogeneic mammal by providing the preferred embodiments described in the following items.

1. Method of prolonging the survival of a recipient organism that is transplanted a genetically modified heart, kidney, lung or liver from a xenogeneic donor organism, wherein the method comprises all or a subset of the following steps:

(i) administering an immunosuppressive induction therapy to the recipient organism according to a dosing regimen, wherein the dosing regimen comprises the administration of one or more immunosuppressive agent(s) before transplantation;

(ii) surgically extracting the heart, kidney, lung or liver from the donor organism and preserving the surgically extracted heart, kidney, lung or liver, respectively, by non-ischemic preservation until transplantation;

(iii) transplanting the surgically extracted, preserved heart, kidney, lung or liver of the donor organism into the recipient organism, so that it functionally substitutes the heart, kidney, lung or liver, respectively, of the recipient organism;

(iv) administering an immunosuppressive maintenance therapy to the recipient organism according to a dosing regimen, wherein the dosing regimen comprises the administration of one or more immunosuppressive agent(s) after transplantation, and wherein the dosing regimen comprises a first administration of a glucocorticoid within 1 day after transplantation;

(v) administering an anti-inflammatory therapy to the recipient organism according to a dosing regimen, wherein the dosing regimen comprises the administration of one or more anti-inflammatory agent(s) after transplantation;

(vi) administering one or more antihypertensive agent(s) to the recipient organism according to a dosing regimen, wherein the dosing regimen comprises the administration of one or more antihypertensive agent(s) after transplantation;

(vii) administering an mTOR inhibitor to the recipient organism according to a dosing regimen, wherein the dosing regimen comprises the administration of an mTOR inhibitor after transplantation; and

(viii) decreasing the dosage (mg/kg/day) of the glucocorticoid administered to the recipient organism according to the dosing regimen of step (iv) by a factor of at least 5, preferably by a factor of at least 10, within 5 days after transplantation;

wherein the donor organism is a mammal; and

wherein the recipient organism is a primate.

The above method according to item 1 may comprise only steps (vi) and (vii), but it preferably comprises steps (ii), (iii), (iv), (vi), (vii) and (viii), and it most preferably comprises all of the steps (i) to (viii).

2. The method of item 1, wherein the method is for preventing heart, kidney, lung or liver overgrowth.

3. The method of item 1 or 2, wherein the functional substitution of step (iii) is an orthotopic substitution.

4. The method of any of items 1 to 3, wherein the genetically modified heart, kidney, lung or liver has the following genetic modifications:

a) homozygous knock-out of the alpha-1,3-galactosyltransferase gene;

b) insertion of the human CD46 gene; and

c) insertion of the human thrombomodulin gene.

5. The method of any of items 1 to 3, wherein the genetically modified heart, kidney, lung or liver has the following genetic modifications:

a) homozygous knock-out of the alpha-1,3-galactosyltransferase gene, CMAH gene and B4GALNT2 gene; and

b) insertion of the human CD46 gene, the human CD55 gene, the human CD59 gene, a hemeoxygenase 1 gene and an A20 gene.

6. The method of item 5, wherein the genetically modified heart, kidney, lung or liver additionally has the following genetic modification:
c) insertion of a LEA gene or a PDL-1 gene.

7. The method of item 6, wherein the LEA gene is LEA29Y as shown in SEQ ID NO: 1.

8. The method of any of items 4 to 7, wherein the genetically modified heart, kidney, lung or liver additionally has the following genetic modification:
d) insertion of a sequence encoding human $\alpha$1,2-fucosyltransferase.

9. The method of any of items 1 to 8, wherein the donor organism belongs to the family *Suidae,* preferably wherein the donor organism belongs to the genus *Sus,* more preferably wherein the donor organism belongs to the species *S. scrofa,* most preferably wherein the donor organism belongs to the subspecies *S. s. domesticus*.

10. The method of any of items 1 to 9, wherein the donor organism belongs to the species *S. scrofa.*

11. The method of any of items 1 to 10, wherein the donor organism is a pig of German Landrace/Large White cross-bred genetic background, a Duroc pig, a Schwäbisch-Hällische pig, a black mini pig, or an Auckland Island pig; preferably wherein the donor organism is a pig of German Landrace/Large White cross-bred genetic background.

12. The method of any of items 1 to 11, wherein the recipient organism belongs to the family *Cercopithecidae,* preferably wherein the recipient organism belongs to the genus *Papio,* more preferably wherein the recipient organism belongs to the species *Papio Anubis* or *Papio Hamadryas,* most preferably wherein the recipient organism belongs to the species *Papio Anubis.*

13. The method of any of items 1 to 11, wherein the recipient organism belongs to the family *Homonidae,* preferably wherein the recipient organism belongs to the genus *Homo,* more preferably wherein the recipient organism is a human.

14. The method of any of items 1 to 13, wherein survival of the recipient organism is prolonged to more than 40 days after transplantation, to more than 50 days after transplantation, to more than 60 days after transplantation, to more than 70 days after transplantation, to more than 80 days after transplantation, or to more than 90 days after transplantation; preferably wherein survival of the recipient organism is prolonged to more than 50 days after transplantation, more preferably to more than 70 days after transplantation, and most preferably to more than 90 days after transplantation.

15. The method of any of items 1 to 14, wherein the dosing regimen of the immunosuppressive induction therapy of step (i) comprises administering one or more immunosuppressive agent(s) selected from the following list: anti-CD20 antibody, anti-thymocyte-globuline (ATG) and anti-CD40 antibody/anti-CD40L pasylated Fab; preferably wherein the dosing regimen comprises administering all of the above immunosuppressive agents.

16. The method of any of items 1 to 15, wherein the non-ischemic preservation of step (ii) is a non-ischemic heart, kidney, lung or liver perfusion.

17. The method of any of items 1 to 16, wherein the dosing regimen of the immunosuppressive maintenance therapy of step (iv) comprises the administration of the glucocorticoid at a dosage of 9 mg/kg/day to 11 mg/kg/day on the day of the first administration of the glucocorticoid.

18. The method of any of items 1 to 17, wherein the dosing regimen of the immunosuppressive maintenance therapy of step (iv) comprises administering a glucocorticoid and one or more immunosuppressive agent(s) selected from the following list: anti-CD20 antibody, anti-CD40 antibody/anti-CD40L pasylated Fab and mycophenolate-mofetil (MMF); preferably wherein the dosing regimen comprises administering all of the above immunosuppressive agents.

19. The method of any of items 1 to 18, wherein the dosing regimen of the anti-inflammatory therapy of step (v) comprises administering one or more anti-inflammatory agent(s) selected from the following list: IL-6-receptor antibody, TNF$\alpha$ inhibitor and IL-1-receptor antagonist; preferably wherein the dosing regimen comprises administering all of the above anti-inflammatory agents.

20. The method of any of items 1 to 19, wherein the dosage (mg/kg/day) of the glucocorticoid administered to the recipient organism according to the dosing regimen of step (iv) is decreased in step (viii) by a factor of at least 10, preferably by a factor of at least 20, within 10 days after transplantation.

21. The method of any of items 1 to 20, wherein the dosing regimen of step (vi) comprises administering one or more antihypertensive agent(s) selected from the following list: diuretics, calcium channel blockers, angiotensin-converting enzyme (ACE) inhibitors, angiotensin II receptor antagonists, adrenergic receptor antagonists, vasodilators, renin inhibitors, aldosterone receptor antagonists, alpha-2 adrenergic receptor agonists and endothelin receptor blockers, preferably wherein the dosing regimen comprises administering one or more antihypertensive agent(s) selected from angiotensin-converting enzyme (ACE) inhibitors and adrenergic receptor antagonists, more preferably wherein the dosing regimen comprises administering enalapril and/or metoprolol tartrate, most preferably wherein the dosing regimen comprises administering enalapril and metoprolol tartrate.

22. The method of any of items 1 to 21, wherein the dosing regimen of step (vi) comprises a first administration of one or more antihypertensive agent(s) within 2 days after transplantation.

23. The method of any of items 1 to 22, wherein the antihypertensive agent(s) of step (vi) are administered so that the mean arterial pressure of the recipient organism is kept within the range of 70 to 90 mmHg, preferably within the range of 75 to 85 mmHg.

24. The method of any of items 1 to 23, wherein the dosing regimen of step (vii) comprises administering an mTOR inhibitor selected from the following list: dactolisib (BEZ235, NVP-BEZ235), rapamycin (sirolimus), everolimus (RAD001), AZD8055, temsirolimus (CCI-779, NSC 683864), SF2523, 3BDO, PI-103, KU-0063794 I, torkinib (PP242), ridaforolimus (deforolimus, MK-8669), sapanisertib (INK 128, MLN0128), voxtalisib (SAR245409, XL765) analogue, torin 1, omipalisib (GSK2126458, GSK458), OSI-027, PF-04691502, apitolisib (GDC-0980, RG7422), GSK1059615, gedatolisib (PF-05212384, PKI-587), WYE-354, vistusertib (AZD2014), torin 2, WYE-125132 (WYE-132), BGT226 (NVP-BGT226), palomid 529 (P529), PP121, WYE-687, WAY-600, ETP-46464, GDC-0349, XL388, zotarolimus (ABT-578), CZ415, MHY1485, voxtalisib (XL765, SAR245409), chrysophanic acid and CC-223, LY3023414; preferably wherein the dosing regimen comprises administering temsirolimus.

25. The method of any of items 1 to 25, wherein the dosing regimen of step (vii) comprises a first administration of the mTOR inhibitor within 10 days after transplantation, preferably between 2 days and 9 days after transplantation, more preferably between 4 days and 7 days after transplantation.

26. The method of any of items 1 to 25, wherein the genetically modified heart, kidney, lung or liver is a genetically modified heart.

27. The method of any of items 1 to 25, wherein the genetically modified heart, kidney, lung or liver is a genetically modified kidney.

28. The method of any of items 1 to 25, wherein the genetically modified heart, kidney, lung or liver is a genetically modified lung.

29. The method of any of items 1 to 25, wherein the genetically modified heart, kidney, lung or liver is a genetically modified liver.

30. Living primate whose heart, kidney, lung or liver is functionally substituted by a transplanted, genetically modified heart, kidney, lung or liver, respectively, from a xenogeneic mammal, wherein said living primate is obtainable (or obtained) by the method according to any of items 1 to 29.

31. Living primate whose heart, kidney, lung or liver is functionally substituted by a transplanted, genetically modified heart, kidney, lung or liver, respectively, from a xenogeneic mammal, wherein the heart, kidney, lung or liver of said living primate has been transplanted more than 40 days ago, more than 50 days ago, more than 60 days ago, more than 70 days ago, more than 80 days ago, or more than 90 days ago, preferably more than 50 days ago, more preferably more than 70 days ago, and most preferably more than 90 days ago.

32. Living primate according to item 31, wherein said living primate is a non-human living primate.

33. Living primate according to item 31 or 32, wherein said living primate belongs to the family *Cercopithecidae,* preferably wherein said living primate belongs to the genus *Papio,* more preferably wherein said living primate belongs to the species *Papio Anubis* or *Papio Hamadryas,* most preferably wherein said living primate belongs to the species *Papio Anubis.*

34. Living primate according to any of items 31 to 33, wherein the xenogeneic mammal belongs to the family *Suidae,* preferably wherein the xenogeneic mammal belongs to the genus *Sus,* more preferably wherein the xenogeneic mammal belongs to the species *S. scrofa,* most preferably wherein the xenogeneic mammal belongs to the subspecies *S. s. domesticus.*

35. Living primate according to any of items 31 to 34, wherein the xenogeneic mammal belongs to the species *S. scrofa.*

36. Living primate according to any of items 31 to 35, wherein the xenogeneic mammal is a pig of German Landrace/Large White cross-bred genetic background, a Duroc pig, a Schwäbisch-Hällische pig, a black mini pig, or an Auckland Island pig; preferably wherein the xenogeneic mammal is a pig of German Landrace/Large White cross-bred genetic background.

37. Living primate according to any of items 31 to 36, wherein the genetically modified heart, kidney, lung or liver has the following genetic modifications:

a) homozygous knock-out of the alpha-1,3-galactosyltransferase gene;

b) insertion of the human CD46 gene; and

c) insertion of the human thrombomodulin gene.

38. Living primate according to any of items 31 to 36, wherein the genetically modified heart, kidney, lung or liver has the following genetic modifications:

a) homozygous knock-out of the alpha-1,3-galactosyltransferase gene, CMAH gene and B4GALNT2 gene; and

b) insertion of the human CD46 gene, the human CD55 gene, the human CD59 gene, a hemeoxygenase 1 gene and an A20 gene.

39. Living primate according to item 38, wherein the genetically modified heart, kidney, lung or liver additionally has the following genetic modification:
c) insertion of a LEA gene or a PDL-1 gene.

40. Living primate according to item 39, wherein the LEA gene is LEA29Y as shown in SEQ ID NO: 1.

41. Living primate according to any of items 37 to 40, wherein the genetically modified heart, kidney, lung or liver additionally has the following genetic modification:
d) insertion of a sequence encoding human $\alpha$1,2-fucosyltransferase.

42. Living primate according to any of items 31 to 41 whose heart is functionally substituted by a transplanted, genetically modified heart.

43. Living primate according to any of items 31 to 41 whose kidney is functionally substituted by a transplanted, genetically modified kidney.

44. Living primate according to any of items 31 to 41 whose lung is functionally substituted by a transplanted, genetically modified lung.

45. Living primate according to any of items 31 to 41 whose liver is functionally substituted by a transplanted, genetically modified liver.

46. Living primate according to item 42, wherein the transplanted heart of said living primate has a left ventricular mass that is equal to or less than 150% of the left ventricular mass of the same heart at the time of transplantation, preferably equal to or less than 140% of the left ventricular mass of the same heart at the time of transplantation, more preferably equal to or less than 130% of the left ventricular mass of the same heart at the time of transplantation, even more preferably equal to or less than 120% of the left ventricular mass of the same heart at the time of transplantation, and most preferably equal to or less than 110% of the left ventricular mass of the same heart at the time of transplantation.

47. Living primate according to any of items 31 to 46, wherein said living primate has a platelet count of between 150 and 300 g/l, preferably between 170 and 250 g/l, more preferably between 180 and 230 g/l.

48. Living primate according to any of items 31 to 47, wherein said living primate has a bilirubin level of equal to or less than 1.2 mg/dl, preferably of equal to or less than 1.0 mg/dl, more preferably of equal to or less than 0.8 mg/dl, most preferably of equal to or less than 0.6 mg/dl.

49. Living primate according to any of items 31 to 48, wherein said living primate has a troponin T (hs) level of equal to or less than 0.3 ng/ml, preferably of equal to or less than 0.2 ng/ml, more preferably of equal to or less than 0.1 ng/ml, most preferably of equal to or less than 0.014 ng/ml.

50. Living primate according to any of items 31 to 49, wherein said living primate has an LDH level of equal to or less than 1500 U/l, preferably of equal to or less than 1000 U/l, more preferably of equal to or less than 700 U/l, most preferably of equal to or less than 500 U/l.

51. Composition comprising an mTOR inhibitor for use in a method of prolonging the survival of a recipient organism that has been transplanted a genetically modified heart, kidney, lung or liver from a xenogeneic donor organism, wherein the donor organism is a mammal and wherein the recipient organism is a primate.

52. Composition comprising an mTOR inhibitor for use according to item 51, wherein the composition is for use in a method of preventing heart, kidney, lung or liver overgrowth.

53. Composition comprising an mTOR inhibitor for use according to item 51 or 52, wherein the recipient organism has been transplanted a genetically modified heart, kidney, lung or liver from a xenogeneic donor organism so that it functionally substitutes the heart, kidney, lung or liver, respectively, of the recipient organism.

54. Composition comprising an mTOR inhibitor for use according to item 53, wherein the functional substitution is an orthotopic substitution.

55. Composition comprising an mTOR inhibitor for use according to any of items 51 to 54, wherein the donor organism belongs to the family *Suidae,* preferably wherein the donor organism belongs to the genus *Sus,* more preferably wherein the donor organism belongs to the species *S. scrofa,* most preferably wherein the donor organism belongs to the subspecies *S. s. domesticus.*

56. Composition comprising an mTOR inhibitor for use according to any of items 51 to 55, wherein the donor organism belongs to the species *S. scrofa.*

57. Composition comprising an mTOR inhibitor for use according to any of items 51 to 56, wherein the donor organism is a pig of German Landrace/Large White cross-bred genetic background, a Duroc pig, a Schwäbisch-Hällische pig, a black mini pig, or an Auckland Island pig; preferably wherein the donor organism is a pig of German Landrace/Large White cross-bred genetic background.

58. Composition comprising an mTOR inhibitor for use according to any of items 51 to 57, wherein the recipient organism belongs to the family *Cercopithecidae,* preferably wherein the recipient organism belongs to the genus *Papio,* more preferably wherein the recipient organism belongs to the species *Papio Anubis* or *Papio Hamadryas,* most preferably wherein the recipient organism belongs to the species *Papio Anubis.*

59. Composition comprising an mTOR inhibitor for use according to any of items 51 to 57, wherein the recipient organism belongs to the family *Homonidae,* preferably wherein the recipient organism belongs to the genus *Homo,* more preferably wherein the recipient organism is a human.

60. Composition comprising an mTOR inhibitor for use according to any of items 51 to 59, wherein survival of the recipient organism is prolonged to more than 40 days after transplantation, to more than 50 days after transplantation, to more than 60 days after transplantation, to more than 70 days after transplantation, to more than 80 days after transplantation, or to more than 90 days after transplantation; preferably wherein survival of the recipient organism is prolonged to more than 50 days after transplantation, more preferably to more than 70 days after transplantation, and most preferably to more than 90 days after transplantation.

61. Composition comprising an mTOR inhibitor for use according to any of items 51 to 60, wherein the genetically modified heart, kidney, lung or liver has the following genetic modifications:

a) homozygous knock-out of the alpha-1,3-galactosyltransferase gene;

b) insertion of the human CD46 gene; and

c) insertion of the human thrombomodulin gene.

62. Composition comprising an mTOR inhibitor for use according to any of items 51 to 60, wherein the genetically modified heart, kidney, lung or liver has the following genetic modifications:

a) homozygous knock-out of the alpha-1,3-galactosyltransferase gene, CMAH gene and B4GALNT2 gene; and

b) insertion of the human CD46 gene, the human CD55 gene, the human CD59 gene, a hemeoxygenase 1 gene and an A20 gene.

63. Composition comprising an mTOR inhibitor for use according to item 62, wherein the genetically modified heart, kidney, lung or liver additionally has the following genetic modification:
c) insertion of a LEA gene or a PDL-1 gene.

64. Composition comprising an mTOR inhibitor for use according item 63, wherein the LEA gene is LEA29Y as shown in SEQ ID NO: 1.

65. Composition comprising an mTOR inhibitor for use according to any of items 61 to 64, wherein the genetically modified heart, kidney, lung or liver additionally has the following genetic modification:
d) insertion of a sequence encoding human $\alpha$1,2-fucosyltransferase.

66. Composition comprising an mTOR inhibitor for use according to any of items 51 to 65, wherein the mTOR inhibitor is selected from the following list: dactolisib (BEZ235, NVP-BEZ235), rapamycin (sirolimus), everolimus (RAD001), AZD8055, temsirolimus (CCI-779, NSC 683864), SF2523, 3BDO, PI-103, KU-0063794 I, torkinib (PP242), ridaforolimus (deforolimus, MK-8669), sapanisertib (INK 128, MLN0128), voxtalisib (SAR245409, XL765) analogue, torin 1, omipalisib (GSK2126458, GSK458), OSI-027, PF-04691502, apitolisib (GDC-0980, RG7422), GSK1059615, gedatolisib (PF-05212384, PKI-587), WYE-354, vistusertib (AZD2014), torin 2, WYE-125132 (WYE-132), BGT226 (NVP-BGT226), palomid 529 (P529), PP121, WYE-687, WAY-600, ETP-46464, GDC-0349, XL388, zotarolimus (ABT-578), CZ415, MHY1485, voxtalisib (XL765, SAR245409), chrysophanic acid and CC-223, LY3023414; preferably wherein the mTOR inhibitor is temsirolimus.

67. Composition comprising an mTOR inhibitor for use according to any of items 51 to 66, wherein the composition is administered according to a dosing regimen comprising a first administration of the composition within 10 days after transplantation, preferably between 2 days and 9 days after transplantation, more preferably between 4 days and 7 days after transplantation.

68. Composition comprising an mTOR inhibitor for use according to any of items 51 to 67, wherein the composition is administered according to a dosing regimen comprising a first administration and subsequent daily administrations.

69. Composition comprising an mTOR inhibitor for use according to any of items 51 to 68, wherein the recipient organism had received an immunosuppressive induction therapy according to a dosing regimen, wherein the dosing regimen had comprised the administration of one or more immunosuppressive agent(s) before transplantation.

70. Composition comprising an mTOR inhibitor for use according to item 69, wherein the dosing regimen of the immunosuppressive induction therapy had comprised administering one or more immunosuppressive agent(s) selected from the following list: anti-CD20 antibody, anti-thymocyte-globuline (ATG) and anti-CD40 antibody/anti-CD40L pasylated Fab; preferably wherein the dosing regimen had comprised administering all of the above immunosuppressive agents.

71. Composition comprising an mTOR inhibitor for use according to any of items 51 to 70, wherein the recipient organism receives an immunosuppressive maintenance therapy according to a dosing regimen, wherein the dosing regimen comprises the administration of one or more immunosuppressive agent(s) after transplantation, and wherein the dosing regimen comprises a first administration of a glucocorticoid that has occurred within 1 day after transplantation.

72. Composition comprising an mTOR inhibitor for use according to item 71, wherein the dosing regimen of the immunosuppressive maintenance therapy comprises the administration of the glucocorticoid at a dosage of 9 mg/kg/day to 11 mg/kg/day on the day of the first administration of the glucocorticoid.

73. Composition comprising an mTOR inhibitor for use according to item 71 or 72, wherein the dosing regimen of the immunosuppressive maintenance therapy comprises administering a glucocorticoid and one or more immunosuppressive agent(s) selected from the following list: anti-CD20 antibody, anti-CD40 antibody/anti-CD40L pasylated Fab and mycophenolate-mofetil (MMF); preferably wherein the dosing regimen comprises administering a glucocorticoid, anti-CD40 antibody/anti-CD40L pasylated Fab and mycophenolate-mofetil (MMF); most preferably wherein the dosing regimen comprises administering a glucocorticoid, anti-CD20 antibody, anti-CD40 antibody/anti-CD40L pasylated Fab and mycophenolate-mofetil (MMF).

74. Composition comprising an mTOR inhibitor for use according to any of items 71 to 73, wherein the dosage (mg/kg/day) of the glucocorticoid administered to the recipient organism according to the dosing regimen has been decreased by a factor of at least 5, preferably by a factor of at least 10, within 5 days after transplantation.

75. Composition comprising an mTOR inhibitor for use according to any of items 51 to 74, wherein the recipient organism receives anti-inflammatory therapy according to a dosing regimen.

76. Composition comprising an mTOR inhibitor for use according to item 75, wherein the dosing regimen of the anti-inflammatory therapy comprises administering one or more anti-inflammatory agent(s) selected from the following list: IL-6-receptor antibody, TNF$\alpha$ inhibitor and IL-1-receptor antagonist; preferably wherein the dosing regimen comprises administering all of the above anti-inflammatory agents.

77. Composition comprising an mTOR inhibitor for use according to any of items 51 to 76, wherein the recipient organism receives antihypertensive treatment according to a dosing regimen.

78. Composition comprising an mTOR inhibitor for use according to item 77, wherein the dosing regimen of the antihypertensive treatment comprises administering one or more antihypertensive agent(s) selected from the following list: diuretics, calcium channel blockers, angiotensin-converting enzyme (ACE) inhibitors, angiotensin II receptor antagonists, adrenergic receptor antagonists, vasodilators, renin inhibitors, aldosterone receptor antagonists, alpha-2 adrenergic receptor agonists and endothelin receptor blockers, preferably wherein the dosing regimen comprises administering one or more antihypertensive agent(s) selected from angiotensin-converting enzyme (ACE) inhibitors and adrenergic receptor antagonists, more preferably wherein the dosing regimen comprises administering enalapril and/or metoprolol tartrate, most preferably wherein the dosing regimen comprises administering enalapril and metoprolol tartrate.

79. Composition comprising an mTOR inhibitor for use according to item 77 or 78, wherein the dosing regimen of the antihypertensive treatment comprises a first administration of one or more antihypertensive agent(s) that has occurred within 2 days after transplantation.

80. Composition comprising an mTOR inhibitor for use according to any of items 77 to 79, wherein the antihypertensive agent(s) are administered so that the mean arterial pressure of the recipient organism is kept within the range of 70 to 90 mmHg, preferably within the range of 75 to 85 mmHg.

81. Composition comprising an mTOR inhibitor for use according to any of items 51 to 80, wherein before transplantation the heart, kidney, lung or liver from the xenogeneic donor organism had been preserved by non-ischemic preservation.

82. Composition comprising an mTOR inhibitor for use according to item 81, wherein the non-ischemic preservation is a non-ischemic heart, kidney, lung or liver perfusion.

83. Composition comprising an mTOR inhibitor for use according to any of items 51 to 82, wherein the genetically modified heart, kidney, lung or liver is a genetically modified heart.

84. Composition comprising an mTOR inhibitor for use according to any of items 51 to 82, wherein the genetically modified heart, kidney, lung or liver is a genetically modified kidney.

85. Composition comprising an mTOR inhibitor for use according to any of items 51 to 82, wherein the genetically modified heart, kidney, lung or liver is a genetically modified lung.

86. Composition comprising an mTOR inhibitor for use according to any of items 51 to 82, wherein the genetically modified heart, kidney, lung or liver is a genetically modified liver.

87. Composition comprising one or more antihypertensive agent(s) for use in a method of prolonging the survival of a recipient organism that has been transplanted a genetically modified heart, kidney, lung or liver from a xenogeneic donor organism, wherein the donor organism is a mammal and wherein the recipient organism is a primate.

88. Composition comprising one or more antihypertensive agent(s) for use according to item 87, wherein the composition is for use in a method of preventing heart, kidney, lung or liver overgrowth.

89. Composition comprising one or more antihypertensive agent(s) for use according to item 87 or 88, wherein the recipient organism has been transplanted a genetically modified heart, kidney, lung or liver from a xenogeneic donor organism so that it functionally substitutes the heart, kidney, lung or liver, respectively, of the recipient organism.

90. Composition comprising one or more antihypertensive agent(s) for use according to item 89, wherein the functional substitution is an orthotopic substitution.

91. Composition comprising one or more antihypertensive agent(s) for use according to any of items 87 to 90, wherein the donor organism belongs to the family *Suidae,* preferably wherein the donor organism belongs to the genus *Sus,* more preferably wherein the donor organism belongs to the species *S. scrofa,* most preferably wherein the donor organism belongs to the subspecies *S. s. domesticus.*

92. Composition comprising one or more antihypertensive agent(s) for use according to any of items 87 to 91, wherein the donor organism belongs to the species *S. scrofa.*

93. Composition comprising one or more antihypertensive agent(s) for use according to any of items 87 to 92, wherein the donor organism is a pig of German Landrace/Large White cross-bred genetic background, a Duroc pig, a Schwäbisch-Hällische pig, a black mini pig, or an Auckland Island pig; preferably wherein the donor organism is a pig of German Landrace/Large White cross-bred genetic background.

94. Composition comprising one or more antihypertensive agent(s) for use according to any of items 87 to 93, wherein the recipient organism belongs to the family *Cercopithecidae,* preferably wherein the recipient organism belongs to the genus *Papio,* more preferably wherein the recipient organism belongs to the species *Papio Anubis* or *Papio Hamadryas,* most preferably wherein the recipient organism belongs to the species *Papio Anubis.*

95. Composition comprising one or more antihypertensive agent(s) for use according to any one of items 87 to 93, wherein the recipient organism belongs to the family *Homonidae,* preferably wherein the recipient organism belongs to the genus *Homo,* more preferably wherein the recipient organism is a human.

96. Composition comprising one or more antihypertensive agent(s) for use according to any of items 87 to 95, wherein survival of the recipient organism is prolonged to more than 40 days after transplantation, to more than 50 days after transplantation, to more than 60 days after transplantation, to more than 70 days after transplantation, to more than 80 days after transplantation, or to more than 90 days after transplantation; preferably wherein survival of the recipient organism is prolonged to more than 50 days after transplantation, more preferably to more than 70 days after transplantation, and most preferably to more than 90 days after transplantation.

97. Composition comprising one or more antihypertensive agent(s) for use according to any of items 87 to 96, wherein the genetically modified heart, kidney, lung or liver has the following genetic modifications:

a) homozygous knock-out of the alpha-1,3-galactosyltransferase gene;

b) insertion of the human CD46 gene; and

c) insertion of the human thrombomodulin gene.

98. Composition comprising one or more antihypertensive agent(s) for use according to any of items 87 to 96, wherein the genetically modified heart, kidney, lung or liver has the following genetic modifications:

a) homozygous knock-out of the alpha-1,3-galactosyltransferase gene, CMAH gene and B4GALNT2 gene; and

b) insertion of the human CD46 gene, the human CD55 gene, the human CD59 gene, a hemeoxygenase 1 gene and an A20 gene.

99. Composition comprising one or more antihypertensive agent(s) for use according to item 98, wherein the genetically modified heart, kidney, lung or liver additionally has the following genetic modification:
c) insertion of a LEA gene or a PDL-1 gene.

100. Composition comprising one or more antihypertensive agent(s) for use according item 99, wherein the LEA gene is LEA29Y as shown in SEQ ID NO: 1.

101. Composition comprising one or more antihypertensive agent(s) for use according to any of items 97 to 100, wherein the genetically modified heart, kidney, lung or liver additionally has the following genetic modification:
d) insertion of a sequence encoding human $\alpha$1,2-fucosyltransferase.

102. Composition comprising one or more antihypertensive agent(s) for use according to any of items 87 to 101, wherein the antihypertensive agent(s) are one or more agent(s) selected from the following list: diuretics, calcium channel blockers, angiotensin-converting enzyme (ACE) inhibitors, angiotensin II receptor antagonists, adrenergic receptor antagonists, vasodilators, renin inhibitors, aldosterone receptor antagonists, alpha-2 adrenergic receptor agonists and endothelin receptor blockers, preferably wherein the antihypertensive agent(s) are selected from angiotensin-converting enzyme (ACE) inhibitors and adrenergic receptor antagonists, more preferably wherein the antihypertensive agent(s) is/are enalapril and/or metoprolol tartrate, most preferably wherein the antihypertensive agents are enalapril and metoprolol tartrate.

103. Composition comprising one or more antihypertensive agent(s) for use according to any of items 87 to 102, wherein the composition is administered according to a dosing regimen comprising a first administration of the composition within 2 days after transplantation.

104. Composition comprising one or more antihypertensive agent(s) for use according to any of items 87 to 103, wherein the composition is administered so that the mean arterial pressure of the recipient organism is kept within the range of 70 to 90 mmHg, preferably within the range of 75 to 85 mmHg.

105. Composition comprising one or more antihypertensive agent(s) for use according to any of items 87 to 104, wherein the recipient organism had received an immunosuppressive induction therapy according to a dosing regimen, wherein the dosing regimen had comprised the administration of one or more immunosuppressive agent(s) before transplantation.

106. Composition comprising one or more antihypertensive agent(s) for use according to item 105, wherein the dosing regimen of the immunosuppressive induction therapy had comprised administering one or more immunosuppressive agent(s) selected from the following list: anti-CD20 antibody, anti-thymocyte-globuline (ATG) and anti-CD40 antibody/anti-CD40L pasylated Fab; preferably wherein the dosing regimen had comprised administering all of the above immunosuppressive agents.

107. Composition comprising one or more antihypertensive agent(s) for use according to any of items 87 to 106, wherein the recipient organism receives an immunosuppressive maintenance therapy according to a dosing regimen, wherein the dosing regimen comprises the administration of one or more immunosuppressive agent(s) after transplantation, and wherein the dosing regimen comprises a first administration of a glucocorticoid that has occurred within 1 day after transplantation.

108. Composition comprising one or more antihypertensive agent(s) for use according to item 107, wherein the dosing regimen of the immunosuppressive maintenance therapy comprises the administration of the glucocorticoid at a dosage of 9 mg/kg/day to 11 mg/kg/day on the day of the first administration of the glucocorticoid.

109. Composition comprising one or more antihypertensive agent(s) for use according to item 107 or 108, wherein the dosing regimen of the immunosuppressive maintenance therapy comprises administering a glucocorticoid and one or more immunosuppressive agent(s) selected from the following list: anti-CD20 antibody, anti-CD40 antibody/anti-CD40L pasylated Fab and mycophenolate-mofetil (MMF); preferably wherein the dosing regimen comprises administering a glucocorticoid, anti-CD40 antibody/anti-CD40L pasylated Fab and mycophenolate-mofetil (MMF); most preferably wherein the dosing regimen comprises administering a glucocorticoid, anti-CD20 antibody, anti-CD40 antibody/anti-CD40L pasylated Fab and mycophenolate-mofetil (MMF).

110. Composition comprising one or more antihypertensive agent(s) for use according to any of items 107 to 109, wherein the dosage (mg/kg/day) of the glucocorticoid administered to the recipient organism according to the dosing regimen has been decreased by a factor of at least 5, preferably by a factor of at least 10, within 5 days after transplantation.

111. Composition comprising one or more antihypertensive agent(s) for use according to any of items 87 to 110, wherein the recipient organism receives anti-inflammatory therapy according to a dosing regimen.

112. Composition comprising one or more antihypertensive agent(s) for use according to item 111, wherein the dosing regimen of the anti-inflammatory therapy comprises administering one or more anti-inflammatory agent(s) selected from the following list: IL-6-receptor antibody, TNF$\alpha$ inhibitor and IL-1-receptor antagonist; preferably wherein the dosing regimen comprises administering all of the above anti-inflammatory agents.

113. Composition comprising one or more antihypertensive agent(s) for use according to any of items 87 to 112, wherein the recipient organism is administered an mTOR inhibitor according to a dosing regimen.

114. Composition comprising one or more antihypertensive agent(s) for use according to item 113, wherein the mTOR inhibitor is selected from the following list: dactolisib (BEZ235, NVP-BEZ235), rapamycin (sirolimus), everolimus (RAD001), AZD8055, temsirolimus (CCI-779, NSC 683864), SF2523, 3BDO, PI-103, KU-0063794 I, torkinib (PP242), ridaforolimus (deforolimus, MK-8669), sapanisertib (INK 128, MLN0128), voxtalisib (SAR245409, XL765) analogue, torin 1, omipalisib (GSK2126458, GSK458), OSI-027, PF-04691502, apitolisib (GDC-0980, RG7422), GSK1059615, gedatolisib (PF-05212384, PKI-587), WYE-354, vistusertib (AZD2014), torin 2, WYE-125132 (WYE-132), BGT226 (NVP-BGT226), palomid 529 (P529), PP121, WYE-687, WAY-600, ETP-46464, GDC-0349, XL388, zotarolimus (ABT-578), CZ415, MHY1485, voxtalisib (XL765, SAR245409), chrysophanic acid and CC-223, LY3023414; preferably wherein the mTOR inhibitor is temsirolimus.

115. Composition comprising one or more antihypertensive agent(s) for use according to item 113 or 114, wherein the dosing regimen comprises a first administration of the mTOR inhibitor that has occurred within 10 days after transplantation, preferably between 2 days and 9 days after transplantation, more preferably between 4 days and 7 days after transplantation.

116. Composition comprising one or more antihypertensive agent(s) for use according to any of items 113 to 115, wherein the mTOR inhibitor is administered according to a dosing regimen comprising a first administration and subsequent daily administrations.

117. Composition comprising one or more antihypertensive agent(s) for use according to any of items 87 to 116, wherein before transplantation the heart, kidney, lung or liver from the xenogeneic donor organism had been preserved by non-ischemic preservation.

118. Composition comprising one or more antihypertensive agent(s) for use according to item 117, wherein the non-ischemic preservation is a non-ischemic heart, kidney, lung or liver perfusion.

119. Composition comprising one or more antihypertensive agent(s) for use according to any of items 87 to 118, wherein the genetically modified heart, kidney, lung or liver is a genetically modified heart.

120. Composition comprising one or more antihypertensive agent(s) for use according to any of items 87 to 118, wherein the genetically modified heart, kidney, lung or liver is a genetically modified kidney.

121. Composition comprising one or more antihypertensive agent(s) for use according to any of items 87 to 118, wherein the genetically modified heart, kidney, lung or liver is a genetically modified lung.

122. Composition comprising one or more antihypertensive agent(s) for use according to any of items 87 to 118, wherein the genetically modified heart, kidney, lung or liver is a genetically modified liver.
It will be appreciated that the features of the living primate in items 30-50 and/or the features of the composition of items 51-122 also may apply to the method or the recipient of the method of items 1-29.
As mentioned above, the inventors have surprisingly found that when the method of prolonging the survival of a recipient organism that is transplanted a genetically modified heart is performed and/or the compositions are administered in accordance with the invention, the survival of the heart recipient is significantly prolonged. Additionally, when the method is performed and/or the compositions are administered in accordance with the invention, the transplanted hearts do not overgrow in the recipients, and no or only minor heart-related macroscopic (e.g., in the appearance of ventricular walls and cavities), microscopic (e.g., in immunohistochemical analyses of the heart), functional (e.g. in echocardiography) or biochemical (e.g., in blood values) anomalies are observed (see Figure 1 and Figure 6, in particular the brief description of Figure 6 below).
In the above-described method of prolonging the survival of a recipient organism, said recipient organism is transplanted a genetically modified heart, kidney, lung or liver from a xenogeneic donor organism. Similarly, the above-described living primate has been transplanted a genetically modified heart, kidney, lung or liver, from a xenogeneic mammal, and the above-described compositions comprising an mTOR inhibitor/antihypertensive agent(s) are for use in a method of prolonging the survival of a recipient organism that has been transplanted a genetically modified heart, kidney, lung or liver from a xenogeneic donor organism. Accordingly, the present invention also provides a genetically modified mammal that can, in one embodiment, serve as xenogeneic donor organism for such genetically modified heart, kidney, lung or liver. This genetically modified mammal has an insertion of a sequence encoding a human complement-regulatory protein as well as an insertion of a sequence encoding human thrombomodulin. Both of these sequences are inserted into the endogenous N-acetyllactosaminide alpha-1,3-galactosyltransferase locus (in the following also referred to as GGTA1 locus or alpha-1,3-galactosyltransferase gene locus), so that the GGTA1 locus is interrupted and GGTA1 is no longer expressed. The sequence encoding the human complement-regulatory protein is inserted such that its expression is under the control of the endogenous GGTA1 promoter. This design provides, *inter alia*, the following advantages: Introducing the sequence encoding the human complement-regulatory protein as well as the sequence encoding human thrombomodulin at a single locus (i.e., the GGTA1 locus) prevents segregation of these genes during breeding of the genetically modified mammal. Additionally, the GGTA1 locus is known to be a transcriptionally active site that allows sufficient transcription from a safe harbor. Accordingly, the present invention provides a genetically modified mammal and methods of producing same by providing the preferred embodiments described in the following items:

123. A genetically modified mammal that has the following genetic modifications:

a) insertion of a sequence encoding a human complement-regulatory protein, and

b) insertion of a sequence encoding human thrombomodulin;

wherein the sequence encoding a human complement-regulatory protein and the sequence encoding human thrombomodulin are inserted at the alpha-1,3-galactosyltransferase gene locus, wherein at least the sequence encoding a human complement-regulatory protein is expressed under the control of the endogenous promoter of the alpha-1,3-galactosyltransferase gene; and

wherein the genetically modified mammal is non-human.

124. The genetically modified mammal of item 123, wherein both the sequence encoding a human complement-regulatory protein and the sequence encoding human thrombomodulin are expressed under the control of the endogenous promoter of the alpha-1,3-galactosyltransferase gene.

125. The genetically modified mammal of item 123, wherein the sequence encoding a human complement-regulatory protein is expressed under the control of the endogenous promoter of the alpha-1,3-galactosyltransferase gene, and wherein the sequence encoding human thrombomodulin is expressed under the control of an exogenous promoter.

126. The genetically modified mammal of item 125, wherein the exogenous promoter that controls the expression of the sequence encoding human thrombomodulin is a ubiquitously active promoter.

127. The genetically modified mammal of any of items 123 to 126, wherein the genetically modified mammal lacks expression of alpha-1,3-galactosyltransferase.

128. The genetically modified mammal of any of items 123 to 127, wherein the sequence encoding a human complement-regulatory protein is a sequence encoding human CD46, human CD55, or human CD59.

129. The genetically modified mammal of any of items 123 to 128, wherein the sequence encoding a human complement-regulatory protein is a sequence encoding human CD46.

130. The genetically modified mammal of any of items 123 to 129, wherein the genetically modified mammal additionally has the following genetic modifications:

c) homozygous knock-out of the CMAH gene, and

d) homozygous knock-out of the B4GALNT2 gene.

131. The genetically modified mammal of any of items 123 to 130, wherein the genetically modified mammal additionally has the following genetic modification:
e) insertion of a selectable marker gene sequence between the sequence encoding a human complement-regulatory protein and the sequence encoding human thrombomodulin.

132. The genetically modified mammal of item 131, wherein the selectable marker gene sequence is flanked by loxP sites.

133. The genetically modified mammal of any of items 123 to 132, wherein genetically modified mammal additionally has the following genetic modification:
f) insertion of a sequence encoding human $\alpha$1,2-fucosyltransferase.

134. The genetically modified mammal of any of items 123 to 133, wherein the genetically modified mammal has one or more additional genetic modification(s).

135. The genetically modified mammal of item 134, wherein the one or more additional genetic modification(s) is/are insertion(s).

136. The genetically modified mammal of item 133 or 135, wherein the one or more insertion(s) is/are at the alpha-1,3-galactosyltransferase gene locus.

137. The genetically modified mammal of any of items 123 to 136, wherein the genetically modified mammal belongs to the family *Suidae,* preferably wherein the genetically modified mammal belongs to the genus *Sus,* more preferably wherein the genetically modified mammal belongs to the species S. *scrofa,* most preferably wherein the genetically modified mammal belongs to the subspecies S. s. *domesticus.*

138. The genetically modified mammal of any of items 123 to 137, wherein the genetically modified mammal belongs to the species *S. scrofa.*

139. The genetically modified mammal of any of items 123 to 138, wherein the genetically modified mammal is a pig of German Landrace/Large White cross-bred genetic background, a Duroc pig, a Swabian-Hall pig, a black mini pig, or an Auckland Island pig; preferably wherein the donor mammal is a pig of German Landrace/Large White cross-bred genetic background.

140. The genetically modified mammal of any of items 123 to 139, wherein the genetically modified mammal is free of porcine endogenous retrovirus C (PERV-C).

141. The genetically modified mammal of any of items 123 to 140, wherein the genetically modified mammal is suitable to serve as donor organism for xenogeneic organ transplants.

142. An organ derived from the genetically modified mammal of any of items 123 to 141.

143. A heart derived from the genetically modified mammal of any of items 123 to 141.

144. A kidney derived from the genetically modified mammal of any of items 123 to 141.

145. A lung derived from the genetically modified mammal of any of items 123 to 141.

146. A liver derived from the genetically modified mammal of any of items 123 to 141.

147. A cell derived from the genetically modified mammal of any of items 123 to 141.

148. The method of any of items 1 to 29, wherein the xenogeneic donor organism is a genetically modified mammal according to any of items 123 to 141.

149. Living primate according to any of items 30 to 50, wherein the xenogeneic mammal is a genetically modified mammal according to any of items 123 to 141.

150. Composition comprising an mTOR inhibitor for use according to any of items 51 to 86, wherein the xenogeneic donor organism is a genetically modified mammal according to any of items 123 to 141.

151. Composition comprising one or more antihypertensive agent(s) for use according to any of items 87 to 122, wherein the xenogeneic donor organism is a genetically modified mammal according to any of items 123 to 141.

152. A method of making a genetically modified mammal comprising the following steps:

(a) inserting a sequence encoding a human complement-regulatory protein and a sequence encoding human thrombomodulin at the alpha-1,3-galactosyltransferase gene locus of a mammalian genome to provide a genetically modified genome;

(b) introducing the genetically modified genome into a cell; and

(c) permitting the cell comprising the genetically modified genome to mature into a genetically modified mammal;

wherein the genetically modified mammal is non-human.

153. The method of item 152, wherein the genome is a somatic cell genome and wherein the genetically modified genome is introduced into the cell by somatic cell nuclear transfer.

154. The method of item 152 or 153, wherein the sequence encoding a human complement-regulatory protein is a sequence encoding human CD46, human CD55, or human CD59.

155. The method of any of items 152 to 154, wherein the sequence encoding a human complement-regulatory protein is a sequence encoding human CD46.

156. The method of any of items 152 to 155, wherein the sequence encoding a human complement-regulatory protein and the sequence encoding human thrombomodulin are located on a single expression vector that is inserted into the alpha-1,3-galactosyltransferase gene locus.

157. The method of item 156, wherein the expression vector that is inserted into the alpha-1,3-galactosyltransferase gene locus additionally comprises a selectable marker gene sequence between the sequence encoding a human complement-regulatory protein and the sequence encoding human thrombomodulin.

158. The method of item 157, wherein the selectable marker gene sequence is flanked by loxP sites.

159. The method of any of items 152 to 158, wherein the sequence encoding a human complement-regulatory protein and the sequence encoding human thrombomodulin are inserted at the alpha-1,3-galactosyltransferase gene locus such that the genetically modified mammal lacks expression of alpha-1,3-galactosyltransferase.

160. The method of any of items 152 to 159, wherein the sequence encoding a human complement-regulatory protein and the sequence encoding human thrombomodulin are inserted at the alpha-1,3-galactosyltransferase gene locus such that at least the sequence encoding a human complement-regulatory protein is expressed under the control of the endogenous promoter of the alpha-1,3-galactosyltransferase gene.

161. The method of any of items 152 to 160, wherein the sequence encoding a human complement-regulatory protein and the sequence encoding human thrombomodulin are inserted at the alpha-1,3-galactosyltransferase gene locus such that both the sequence encoding a human complement-regulatory protein and the sequence encoding human thrombomodulin are expressed under the control of the endogenous promoter of the alpha-1,3-galactosyltransferase gene.

162. The method of any of items 152 to 160, wherein the sequence encoding a human complement-regulatory protein and the sequence encoding human thrombomodulin are inserted at the alpha-1,3-galactosyltransferase gene locus such that the sequence encoding a human complement-regulatory protein is expressed under the control of the endogenous promoter of the alpha-1,3-galactosyltransferase gene and the sequence encoding human thrombomodulin is expressed under the control of an exogenous promoter.

163. The method of item 162, wherein the exogenous promoter is a ubiquitously active promoter.

164. The method of any of items 152 to 163, wherein the method further comprises knocking-out the CMAH and B4GALNT2 genes of the mammalian genome before step (b).

165. The method of any of items 152 to 164, wherein the method further comprises inserting a sequence encoding human $\alpha$1,2-fucosyltransferase into the mammalian genome before step (b).

166. The method of item 165, wherein the sequence encoding a human complement-regulatory protein, the sequence encoding human thrombomodulin and the sequence encoding human $\alpha$1,2-fucosyltransferase are located on a single expression vector that is inserted into the alpha-1,3-galactosyltransferase gene locus.

167. The method of any of items 152 to 166, wherein the method further comprises introducing one or more additional genetic modification(s) into the mammalian genome before step (b).

168. The method of item 167, wherein the one or more additional genetic modification(s) is/are genetic insertion(s).

169. The method of item 168, wherein the sequence encoding a human complement-regulatory protein, the sequence encoding human thrombomodulin and the one or more additional insertion(s) are located on a single expression vector that is inserted into the alpha-1,3-galactosyltransferase gene locus.

170. The method of any of items 152 to 169, wherein the genetic modifications are introduced using CRISPR/Cas9, ZFN or TALEN.

171. The method of any of items 152 to 170, wherein the genetic modifications are introduced using CRISPR/Cas9.

172. The method of any of items 152 to 171, wherein the mammal belongs to the family *Suidae,* preferably wherein the mammal belongs to the genus *Sus,* more preferably wherein the mammal belongs to the species *S. scrofa,* most preferably wherein the mammal belongs to the subspecies *S. s. domesticus.*

173. The method of any of items 152 to 172, wherein the mammal belongs to the species *S. scrofa.*

174. The method of any of items 152 to 173, wherein the mammal is a pig of German Landrace/Large White cross-bred genetic background, a Duroc pig, a Swabian-Hall pig, a black mini pig, or an Auckland Island pig; preferably wherein the mammal is a pig of German Landrace/Large White cross-bred genetic background.

175. The method of any of items 152 to 174, wherein the mammal is free of porcine endogenous retrovirus C (PERV-C).

176. The method of any of items 152 to 175, wherein the genetically modified mammal is suitable to serve as donor organism for xenogeneic organ transplants.

177. A genetically modified mammal that is obtainable (or obtained) by the method of any of items 152 to 176.

178. The method of any of items 1 to 29, wherein the xenogeneic donor organism is a genetically modified mammal according to item 177.

179. Living primate according to any of items 30 to 50, wherein the xenogeneic mammal is a genetically modified mammal according to item 177.

180. Composition comprising an mTOR inhibitor for use according to any of items 51 to 86, wherein the xenogeneic donor organism is a genetically modified mammal according to item 177.

181. Composition comprising one or more antihypertensive agent(s) for use according to any of items 87 to 122, wherein the xenogeneic donor organism is a genetically modified mammal according to item 177.

Organ overgrowth in the recipient organism is a major problem when a genetically modified organ from a xenogeneic donor organism (e.g., from a pig) is transplanted into a recipient organism (e.g., into a baboon). In accordance with the present invention, organ overgrowth is counteracted by a combination of treatments: (i) decreasing the blood pressure of the recipient (e.g., of the baboon) to match the lower levels of the donor (e.g., of the pig); (ii) tapering cortisone at an early stage; and/or (iii) using the sirolimus prodrug temsirolimus to mitigate myocardial hypertrophy (cf. Examples 1 to 4). However, the present inventors have found that the growth of pigs (and thus of their organs) can also be counteracted genetically, by knocking out the growth hormone receptor (GHR) gene. The present inventors have found that the body weight of GHR-KO (GHR-knock out) pigs was reduced by 60% compared with controls, and most organs weights were reduced proportionally (cf. Example 7). Moreover, GHR-KO pigs had markedly reduced serum insulin-like growth factor 1 (IGF1) levels. Strikingly, by treating GHR-KO pigs with IGF1 it will be possible to promote their growth (shown in references 88, 108, 109 and 110 for patients with Laron syndrome). Hence, by treating GHR-KO pigs with IGF1, the size of the pigs and their organs can be adjusted at will.

In view of the above, the present inventors have knocked out the GHR gene in GT-KO/hCD46/hTM pigs that can be used as donor organisms for xeno-transplantation into primates (cf. Example 7). Hence, the present invention provides GHR-KO mammals (e.g., pigs) that are suitable donors for xeno-transplantation, and whose size/organ size can be adjusted by treating them with IGF1. Using such donor mammals in the xeno-transplantation in accordance with the present invention may cooperate with the above-mentioned drug treatments of the present invention to counteract organ overgrowth. Additionally, using GHR-KO mammals as donors could avoid long-term treatment of the recipient with an mTOR inhibitor. Finally, being able to adjust the size of the donor organs to the needs of the recipient may be particularly useful for recipients of relatively small size, who require relatively small donor organs.

Accordingly, the present invention provides a donor organism for xenogeneic organ transplants and methods of producing same by providing the preferred embodiments described in the following items:

182. A donor organism for xenogeneic organ transplants, wherein the donor organism has the following genetic modification:

a) homozygous knock-out of the growth hormone receptor gene;

and wherein the donor organism is a non-human mammal.

183. The donor organism of item 182, wherein the donor organism additionally has the following genetic modifications:

b) homozygous knock-out of the alpha-1,3-galactosyltransferase gene;

c) insertion of a sequence encoding human CD46; and

d) insertion of a sequence encoding human thrombomodulin.

184. The donor organism of item 182, wherein the donor organism additionally has the following genetic modifications:

b) homozygous knock-outs of the alpha-1,3-galactosyltransferase gene, CMAH gene and B4GALNT2 gene; and

c) insertions of a sequence encoding human CD46, a sequence encoding human CD55, a sequence encoding human CD59, a sequence encoding hemeoxygenase 1 and a sequence encoding A20.

185. The donor organism of item 184, wherein the sequence encoding hemeoxygenase 1 encodes human hemeoxygenase 1, and wherein the sequence encoding A20 encodes human A20.

186. The donor organism of item 184 or 185, wherein the donor organism additionally has the following genetic modification:
d) insertion of a sequence encoding LEA29Y or PD-L1.

187. The donor organism of item 186, wherein the sequence encoding LEA29Y is the sequence shown in SEQ ID NO: 1.

188. The donor organism of item 186, wherein the sequence encoding PD-L1 encodes human PD-L1.

189. The donor organism of any of items 182 to 188, wherein the donor organism additionally has the following genetic modification:
e) insertion of a sequence encoding human $\alpha$1,2-fucosyltransferase.

190. The donor organism of any of items 182 to 189, wherein the donor organism belongs to the family *Suidae,* preferably wherein the donor organism belongs to the genus *Sus,* more preferably wherein the donor organism belongs to the species *S. scrofa,* most preferably wherein the donor organism belongs to the subspecies *S. s. domesticus.*

191. The donor organism of any of items 182 to 190, wherein the donor organism belongs to the species *S. scrofa*.

192. The donor organism of any of items 182 to 191, wherein the donor organism is a pig of German Landrace/Large White cross-bred genetic background, a Duroc pig, a Swabian-Hall pig, a black mini pig, or an Auckland Island pig; preferably wherein the donor organism is a pig of German Landrace/Large White cross-bred genetic background.

193. The donor organism of any of items 182 to 192, wherein the donor organism is free of porcine endogenous retrovirus C (PERV-C).

194. The donor organism of any of items 182 to 193, wherein the donor organism is a genetically modified organism according to any of items 123 to 141.

195. The method of any of items 1 to 29, wherein the xenogeneic donor organism is a donor organism according to any of items 182 to 194.

196. Living primate according to any of items 30 to 50, wherein the xenogeneic mammal is a donor organism according to any of items 182 to 194.

197. Composition comprising an mTOR inhibitor for use according to any of items 51 to 86, wherein the xenogeneic donor organism is a donor organism according to any of items 182 to 194.

198. Composition comprising one or more antihypertensive agent(s) for use according to any of items 87 to 122, wherein the xenogeneic donor organism is a donor organism according to any of items 182 to 194.

199. A method of making a donor organism for xenogeneic organ transplants comprising the following step (i):

(i) adjusting the size of the donor organism by treating the donor organism with IGF1;

wherein the donor organism has the following genetic modification:

a) homozygous knock-out of the growth hormone receptor gene;

and wherein the donor organism is a non-human mammal.

200. The method of item 199, wherein the treatment with IGF1 is initiated after birth of the donor organism.

201. The method of item 199 or 200, wherein the donor organism additionally has the following genetic modifications:

b) homozygous knock-out of the alpha-1,3-galactosyltransferase gene;

c) insertion of a sequence encoding human CD46; and

d) insertion of a sequence encoding human thrombomodulin.

202. The method of item 199 or 200, wherein the donor organism additionally has the following genetic modifications:

b) homozygous knock-outs of the alpha-1,3-galactosyltransferase gene, CMAH gene and B4GALNT2 gene; and

c) insertions of a sequence encoding human CD46, a sequence encoding human CD55, a sequence encoding human CD59, a sequence encoding hemeoxygenase 1 and a sequence encoding A20.

203. The method of item 202, wherein the sequence encoding hemeoxygenase 1 encodes human hemeoxygenase 1 and the sequence encoding A20 encodes human A20.

204. The method of item 202 or 203, wherein the donor organism additionally has the following genetic modification: d) insertion of a sequence encoding LEA29Y or PD-L1.

205. The method of item 204, wherein the sequence encoding LEA29Y is the sequence shown in SEQ ID NO: 1.

206. The method of item 204, wherein the sequence encoding PD-L1 encodes human PD-L1.

207. The method of any of items 199 to 206, wherein the donor organism additionally has the following genetic modification:
e) insertion of a sequence encoding human $\alpha$1,2-fucosyltransferase.

208. The method of any of items 199 to 207, wherein the donor organism belongs to the family *Suidae,* preferably wherein the donor organism belongs to the genus *Sus,* more preferably wherein the donor organism belongs to the species *S. scrofa,* most preferably wherein the donor organism belongs to the subspecies *S. s. domesticus.*

209. The method of any of items 199 to 208, wherein the donor organism belongs to the species *S. scrofa.*

210. The method of any of items 199 to 209, wherein the donor organism is a pig of German Landrace/Large White cross-bred genetic background, a Duroc pig, a Swabian-Hall pig, a black mini pig, or an Auckland Island pig; preferably wherein the donor organism is a pig of German Landrace/Large White cross-bred genetic background.

211. The method of any of items any of items 199 to 210, wherein the donor organism is free of porcine endogenous retrovirus C (PERV-C).

212. The method of any of items any of items 199 to 210, wherein the donor organism is a genetically modified organism according to any of items 123 to 141.

213. A donor organism for xenogeneic organ transplants, wherein the donor organism is obtainable (or obtained) according to the method of any of items 199 to 212.

214. The method of any of items 1 to 29, wherein the xenogeneic donor organism is a donor organism according to item 213.

215. Living primate according to any of items 30 to 50, wherein the xenogeneic mammal is a donor organism according to item 213.

216. Composition comprising an mTOR inhibitor for use according to any of items 51 to 86, wherein the xenogeneic donor organism is a donor organism according to item 213.

217. Composition comprising one or more antihypertensive agent(s) for use according to any of items 87 to 122, wherein the xenogeneic donor organism is a donor organism according to item 213.

## BRIEF DESCRIPTION OF THE DRAWINGS

**Figure 1A-L**, illustrations of results reported in Example 2: A, High sensitive cardiac troponin T and **B**, bilirubin levels of the three experimental groups (I, light grey dots; II, dark grey dashes; III, black lines). In contrast to animals of group I (crystalloid cardioplegia) and II (non-ischemic heart perfusion), those of group III (non-ischemic heart perfusion and treatment for cardiac hypertrophy) had low levels of troponin T and bilirubin throughout the experiments, indicating normal graft and liver function. **C**, Kaplan-Meier curve of survival after orthotopic xenotransplantation of groups I, II and III; the longest experiment was electively terminated after reaching the study endpoint of 90 days; log-rank test, p=0.0236. **D**, LV mass increases of donor hearts as estimated by echocardiography. LV mass of group II experiment 64 had quadrupled after 40 days, whereas LV mass of 67 (group III) remained unchanged after 90 days. The control heart (donor sibling of experiment 64, Co) had tripled after 33 days. **E**, Image of the porcine donor heart of experiment 57 (group I) after explanation (left) on POD 30 and the animal's own heart (right) for comparison. The graft had reached 230% of its initial weight at the end of the experiment; donor and recipient hearts had approximately the same size at the day of transplantation. **F**, Same hearts as in **E**, after preservation in formalin and tissue sampling. Transverse cuts approximately 1 cm below the mitral valve; the graft's LV was extensively hypertrophied and LV cavity diminished. **G**, Image of the porcine donor heart of experiment 67 (group III) after explantation (left) on POD 90 and the animal's own heart (right). Transverse cuts approximately 1 cm below the mitral valve. LV wall thickness increase was only minor at the end of experiment, LV cavity was not diminished. **H**, HE staining of porcine donor LV myocardium (left) and recipient liver (right) of experiment 64 on POD 40. Myocardium: multifocal cell necroses with hypereosinophilia, moderate interstitial infiltrations of lymphocytes, neutrophiles and macrophages, sporadic thromboses of small vessels; liver: multifocal centrolobular cell vacuolizations and necroses, multifocal intralesional hemorrhages. **I**, HE staining of porcine donor LV myocardium and recipient liver of experiment 67 on POD 90. Myocardium: very sporadic infiltrations of lymphocytes, multifocal minor interstitial edema; liver: small vacuolar degeneration of hepatocytes (lipid type). **J**, Myocyte size (WGA staining, scale bar = 50 $\mu$m) displayed in representative myocardial sections of a sham operated porcine heart (control, left), experiments 64 (group II, center) and 67 (group III, right). **K**, compared to a sham operated heart (Co), myocyte cross-sectional area increased in experiment 64 but not in experiment 67 (data presented as mean$\pm$SD; p values as indicated, one-way ANOVA with Holm-Sidak's multiple comparisons test). **L**, Western blots (myocardium) of phosphorylated mTOR and PCNA, both downregulated in experiment 67 after treatment with temsirolimus as compared to control (Co). Co, control; HE, hematoxylin eosin; LV, left ventricular; mTOR, mechanistic target of rapamycin; POD, postoperative day; PCNA, proliferating cell nuclear antigen; WGA, wheat germ agglutinin.

**Figure 2A-K:** Immunofluorescence stainings in myocardial tissues (**A - D**) their quantitative measurements (**E - I**) and non-Gal antibody levels (**J - K**). Exemplary stainings of the three different groups: experiments #57 (group I, upper row), #64 (group II, middle row) and #67 (group III, lower row). IgM depositions (**A**, shown in green, faint in groups I, II, absent in group III), of IgG depositions (**B**, absent in all groups); nuclei are always stained with DAPI (blue), scale bars represent always 25μm. Little depositions of C3b/c (red), C4b/c (green) (**C**) and fibrin (green, **D**) in porcine myocardial tissues of groups I, II, no such findings in group III. **E -** I, Fluorescence intensities (n=5 each) were measured using ImageJ and the corresponding values plotted using GraphPad Prism. Three groups are shown: group I (#57); group II (#60, 63, 64), and group III (#66, 67). **E**, IgM. **F**, IgG. **G**, C3b/c. **H**, C4b/c. **I**, fibrin; C3b/c and C4b/c values are compared to those of controls (Co) measured in a healthy pig heart; bars are means ± standard deviations. **J** and **K**, Non-Gal IgM and IgG antibody levels in baboon plasma; antibody binding to GT-KO/hCD46/hTM PAEC; the cells were incubated in heat inactivated baboon plasma, stained for IgG and IgM and analysed by fluorescence-activated cell sorting (FACS). Values are expressed as median fluorescence intensity (MFI). Baboon #46 experienced humural rejection of its (heterotopically transplanted intrathoracic) donor heart which served as positive control (grey); POD=postoperative day; Gal, α1,3-galactosyl.

**Figure 3A-D:** Hemodynamic data, measured by transpulmonary thermodilution and postoperative catecholamine support. Hemodynamic measurements were taken after induction of anesthesia (pre CPB) and 60 minutes after termination of cardiopulmonary bypass (post CPB). Donor hearts of group I (light grey) received crystalloid cardioplegia, donor hearts of groups II (dark grey) and III (black) were preserved with continuous cold hyperoncotic perfusion; data presented as scatter plots with median bars and analysed for significance with paired and unpaired t-tests. **A**, SVI and **B**, CI before and after CPB. Both parameters decreased in group I and were lower in group I hearts after CPB when compared to groups II and III. **C**, Dosages of catecholamines 60 minutes after termination of CPB and **D**, the durations of postoperative vasopressive and inotropic support. Group I needed more noradrenaline (NOR) and epinephrine (EPI) when compared to groups II and III. Animals of group I needed inotropic support with epinephrine for a longer time. CI, cardiac index; CPB, cardiopulmonary bypass; EPI, epinephrine; SVI, stroke volume index; NOR, noradrenaline.

**Figure 4A-C:** Graphics of LV sizes during diastole (left) and systole (right), derived from transthoracic echocardiographies. **A**, Experiment 64 (group II, survival 40 days): LV mass had increased by 303% on POD 38, diastolic and systolic functions were greatly impaired due to myocardial hypertrophy and decreased intraventricular cavity. LV FS were 32% and 14% on POD 1 and POD 38. The animal had signs of heart failure. These findings were similar to other experiments in group I and II. **B**, Experiment 67 (group III, survival 90 days): in contrast to experiment 64, LV mass had increased by only 22% on POD 82, diastolic and systolic functions were preserved. LV FS were 27% and 34% on POD 1 and POD 82. The animal had no signs of heart failure. **C**, Pig 5157 (control, donor sibling of experiment 64): LV mass had increased by 187% on POD 33, diastolic and systolic function were however preserved. LV FS were 32% and 41% on POD 1 and POD 33. When compared to the transplanted sibling heart (experiment 64), the LV had grown less in size and showed nor myocardial hypertrophy or heart failure. FS, fractional shortening; LV, left ventricular; POD, postoperative day

**Figure 5A-B:** Correlations between LV mass (black dots), thrombocyte counts (dark grey line) respectively LDH values (light grey dashes). **A**, Around POD 20 in recipient 64 (group II), LV mass increased more vigorously, while the thrombocytes dropped, LDH increased. **B**, These findings were in contradistinction to recipient 67 (group III), whose LV mass remained stable. LV, left ventricular; POD, postoperative day.

**Figure 6A-B**: Transthoracic echocardiographic midpapillary short axis views of porcine grafts after cardiac xenotransplantation (video stills). **A**, Example of the overgrowth of a transplanted organ, Experiment 64 (group II, POD 30): the LV muscular wall had thickened and cardiac function deteriorated; LV cavity was severely diminished; wall motion was diminished; there were signs of terminal heart failure; consecutive terminal liver failure. These findings were similar to other experiments in group I and II. **B**, Experiment 67 (group III, POD 57): in contrast to experiment 64, LV muscular wall remained unchanged, LV and RV functions were well preserved; LV cavity was normal in size; there was almost normal pump function; no signs of heart failure or of liver failure. RV, right ventricular; LV, left ventricular

**Figure 7**: Flowchart of exemplary dosing regimen of immunosuppressive induction and maintenance therapy as well as of exemplary dosing regimen of mTOR inhibitor administration. The marks on each time line represent administrations. The numbers below the marks of the methylprednisolone time line represent the respective dosages (mg/kg/day). HTX, heart transplantation; ATG, anti-thymocyte globulin; i.v., intravenous.

**Figure 8**: Flowchart of exemplary dosing regimen of anti-inflammatory therapy. HTX, heart transplantation; i.v., intravenous; s.c., subcutaneous. The marks on each time line represent administrations.

**Figure 9**: Flowchart of exemplary dosing regimen of anti-hypertensive treatment. HTX, heart transplantation; i.v., intravenous. The marks on each time line represent administrations.

**Figure 10A-C**: Exemplary cardiac xenotransplantation techniques. **A**, Orthotopic cardiac transplantation. The recipient heart is removed at the atrial level; ascending aorta and the pulmonary trunk are cut. The donor organ is then connected accordingly. **B**, Intrathoracic heterotopic cardiac transplantation. The donor organ is placed to the right of the recipient heart, within the right chest. There are two common atria, since they are end-to-end anastomosed. The blood volume is ejected by both hearts via two end-to-side connections at the levels of the ascending aortas, the pulmonary artery trunks (the latter needs a graft interposition). Both organs work non-synchronously. Their shares of the cardiac output depend on the respective preloads, which in turn are related to the parts of the cycles of the two hearts. **C**, Heterotopic abdominal transplantation. Only the coronary arteries are perfused (via the abdominal aorta), coronary venous blood enters the right atrium, then the ventricle. It is ejected via the pulmonary trunk into the inferior vena cava. The heart is beating, but non-working. Figure adapted from Mohiuddin, Reichart et al., 2015.

**Figure 11A-F**: Survival, laboratory parameters, necropsy and histology after orthotopic xenotransplantation. **A**, Kaplan-Meier curve of survival of groups I (light grey dots; n = 5 animals), II (dark grey dashes; n = 4 animals) and III (black lines; n = 5 animals). Two-sided log-rank test, P = 0.0007. **B**, **C**, Serum concentrations of cardiac troponin T (**B**) and bilirubin (**C**). **D**, Left ventricular (LV) masses of xenografted hearts from animals 9 (group II), 11 and 13 (both group III); note increased graft growth after discontinuation of temsirolimus (arrow). **E**, Quantitative analysis of cardiomyocyte cross-sectional areas. Data are mean $\pm$ s.d., P values are indicated, one-way analysis of variance (ANOVA) with Holm-Sidak's multiple comparisons test (n = 3 biologically independent samples with 5-8 measurements each). **F**, Western blot analysis of myocardium from transplanted hearts of animals 11 and 12 showed reduced mTOR phosphorylation (p-mTOR) compared to age-matched control samples. n = 2, group III; n = 2, controls.

**Figure 12A-G**: Quantitative evaluation of antibodies, complement and fibrin in myocardial tissue and serum levels of non-galactose-$\alpha$1,3-galactose xenoreactive antibodies. **A-E**, Quantitative evaluation of fluorescence intensities (n = 9 biologically independent samples with 5-10 measurements per experiment; for representative images see Fig. 2A-D). Raw integrated densities are shown for IgM (**A**), IgG (**B**), C3b/c (**C**), C4b/c (**D**) and fibrin (**E**). Group I (animal 3); group II (animals 6, 8, 9); group III (animals 11-14). C3b/c and C4b/c values are compared to those of controls measured in healthy pig hearts. Data are mean $\pm$ s.d. **F**, **G**, Levels of non-galactose-$\alpha$1,3-galactose xenoreactive IgM and IgG antibodies in baboon plasma; antibody binding to $\alpha$-galactosyltransferase-knockout porcine aortic endothelial cells that express human CD46 and thrombomodulin was analysed by fluorescence-activated cell sorting. Values are expressed as mean fluorescence intensity. Animals 6, 9 and 10 received an anti-CD40L PASylated Fab, the others were treated with an anti-CD40 monoclonal antibody. Plasma from a baboon who rejected a heterotopically intrathoracic transplanted pig heart served as positive control.

**Figure 13A-D**: Hemodynamic data, measured by transpulmonary thermodilution and post-operative catecholamine support. Measurements were taken after induction of anaesthesia (before) and 60 min after termination of CPB (after). Donor hearts of group I (light grey) received crystalloid cardioplegia, donor hearts of groups II (dark grey) and III (black) were preserved with continuous cold hyperoncotic perfusion; data are presented as scatter plots with mean $\pm$ s.d. with individuals shown as dots; n = 14 animals, two-sided paired and unpaired t-tests, P values as indicated. **A**, Stroke volume index. **B**, Cardiac index before and after CPB. Both parameters decreased in group I and were lower in group I after CPB than in group II and III. **C**, Dosages of catecholamines 60 min after termination of CPB. **D**, Durations of post-operative vasopressive and inotropic support. Animals in group I required more noradrenaline and adrenaline than those in group II and III. Animals in group I required inotropic support with adrenaline for a longer time.

**Figure 14A-B**: Additional laboratory parameters. **A**, **B**, Serum concentrations of lactate dehydrogenase (**A**) and platelet counts (**B**) in animals of groups I (light grey dots in black/white version of figures; black line in coloured version of figures), II (dark grey dashed line in black/white version of figures; red line in coloured version of figures) and III (black line in black/white version of figures; magenta line in coloured version of figures). At the end of experiments in groups I and II, platelet counts decreased whereas LDH increased. Group III animals did not show these alterations.

**Figure 15A-B**: Immunohistochemistry of post-mortem myocardial specimens. **A**, **B**, Expression of human membrane cofactor protein (hCD46) (**A**) and human thrombomodulin (hTM) (**B**) was consistent in all donor organs (1-14). Scale bars, 50 $\mu$m. n = 14 GTKO/hCD46/hTM pigs; n = 1 wild-type pig (control). Biological samples from all animals are shown.

**Figure 16:** Nanopore sequencing of the hCD46 transgene locus in GGTA1-KO/hCD46 transgenic pigs provided by Revivicor reveals the presence of at least two integrants of a human FUT1 coding sequence in a murine MHC promoter driven expression cassette. The two integrants (labelled with 1 and 2 on the right side) can be clearly differentiated by the orientation of flanking hCD46 transgene copies.

**Figure 17**: Mean transcript abundance of GGTA1, CMAH and B4GALNT2 in different tissues from five 2-year-old female wild-type pigs. Expression profiling was performed by RNA-sequencing. TPM (Transcripts Per Million; cf. reference 106) is a transcript abundance metric useful for comparing the expression levels of different genes within a sample. TPM takes into account the depth of your library by normalizing per million reads, and it also controls for the length of the gene by dividing each data point by the length of the transcript in number of bases.

**Figure 18**: Strategy for genetic engineering of donor pigs. The GGTA1, CMAH and B4GALNT2 genes will be inactivated by CRISPR/Cas9. Then a cassette with a promoterless hCD46 minigene, a floxed selectable marker gene (SMG), and an hTM expression cassette is inserted into the GGTA1 locus such that expression of hCD46 is driven by GGTA1 regulatory sequences. Additional transgenes can be inserted via Cre-mediated cassette exchange with an engineered BAC that contains one or several additional transgenes.

**Figure 19**: Generation of a GHR-deficient pig model using CRISPR/Cas technology. Partial DNA sequence of GHR exon 3. The sgRNA binding site is indicated by the solid line under the sequence, and the protospacer adjacent motif (PAM) by the dashed line under the sequence. Insertions (double line under the sequence) of 1 bp (founder #2529) or 7 bp (founder #2533) lead to a shift of the reading frame. WT = wild type.

**Figure 20**: Generation of a GHR-deficient pig model using CRISPR/Cas technology. Ligand immunohistochemistry demonstrating the absence of functional GHR (dark staining in control) in GHR-KO pigs. Chromogen: DAB; counterstain: Mayer's hemalum; bar = 10 $\mu$m.

**Figure 21A-C**: Serum IGF1, IGFBP and GH concentrations of GHR-KO compared with control pigs. **A**, Means and standard deviations of serum IGF1 values (GHR-KO: n = 42; control: n = 69). **B**, Quantification of IGFBP3 and IGFBP2 in serum from GHR-KO (n = 10) and control pigs (n = 12). The figure shows medians, 25th and 75th percentiles (box), and extremes (whiskers). **C**, Area under the GH curve (AUC; means and standard deviations for 6 female GHR-KO and 5 female/1 male control pigs). AU = arbitrary units. *p <0.05; ***p <0.001.

**Figure 22A-B**: Body weight gain and growth of GHR-KO compared with control pigs. **A**, GHR-KO pig (front) and control littermate aged 6 months. **B**, Body weight gain. The parameters were determined in 12 GHR-KO and 25 control pigs. Panel B shows least squares means (LSMs) and standard errors of LSMs estimated for group*age. *p <0.05; **p <0.01; ***p <0.001; ns = not significant.

**Figure 23A-B**: Disproportionate organ growth in GHR-KO compared with control pigs. GHR-deficiency led to a proportionate and disproportionate reduction in organ sizes. **A**, Representative organs from control (left) and GHR-KO pigs (right). **B**, Relative differences between GHR-KO and control pigs in absolute organ weights and in organ weight-to-body weight ratios (relative organ weights). These parameters were determined in 9 GHR-KO and 25 control pigs, and least squares means (LSMs) and standard errors of LSMs were estimated for the 2 groups. *p <0.05; **p <0.01; ***p <0.001.

**Figure 24A-D: A**, Body composition of 6-month-old GHR-KO compared with control pigs. DXA analysis revealed a significantly higher amount of total body fat in GHR-KO pigs (GHR-KO: n = 12; control: n = 25; ***p <0.001). Panel A shows at least squares means (LSMs) and standard errors of LSMs estimated for the 2 groups. **B-D**, Age-dependent changes in glucose and lipid homeostasis parameters in GHR-KO and control pigs. **B**, Transient juvenile hypoglycemia in GHR-KO pigs. Serum concentrations of (**C**) triglycerides and (**D**) cholesterol were significantly lower in young GHR-KO pigs than in age-matched controls, but normalized with age. At least 6 animals per group and age-class were investigated. Panels B-D show least squares means (LSMs) and standard errors of LSMs estimated for group*age. *p <0.05; **p <0.01.

**Figure 25**: Generation of GHR-deficient GGTA1-KO, hCD46/hTBM transgenic pigs. Primary cells were isolated from a GGTA1-KO, hCD46/hTBM transgenic pig and co-transfected with a Cas9 expression plasmid and a plasmid transcribing a guide RNA specific for the porcine GHR exon 3. A biallelic GHR mutant cell clone was used for somatic cell nuclear transfer, and cloned embryos were transferred to estrous-cycle synchronized recipient gilts, resulting in the birth of small GHR-deficient GGTA1-KO, hCD46/hTBM transgenic pigs.

## DETAILED DESCRIPTION OF THE INVENTION

Unless otherwise defined below, the terms used in the present invention shall be understood in accordance with their common meaning known to the person skilled in the art.

In the present invention, the terms "comprise" and "consist of" have the meaning known in the art. Optionally, on each occurrence the term "comprise(s)" as used in the invention can be replaced by the term "consist(s) of".

All publications, patents and patent applications cited herein are hereby incorporated by reference in their entirety for all purposes.

Definitions

The term "xenogeneic" is used according to its general meaning known in the art. Accordingly, a "xenogeneic donor" as used in the present invention is a donor that is a member of a different species than the recipient organism. A "xenogeneic transplantation" is a transplantation wherein the graft is derived from a species that is different from the species of the recipient organism.

The term "overgrowth" as used herein generally refers to any increase in organ or tissue mass or volume that is detrimental for organ or tissue function, in particular due to exogenous factors (e.g. compression due to space limitation) or due to endogenous factors (e.g. hypoxia due to insufficient microvascular circulation). Thus, when a heart, kidney or liver is transplanted at a weight within a physiological range of the respective organ of the recipient organism, in one embodiment of the present invention the term "overgrowth" means that the weight of the organ has increased to reach a weight that is 50% higher than the physiological weight. When a lung is transplanted at a volume within a physiological range of the lung of the recipient organism, in one embodiment the term "overgrowth" means that the volume of the lung has increased to reach a volume that is 50% larger than the physiological volume. A weight or volume within a physiological range as used herein means a weight or volume, respectively, that does not deviate by more than +/- 20%, preferably by not more than +/- 10% from the physiological weight or volume of the respective organ of the respective recipient organism. The physiological weight or volume of an organ can be determined by the skilled person for any given recipient based on common general knowledge. Exemplary physiological weights and volumes of organs are given in references 116 and 117. In a preferred embodiment, the term "overgrowth" as used herein means that the transplanted organ (e.g., heart) triples to quadruples in volume and weight within approximately one month after transplantation.

The term "genetically modified heart/kidney/lung/liver" as used herein refers to a heart/kidney/lung/liver whose cells carry an artificially modified genome. Without being limited thereto, such artificial modification may be, for example, an artificial insertion of exogenous nucleic acid(s), and/or a deletion of endogenous nucleic acid(s). The skilled person will be aware of various methods for introducing such genetic modifications to obtain a genetically modified heart/kidney/lung/liver. For example, mutations can be artificially introduced into the germ line of animals so that all cells of such genetically modified animal carry the respective mutations in their genomes. The heart/kidney/lung/liver of such genetically modified animal would be a genetically modified heart/kidney/lung/liver in accordance with the present invention.

The term "immunosuppressive induction therapy" as used herein refers to any immunosuppressive therapy that is administered to a recipient organism before said recipient organism is transplanted a graft. Hence, in the present invention the term "immunosuppressive induction therapy" refers to an immunosuppressive therapy that is administered to a recipient organism before heart/kidney/lung/liver transplantation.

The term "immunosuppressive maintenance therapy" as used herein refers to any immunosuppressive therapy that is administered to a recipient organism after said recipient organism is transplanted a graft. Hence, in the present invention the term "immunosuppressive maintenance therapy" refers to an immunosuppressive therapy that is administered to a recipient organism after heart/kidney/lung/liver transplantation.

The term "non-ischemic preservation" as used herein refers to the *ex vivo* preservation of an organ wherein during said preservation it is ensured that the cells of said organ receive sufficient oxygen supply, nutrients and hormones. For example, in one embodiment of the invention "non-ischemic preservation" of a surgically extracted heart/kidney/lung/liver is carried out by "non-ischemic heart/kidney/lung/liver perfusion". As used herein, the term "non-ischemic heart/kidney/lung/liver perfusion" refers to an *ex vivo* heart/kidney/lung/liver preservation wherein oxygen-carrying blood or an oxygen-carrying blood-like fluid, supplemented with nutrients and hormones, is pumped through the blood vessels of said heart/kidney/lung/liver in order to ensure sufficient oxygen supply and energy, hormonal demands of the cells of said heart/kidney/lung/liver.

The term "functionally substituting" a heart/kidney/lung/liver by a transplanted heart/kidney/lung/liver as used herein preferably refers to an orthotopic substitution, but may also refer to a heterotopic support, and in the case of a transplanted heart to an intrathoracic heterotopic support. If the substitution is an orthotopic substitution, the substitution is a replacement wherein the transplanted heart/kidney/lung/liver takes over the functions of the recipient's own heart/kidney/lung/liver. Such functional substitution requires that the heart/kidney/lung/liver of the recipient organism is removed prior to transplantation, and that the donor heart/kidney/lung/liver is placed in an orthotopic (i.e., in a natural) position in the recipient organism. In the case of a transplanted heart, if the substitution is an intrathoracic heterotopic support, the substitution is in support of a recipient's heart which is, e.g., diseased in a human situation (cf. reference 24, Figure 10 B). In the heterotopic intrathoracic substitution, the recipient's own heart remains in place, while the donor heart is hemodynamically connected in parallel and to the right; in other words: the donor organ supports (as just mentioned) the recipient's heart (which, e.g., in a clinical situation would be terminally diseased). As will be clear to the skilled person, the functional parameters (e.g., heart rate, cardiac output, ejection fraction, stroke volume, etc.) of the transplanted heart may not be identical to the functional parameters of the recipient's own heart. Nevertheless, the transplanted heart is considered to functionally substitute the recipient's heart if its functional parameters are within a range that allows survival of the recipient.

The term "anti-inflammatory therapy" as used herein is known in the art and refers to the administration of anti-inflammatory agent(s). As will be known to the skilled person, the anti-inflammatory properties of agents can be evaluated *in silico*, *in vitro*, and/or *in vivo.* For example, an agent would be considered to have anti-inflammatory properties (and thus be an anti-inflammatory agent) if said agent reduces or inhibits, or would be expected to reduce or inhibit, a pro-inflammatory signal mediated by a xenogeneic protein and/or if said agent reduces or inhibits inflammation in a subject.

The time point of heart/kidney/lung/liver transplantation as defined in this invention is the time point at which the transplanted heart/kidney/lung/liver starts functionally substituting the heart/kidney/lung/liver of the recipient organism. If, for example, a glucocorticoid is administered "within 1 day after transplantation", such administration of the glucocorticoid occurs within a time period that begins as soon as the transplanted heart/kidney/lung/liver starts functionally substituting the heart/kidney/lung/liver of the recipient organism, and ends at the end of the subsequent day. Hence, if the transplantation is performed in the morning, the time period "within 1 day after transplantation" ends at the end of the subsequent day, i.e. more than 24 hours later. Similarly, the time period of, e.g., "between 2 days and 9 days after transplantation" or "between 2 and 9 days after transplantation" or "2 to 9 days after transplantation" begins at the beginning of the $2^{nd}$ day after transplantation and ends at the end of the $9^{th}$ day after transplantation. If an administration is said to occur, e.g., "5 days after transplantation", this refers to an administration on the $5^{th}$ day after transplantation.

The term "dosage (mg/kg/day)" as used herein refers to the dosage of a substance in milligram (mg) that is administered to a subject per kilogram (kg) bodyweight of the subject and per day. Such dosage may be administered, e.g., with a single administration, or with several administrations that are distributed over the day, or it may be administered by continuous administration. When decreasing the dosage (mg/kg/day) by a specific factor, the decreased dosage is calculated by dividing the dosage of the first administration by said factor. For example, if a glucocorticoid is administered as part of the immunosuppressive maintenance therapy at an initial dosage of 10 mg/kg/day, decreasing that dosage to 2 mg/kg/day would represent a decrease by a factor of 5.

The term "mean arterial pressure" is used according to its general meaning known in the art and refers to the average blood pressure in the arteries of a subject during a single cardiac cycle. The skilled person will be aware of numerous methods for determining the mean arterial pressure.

The term "endogenous promoter" as used herein refers to a promoter that is naturally present in the genome of the respective mammal. In contrast, the term "exogenous promoter" as used herein refers to a promoter that is not naturally present in the genome of the respective mammal.

The term "ubiquitously active promoter" as used herein refers to a promoter that drives the expression of a gene in multiple tissues and cell types. In other words, when the expression of a gene is controlled by a ubiquitously active promoter, the expression is not tissue-specific.

The term "heart/kidney/lung/liver" as used herein means one or more organ(s) selected from the group consisting of heart, kidney, lung and liver. In a preferred embodiment, the term "heart/kidney/lung/liver" as used herein means heart, kidney, lung or liver.

## Embodiments

*Method of prolonging the survival of a primate following heart/kidney/lung/liver transplantation*

The present invention relates to a method of prolonging the survival of a recipient organism that is transplanted a genetically modified heart/kidney/lung/liver from a xenogeneic donor organism. *Inter alia* each of the following aspects of the method in accordance with the invention contribute to the prolonged survival of the recipient organism: (1) Administering an immunosuppressive induction therapy to the recipient before transplantation. (2) Preserving the surgically extracted heart/kidney/lung/liver from the donor organism by non-ischemic preservation until transplantation. (3) Administering an immunosuppressive maintenance therapy to the recipient organism which comprises a first administration of a glucocorticoid within 1 day after transplantation, and wherein the dosage (mg/kg/day) of said glucocorticoid is decreased by a factor of at least 5 within 5 days after transplantation. (4) Administering one or more antihypertensive agent(s) to the recipient organism after transplantation. (5) Administering an mTOR inhibitor to the recipient organism after transplantation. The above aspects (alone or in combination) prolong the survival of a recipient organism that is transplanted a heart/kidney/lung/liver from a xenogeneic donor organism as compared to the survival of a recipient organism that is transplanted a heart/kidney/lung/liver from a xenogeneic donor organism but is not treated in accordance with the present invention. In one embodiment of the invention, the method of the present invention prolongs the survival of the recipient organism to more than 40 days after transplantation, to more than 50 days after transplantation, to more than 60 days after transplantation, to more than 70 days after transplantation, to more than 80 days after transplantation, or to more than 90 days after transplantation. Preferably, the method of the present invention prolongs the survival of the recipient organism to more than 50 days after transplantation, more preferably to more than 70 days after transplantation, and most preferably to more than 90 days after transplantation.

In the present invention, the recipient organism is a primate, preferably the recipient organism belongs to the family *Cercopithecidae,* more preferably the recipient organism belongs to the genus *Papio,* even more preferably the recipient organism belongs to the species *Papio Anubis* or *Papio Hamadryas,* most preferably the recipient organism belongs to the species *Papio Anubis.* The baboon (*Papio*) can serve as a model organism for heart/kidney/lung/liver transplantations into humans. Hence, in another embodiment of the present invention the recipient organism belongs to the family *Homonidae,* preferably the recipient organism belongs to the genus *Homo,* more preferably the recipient organism is a human. In an alternative embodiment of the present invention, the recipient organism belongs to the family *Cercopithecidae,* preferably to the genus Macaca, most preferably to the species *M. mulatta.*

In the present invention the donor organism is a mammal, preferably the donor organism belongs to the family *Suidae,* more preferably the donor organism belongs to the genus *Sus,* even more preferably the donor organism belongs to the species *S. scrofa,* and most preferably the donor organism belongs to the subspecies *S. s. domesticus.* Preferably, the donor organism is a pig of German Landrace/Large White cross-bred genetic background, a Duroc pig, a Schwäbisch-Hällische (Swabian-Hall) pig, a black mini pig, or an Auckland Island pig (which has fewer infectious microorganisms, cf. reference 26); more preferably the donor organism is a pig of German Landrace/Large White cross-bred genetic background. In accordance with all other aspects of this embodiment, the donor organism is juvenile.

When a heart, kidney or liver is transplanted in accordance with the present invention, it is preferable that the donor organism is selected such that the weight of the organ to be transplanted is within a physiological range of the respective organ of the recipient organism. A weight within a physiological range as used herein means that the weight of the organ does not deviate by more than +/- 20%, preferably by not more than +/- 10% from the physiological weight of the organ of the respective recipient organism. When a lung is transplanted in accordance with the present invention, it is preferable that the donor organism is selected such that the volume of the lung is within a physiological range of the lung of the recipient organism. A volume within a physiological range as used herein means that the volume of the lung does not deviate by more than +/- 20%, preferably by not more than +/- 10% from the physiological volume of the lung of the respective recipient organism. The physiological weight or volume of an organ can be determined by the skilled person for any given recipient based on common general knowledge. Exemplary physiological weights and volumes of organs are given in references 116 and 117. As will be clear to the skilled person, the body weight of an organism can serve as an indication for the weight and volume of its organs. Thus, in another embodiment of the present invention, the body weight of the donor organism does not deviate by more than +/- 20% preferably by not more than +/- 10% from the body weight of the recipient organism.

The heart/kidney/lung/liver in accordance with the present invention is genetically modified in order to avoid transplant rejection by the recipient organism. The skilled person will be aware of suitable genetic modifications depending on the respective donor organism and the respective recipient organism in accordance with the invention. In one embodiment, the genetically modified heart/kidney/lung/liver of the present invention has the following genetic modifications: (1) homozygous knock-out of the alpha-1,3-galactosyltransferase gene; (2) insertion of the human CD46 gene; and (3) insertion of the human thrombomodulin gene. When the donor organism belongs to the family *Suidae* and the recipient organism belongs to the genus *Papio,* the genetically modified heart/kidney/lung/liver may optionally only have the aforementioned genetic modification (1), i.e. the homozygous knock-out of the alpha-1,3-galactosyltransferase gene. When the donor organism belongs to the family *Suidae* and the recipient organism is human, the genetically modified heart/kidney/lung/liver preferably has all of the aforementioned genetic modifications (1) to (3). In another embodiment, the genetically modified heart/kidney/lung/liver of the present invention has the following genetic modifications: (1) homozygous knock-out of the alpha-1,3-galactosyltransferase gene, the CMAH gene and the B4GALNT2 gene; (2) insertion of the human CD46 gene, the human CD55 gene, the human CD59 gene, a hemeoxygenase 1 gene and an A20 gene; and optionally (3) insertion of a LEA gene or a PDL-1 (PD-L1, PDL1, CD274) gene. Details of the genetic modifications of this embodiment can be found in reference 23 (which is incorporated herein in its entirety). In this embodiment, the hemeoxygenase 1 gene, the A20 gene, the LEA gene and the PDL-1 gene can be from any organism, but preferably they are the human hemeoxygenase 1 gene, the human A20 gene, the human LEA gene and the human PDL-1 gene, respectively. In the above embodiments, when inserting the human CD46 gene (huCD46) preferably transcripts according to any transcript produced from the following genomic locus/coding region are expressed: GRCH38p7, NC_000001, region 207752038 - 207795516. More preferably, when inserting the human CD46 gene at least one of the following transcripts is expressed from a transgene expression cassette: NM_002389, NM_172359, NM_172351, NM_153826, NM_172352, NM_172353, NM_172350 and NM_172361. In the above embodiments, when inserting the human thrombomodulin gene (huTHBD, huTBM) preferably transcripts according to any transcript produced from the following genomic locus/coding region are expressed: GRCH38p7, NC_000020, region 23045633 - 23049664. More preferably, when inserting the human thrombomodulin gene the following transcript is expressed from a transgene expression cassette: NM_000361. In the above embodiments, when inserting the human CD55 gene (huCD55) preferably transcripts according to any transcript produced from the following genomic locus/coding region are expressed: GRCH38p7, NC_000001, region 207321472 - 207360966. More preferably, when inserting the human CD55 gene at least one of the following transcripts is expressed from a transgene expression cassette: NM_001114752, NM_001300903, NM_001300904, NM_000574 and NM_001300902. In the above embodiments, when inserting the human CD59 gene (huCD59) preferably transcripts according to any transcript produced from the following genomic locus/coding region are expressed: GRCH38p7, NC_000011, region 33703010 - 33736479. More preferably, when inserting the human CD59 gene at least one of the following transcripts is expressed from a transgene expression cassette: NM_000611, NM_203331, NM_203329, NM_203330, NM001127227, NM_001127226, NM_001127225 and NM_001127223. In the above embodiments, when inserting the human hemeoxygenase 1 gene (huHMOX1, huHO1) preferably transcripts according to any transcript produced from the following genomic locus/coding region are expressed: GRCH38p7, NC_000022, region 35381067 - 35394214. More preferably, when inserting the human hemeoxygenase 1 gene the following transcript is expressed from a transgene expression cassette: NM_002133. In the above embodiments, when inserting the human A20 gene (huA20, huTNFAIP3) preferably transcripts according to any transcript produced from the following genomic locus/coding region are expressed: GRCH38p7, NC_000006.12, region 137866317 - 137883314. More preferably, when inserting the human A20 gene at least one of the following transcripts is expressed from a transgene expression cassette: NM_001270508; NM_001270507; NM_006290. As will be clear to the person skilled in the art, the term "LEA gene" as used herein refers to a synthetic sequence coding for an artificial CTLA4-Ig protein, which has been optimized for high affinity to CD80/CD86. In the above embodiments, when inserting the human LEA gene (i.e., sequence) preferably the following codon-optimized variant LEA29Y is expressed, e.g. from a transgene expression cassette:

atgggggtactgctcacacagaggacgctgctcagtctggtccttgcactcctgtttccaagcatggcgagcatggcgatgcacgtggcccagcctgctgtg
gtactggccagcagccgaggcatcgccagctttgtgtgtgagtatgcatctccaggcaaatatactgaggtccgggtgacagtgcttcggcaggctgacag
ccaggtgactgaagtctgtgcggcaacctacatgatggggaatgagttgaccttcctagatgattccatctgcacgggcacctccagtggaaatcaagtga
acctcactatccaaggactgagggccatggacacgggactctacatctgcaaggtggagctcatgtacccaccgccatactacgagggcataggcaac
ggaacccagatttatgtaattgatccagaaccgtgcccagattctgatcaggagcccaaatcttctgacaaaactcacacatccccaccgtccccagcacc
tgaactcctgggggggatcgtcagtcttcctcttccccccaaaacccaaggacaccctcatgatctcccggacccctgaggtcacatgcgtggtggtggacgt
gagccacgaagaccctgaggtcaagttcaactggtacgtggacggcgtggaggtgcataatgccaagacaaagccgcgggaggagcagtacaaca
gcacgtaccgggtggtcagcgtcctcaccgtcctgcaccaggactggctgaatggcaaggagtacaagtgcaaggtctccaacaaagccctcccagcc
cccatcgagaaaaccatctccaaagccaaagggcagccccgagaaccacaggtgtacaccctgcccccatcccgggatgagctgaccaagaacca
ggtcagcctgacctgcctggtcaaaggcttctatcccagcgacatcgccgtggagtgggagagcaatgggcagccggagaacaactacaagaccacg
cctcccgtgctggactccgacggctccttcttcctctacagcaagctcaccgtggacaagagcaggtggcagcaggggaacgtcttctcatgctccgtgatg
catgaggctctgcacaaccactacacgcagaagagcctctccctgtctccgggtaaatga (SEQ ID NO: 1).

In the above embodiments, when inserting the human PDL-1 gene (huPD-L1, huPDL1, huCD274) preferably transcripts according to any transcript produced from the following genomic locus/coding region are expressed: GRCH38p7, NC_000009, region 5450503 - 5470567. More preferably, when inserting the human PDL-1 gene at least one of the following transcripts is expressed from a transgene expression cassette: NM_014143; NM_001267706; NM_00134029.

As will be clear to the person skilled in the art, and as directly follows from the above, when reference is made to the "insertion of a gene" in accordance with the present invention, in a preferred embodiment not the entire gene is inserted. In this embodiment, any sequence (e.g., in an expression cassette) may be inserted that allows for the transcript of the respective gene to be expressed.

In another embodiment that can be combined with any of the embodiments described above, the genetically modified heart/kidney/lung/liver of the present invention additionally has an insertion of a sequence encoding human $\alpha$1,2-fucosyltransferase. Preferably, the human $\alpha$1,2-fucosyltransferase is the protein expressed from the following gene: Official Symbol: FUT1; Official Full Name: fucosyltransferase 1 (H blood group); Primary source: HGNC:HGNC:4012; See related: Ensembl:ENSG00000174951, MIM:211100.

In the present invention, when reference is made to an insertion of a gene/sequence, this insertion may be a heterozygous or homozygous insertion. As will be clear to a person skilled in the art, if the expression level is not high enough with a heterozygous insertion, a homozygous insertion is preferred.

Preferably, the genetically modified heart/kidney/lung/liver in accordance with the present invention is obtained from a genetically modified donor organism, i.e. from a donor organism that carries the respective genetic modifications in its germ line. Methods of obtaining such genetically modified donor organisms (e.g. pigs) are known to a person skilled in the art (cf. references 92, 103, 104 and 105, all of which are incorporated herein in their entireties). As an alternative to conventional techniques, genetically modified pigs can also be obtained using new techniques, such as CRISPR/CAS 9, which could provide for improved insertion loci.

The immunosuppressive induction therapy, the immunosuppressive maintenance therapy, the anti-inflammatory therapy, the antihypertensive agent(s) and the mTOR inhibitor in accordance with the present invention are administered according to dosing regimens. Each dosing regimen may comprise the administration of only one pharmaceutically active agent, or the administration of several pharmaceutically active agents. If a dosing regimen comprises the administration of several pharmaceutically active agents, said agents can be mixed into a single composition before administration, or they can be administered separately. If administered separately, said pharmaceutically active agents can be administered at substantially the same time (e.g., simultaneously or consecutively), or they can be administered at different times. Moreover, regardless of whether a dosing regimen comprises the administration of only one pharmaceutically active agent or of several pharmaceutically active agents, each dosing regimen may comprise a single administration of each pharmaceutically active agent, or several administrations of each pharmaceutically active agent. If a dosing regimen comprises the administration of several pharmaceutically active agents, and if several pharmaceutically active agents are administered several times, the time intervals between the administrations of the different pharmaceutically active agents may differ. For example, if a dosing regimen comprises several administrations of pharmaceutically active agent A and pharmaceutically active agent B, pharmaceutically active agent A may be administered every day (or after a fixed interval of one or several days), whereas pharmaceutically active agent B may be administered every other day. Any first administration of a pharmaceutically active agent within a dosing regimen in accordance with the present invention is referred to as a "first administration". Of course, such "first administration" does not exclude that the subject had been administered the same pharmaceutically active agent before the dosing regimen in accordance with the present invention had been initiated.

As will be clear to the skilled person, when the present invention refers to a single administration of a pharmaceutically active agent that occurs on a given day after transplantation, such single administration can also be split up into several administrations, as long as these several administrations occur on the same day. Of course, if a dose is given for such single administration and the single administration is split up into several administrations that occur on the same day, the doses of the several administrations need to add up to the dose of the single administration that is indicated in this invention. As will be clear to the skilled person, an administration may also be a continuous administration, wherein the pharmaceutically active agent is administered continuously throughout the whole day. If a dose is given for such continuous administration, the dose represents the cumulative dose that is administered throughout the whole day. If continuous administrations occur on consecutive days, the pharmaceutically active agent is administered continuously for several days.

The immunosuppressive induction therapy in accordance with the present invention is administered to the recipient organism according to a dosing regimen. Said dosing regimen comprises the administration of one or more immunosuppressive agent(s) before transplantation. Exemplary immunosuppressive agents that may be administered according to the dosing regimen of the immunosuppressive induction therapy include anti-CD20 antibody, anti-thymocyte-globuline and anti-CD40 antibody/anti-CD40L pasylated (PASylated) Fab (fragment antigen binding).

In one embodiment of the invention, the dosing regimen of the immunosuppressive induction therapy comprises the administration of anti-CD20 antibody. In a preferred embodiment, said anti-CD20 antibody is the anti-CD20 antibody Mabthera that is commercially available from Roche Pharma AG (Grenzach-Wyhlen, Germany). The dosing regimen of the immunosuppressive induction therapy may comprise a single or several administrations of anti-CD20 antibody. In a preferred embodiment, the dosing regimen comprises a single administration of anti-CD20 antibody. More preferably, said single administration of anti-CD20 antibody occurs between 5 and 9 days before heart/kidney/lung/liver transplantation, most preferably said single administration occurs 7 days before heart/kidney/lung/liver transplantation. The preferred dose of an anti-CD20 antibody administration is 19 mg per kg body weight. In accordance with all other aspects of this embodiment, the preferred route of anti-CD20 antibody administration is intravenous (i.v.) short infusion.

In another embodiment of the invention, the dosing regimen of the immunosuppressive induction therapy comprises the administration of anti-thymocyte-globuline (ATG). In a preferred embodiment, said anti-thymocyte-globuline is the anti-thymocyte-globuline Thymoglobuline that is commercially available from Sanofi-Aventis Germany GmbH (Frankfurt, Germany). The dosing regimen of the immunosuppressive induction therapy may comprise a single or several administrations of anti-thymocyte-globuline. In a preferred embodiment, the dosing regimen comprises several administrations of anti-thymocyte-globuline, in an even more preferred embodiment the dosing regimen comprises two administrations of anti-thymocyte-globuline. Preferably, said two administrations of anti-thymocyte-globuline occur within 3 days before heart/kidney/lung/liver transplantation, most preferably they occur 1 and 2 days before heart/kidney/lung/liver transplantation, respectively. The preferred dose of an anti-thymocyte-globuline administration is 50 mg per kg body weight. In another embodiment, the preferred dose of an anti-thymocyte-globuline administration is 5 mg per kg body weight. In accordance with all other aspects of this embodiment, the preferred route of anti-thymocyte-globuline administration is intravenous (i.v.) short infusion.

In another embodiment of the invention, the dosing regimen of the immunosuppressive induction therapy comprises the administration of anti-CD40 antibody or anti-CD40L pasylated (PASylated) Fab. In a preferred embodiment, said anti-CD40 antibody is the mouse/rhesus chimeric IgG4 clone 2C10R4 that is available from NIH Nonhuman Primate Reagent Resource, Mass Biologicals, Boston, MA, USA; courtesy of Keith Reimann. In another preferred embodiment, said anti-CD40L pasylated Fab is humanised anti-CD40L pasylated Fab that is produced by Wacker-Chemie (Burghausen, Germany) and purified by XL-protein GmbH (Freising, Germany) as described in Binder & Skerra (cf. reference 9). The dosing regimen of the immunosuppressive induction therapy may comprise a single or several administrations of anti-CD40 antibody/anti-CD40L pasylated Fab. In a preferred embodiment, the dosing regimen comprises a single administration of anti-CD40 antibody/anti-CD40L pasylated Fab. More preferably, said single administration of anti-CD40 antibody/anti-CD40L pasylated Fab occurs within 2 days before heart/kidney/lung/liver transplantation, most preferably said single administration occurs 1 day before heart/kidney/lung/liver transplantation. The preferred dose of an administration is 50 mg per kg body weight anti-CD40 antibody, or 20 mg per kg body weight anti-CD40L pasylated Fab. In accordance with all other aspects of this embodiment, the preferred route of anti-CD40 antibody/anti-CD40L pasylated Fab administration is intravenous (i.v.) short infusion.

In a preferred embodiment of the invention, the dosing regimen of the immunosuppressive induction therapy comprises the administration of anti-CD20 antibody, anti-thymocyte-globuline and anti-CD40 antibody/anti-CD40L pasylated (PASylated) Fab. In this preferred embodiment, the preferred anti-CD20 antibody, the preferred anti-thymocyte-globuline and the preferred anti-CD40 antibody/anti-CD40L pasylated Fab are the same as described for the respective embodiments above. Moreover, in this preferred embodiment also the preferred frequencies, the preferred time points, the preferred doses and the preferred routes of administration are the same as described for the respective embodiments above. A particularly preferred dosing regimen of the immunosuppressive induction therapy in accordance with the present invention is depicted in Figure 7.

The surgical heart/kidney/lung/liver extraction from the donor organism in accordance with the present invention is performed as will be known to a person skilled in the art. Subsequently, the surgically extracted heart/kidney/lung/liver is preserved by non-ischemic preservation until transplantation. The duration of non-ischemic preservation is preferably shorter than 24 hours, more preferably shorter than 15 hours, even more preferably shorter than 10 hours, even more preferably shorter than 5 hours, and most preferably shorter than 4 hours. Preferably, said non-ischemic preservation is a non-ischemic heart/kidney/lung/liver perfusion, e.g. as described for non-ischemic heart perfusion by Steen et al. in reference 10 (incorporated herein in its entirety). For such non-ischemic heart perfusion, 3.5 L of an oxygenated albumin-containing hyperoncotic cardioplegic nutrition solution with hormones and erythrocytes may be used at a temperature of 8°C in a portable extracorporal heart preservation system consisting of a pressure- and flow-controlled roller pump, an $O_2$/$CO_2$ exchanger, a leukocyte filter, an arterial filter and a cooler-heater unit. After aortic crossclamping, the heart may be perfused with 600 ml of preservation medium, then excised and moved into the cardiac preservation system. A large cannula may be introduced into the ascending aorta and the mitral valve may be made temporarily incompetent to prevent left ventricular dilation; the superior caval vein may be occluded, inferior caval vein, pulmonary artery and pulmonary veins may however be left open for free outlet of perfusate. The heart may be submersed in a reservoir filled with cold perfusion medium and antegrade coronary perfusion may commence via the already placed aortic canula. The perfusion pressure may remain regulated at precisely 20 mmHg. During implantation, the heart may be intermittently perfused for 2 min every 20 min.

Also the heart/kidney/lung/liver transplantation into the recipient organism in accordance with the present invention is performed as will be known to a person skilled in the art. Preferably, the recipient organism receives anesthesia and analgesia treatment that may comprise administering ketamine hydrochloride, midazolam, propofol, fentanyl, sevoflurane and/or metamizole, e.g. as described in reference 11 (incorporated herein in its entirety). In a preferred embodiment, the heart transplantation is performed applying Shumway's and Lower's technique as described in reference 8, which is incorporated herein in its entirety.

In the methods of the present invention, the surgically extracted, preserved heart/kidney/lung/liver of the donor organism is transplanted into the recipient organism so that it functionally substitutes the heart/kidney/lung/liver of the recipient organism. Such functional substitution is preferably an orthotopic substitution, but may also be a heterotopic support, and in the case of a transplanted heart an intrathoracic heterotopic support. Orthotopic heart substitution can be performed as described in Example 1. Intrathoracic heterotopic heart transplantation is also life supporting and would be an alternative. In brief: Intrathoracic heterotopic heart transplantation leaves the recipient's heart in place and as a back-up in case the transplanted xenogeneic organ fails - an important strategy that may be applied at least at the very beginning of a clinical pilot study (and re-transplantation would be possible). Intrathoracic heterotopic xenogeneic heart transplantation can be performed using a technique (Figure 10B) based on the method developed by C.N. Barnard (cf. references 24, 25). This procedure requires the support of a heart-lung machine. The donor heart (e.g., porcine heart) is placed to the right of the recipient (e.g., baboon) heart, thereby compressing parts of the right lung. Implantation begins with anastomoses of the left and right atria. Both aortas are then combined in an end-to-side fashion. Finally, both pulmonary arteries are connected with the help of an interposition graft (cf. Figure 10B). In contrast with abdominally transplanted hearts, which are contracting but not ejecting (cf. Figure 10C, immunologic model), intrathoracically placed transplants eject blood. Postoperative echocardiographies prove systolic and diastolic movements of the aortic and mitral valves, and aortic systolic pressure curves correlate with the donor ECG. After intrathoracic heterotopic procedures, both the donor and the recipient heart (which in clinical situations may have impaired function) contribute to cardiac output according to their respective cardiac cycles (their preloads). Intrathoracic heterotopic xenogeneic heart transplantation will be performed as described in reference 25. Of course, the intrathoracic heterotopic xenogeneic heart transplantation described in reference 25 is preferably combined with non-ischemic heart preservation in accordance with the present invention, with administering an immunosuppressive induction and maintenance therapy in accordance with the present invention, with decreasing the dosage of the glucocorticoid administered as part of the immunosuppressive maintenance therapy in accordance with the present invention, with administering an anti-inflammatory therapy in accordance with the present invention, with administering one or more antihypertensive agent(s) in accordance with the present invention, and/or with administering an mTOR inhibitor in accordance with the present invention.

The immunosuppressive maintenance therapy in accordance with the present invention is administered to the recipient organism according to a dosing regimen. Said dosing regimen comprises the administration of one or more immunosuppressive agent(s) after transplantation, wherein the dosing regimen comprises a first administration of a glucocorticoid within 1 day after transplantation, preferably on the day of transplantation. In addition to the glucocorticoid, exemplary immunosuppressive agents that may be administered in accordance with the dosing regimen of the immunosuppressive maintenance therapy include anti-CD20 antibody, mycophenolate mofetil and anti-CD40 antibody/anti-CD40L pasylated Fab.

The dosing regimen of the immunosuppressive maintenance therapy in accordance with the present invention optionally comprises the administration of a glucocorticoid. A first administration of the glucocorticoid occurs within 1 day after transplantation, preferably on the day of transplantation. The dosing regimen of the immunosuppressive maintenance therapy comprises several administrations of the glucocorticoid, preferably one administration on each day after the first administration. The preferred dosage of the glucocorticoid on the day of the first administration is 5 mg/kg/day to 15 mg/kg/day, preferably 7 mg/kg/day to 13 mg/kg/day, more preferably 9 mg/kg/day to 11 mg/kg/day, most preferably 10 mg/kg/day. The dosing regimen of the immunosuppressive maintenance therapy comprises decreasing the dosage (mg/kg/day) of the glucocorticoid administered to the recipient organism by a factor of at least 5, preferably by a factor of at least 10, within 5 days after transplantation. For example, if the dosage of the glucocorticoid on the day of the first administration is 10 mg/kg/day, the maximum dosage on the $5^{th}$ day after transplantation is 2 mg/kg/day, preferably 1 mg/kg/day. In a preferred embodiment, the dosing regimen of the immunosuppressive maintenance therapy comprises decreasing the dosage (mg/kg/day) of the glucocorticoid administered to the recipient organism by a factor of at least 10, preferably by a factor of at least 20, within 10 days after transplantation, in various aspects. In another preferred embodiment, the dosing regimen of the immunosuppressive maintenance therapy comprises decreasing the dosage (mg/kg/day) of the glucocorticoid administered to the recipient organism by a factor of at least 50, preferably by a factor of at least 100, within 20 days after transplantation. In principle any corticosteroid with glucocorticoid potency may be used in the present invention. However, in a preferred embodiment the glucocorticoid is cortisol, cortisone, prednisone, prednisolone, methylprednisolone, dexamethasone, betamethasone or triamcinolone, preferably the glucocorticoid is prednisolone or methylprednisolone, most preferably the glucocorticoid is methylprednisolone. Methylprednisolone is commercially available, e.g., as Urbasone solubile from Sanofi-Aventis Germany GmbH (Frankfurt, Germany). In accordance with all other aspects of this embodiment, the preferred route of administration of the glucocorticoid is intravenous (i.v.) bolus injection.

In one embodiment of the invention, the dosing regimen of the immunosuppressive maintenance therapy comprises the administration of anti-CD20 antibody. If anti-CD20 antibody had already been administered as part of the preceding immunosuppressive induction therapy, the anti-CD20 antibody administered as part of the immunosuppressive maintenance therapy is preferably the same anti-CD20 antibody that had been administered as part of the immunosuppressive induction therapy. In a preferred embodiment, the anti-CD20 antibody of the immunosuppressive induction therapy and of the immunosuppressive maintenance therapy is the anti-CD20 antibody Mabthera that is commercially available from Roche Pharma AG (Grenzach-Wyhlen, Germany). The dosing regimen of the immunosuppressive maintenance therapy may comprise a single or several administrations of anti-CD20 antibody. In a preferred embodiment, the dosing regimen comprises several administrations of anti-CD20 antibody. More preferably, the dosing regimen comprises 2 to 4 administrations of anti-CD20 antibody, most preferably the dosing regimen comprises 3 administrations of anti-CD20 antibody. In one embodiment, the dosing regimen of the immunosuppressive maintenance therapy comprises an administration of anti-CD20 antibody within 1 day after transplantation, preferably the dosing regimen of the immunosuppressive maintenance therapy comprises an administration of anti-CD20 antibody on the day of transplantation. In another embodiment, if the dosing regimen comprises two or more administrations of anti-CD20 antibody, the dosing regimen comprises an administration of anti-CD20 antibody within 1 day after transplantation and an administration of anti-CD20 antibody between 6 and 8 days after transplantation, preferably an administration of anti-CD20 antibody on the day of transplantation and an administration of anti-CD20 antibody 7 days after transplantation. In another embodiment, if the dosing regimen comprises three or more administrations of anti-CD20 antibody, the dosing regimen comprises an administration of anti-CD20 antibody within 1 day after transplantation, an administration of anti-CD20 antibody between 6 and 8 days after transplantation and an administration of anti-CD20 antibody between 13 and 15 days after transplantation, preferably an administration of anti-CD20 antibody on the day of transplantation, an administration of anti-CD20 antibody 7 days after transplantation and an administration of anti-CD20 antibody 14 days after transplantation. The preferred dose of an anti-CD20 antibody administration is 19 mg per kg body weight. In accordance with all other aspects of this embodiment, the preferred route of anti-CD20 antibody administration is intravenous (i.v.) short infusion.

In another embodiment of the invention, the dosing regimen of the immunosuppressive maintenance therapy comprises the administration of mycophenolate mofetil (MMF). In a preferred embodiment, the mycophenolate mofetil of the immunosuppressive maintenance therapy is the mycophenolate mofetil CellCept that is commercially available from Roche (Basle, Switzerland). The dosing regimen of the immunosuppressive maintenance therapy preferably comprises several administrations of mycophenolate mofetil, wherein one administration occurs within 1 day after transplantation, preferably on the day of transplantation. In a preferred embodiment, the dosing regimen of the immunosuppressive maintenance therapy comprises one administration of mycophenolate mofetil on each day after transplantation. The preferred dose of a mycophenolate mofetil administration is 40 mg per kg body weight. Even more preferably, the dose is adjusted to a trough level of 2-3 μg/ml. In accordance with all other aspects of this embodiment, the preferred route of mycophenolate mofetil administration is continuous intravenous (i.v.) infusion. In a preferred embodiment, when mycophenolate mofetil is administered according to a dosing regimen of the immunosuppressive maintenance therapy, mycophenolate mofetil is additionally administered already before transplantation, in order to reach therapeutic drug levels already at the time of transplantation. For example, mycophenolate mofetil may be administered once or several times before transplantation. Preferably, mycophenolate mofetil is administered several times before transplantation, and even more preferably mycophenolate mofetil is administered twice before transplantation. Preferably, said two administrations of mycophenolate mofetil occur within 3 days before transplantation, most preferably they occur 1 and 2 days before heart/kidney/lung/liver transplantation, respectively. The preferred mycophenolate mofetil, the preferred dose of mycophenolate mofetil administration and the preferred route of administration are as described above for the mycophenolate mofetil administration in accordance with the dosing regimen of the immunosuppressive maintenance therapy.

In another embodiment of the invention, the dosing regimen of the immunosuppressive maintenance therapy comprises the administration of anti-CD40 antibody or anti-CD40L pasylated (PASylated) Fab. If anti-CD40 antibody or anti-CD40L pasylated Fab had already been administered as part of the preceding immunosuppressive induction therapy, the anti-CD40 antibody or anti-CD40L pasylated Fab administered as part of the immunosuppressive maintenance therapy is preferably the same anti-CD40 antibody or anti-CD40L pasylated Fab that had been administered as part of the immunosuppressive induction therapy. In a preferred embodiment, said anti-CD40 antibody is the mouse/rhesus chimeric IgG4 clone 2C10R4 that is available from NIH Nonhuman Primate Reagent Resource, Mass Biologicals, Boston, MA, USA; courtesy of Keith Reimann. In another preferred embodiment, said anti-CD40L pasylated Fab is humanised anti-CD40L pasylated Fab that is produced by Wacker-Chemie (Burghausen, Germany) and purified by XL-protein GmbH (Freising, Germany) as described in Binder & Skerra (cf. reference 9). The dosing regimen of the immunosuppressive maintenance preferably comprises several administrations of anti-CD40 antibody/anti-CD40L pasylated Fab, wherein one administration occurs within 1 day after transplantation, preferably on the day of transplantation. In a preferred embodiment, the dosing regimen of the immunosuppressive maintenance therapy comprises one administration within 1 day after transplantation, 4 administrations within 8 days after transplantation, 6 administrations within 15 days after transplantation, 7 administrations within 20 days after transplantation and subsequent administrations every 6 to 8 days. The preferred dose of an administration is 50 mg per kg body weight anti-CD40 antibody, or 20 mg per kg body weight anti-CD40L pasylated Fab. In accordance with all other aspects of this embodiment, the preferred route of anti-CD40 antibody/anti-CD40L pasylated Fab administration is intravenous (i.v.) short infusion.

In a preferred embodiment of the invention, the dosing regimen of the immunosuppressive maintenance therapy comprises the administration of a glucocorticoid, anti-CD20 antibody, mycophenolate mofetil and anti-CD40 antibody/anti-CD40L pasylated (PASylated) Fab. In this preferred embodiment, the preferred glucocorticoid, the preferred anti-CD20 antibody, the preferred mycophenolate mofetil and the preferred anti-CD40 antibody/anti-CD40L pasylated Fab are the same as described for the respective embodiments above. Moreover, in this preferred embodiment also the preferred frequencies, the preferred time points, the preferred doses (or dosages) and the preferred routes of administration are the same as described for the respective embodiments above. A particularly preferred dosing regimen of the immunosuppressive maintenance therapy in accordance with the present invention is depicted in Figure 7.

The anti-inflammatory therapy in accordance with the present invention is administered to the recipient organism according to a dosing regimen. Said dosing regimen comprises the administration of one or more anti-inflammatory agent(s) after transplantation. Exemplary anti-inflammatory agents that may be administered in accordance with the dosing regimen of the anti-inflammatory therapy include an IL-1 receptor antagonist, a TNF-$\alpha$ (= TNFa = TNF$\alpha$) inhibitor and an IL-6 receptor antibody. In the present invention, anti-inflammatory treatment should counteract typical unspecific xenogeneic inflammatory reactions like pleural/pericardial effusions.

In one embodiment of the invention, the dosing regimen of the anti-inflammatory therapy comprises the administration of an IL-1 receptor antagonist. In a preferred embodiment, said IL-1 receptor antagonist is the IL-1 receptor antagonist Kineret that is commercially available from Swedish Orphan Biovitrum GmbH (Martinsried, Germany). In a preferred embodiment, the dosing regimen of the anti-inflammatory therapy comprises several administrations of IL-1 receptor antagonist, wherein one administration occurs within 1 day after transplantation, preferably on the day of transplantation. In a more preferred embodiment, the dosing regimen of the anti-inflammatory therapy comprises several administrations of IL-1 receptor antagonist, wherein one administration occurs within 1 day after transplantation, preferably on the day of transplantation, and wherein one administration occurs on each subsequent day. The preferred dose of an IL-1 receptor antagonist administration is 1.3 mg per kg body weight. In accordance with all other aspects of this embodiment, the preferred route of IL-1 receptor antagonist administration is subcutaneous (s.c.) or intravenous (i.v.) bolus injection.

In another embodiment of the invention, the dosing regimen of the anti-inflammatory therapy comprises the administration of a TNF-$\alpha$ inhibitor. In a preferred embodiment, said TNF-$\alpha$ inhibitor is the TNF-$\alpha$ inhibitor Enbrel that is commercially available from Pfizer Pharma GmbH (Berlin, Germany). In a preferred embodiment, the dosing regimen of the anti-inflammatory therapy comprises several administrations of TNF-$\alpha$ inhibitor, wherein one administration occurs within 1 day after transplantation, preferably on the day of transplantation. In a more preferred embodiment, the dosing regimen of the anti-inflammatory therapy comprises several administrations of TNF-$\alpha$ inhibitor, wherein one administration occurs within 1 day after transplantation and one administration occurs every 6 to 8 days thereafter, preferably wherein one administration occurs on the day of transplantation and one administration occurs every 7 days thereafter. The preferred dose of a TNF-$\alpha$ inhibitor administration is 0.7 mg per kg body weight. In accordance with all other aspects of this embodiment, the preferred route of TNF-$\alpha$ inhibitor administration is subcutaneous (s.c.) bolus injection.

In another embodiment of the invention, the dosing regimen of the anti-inflammatory therapy comprises the administration of an IL-6 receptor antibody. In a preferred embodiment, said IL-6 receptor antibody is the IL-6 receptor antibody RoActemra that is commercially available from Roche Pharma AG (Grenzach-Wyhlen, Germany). The dosing regimen of the anti-inflammatory therapy may comprise a single or several administrations of IL-6 receptor antibody. In a preferred embodiment the dosing regimen of the anti-inflammatory therapy comprises several administrations of IL-6 receptor antibody, wherein one administration occurs within 1 day after transplantation, preferably on the day of transplantation. In a more preferred embodiment, the dosing regimen of the anti-inflammatory therapy comprises several administrations of IL-6 receptor antibody, wherein one administration occurs within 1 day after transplantation and one administration occurs every 20 to 40 days thereafter, preferably wherein one administration occurs on the day of transplantation and one administration occurs every 25 to 35 days thereafter. The preferred dose of an IL-6 receptor antibody administration is 8 mg per kg body weight. In accordance with all other aspects of this embodiment, the preferred route of IL-6 receptor antibody administration is intravenous (i.v.) short infusion.

In a preferred embodiment of the invention, the dosing regimen of the anti-inflammatory therapy comprises the administration IL-1 receptor antagonist, TNF-$\alpha$ inhibitor and IL-6 receptor antibody. In this preferred embodiment, the preferred IL-1 receptor antagonist, the preferred TNF-$\alpha$ inhibitor and the preferred IL-6 receptor antibody are the same as described for the respective embodiments above. Moreover, in this preferred embodiment also the preferred frequencies, the preferred time points, the preferred doses and the preferred routes of administration are the same as described for the respective embodiments above. A particularly preferred dosing regimen of the anti-inflammatory therapy in accordance with the present invention is depicted in Figure 8.

The one or more antihypertensive agent(s) in accordance with the present invention is/are administered to the recipient organism according to a dosing regimen. Said dosing regimen comprises the administration of one or more antihypertensive agent(s) after transplantation. Exemplary antihypertensive agent(s) that may be administered in accordance with the dosing regimen of the anti-inflammatory therapy include diuretics such as the loop diuretics furosemide and ethacrynic acid, the thiazide/thiazide-like diuretics hydrochlorothiazide and indapamide, and the potassium-sparing diuretics triamterene and amiloride; calcium channel blockers such as the dihydropyridines amlodipine and nifedipine, and the non-dihydropyridines verapamil and diltiazem; angiotensin-converting enzyme (ACE) inhibitors such as enalapril and ramipril; angiotensin II receptor antagonists such as valsartan and candesartan; adrenergic receptor antagonists such as the beta blockers bisoprolol and metoprolol, the alpha blockers doxazosin and phenoxybenzamine, and the mixed alpha/beta blockers carvedilol and labetalol; vasodilators such as sodium nitroprusside and hydralazine; renin inhibitors such as aliskiren; aldosterone receptor antagonists such as spironolactone and eplerenone; alpha-2 adrenergic receptor agonists such as clonidine and methyldopa; and endothelin receptor blockers such as bosentan. In a preferred embodiment, the dosing regimen comprises the administration of one or more antihypertensive agent(s) selected from angiotensin-converting enzyme (ACE) inhibitors and adrenergic receptor antagonists. Preferably, the dose of the one or more antihypertensive agent(s) is adjusted so that the mean arterial pressure of the recipient organism is kept within the range of 70 to 90 mmHg, preferably within the range of 75 to 85 mmHg. Even more preferably, the dose is adjusted so that the mean arterial pressure of the recipient organism is kept within the range of 70 to 90 mmHg, preferably within the range of 75 to 85 mmHg, and so that the heart rate is kept within the range of 80 to 120 bpm, preferably within the range of 90 to 110 bpm.

In one embodiment of the invention, the dosing regimen of administering the one or more antihypertensive agent(s) comprises the administration of an angiotensin-converting enzyme (ACE) inhibitor. In a preferred embodiment, said angiotensin-converting enzyme (ACE) inhibitor is enalapril. Enalapril is commercially available, e.g., as Enahexal from Hexal AG (Holzkirchen, Germany). In a preferred embodiment, the dosing regimen of administering the one or more antihypertensive agent(s) comprises several administrations of an angiotensin-converting enzyme (ACE) inhibitor, wherein one administration occurs within 2 days after transplantation, preferably within 1 day after transplantation. In a more preferred embodiment, the dosing regimen of administering the one or more antihypertensive agent(s) comprises several administrations of an angiotensin-converting enzyme (ACE) inhibitor, wherein one administration occurs within 2 days after transplantation, preferably within 1 day after transplantation, and wherein one administration occurs on each subsequent day. In accordance with all other aspects of this embodiment, the preferred route of angiotensin-converting enzyme (ACE) inhibitor administration is intravenous (i.v.) administration.

In another embodiment of the invention, the dosing regimen of administering the one or more antihypertensive agent(s) comprises the administration of an adrenergic receptor antagonist. In a preferred embodiment, said adrenergic receptor antagonist is metoprolol tartrate. Metoprolol tartrate is commercially available, e.g., as Beloc i.v. from AstraZeneca GmbH (Wedel, Germany). In a preferred embodiment, the dosing regimen of administering the one or more antihypertensive agent(s) comprises several administrations of an adrenergic receptor antagonist, wherein one administration occurs within 2 days after transplantation, preferably within 1 day after transplantation. In a more preferred embodiment, the dosing regimen of administering the one or more antihypertensive agent(s) comprises several administrations of an adrenergic receptor antagonist, wherein one administration occurs within 2 days after transplantation, preferably within 1 day after transplantation, and wherein one administration occurs on each subsequent day. In accordance with all other aspects of this embodiment, the preferred route of adrenergic receptor antagonist administration is intravenous (i.v.) administration.

In a preferred embodiment of the invention, the dosing regimen of administering the one or more antihypertensive agent(s) comprises the administration of an angiotensin-converting enzyme (ACE) inhibitor and an adrenergic receptor antagonist. In this preferred embodiment, the preferred angiotensin-converting enzyme (ACE) inhibitor and the preferred adrenergic receptor antagonist are the same as described for the respective embodiments above. Moreover, in this preferred embodiment also the preferred frequencies, the preferred time points and the preferred routes of administration are the same as described for the respective embodiments above. A particularly preferred dosing regimen of administering the one or more antihypertensive agent(s) in accordance with the present invention is depicted in Figure 9. The antihypertensive agent(s) in accordance with the present invention is/are administered to the recipient organism in order to decrease the blood pressure to the (lower) level of the donor (e.g. pig) organism.

The mTOR inhibitor in accordance with the present invention is administered to the recipient organism according to a dosing regimen. Said dosing regimen comprises the administration of the mTOR inhibitor after transplantation. Exemplary mTOR inhibitors that may be administered in accordance with the dosing regimen of administering an mTOR inhibitor include dactolisib (BEZ235, NVP-BEZ235), rapamycin (sirolimus), everolimus (RAD001), AZD8055, temsirolimus (CCI-779, NSC 683864), SF2523, 3BDO, PI-103, KU-0063794 I, torkinib (PP242), ridaforolimus (deforolimus, MK-8669), sapanisertib (INK 128, MLN0128), voxtalisib (SAR245409, XL765) analogue, torin 1, omipalisib (GSK2126458, GSK458), OSI-027, PF-04691502, apitolisib (GDC-0980, RG7422), GSK1059615, gedatolisib (PF-05212384, PKI-587), WYE-354, vistusertib (AZD2014), torin 2, WYE-125132 (WYE-132), BGT226 (NVP-BGT226), palomid 529 (P529), PP121, WYE-687, WAY-600, ETP-46464, GDC-0349, XL388, zotarolimus (ABT-578), CZ415, MHY1485, voxtalisib (XL765, SAR245409), chrysophanic acid and CC-223, LY3023414. In a preferred embodiment, the dosing regimen comprises the administration of the mTOR inhibitor temsirolimus. Temsirolimus is available for intravenous application, e.g., as Torisel from Pfizer Pharma GmbH (Berlin, Germany). Temsirolimus may be long-acting, similar to anti-CD40 antibody/anti-CD40L pasylated Fab. This is a handling advantage in non-human primate experiments. The mTOR inhibitor in accordance with the present invention is administered to the recipient organism to inhibit growth hormones, in order to inhibit overgrowth of the transplanted heart/kidney/lung/liver.

In a preferred embodiment, the dosing regimen of administering the mTOR inhibitor comprises several administrations of the mTOR inhibitor, wherein the first administration occurs within 10 days after transplantation, preferably between 2 days and 9 days after transplantation, more preferably between 4 days and 7 days after transplantation. In a more preferred embodiment, the dosing regimen of administering the mTOR inhibitor comprises several administrations of the mTOR inhibitor, wherein the first administration occurs within 10 days after transplantation, preferably between 2 days and 9 days after transplantation, more preferably between 4 days and 7 days after transplantation, and wherein one administration occurs on each subsequent day. As will be clear to the skilled person, the time point of the first administration of the mTOR inhibitor depends on the general condition of the recipient (e.g. whether there are pericardial/pleural effusions). Preferably, the dose of mTOR inhibitor administration is adjusted to a trough level of 5-10 ng/ml rapamycin. In accordance with all other aspects of this embodiment, the preferred route of mTOR inhibitor administration is intravenous (i.v.) short infusion. A particularly preferred dosing regimen of administering an mTOR inhibitor in accordance with the present invention is depicted in Figure 7.

In one embodiment of the present invention, the recipient organism that is transplanted a genetically modified heart/kidney/lung/liver from a xenogeneic donor organism receives additive therapy according to a dosing regimen. Said dosing regimen comprises the administration of one or more pharmaceutically active agent(s). Exemplary pharmaceutically active agents that may be administered according to the dosing regimen of the additive therapy include acetylsalicylic acid, unfractionated heparin, C1 esterase inhibitor, ganciclovir, cefuroxime and epoetin beta.

In one embodiment of the invention, the dosing regimen of the additive therapy comprises the administration of acetylsalicylic acid. In a preferred embodiment, said acetylsalicylic acid is the acetylsalicylic acid Aspirin that is commercially available from Bayer Vital GmbH (Leverkusen, Germany). The dosing regimen of the additive therapy preferably comprises several administrations of acetylsalicylic acid. In a preferred embodiment, the dosing regimen comprises an acetylsalicylic acid administration each day after transplantation. The preferred dose of an acetylsalicylic acid administration is 2 mg per kg body weight. In accordance with all other aspects of this embodiment, the preferred route of acetylsalicylic acid administration is intravenous (i.v.) bolus injection. In the present invention, acetylsalicylic acid is used as an anti-coagulant.

In another embodiment of the invention, the dosing regimen of the additive therapy comprises the administration of unfractionated heparin. In a preferred embodiment, said unfractionated heparin is the unfractionated heparin Heparin-Natrium-25000-ratiopharm that is commercially available from ratiopharm GmbH (Ulm, Germany). The dosing regimen of the additive therapy preferably comprises several administrations of unfractionated heparin, wherein the first administration of unfractionated heparin occurs 4 to 6 days after transplantation, preferably 5 days after transplantation. In a more preferred embodiment, the dosing regimen of the additive therapy comprises several administrations of unfractionated heparin, wherein the first administration of unfractionated heparin occurs 4 to 6 days after transplantation, preferably 5 days after transplantation, and wherein one administration occurs on each subsequent day. The preferred dose of unfractionated heparin administration is 480 to 960 U per kg bodyweight per day. In accordance with all other aspects of this embodiment, the preferred route of unfractionated heparin administration is continuous intravenous (i.v.) infusion. Thus, the unfractionated heparin is preferably administered continuously for 24 hours a day at 20 to 40 U per kg bodyweight per hour. In the present invention, unfractionated heparin is used as an anti-coagulant.

In another embodiment of the invention, the dosing regimen of the additive therapy comprises the administration of C1 esterase inhibitor. In a preferred embodiment, said C1 esterase inhibitor is the C1 esterase inhibitor Berinert that is commercially available from CSL Behring GmbH (Hattersheim, Germany). The dosing regimen of the additive therapy preferably comprises several administrations of C1 esterase inhibitor, wherein the first administration occurs within 1 day after transplantation, preferably on the day of transplantation. In a more preferred embodiment, the dosing regimen of the additive therapy comprises several administrations of C1 esterase inhibitor, wherein the first administration occurs within 1 day after transplantation, another administration occurs between 6 and 8 days after transplantation, and another administration occurs between 13 and 15 days after transplantation. In the most preferred embodiment, the dosing regimen of the additive therapy comprises several administrations of C1 esterase inhibitor, wherein the first administration occurs on the day of transplantation, another administration occurs 7 days after transplantation, and another administration occurs 14 days after transplantation. The preferred dose of a C1 esterase inhibitor administration is 17.5 U per kg body weight. In accordance with all other aspects of this embodiment, the preferred route of C1 esterase inhibitor administration is intravenous (i.v.) short infusion. In the present invention, the C1 esterase inhibitor is used as a complement inhibitor.

In another embodiment of the invention, the dosing regimen of the additive therapy comprises the administration of ganciclovir. In a preferred embodiment, said ganciclovir is the ganciclovir Cymevene that is commercially available from Roche Pharma AG (Grenzach-Wyhlen, Germany). The dosing regimen of the additive therapy preferably comprises several administrations of ganciclovir. In a preferred embodiment, the dosing regimen comprises a ganciclovir administration on each day after transplantation. The preferred dose of a ganciclovir administration is 5 mg per kg body weight. In accordance with all other aspects of this embodiment, the preferred route of ganciclovir administration is continuous intravenous (i.v.) infusion. In the present invention, ganciclovir is used to block primate cyclomegalovirus infection.

In another embodiment of the invention, the dosing regimen of the additive therapy comprises the administration of cefuroxim. In a preferred embodiment, said cefuroxim is Cefuroxim Hikma that is commercially available from Hikma Pharma GmbH (Martinsried, Germany). The dosing regimen of the additive therapy preferably comprises several administrations of cefuroxim. In a preferred embodiment, the dosing regimen comprises a cefuroxim administration within 1 day after transplantation, preferably on the day of transplantation. In a more preferred embodiment, the dosing regimen comprises a cefuroxim administration on the day of transplantation and a cefuroxim administration on each subsequent day until (and including) day 5 after transplantation. The preferred dose of a cefuroxim administration is 50 mg per kg body weight. In accordance with all other aspects of this embodiment, the preferred route of cefuroxim administration is continuous intravenous (i.v.) infusion. In the present invention, cefuroxim (a cephalosporin) is used as a broad antibiotic prophylaxis against bacterial infections that may be contracted during surgery.

In another embodiment of the invention, the dosing regimen of the additive therapy comprises the administration of epoetin beta. In a preferred embodiment, said epoetin beta is the epoetin beta NeoRecormon 5000IU that is commercially available from Roche Pharma AG (Grenzach-Wyhlen, Germany). The dosing regimen of the additive therapy may comprise a single administration or several administrations of epoetin beta. In a preferred embodiment, the dosing regimen comprises an epoetin beta administration 6 to 8 days before transplantation and an epoetin beta administration between 1 day before and 1 day after transplantation. In a more preferred embodiment, the dosing regimen comprises an epoetin beta administration 7 days before transplantation and an epoetin beta administration on the day of transplantation. The preferred dose of an epoetin beta administration is 2000 U. In accordance with all other aspects of this embodiment, the preferred route of epoetin beta administration is subcutaneous (s.c.) or intravenous (i.v.) bolus injection. In the present invention, erythropoetin is used as a hormone stimulating hemoglobulin production (i.e. it may counteract blood losses).

In a preferred embodiment of the invention, the dosing regimen of the additive therapy comprises the administration of acetylsalicylic acid, unfractionated heparin, C1 esterase inhibitor, ganciclovir, cefuroxime and epoetin beta. In this preferred embodiment, the preferred acetylsalicylic acid, the preferred unfractionated heparin, the preferred C1 esterase inhibitor, the preferred ganciclovir, the preferred cefuroxime and the preferred epoetin beta are the same as described for the respective embodiments above. Moreover, in this preferred embodiment also the preferred frequencies, the preferred time points, the preferred doses and the preferred routes of administration are the same as described for the respective embodiments above.

*Primate with a transplanted heart/kidney/lung/liver*

The present invention also relates to a living primate (e.g., a non-human living primate) whose heart/kidney/lung/liver is functionally substituted by a transplanted, genetically modified heart/kidney/lung/liver from a xenogeneic mammal, wherein said living primate is obtainable by the method of prolonging the survival of a primate following heart/kidney/lung/liver transplantation as described above.

Additionally, the present invention relates to a living primate whose heart/kidney/lung/liver is functionally substituted by a transplanted, genetically modified heart/kidney/lung/liver from a xenogeneic mammal, wherein the heart/kidney/lung/liver of said living primate has been transplanted more than 40 days ago, more than 50 days ago, more than 60 days ago, more than 70 days ago, more than 80 days ago, or more than 90 days ago, preferably more than 50 days ago, more preferably more than 70 days ago and most preferably more than 90 days ago.

In one embodiment, the living primate belongs to the family *Cercopithecidae*, preferably to the genus *Papio*, more preferably to the species *Papio Anubis* or *Papio Hamadryas*, most preferably to the species *Papio Anubis.* In an alternative embodiment, the living primate belongs to the family *Cercopithecidae*, preferably to the genus Macaca, most preferably to the species *M. mulatta.*

In another embodiment, the xenogeneic mammal belongs to the family *Suidae,* more preferably to the genus *Sus*, even more preferably to the species *S. scrofa,* and most preferably to the subspecies *S. s. domesticus.* Preferably, the xenogeneic mammal is a pig of German Landrace/Large White cross-bred genetic background, a Duroc pig, a Schwäbisch-Hällische (Swabian-Hall) pig, a black mini pig, or an Auckland Island pig; more preferably the xenogeneic mammal is a pig of German Landrace/Large White cross-bred genetic background. In accordance with all other aspects of this embodiment, the xenogeneic mammal was juvenile at the time of transplantation.

The heart/kidney/lung/liver in accordance with the present invention is genetically modified in order to avoid transplant rejection by the living primate. The skilled person will be aware of suitable genetic modifications depending on the respective donor organism and the respective living primate in accordance with the invention. In one embodiment, the genetically modified heart/kidney/lung/liver of the living primate has the following genetic modifications: (1) homozygous knock-out of the alpha-1,3-galactosyltransferase gene; (2) insertion of the human CD46 gene; and (3) insertion of the human thrombomodulin gene. When the donor organism belongs to the family *Suidae* and the living primate belongs to the genus *Papio*, the genetically modified heart/kidney/lung/liver may optionally only have the aforementioned genetic modification (1), i.e. the homozygous knock-out of the alpha-1,3-galactosyltransferase gene. In another embodiment, the genetically modified heart/kidney/lung/liver of the living primate has the following genetic modifications: (1) homozygous knock-out of the alpha-1,3-galactosyltransferase gene, the CMAH gene and the B4GALNT2 gene; (2) insertion of the human CD46 gene, the human CD55 gene, the human CD59 gene, a hemeoxygenase 1 gene and an A20 gene; and optionally (3) insertion of a LEA gene or a PDL-1 (PD-L1, PDL1, CD274) gene. Details of the genetic modifications of this embodiment can be found in reference 23 (which is incorporated herein in its entirety). In this embodiment, the hemeoxygenase 1 gene, the A20 gene, the LEA gene and the PDL-1 gene can be from any organism, but preferably they are the human hemeoxygenase 1 gene, the human A20 gene, the human LEA gene and the human PDL-1 gene, respectively. In the above embodiments, when inserting the human CD46 gene (huCD46) preferably transcripts according to any transcript produced from the following genomic locus/coding region are expressed: GRCH38p7, NC_000001, region 207752038 - 207795516. More preferably, when inserting the human CD46 gene at least one of the following transcripts is expressed from a transgene expression cassette: NM_002389, NM_172359, NM_172351, NM_153826, NM_172352, NM_172353, NM_172350 and NM_172361. In the above embodiments, when inserting the human thrombomodulin gene (huTHBD, huTBM) preferably transcripts according to any transcript produced from the following genomic locus/coding region are expressed: GRCH38p7, NC_000020, region 23045633 - 23049664. More preferably, when inserting the human thrombomodulin gene the following transcript is expressed from a transgene expression cassette: NM_000361. In the above embodiments, when inserting the human CD55 gene (huCD55) preferably transcripts according to any transcript produced from the following genomic locus/coding region are expressed: GRCH38p7, NC_000001, region 207321472 - 207360966. More preferably, when inserting the human CD55 gene at least one of the following transcripts is expressed from a transgene expression cassette: NM_001114752, NM_001300903, NM_001300904, NM_000574 and NM_001300902. In the above embodiments, when inserting the human CD59 gene (huCD59) preferably transcripts according to any transcript produced from the following genomic locus/coding region are expressed: GRCH38p7, NC_000011, region 33703010 - 33736479. More preferably, when inserting the human CD59 gene at least one of the following transcripts is expressed from a transgene expression cassette: NM_000611, NM_203331, NM_203329, NM_203330, NM001127227, NM_001127226, NM_001127225 and NM_001127223. In the above embodiments, when inserting the human hemeoxygenase 1 gene (huHMOX1, huHO1) preferably transcripts according to any transcript produced from the following genomic locus/coding region are expressed: GRCH38p7, NC_000022, region 35381067 - 35394214. More preferably, when inserting the human hemeoxygenase 1 gene the following transcript is expressed from a transgene expression cassette: NM_002133. In the above embodiments, when inserting the human A20 gene (huA20, huTNFAIP3) preferably transcripts according to any transcript produced from the following genomic locus/coding region are expressed: GRCH38p7, NC_000006.12, region 137866317 - 137883314. More preferably, when inserting the human A20 gene at least one of the following transcripts is expressed from a transgene expression cassette: NM_001270508; NM_001270507; NM_006290. As will be clear to the person skilled in the art, the term "LEA gene" as used herein refers to a synthetic sequence coding for an artificial CTLA4-Ig protein, which has been optimized for high affinity to CD80/CD86. In the above embodiments, when inserting the human LEA gene preferably the following codon-optimized variant LEA29Y is expressed, e.g. from a transgene expression cassette:

atgggggtactgctcacacagaggacgctgctcagtctggtccttgcactcctgtttccaagcatggcgagcatggcgatgcacgtggcccagcctgctgtg
gtactggccagcagccgaggcatcgccagctttgtgtgtgagtatgcatctccaggcaaatatactgaggtccgggtgacagtgcttcggcaggctgacag
ccaggtgactgaagtctgtgcggcaacctacatgatggggaatgagttgaccttcctagatgattccatctgcacgggcacctccagtggaaatcaagtga
acctcactatccaaggactgagggccatggacacgggactctacatctgcaaggtggagctcatgtacccaccgccatactacgagggcataggcaac
ggaacccagatttatgtaattgatccagaaccgtgcccagattctgatcaggagcccaaatcttctgacaaaactcacacatccccaccgtccccagcacc
tgaactcctggggggatcgtcagtcttcctcttccccccaaaacccaaggacaccctcatgatctcccggacccctgaggtcacatgcgtggtggtggacgt
gagccacgaagaccctgaggtcaagttcaactggtacgtggacggcgtggaggtgcataatgccaagacaaagccgcgggaggagcagtacaaca
gcacgtaccgggtggtcagcgtcctcaccgtcctgcaccaggactggctgaatggcaaggagtacaagtgcaaggtctccaacaaagccctcccagcc
cccatcgagaaaaccatctccaaagccaaagggcagccccgagaaccacaggtgtacaccctgcccccatcccgggatgagctgaccaagaacca
ggtcagcctgacctgcctggtcaaaggcttctatcccagcgacatcgccgtggagtgggagagcaatgggcagccggagaacaactacaagaccacg
cctcccgtgctggactccgacggctccttcttcctctacagcaagctcaccgtggacaagagcaggtggcagcaggggaacgtcttctcatgctccgtgatg
catgaggctctgcacaaccactacacgcagaagagcctctccctgtctccgggtaaatga (SEQ ID NO: 1).

In the above embodiments, when inserting the human PDL-1 gene (huPD-L1, huPDL1, huCD274) preferably transcripts according to any transcript produced from the following genomic locus/coding region are expressed: GRCH38p7, NC_000009, region 5450503 - 5470567. More preferably, when inserting the human PDL-1 gene at least one of the following transcripts is expressed from a transgene expression cassette: NM_014143; NM_001267706; NM_00134029.

As will be clear to the person skilled in the art, and as directly follows from the above, when reference is made to the "insertion of a gene" in accordance with the present invention, in a preferred embodiment not the entire gene is inserted. In this embodiment, any sequence (e.g., in an expression cassette) may be inserted that allows for the transcript of the respective gene to be expressed.

In another embodiment that can be combined with any of the embodiments described above, the genetically modified heart/kidney/lung/liver of the present invention additionally has an insertion of a sequence encoding human $\alpha$1,2-fucosyltransferase. Preferably, the human $\alpha$1,2-fucosyltransferase is the protein expressed from the following gene: Official Symbol: FUT1; Official Full Name: fucosyltransferase 1 (H blood group); Primary source: HGNC:HGNC:4012; See related: Ensembl:ENSG00000174951, MIM:211100.

In the present invention, when reference is made to an insertion of a gene/sequence, this insertion may be a heterozygous or homozygous insertion. As will be clear to a person skilled in the art, if the expression level is not high enough with a heterozygous insertion, a homozygous insertion is preferred.

Preferably, the genetically modified heart/kidney/lung/liver in accordance with the present invention is obtained from a genetically modified donor organism, i.e. from a donor organism that carries the respective genetic modifications in its germ line. Methods of obtaining such genetically modified donor organisms (e.g. pigs) are known to a person skilled in the art. As an alternative to conventional techniques, genetically modified pigs can also be obtained using new techniques, such as CRISPR/CAS 9, which could provide for improved insertion loci.

In one embodiment of the primate with a transplanted heart, the transplanted heart of the living primate in accordance with the present invention has a left ventricular mass that is equal to or less than 150% of the left ventricular mass of the same heart at the time of transplantation. Preferably, the transplanted heart of said living primate has a left ventricular mass that is equal to or less than 140% of the left ventricular mass of the same heart at the time of transplantation, more preferably equal to or less than 130% of the left ventricular mass of the same heart at the time of transplantation, even more preferably equal to or less than 120% of the left ventricular mass of the same heart at the time of transplantation, and most preferably equal to or less than 110% of the left ventricular mass of the same heart at the time of transplantation. In this embodiment, the left ventricular (LV) mass, LV mass increase and fractional shortening (FS) are quantified as follows. Transthoracic echocardiographies are done in analgosedation at regular intervals, e.g. using an HP Sonos 7500 (HP Inc., Palo Alto, CA, USA); midpapillary short axis views are recorded. At end-diastole and end-systole, LV diameters (LVEDD, LVESD), diastolic and systolic interventricular (IVSd, IVSs), posterior wall thicknesses (PWd, PWs) are measured; the mean of three measurements is used for further calculations and visualization (Excel and PowerPoint, Microsoft, Redmond, Washington, USA). LV mass is calculated using the below formula 1, relative LV mass increase and LV FS using the below formulas 2 and 3 (cf. references 11, 12).

$$\text{(Formula 1) LV mass (g)} = 0.8*(1.04([LVEDD+IVSd+PWd]^3-[LVEDD]^3))+0.6$$

$$\text{(Formula 2) LV mass increase (\%)} = ([\text{LV mass}_{end}/\text{LV mass}_{start}] - 1)*100$$

$$\text{(Formula 3) FS (\%)} = ([LVEDD - LVESD]/LVEDD)*100$$

IVSd, diastolic intraventricular septum thickness; IVSs, systolic intraventricular septum thickness; LVEDD, left ventricular end-diastolic diameter; LVESD, left ventricular end-systolic diameter; PWd, diastolic posterior wall thickness; PWs, systolic posterior wall thickness.

In one embodiment, the living primate with a transplanted heart/kidney/lung/liver in accordance with the present invention has a platelet count of between 150 and 300 g/l, preferably between 170 and 250 g/l, more preferably between 180 and 230 g/l. Methods of determining the platelet count are known in the art.

In another embodiment, the living primate in accordance with the present invention has a bilirubin level of equal to or less than 1.2 mg/dl, preferably of equal to or less than 1.0 mg/dl, more preferably of equal to or less than 0.8 mg/dl, most preferably of equal to or less than 0.6 mg/dl. Methods of determining the bilirubin level are known in the art.

In another embodiment, the living primate in accordance with the present invention has a troponin T (hs) level of equal to or less than 0.3 ng/ml, preferably of equal to or less than 0.2 ng/ml, more preferably of equal to or less than 0.1 ng/ml, most preferably of equal to or less than 0.014 ng/ml. Methods of determining the troponin T (hs) level are known in the art.

In another embodiment, the living primate in accordance with the present invention has an LDH level of equal to or less than 1500 U/l, preferably of equal to or less than 1000 U/l, more preferably of equal to or less than 700 U/l, most preferably of equal to or less than 500 U/l. Methods of determining the LDH level are known in the art.

*mTOR inhibitor composition for use in a method of prolonging the survival of a primate following heart/kidney/lung/liver transplantation*

Additionally, the present invention relates to a composition comprising an mTOR inhibitor for use in a method of prolonging the survival of a recipient organism that has been transplanted a genetically modified heart/kidney/lung/liver from a xenogeneic donor organism, wherein the donor organism is a mammal and wherein the recipient organism is a primate. Preferably, said recipient organism has been transplanted the genetically modified heart/kidney/lung/liver so that it functionally substitutes the heart/kidney/lung/liver of the recipient organism.

As the method wherein the composition comprising an mTOR inhibitor is used resembles the method of prolonging the survival of a recipient organism described above, the respective embodiments of the above described method of prolonging the survival of a recipient organism are also embodiments of the composition comprising an mTOR inhibitor for use in a method of prolonging the survival of a recipient organism, and vice versa. In particular, the donor organism, the recipient organism and the genetically modified heart/kidney/lung/liver are as described above for the respective embodiments of the method of prolonging the survival of a recipient organism. In another preferred embodiment, the method wherein the mTOR inhibitor is used prolongs the survival of the recipient organism to more than 40 days after transplantation, to more than 50 days after transplantation, to more than 60 days after transplantation, to more than 70 days after transplantation, to more than 80 days after transplantation, or to more than 90 days after transplantation. Preferably, the method wherein the mTOR inhibitor is used prolongs the survival of the recipient organism to more than 50 days after transplantation, more preferably to more than 70 days after transplantation, and most preferably to more than 90 days after transplantation.

In one embodiment, the composition comprising an mTOR inhibitor in accordance with the present invention comprises an mTOR inhibitor selected from dactolisib (BEZ235, NVP-BEZ235), rapamycin (sirolimus), everolimus (RAD001), AZD8055, temsirolimus (CCI-779, NSC 683864), SF2523, 3BDO, PI-103, KU-0063794 I, torkinib (PP242), ridaforolimus (deforolimus, MK-8669), sapanisertib (INK 128, MLN0128), voxtalisib (SAR245409, XL765) analogue, torin 1, omipalisib (GSK2126458, GSK458), OSI-027, PF-04691502, apitolisib (GDC-0980, RG7422), GSK1059615, gedatolisib (PF-05212384, PKI-587), WYE-354, vistusertib (AZD2014), torin 2, WYE-125132 (WYE-132), BGT226 (NVP-BGT226), palomid 529 (P529), PP121, WYE-687, WAY-600, ETP-46464, GDC-0349, XL388, zotarolimus (ABT-578), CZ415, MHY1485, voxtalisib (XL765, SAR245409), chrysophanic acid and CC-223, LY3023414. In a preferred embodiment, the mTOR inhibitor is temsirolimus. Temsirolimus is commercially available, e.g., as Torisel from Pfizer Pharma GmbH (Berlin, Germany). As will be clear to the skilled person, the composition comprising an mTOR inhibitor in accordance with the present invention may additionally comprise pharmaceutically acceptable carriers and/or excipients.

Preferably, the composition comprising an mTOR inhibitor in accordance with the present invention is administered to the recipient organism according to a dosing regimen. More preferably, said dosing regimen comprises the administration of the composition after transplantation.

In a preferred embodiment, the dosing regimen of administering the composition comprising an mTOR inhibitor in accordance with the present invention comprises several administrations of the composition, wherein the first administration occurs within 10 days after transplantation, preferably between 2 days and 9 days after transplantation, more preferably between 4 days and 7 days after transplantation. In a more preferred embodiment, the dosing regimen of administering the composition comprising an mTOR inhibitor comprises several administrations of the composition, wherein the first administration occurs within 10 days after transplantation, preferably between 2 days and 9 days after transplantation, more preferably between 4 days and 7 days after transplantation, and wherein one administration occurs on each subsequent day. As will be clear to the skilled person, the time point of the first administration of the mTOR inhibitor depends on the general condition of the recipient (e.g. whether there are pericardial/pleural effusions). Preferably, the dose of the composition comprising an mTOR inhibitor administration is adjusted to a trough level of 5-10 ng/ml rapamycin. In accordance with all other aspects of this embodiment, the preferred route of administration is intravenous (i.v.) short infusion. A particularly preferred dosing regimen of administering the composition comprising an mTOR inhibitor in accordance with the present invention is depicted in Figure 7.

In one embodiment of the invention, the composition comprising an mTOR inhibitor for use in a method of prolonging the survival of a recipient organism in accordance with the present invention is administered to a recipient organism that had received immunosuppressive induction therapy according to a dosing regimen, and/or or to a recipient organism that is receiving immunosuppressive maintenance therapy, anti-inflammatory therapy, antihypertensive agent(s), and/or additive therapy according to respective dosing regimens. Considering that the present inventors have found that administering one or more antihypertensive agent(s) to the recipient after transplantation, administering an mTOR inhibitor to the recipient after transplantation and decreasing the dosage of a glucocorticoid, which is administered to the recipient as part of an immunosuppressive maintenance therapy, early after transplantation prevents the overgrowth of the donor heart in the recipient organism, the composition comprising an mTOR inhibitor for use in a method of prolonging the survival of a recipient organism in accordance with the present invention is preferably administered to a recipient organism that is receiving immunosuppressive maintenance therapy and antihypertensive agent(s) according to respective dosing regimens. The respective dosing regimens of any of the above treatments and their (preferred) embodiments are as described above for the method of prolonging the survival of a recipient organism. As will be clear to the skilled person, if the recipient organism is receiving one of the above treatments according to a respective dosing regimen, the first administration comprised in said dosing regimen has already occurred. Preferably, if the recipient organism is receiving immunosuppressive maintenance therapy according to a dosing regimen, and if said dosing regimen comprises decreasing the dosage (mg/kg/day) of a glucocorticoid by a specific factor within a certain period of time, such decrease has already occurred.

In another embodiment of the invention, the composition comprising an mTOR inhibitor for use in a method of prolonging the survival of a recipient organism is administered to a recipient organism whose genetically modified heart/kidney/lung/liver from the xenogeneic donor organism had been preserved by non-ischemic preservation, preferably by non-ischemic heart/kidney/lung/liver perfusion, before transplantation, e.g. as described above for the method of prolonging the survival of a recipient organism.

*Antihypertensive agent composition for use in a method of prolonging the survival of a primate following heart/kidney/lung/liver transplantation*

Additionally, the present invention relates to a composition comprising one or more antihypertensive agent(s) for use in a method of prolonging the survival of a recipient organism that has been transplanted a genetically modified heart/kidney/lung/liver from a xenogeneic donor organism, wherein the donor organism is a mammal and wherein the recipient organism is a primate. Preferably, said recipient organism has been transplanted the genetically modified heart/kidney/lung/liver so that it functionally substitutes the heart/kidney/lung/liver of the recipient organism.

As the method wherein the composition comprising one or more antihypertensive agent(s) is used resembles the method of prolonging the survival of a recipient organism described above, the respective embodiments of the above described method of prolonging the survival of a recipient organism are also embodiments of the composition comprising one or more antihypertensive agent(s) for use in a method of prolonging the survival of a recipient organism. In particular, the donor organism, the recipient organism and the genetically modified heart/kidney/lung/liver are as described above for the respective embodiments of the method of prolonging the survival of a recipient organism. In another preferred embodiment, the method wherein the one or more antihypertensive agent(s) is/are used prolongs the survival of the recipient organism to more than 40 days after transplantation, to more than 50 days after transplantation, to more than 60 days after transplantation, to more than 70 days after transplantation, to more than 80 days after transplantation, or to more than 90 days after transplantation. Preferably, the method wherein the one or more antihypertensive agent(s) is/are used prolongs the survival of the recipient organism to more than 50 days after transplantation, more preferably to more than 70 days after transplantation, and most preferably to more than 90 days after transplantation.

In one embodiment, the composition comprising one or more antihypertensive agent(s) in accordance with the present invention comprises one or more antihypertensive agent(s) selected from diuretics such as the loop diuretics furosemide and ethacrynic acid, the thiazide/thiazide-like diuretics hydrochlorothiazide and indapamide, and the potassium-sparing diuretics triamterene and amiloride; calcium channel blockers such as the dihydropyridines amlodipine and nifedipine, and the non-dihydropyridines verapamil and diltiazem; angiotensin-converting enzyme (ACE) inhibitors such as enalapril and ramipril; angiotensin II receptor antagonists such as valsartan and candesartan; adrenergic receptor antagonists such as the beta blockers bisoprolol and metoprolol, the alpha blockers doxazosin and phenoxybenzamine, and the mixed alpha/beta blockers carvedilol and labetalol; vasodilators such as sodium nitroprusside and hydralazine; renin inhibitors such as aliskiren; aldosterone receptor antagonists such as spironolactone and eplerenone; alpha-2 adrenergic receptor agonists such as clonidine and methyldopa; and endothelin receptor blockers such as bosentan. In a preferred embodiment, the composition comprising one or more antihypertensive agent(s) comprises one or more antihypertensive agent(s) selected from angiotensin-converting enzyme (ACE) inhibitors and adrenergic receptor antagonists.

In a preferred embodiment of the invention, the composition comprising one or more antihypertensive agent(s) in accordance with the present invention comprises an angiotensin-converting enzyme (ACE) inhibitor, preferably enalapril. Enalapril is commercially available, e.g., as Enahexal from Hexal AG (Holzkirchen, Germany). In another preferred embodiment of the invention, the composition comprising one or more antihypertensive agent(s) in accordance with the present invention comprises an adrenergic receptor antagonist, preferably metoprolol tartrate. Metoprolol tartrate is commercially available, e.g., as Beloc i.v. from AstraZeneca GmbH (Wedel, Germany). In another preferred embodiment, the composition comprising one or more antihypertensive agent(s) in accordance with the present invention comprises an angiotensin-converting enzyme (ACE) inhibitor, preferably enalapril, and an adrenergic receptor antagonist, preferably metoprolol tartrate.

As will be clear to the skilled person, the composition comprising one or more antihypertensive agent(s) in accordance with the present invention may additionally comprise pharmaceutically acceptable carriers and/or excipients.

Preferably, the composition comprising one or more antihypertensive agent(s) in accordance with the present invention is administered to the recipient organism according to a dosing regimen. More preferably, said dosing regimen comprises the administration of the composition after transplantation.

In a preferred embodiment, the dosing regimen of administering the composition comprising the one or more antihypertensive agent(s) comprises several administrations of the composition, wherein one administration occurs within 2 days after transplantation, preferably within 1 day after transplantation. In a more preferred embodiment, the dosing regimen of administering the composition comprising one or more antihypertensive agent(s) comprises several administrations of the composition, wherein one administration occurs within 2 days after transplantation, preferably within 1 day after transplantation, and wherein one administration occurs on each subsequent day. In accordance with all other aspects of this embodiment, the preferred route of administering the composition comprising one or more antihypertensive agent(s) is intravenous (i.v.) administration.

Preferably, the dose of the composition comprising one or more antihypertensive agent(s) is adjusted so that the mean arterial pressure of the recipient organism is kept within the range of 70 to 90 mmHg, preferably within the range of 75 to 85 mmHg. Even more preferably, the dose is adjusted so that the mean arterial pressure of the recipient organism is kept within the range of 70 to 90 mmHg, preferably within the range of 75 to 85 mmHg, and so that the heart rate is kept within the range of 80 to 120 bpm, preferably within the range of 90 to 110 bpm. A particularly preferred dosing regimen of administering the composition comprising one or more antihypertensive agent(s) in accordance with the present invention is depicted in Figure 9.

In one embodiment of the invention, the composition comprising one or more antihypertensive agent(s) for use in a method of prolonging the survival of a recipient organism is administered to a recipient organism that had received immunosuppressive induction therapy according to a dosing regimen, and/or or to a recipient organism that is receiving immunosuppressive maintenance therapy, anti-inflammatory therapy, an mTOR inhibitor and/or and/or additive therapy according to respective dosing regimens. Considering that the present inventors have found that administering one or more antihypertensive agent(s) to the recipient after transplantation, administering an mTOR inhibitor to the recipient after transplantation and decreasing the dosage of a glucocorticoid, which is administered to the recipient as part of an immunosuppressive maintenance therapy, early after transplantation prevents the overgrowth of the donor heart in the recipient organism, the composition comprising one or more antihypertensive agent(s) for use in a method of prolonging the survival of a recipient organism in accordance with the present invention is preferably administered to a recipient organism that is receiving immunosuppressive maintenance therapy and an mTOR inhibitor according to respective dosing regimens. The respective dosing regimens of any of the above treatments and their (preferred) embodiments are as described above for the method of prolonging the survival of a recipient organism. As will be clear to the skilled person, if the recipient organism is receiving one of the above treatments according to the respective dosing regimen, the first administration comprised in said dosing regimen has already occurred. Preferably, if the recipient organism is receiving immunosuppressive maintenance therapy according to a dosing regimen, and if said dosing regimen comprises decreasing the dosage (mg/kg/day) of a glucocorticoid by a specific factor within a certain period of time, such decrease has already occurred.

In another embodiment of the invention, the composition comprising one or more antihypertensive agent(s) for use in a method of prolonging the survival of a recipient organism is administered to a recipient organism whose genetically modified heart/kidney/lung/liver from the xenogeneic donor organism had been preserved by non-ischemic preservation, preferably by non-ischemic heart/kidney/lung/liver perfusion, before transplantation, e.g. as described above for the method of prolonging the survival of a recipient organism.

*Method of preventing or treating heart/kidney/lung/liver failure, primate obtained by such method and compositions for use in such method*

The present invention also relates to a method of preventing or treating heart/kidney/lung/liver failure in a recipient organism that is transplanted a genetically modified heart/kidney/lung/liver from a xenogeneic donor organism, wherein said method comprises all of the steps and preferred embodiments described above for the method of prolonging the survival of a recipient organism that is transplanted a genetically modified heart/kidney/lung/liver from a xenogeneic donor organism.

The present invention also relates to a living primate whose heart/kidney/lung/liver is functionally substituted by a transplanted, genetically modified heart/kidney/lung/liver from a xenogeneic mammal, wherein said living primate is obtainable by the above described method of preventing or treating heart/kidney/lung/liver failure in a recipient organism that is transplanted a genetically modified heart/kidney/lung/liver from a xenogeneic donor organism. Said living primate and its preferred embodiments are as described above for the living primate obtainable by the method of prolonging the survival of a recipient organism that is transplanted a genetically modified heart/kidney/lung/liver from a xenogeneic donor organism.

The present invention also relates to a composition comprising an mTOR inhibitor for use in a method of preventing or treating heart/kidney/lung/liver failure in a recipient organism that has been transplanted a genetically modified heart/kidney/lung/liver from a xenogeneic donor organism, wherein the donor organism is a mammal and wherein the recipient organism is a primate. Said composition comprising an mTOR inhibitor and its preferred embodiments are as described above for the composition comprising an mTOR inhibitor for use in a method of prolonging the survival of a recipient organism that has been transplanted a genetically modified heart/kidney/lung/liver from a xenogeneic donor organism.

The present invention also relates to a composition comprising one or more antihypertensive agent(s) for use in a method of preventing or treating heart/kidney/lung/liver failure in a recipient organism that has been transplanted a genetically modified heart/kidney/lung/liver from a xenogeneic donor organism, wherein the donor organism is a mammal and wherein the recipient organism is a primate. Said composition comprising one or more antihypertensive agent(s) and its preferred embodiments are as described above for the composition comprising one or more antihypertensive agent(s) for use in a method of prolonging the survival of a recipient organism that has been transplanted a genetically modified heart/kidney/lung/liver from a xenogeneic donor organism.

*Method of treating a recipient organism that is transplanted a genetically modified heart/kidney/lung/liver from a xenogeneic donor organism, primate obtained by such method and compositions for use in such method*

The present invention also relates to a method of treating a recipient organism that is transplanted a genetically modified heart/kidney/lung/liver from a xenogeneic donor organism, wherein said method comprises all of the steps and preferred embodiments described above for the method of prolonging the survival of a recipient organism that is transplanted a genetically modified heart/kidney/lung/liver from a xenogeneic donor organism.

The present invention also relates to a living primate whose heart/kidney/lung/liver is functionally substituted by a transplanted, genetically modified heart/kidney/lung/liver from a xenogeneic mammal, wherein said living primate is obtainable by the above described method of treating a recipient organism that is transplanted a genetically modified heart/kidney/lung/liver from a xenogeneic donor organism. Said living primate and its preferred embodiments are as described above for the living primate obtainable by the method of prolonging the survival of a recipient organism that is transplanted a genetically modified heart/kidney/lung/liver from a xenogeneic donor organism.

The present invention also relates to a composition comprising an mTOR inhibitor for use in a method of treating a recipient organism that has been transplanted a genetically modified heart/kidney/lung/liver from a xenogeneic donor organism, wherein the donor organism is a mammal and wherein the recipient organism is a primate. Said composition comprising an mTOR inhibitor and its preferred embodiments are as described above for the composition comprising an mTOR inhibitor for use in a method of prolonging the survival of a recipient organism that has been transplanted a genetically modified heart/kidney/lung/liver from a xenogeneic donor organism.

The present invention also relates to a composition comprising one or more antihypertensive agent(s) for use in a method of treating a recipient organism that has been transplanted a genetically modified heart/kidney/lung/liver from a xenogeneic donor organism, wherein the donor organism is a mammal and wherein the recipient organism is a primate. Said composition comprising one or more antihypertensive agent(s) and its preferred embodiments are as described above for the composition comprising one or more antihypertensive agent(s) for use in a method of prolonging the survival of a recipient organism that has been transplanted a genetically modified heart/kidney/lung/liver from a xenogeneic donor organism.

*Method of preventing heart/kidney/lung/liver overgrowth in a recipient organism that is transplanted a genetically modified heart/kidney/lung/liver from a xenogeneic donor organism, primate obtained by such method and compositions for use in such method*

Overgrowth of the transplanted heart/kidney/lung/liver in the recipient organism is a major problem when a genetically modified heart/kidney/lung/liver from a xenogeneic donor organism is transplanted into a recipient organism. Overgrowth refers to any increase in organ or tissue mass or volume that is detrimental for organ or tissue function, in particular due to exogenous factors (e.g. compression due to space limitation) or due to endogenous factors (e.g. hypoxia due to insufficient microvascular circulation). In one embodiment, heart overgrowth means that the transplanted heart triples to quadruples in volume and weight within approximately one month after transplantation. The surrounding anatomical structures, the recipient's chest finally, limit this expansion. As a result, the heart function is severely restricted; the blood flow is limited, obstructed, backlogged, congested. Since the liver is the first organ hemodynamically below (behind) the heart, liver function is impaired first. The recipient succumbs to terminal heart and liver dysfunction. Reason for the overgrowth is that a mammalian heart (e.g., a porcine heart) grows rapidly in the donor (means, proportionally to the rapidly growing whole body, which may be at the time of the transplantation approximately 20 kg). The Boston Harvard group saw this phenomenon also after kidney and lung transplantation and called it "intrinsic growth" (the heart must grow rapidly at that time of a body development; it does not stop growing even if transplanted in a primate's body; cf. reference 15).

The present inventors have found that in particular administering one or more antihypertensive agent(s) to the recipient after transplantation, administering an mTOR inhibitor to the recipient after transplantation and decreasing rapidly the dosage of a glucocorticoid, which is administered to the recipient as part of an immunosuppressive maintenance therapy, early after transplantation prevents the overgrowth of the donor heart in the recipient organism (see, e.g. Figures 5 and 6). Thus, the present invention also relates to a method of preventing heart/kidney/lung/liver overgrowth in a recipient organism that is transplanted a genetically modified heart/kidney/lung/liver from a xenogeneic donor organism, wherein said method comprises all of the steps and preferred embodiments described above for the method of prolonging the survival of a recipient organism that is transplanted a genetically modified heart/kidney/lung/liver from a xenogeneic donor organism.

The present invention also relates to a living primate whose heart/kidney/lung/liver is functionally substituted by a transplanted, genetically modified heart/kidney/lung/liver from a xenogeneic mammal, wherein said living primate is obtainable by the above described method of preventing heart/kidney/lung/liver overgrowth in a recipient organism that is transplanted a genetically modified heart/kidney/lung/liver from a xenogeneic donor organism. Said living primate and its preferred embodiments are as described above for the living primate obtainable by the method of prolonging the survival of a recipient organism that has is transplanted a genetically modified heart/kidney/lung/liver from a xenogeneic donor organism.

The present invention also relates to a composition comprising an mTOR inhibitor for use in a method of preventing heart/kidney/lung/liver overgrowth in a recipient organism that has been transplanted a genetically modified heart/kidney/lung/liver from a xenogeneic donor organism, wherein the donor organism is a mammal and wherein the recipient organism is a primate. Said composition comprising an mTOR inhibitor and its preferred embodiments are as described above for the composition comprising an mTOR inhibitor for use in a method of prolonging the survival of a recipient organism that has been transplanted a genetically modified heart/kidney/lung/liver from a xenogeneic donor organism.

The present invention also relates to a composition comprising one or more antihypertensive agent(s) for use in a method of preventing heart/kidney/lung/liver overgrowth in a recipient organism that has been transplanted a genetically modified heart/kidney/lung/liver from a xenogeneic donor organism, wherein the donor organism is a mammal and wherein the recipient organism is a primate. Said composition comprising one or more antihypertensive agent(s) and its preferred embodiments are as described above for the composition comprising one or more antihypertensive agent(s) for use in a method of prolonging the survival of a recipient organism that has been transplanted a genetically modified heart/kidney/lung/liver from a xenogeneic donor organism.

*Applicability of the present invention to xenogeneic transplantation of other organs*

As described above, the Boston Harvard group saw the phenomenon of donor organ overgrowth in the recipient also after kidney and lung transplantation (cf. reference 15). The recipients of such transplantations would suffer from the respective terminal diseases. Thus, the methods described in the present invention (in particular administering one or more antihypertensive agent(s) to the recipient after transplantation, administering an mTOR inhibitor to the recipient after transplantation and decreasing the dosage of a glucocorticoid, which is administered to the recipient as part of an immunosuppressive maintenance therapy, early after transplantation) are useful for any xenogeneic organ transplantation, including kidney transplantation, lung transplantation and liver transplantation. For example, the presented techniques should also work in rhesus monkeys after preclinical kidney, lung (and also liver) transplantations (cf. reference 15).

*Genetically modified mammal and method of producing same*

Major progress in genetic engineering and genome editing of livestock species has extended their use to biomedical applications the most notable being: tailored large animal models for translational medicine; porcine cells, tissues and organs for xenotransplantation; and production of pharmaceutical proteins in transgenic large animals. The translation of novel discoveries from basic research to clinical application is a long, often inefficient and costly process. Appropriate animal models are critical for the success of translational research. Although rodent models are widely used, they often do not accurately represent the human disease. Thus, additional animal models that more closely mimic aspects of human anatomy and physiology are required. Several genetically engineered pig models have been generated, many of which represent human disease mechanisms and phenotypes more closely than existing rodent models. In addition, pigs are the most promising donor species for xenotransplantation. Since multiple genetic modifications are required to prevent immune rejection, overcome physiological incompatibilities of xeno-organs, and to eliminate potential risk factors such as porcine endogenous retroviruses (PERV), genome editing is speeding progress in this field. Last but not least, genetic engineering of large animal species as bioreactors for the production of pharmaceutical proteins is still an interesting option, though only a few such products are on the market. In summary, genetically engineered large animals are playing an increasingly important role in biomedicine. For all areas of application, consistent and reliable transgene expression is critical for the value of genetically engineered pigs. The present invention provides a genetically modified mammal with consistent and reliable transgene expression.

In the method of prolonging the survival of a recipient organism described herein, said recipient organism is transplanted a genetically modified heart, kidney, lung or liver from a xenogeneic donor organism. Similarly, the above-described living primate has been transplanted a genetically modified heart, kidney, lung or liver, from a xenogeneic mammal, and the above-described compositions comprising an mTOR inhibitor/antihypertensive agent(s) are for use in a method of prolonging the survival of a recipient organism that has been transplanted a genetically modified heart, kidney, lung or liver from a xenogeneic donor organism. The genetically modified mammal with consistent and reliable transgene expression provided herein can, in one embodiment, serve as xenogeneic donor organism for such genetically modified heart, kidney, lung or liver.

The expression of transgenes randomly inserted into the genome is largely dependent on the context of neighboring chromatin. These so-called position effects result in variable transgene expression between different transgenic lines and often in inconsistent expression within one line. A strategy avoiding these disadvantages of random transgene insertion is the targeted placement of expression cassettes into so-called "safe harbor" loci that have an open configuration and ensure stable transgene expression. The most prominent example is the Rosa26 locus in mice that has also been discovered and used in pig (cf. reference 27). The present invention provides the GGTA1 locus as an advantageous safe harbor for reliable transgene expression. This provides, *inter alia*, the following advantages:

i) GGTA1 is highly expressed in multiple tissues and cell types, most importantly in endothelial cells: in pig endothelial wild-type cells the transcriptional level of CMAH is similar to that of B4GALNT2, whereas the transcriptional level of GGTA1 is approximately 5-fold higher (cf. also Example 6);

ii) GGTA1 is dispensable for normal development as demonstrated by humans and Old World monkeys who lack GGTA1 and multiple lines of GGTA1 knockout pigs;

iii) mutation of GGTA1 using targeted nucleases is highly efficient, even after injection into oocytes (cf. reference 28);

iv) for the generation of donor pigs for xenotransplantation, it is preferable to inactivate GGTA1 anyway to avoid hyperacute rejection of pig-to-primate xenografts; and

v) targeted integration of xenoprotective transgenes into the GGTA1 locus prevents their segregation during breeding.

In view of the above, the present invention provides a genetically modified mammal wherein transgenes are inserted at the alpha-1,3-galactosyltransferase gene (GGTA1) locus, and wherein at least one of the transgenes is expressed under the control of the endogenous promoter of the alpha-1,3-galactosyltransferase gene.

More than 40 different genetic modifications have been introduced into pigs to prevent immune rejection of xenografts, overcome physiological incompatibilities, and reduce risk of transmitting zoonotic pathogens (reviewed in reference 29). The challenge of combining multiple transgenes to enable practical animal breeding, avoiding segregation of independent integration sites, may be overcome by "combineering" and gene stacking, i.e. random or targeted placement of multiple expression cassettes at a single genomic locus (cf. references 23, 30). Accordingly, the present invention provides a genetically modified mammal wherein several transgenes are inserted at the same locus, i.e. at the alpha-1,3-galactosyltransferase gene (GGTA1) locus.

Insertion of transgenes at the GGTA1 locus disrupts the GGTA1 gene and therefore abolishes expression of alpha-1,3-galactosyltransferase. Disruption of GGTA1 is preferable in the genetically modified heart, kidney, lung or liver transplanted in accordance with the present invention, because it helps to avoid hyperacute rejection of the xenografts. Hyperacute rejection of pig-to-primate xenografts is triggered by binding of preformed antibodies to specific xeno-antigens. Subsequent activation of the complement system cannot be controlled due to species incompatibilities between the regulators on the xenograft and the recipient's effector molecules (reviewed in reference 31). The major xeno-antigen is galactose-$\alpha$1,3-galactose ($\alpha$Gal) synthesised by N-acetyllactosaminide alpha-1,3-galactosyltransferase (GGTA1). This enzyme is defective in humans and old-world monkeys, and $\alpha$Gal is consequently absent. Immunogenic contact with bacterial $\alpha$Gal epitopes in the intestinal tract cause humans and old-world monkeys to raise anti-$\alpha$Gal antibodies in early life.

The transgenes inserted into the GGTA1 locus are a human complement-regulatory protein transgene (i.e., a sequence encoding a human complement-regulatory protein) and a human thrombomodulin transgene (i.e., a sequence encoding human thrombomodulin). Expression of human complement-regulatory proteins in transgenic donor mammals (e.g., pigs) is desirable to overcome hyperacute rejection of mammal (e.g., pig)-to-primate xenografts. Human complement-regulatory proteins attenuate complement activation and significantly prolong survival of mammal (e.g., pig)-to-primate xenografts (reviewed in reference 29). Exemplary human complement-regulatory proteins in accordance with the present invention are CD46 (membrane cofactor protein; MCP), CD55 (complement decay-accelerating factor, DAF), and CD59 (membrane inhibitor of reactive lysis, MIRL). A particularly preferred human complement-regulatory protein in accordance with the present invention is CD46. However, the genetically modified mammal in accordance with the present invention may also have all of CD46, CD55 and CD59 inserted into the GGTA1 locus.

Dysregulation of coagulation and disordered haemostasis are frequent complications in preclinical mammal (e.g., pig)-to-nonhuman primate xenotransplantation (reviewed in reference 32). Manifestations range from acute life-threatening consumptive coagulopathy with thrombocytopenia and bleeding, to thrombotic microangiopathy leading to loss of the xenograft. Proposed causes include inflammation, vascular injury, innate, humoral and cellular immune responses, and in particular molecular incompatibilities between mammalian (e.g., porcine) and primate regulators of coagulation (cf. reference 33). Human thrombomodulin (hTM) is a key endothelial anticoagulant/antithrombotic protein that can be supplemented by genetic engineering of donor mammals (e.g., pigs). Porcine TM binds human thrombin, but is a poor co-factor for activation of human protein C (cf. reference 34). To overcome this problem, lines of transgenic mammals (e.g., pigs) can be generated that express human TM under various promoters: CMV (cf. reference 35), CAGGS (cf. reference 36), human ICAM (cf. reference 5), and porcine THBD (cf. reference 37). A GGTA1 knockout, hCD46 transgenic pig heart with the latter hTM expression vector survived for more than 900 days after heterotopic abdominal transplantation into a baboon with appropriate immunosuppression (induction with anti-thymocyte globulin and anti-CD20 antibody, maintenance with mycophenolate mofetil and intensively dosed anti-CD40 (2C10R4) antibody) (cf. reference 5).

The genetically modified mammal in accordance with the present invention may have additional genetic modifications. In one embodiment, the genetically modified mammal in accordance with the present invention additionally has homozygous knock-outs of the CMAH and the B4GALNT2 genes. Cytidine monophosphate-N-acetylneuraminic acid hydroxylase (CMAH) synthesises N-acetylneuraminic acid (Neu5Gc, also called Flanganutziu-Deicher antigen), and porcine $\beta$-1,4-N-acetyl-galactosaminyl transferase 2 (B4GALNT2) makes an Sd(a)-like glycan. Both are other prominent xeno-antigens (reviewed in reference 38). Cells from GGTA1/CMAH/B4GALNT2 deficient pigs exhibit reduced human IgM and IgG binding compared to cells lacking only GGTA1 and CMAH.

In another embodiment, the genetically modified mammal in accordance with the present invention additionally has insertions of a sequence encoding hemeoxygenase 1 (preferably human hemeoxygenase 1) and of a sequence encoding A20 (preferably human A20). Preferably, these insertions are also at the GGTA1 locus.

In another embodiment, the genetically modified mammal in accordance with the present invention additionally has insertions of a sequence encoding LEA29Y or PD-L1 (preferably human PD-L1). Preferably, these insertions are also at the GGTA1 locus. In another embodiment, the genetically modified mammal in accordance with the present invention additionally has an insertion of a sequence encoding human $\alpha$1,2-fucosyltransferase. Preferably, this insertion is also at the GGTA1 locus.

In the genetically modified mammal in accordance with the present invention, the sequence encoding a human complement-regulatory protein and the sequence encoding human thrombomodulin are inserted at the alpha-1,3-galactosyltransferase gene locus, such that at least the sequence encoding a human complement-regulatory protein is expressed under the control of the endogenous promoter of the alpha-1,3-galactosyltransferase gene. As mentioned above, GGTA1 is highly expressed in multiple tissues and cell types, most importantly in endothelial cells. Hence, inserting the sequence encoding a human complement-regulatory protein into the GGTA1 locus such that it is expressed under the control of the endogenous promoter of the alpha-1,3-galactosyltransferase gene ensures that the human complement-regulatory protein is highly expressed.

In another embodiment of the genetically modified mammal in accordance with the present invention, both the sequence encoding a human complement-regulatory protein and the sequence encoding human thrombomodulin are inserted such that they are expressed under the control of the endogenous promoter of the alpha-1,3-galactosyltransferase gene, so that both genes are highly expressed in multiple tissues and cell types. Expression of the human complement-regulatory protein and human thrombomodulin in multiple tissues and cell types ensures that the genetically modified mammal in accordance with the present invention can serve as xenogeneic donor organism for various organs, i.e. for each of a genetically modified heart, kidney, lung or liver.

In an alternative embodiment of the genetically modified mammal in accordance with the present invention, the sequence encoding a human complement-regulatory protein is expressed under the control of the endogenous promoter of the alpha-1,3-galactosyltransferase gene, and the sequence encoding human thrombomodulin is expressed under the control of a porcine THBD promoter or an exogenous promoter. For example, the exogenous promoter may be a CMV promoter, a CAGGS promoter, or a human ICAM promoter. Preferably, such exogenous promoter is a ubiquitously active promoter, i.e. a promoter that is expressed in multiple tissues and cell types. Again, expression of the sequence encoding human thrombomodulin in multiple tissues and cell types ensures that the genetically modified mammal in accordance with the present invention can serve as xenogeneic donor organism for various organs.

In another embodiment of the genetically modified mammal in accordance with the present invention, the genetically modified mammal additionally has an insertion of a selectable marker gene sequence between the sequence encoding a human complement-regulatory protein and the sequence encoding human thrombomodulin. Preferably, this selectable marker gene sequence is flanked by loxP sites. This ensures that cells from the genetically modified mammal can be isolated and used for targeted integration of additional expression vectors. Additional expression vectors can be inserted via Cre-mediated cassette exchange with an engineered bacterial artificial chromosome (BAC) that contains one or several additional transgenes (cf. Figure 18).

In another embodiment of the genetically modified mammal in accordance with the present invention, the genetically modified mammal belongs to the family *Suidae,* preferably to the genus *Sus*, more preferably to the species *S. scrofa,* and most preferably to the subspecies *S. s. domesticus.* In a preferred embodiment of the genetically modified mammal in accordance with the present invention, the genetically modified mammal belongs to the species *S. scrofa.*

In another embodiment of the genetically modified mammal in accordance with the present invention, the genetically modified mammal is a pig of German Landrace/Large White cross-bred genetic background, a Duroc pig, a Schwäbisch-Hällische (Swabian-Hall) pig, a black mini pig, or an Auckland Island pig; preferably the genetically modified mammal is a pig of German Landrace/Large White cross-bred genetic background.

In another embodiment of the genetically modified mammal in accordance with the present invention, the genetically modified mammal (e.g., pig) is free of porcine endogenous retrovirus C (PERV-C). Porcine endogenous retroviruses (PERVs) cannot easily be eliminated, since they are integrated into the porcine genome, and thus pose the most refractory source of risk (reviewed in reference 39). PERV-A and PERV-B are able to infect some human tumour cells and to a lesser extent human primary cells. PERV-C infects only pig cells, but recombinants with PERV-A can infect human cells and are characterised by a high replication rate. Since PERV-C is not present in all pigs, it is possible to identify and select PERV-C-free animals as potential donors. This is preferred in particular when the genetically modified mammal (e.g., pig) in accordance with the present invention serves as xenogeneic donor organism for organs to be transplanted into primates.

The present invention also provides a method of making a genetically modified mammal that can serve as xenogeneic donor organism for organs transplanted into primates. This method comprises inserting a sequence encoding a human complement-regulatory protein and a sequence encoding human thrombomodulin at the alpha-1,3-galactosyltransferase gene locus of a mammalian genome to provide a genetically modified genome; introducing the genetically modified genome into a cell; and permitting the cell comprising the genetically modified genome to mature into a genetically modified mammal. Preferably, the genome is a somatic cell genome and the genetically modified genome is introduced into the cell by somatic cell nuclear transfer (for details on how to obtain such genetically modified mammal see, e.g., reference 92, which is incorporated herein its entirety).

In one embodiment of the method of making a genetically modified mammal in accordance with the present invention, the sequence encoding a human complement-regulatory protein and the sequence encoding human thrombomodulin are located on a single expression vector that is inserted into the alpha-1,3-galactosyltransferase gene locus. Preferably, the expression vector that is inserted into the alpha-1,3-galactosyltransferase gene locus additionally comprises a selectable marker gene sequence between the sequence encoding a human complement-regulatory protein and the sequence encoding human thrombomodulin. As will be known to the skilled person, this marker gene sequence allows for selection of a cell comprising the genetically modified genome.

In another embodiment of the method of making a genetically modified mammal in accordance with the present invention, the method further comprises inserting a sequence encoding human α1,2-fucosyltransferase, and/or introducing one or more additional genetic modification(s) into the mammalian genome. When these genetic modifications are insertions, it is preferred that the sequences are inserted at the GGTA1 locus. Even more preferably, the sequences to be inserted are located on a single expression vector together with the sequence encoding a human complement-regulatory protein and the sequence encoding human thrombomodulin that is inserted into the alpha-1,3-galactosyltransferase gene locus in accordance with the present invention.

In a preferred embodiment of the method of making a genetically modified mammal in accordance with the present invention, the genetic modifications are introduced using CRISPR/Cas9. CRISPR/Cas9 allows for targeted insertion and/or deletion of genes within a genome. Alternatively, the genetic modifications may be introduced using zinc finger nucleases (ZFN) or transcription activator-like effector nuclease (TALEN). The use of these techniques is described in reference 107, which is incorporated herein in its entirety.

The embodiments described above for the genetically modified mammal in accordance with the present invention also apply to the method of making a genetically modified mammal in accordance with the present invention, *mutatis mutandis*. In particular, in a preferred embodiment of the method of making a genetically modified mammal in accordance with the present invention, the mammal belongs to the species *S. scrofa*.

Both for the genetically modified mammal in accordance with the present invention and for the method of making a genetically modified mammal in accordance with the present invention, details relating to the above-mentioned sequences that are inserted into the genetically modified mammal are found in the section relating to the method of prolonging the survival of a primate following heart/kidney/lung/liver transplantation in accordance with the present invention, and in the section relating to the primate with a transplanted heart/kidney/lung/liver in accordance with the present invention. In particular, the transcripts that are preferably expressed when inserting the genes which are described in those sections are also the transcripts that are preferably expressed when inserting the corresponding sequences into the genetically modified mammal in accordance with the present invention.

In one embodiment of the genetically modified mammal in accordance with the present invention, the genetically modified mammal has one or more additional genetic modification(s). Similarly, in one embodiment of the method of making a genetically modified mammal in accordance with the present invention, the method further comprises introducing one or more additional genetic modification(s). When the genetically modified mammal is supposed to serve as xenogeneic donor organism for genetically modified organs for xeno-transplantation, these one or more additional genetic modification(s) is/are preferably selected from (1) genetic modifications to overcome hyperacute and acute vascular rejection of mammal (e.g., pig)-to-primate xenografts; (2) genetic modifications to overcome cellular rejection of mammal (e.g., pig)-to-primate xenografts; (3) genetic modifications to overcome dysregulation of coagulation and inflammation; and/or (4) genetic modifications to decrease the risk for zoonoses. In a preferred embodiment, when these genetic modifications represent genetic insertions, these are at the GGTA1 locus. The above groups of potential genetic modifications in accordance with the present invention are described in the following, in particular with regard to pig-to-primate xeno-transplantations. Any of these genetic modifications could be the one or more additional genetic modification(s) in accordance with the present invention:

(1) Genetic modifications to overcome hyperacute and acute vascular rejection of pig-to-primate xenografts.

Hyperacute rejection of pig-to primate xenografts is triggered by binding of preformed antibodies to specific xeno-antigens. Subsequent activation of the complement system cannot be controlled due to species incompatibilities between the regulators on the xenograft and the recipient's effector molecules (reviewed in reference 31). The major xeno-antigen is galactose-α1,3-galactose (αGal) synthesised by N-acetyllactosaminide alpha-1,3-galactosyltransferase (GGTA1). This enzyme is defective in humans and old-world monkeys, and αGal is consequently absent. Immunogenic contact with bacterial aGal epitopes in the intestinal tract cause humans and old-world monkeys to raise anti-aGal antibodies in early life. Other prominent xeno-antigens are N-acetylneuraminic acid (Neu5Gc, also called Flanganutziu-Deicher antigen) synthesised by cytidine monophosphate-N-acetylneuraminic acid hydroxylase (CMAH) and an Sd(a)-like glycan made by porcine β-1,4-N-acetyl-galactosaminyl transferase 2 (B4GALNT2) (reviewed in reference 38). A first step to overcome hyperacute rejection of pig-to-primate xenografts was the generation of transgenic donor pigs that express human complement-regulatory proteins, such as CD46 (membrane cofactor protein; MCP), CD55 (complement decay-accelerating factor, DAF), CD59 (membrane inhibitor of reactive lysis, MIRL), singly or in combination. A CD46 transgenic pig is characterized in reference 113. These transgenic donor pigs attenuate complement activation and significantly prolong survival of pig-to-primate xenografts (reviewed in reference 29). A major step towards long-term survival of vascularised pig-to-primate xenografts was the inactivation of the porcine GGTA1 gene. Following the first publication of GGTA1 (and consequently aGal) deficient pigs (cf. reference 40), multiple GGTA1 knock-out pig lines were generated, initially by gene targeting (reviewed in reference 41) and later by gene editing (e.g. reference 42). To remove the Neu5Gc xeno-antigen, pigs lacking CMAH were generated (cf. reference 43) and have been combined with GGTA1 deficiency (cf. references 44-46). The porcine B4GALNT2 gene was also inactivated to eliminate an Sd(a)-like xeno-antigen (cf. reference 47). The authors showed that cells from GGTA1/CMAH/B4GALNT2 deficient pigs exhibited reduced human IgM and IgG binding compared to cells lacking only GGTA1 and CMAH.

(2) Genetic modifications to overcome cellular rejection of pig-to-primate xenografts

Both innate and adaptive components of the cellular immune system contribute to xenograft rejection (reviewed in reference 48). Immune cell infiltration of tissue and solid organ xenografts starts with neutrophils, followed by macrophages and T cells (reviewed in reference 49). In addition, natural killer (NK) cells may induce endothelial cell activation in the xenograft (cf. reference 50) and lyse porcine cells directly and via antibody-dependent cytotoxicity (reviewed in reference 51).

Cellular xenografts such as porcine islets in non-human primates are mainly rejected by CD4+ T cells. Their activation can be induced by direct binding of primate T-cell receptors to SLA class 1 and class 2 molecules of porcine cells, or indirectly by antigen-presenting cells (APCs) of the recipient expressing MHCs with processed xeno-antigens (reviewed in reference 49). In addition to this T-cell receptor mediated signal, T-cell activation is regulated by a co-stimulatory signal, which may - depending on its nature - induce and amplify an effective immune response, or exhibit an inhibitory tolerogenic function. In the context of xenotransplantation, the best-studied T-cell co-stimulatory signalling complexes are CD80/CD86-CD28 and CD40-CD154, with CD28 and CD154 (=CD40L) being localised on T cells and CD80/CD86 and CD40 on APCs. The CD80/CD86-CD28 co-stimulation pathway can be blocked by systemic treatment with CTLA4-Ig (abatacept®) or LEA29Y (belatacept®), which have markedly improved the long-term outcome of allogeneic and xenogeneic tissue grafts (reviewed in reference 52). These molecules can also be expressed in genetically modified donor pigs, opening the prospect of inhibiting T-cell activation locally at the graft site, thus avoiding systemic immunosuppression of the recipient and the consequent risk of infection (commented in reference 53). LEA29Y expressing transgenic porcine neonatal islet cell clusters (NICCs) transplanted into immunodeficient diabetic mice normalised blood glucose levels and, in contrast to wild-type NICCs, were not rejected after the recipient mice were reconstituted with human immune cells (cf. reference 54). A subsequent study using diabetic mice with a long-term "humanised" immune system as recipients showed that xenografted porcine islets that expressed LEA29Y survived for several months and normalised the recipients' blood glucose levels, whereas wild-type islets did not engraft in this model (cf. reference 55).

Transgenic pigs expressing human TNF related apoptosis inducing ligand (TRAIL) (cf. references 56, 57), human FAS ligand (FASL) (cf. reference 58), and HLA-E/beta2-microglobulin have also been generated to prevent cellular rejection. Cells from the HLA-E/beta2-microglobulin pigs were effectively protected against human NK-cell mediated cytotoxicity, depending on the level of CD94/NKG2A expression on the NK cells (cf. reference 51). To control macrophage activity, human CD47 has been expressed on porcine cells to activate the "don't eat me signal" receptor SIRP$\alpha$ on (human) monocytes/macrophages and to suppress phagocytic activity (reviewed in reference 29).

(3) Genetic modifications to overcome dysregulation of coagulation and inflammation

Dysregulation of coagulation and disordered hemostasis are frequent complications in preclinical pig-to-non-human primate xenotransplantation (reviewed in reference 32). Manifestations range from acute life-threatening consumptive coagulopathy with thrombocytopenia and bleeding, to thrombotic microangiopathy leading to loss of the xenograft. Proposed causes include inflammation, vascular injury, innate, humoral and cellular immune responses, and in particular molecular incompatibilities between porcine and primate regulators of coagulation (cf. reference 33).

Key endothelial anticoagulant/antithrombotic proteins that have been modified/supplemented by genetic engineering of donor pigs include human thrombomodulin (hTM), endothelial protein C receptor (EPCR), tissue factor pathway inhibitor (TFPI), and ectonucleoside triphosphate diphosphohydrolase 1 (ENTPD1 alias CD39) (reviewed in reference 33).

Porcine TM binds human thrombin, but is a poor co-factor for activation of human protein C (cf. reference 34). To overcome this problem, lines of transgenic pigs have been generated that express human TM under various promoters: CMV (cf. reference 35), CAGGS (cf. reference 36), human ICAM (cf. reference 5), and porcine THBD (cf. reference 37). A GGTA1 knockout, hCD46 transgenic pig heart with the latter hTM expression vector survived for more than 900 days after heterotopic abdominal transplantation into a baboon with appropriate immunosuppression (induction with anti-thymocyte globulin and anti-CD20 antibody, maintenance with mycophenolate mofetil and intensively dosed anti-CD40 (2C10R4) antibody) (cf. reference 5).

EPCR binds protein C and presents it to the THBD/thrombin complex for activation, enhancing the generation of activated protein C (cf. reference 59). Expression of human EPCR in porcine aortic endothelial cells reduced human platelet aggregation almost as efficiently as human THBD (cf. reference 60). The authors concluded that transgenic expression of human EPCR can enhance the effect of porcine THBD, but would have an even greater effect when co-expressed with human THBD. Porcine TFPI is a less efficient inhibitor of human TF/factor Vila than human TFPI (reviewed in reference 33). A recent study demonstrated that Kunitz domain 1 is critical for the species incompatibility between pig TFPI and human tissue factor (TF), and that clotting can be inhibited by human TFPI-transfected porcine bone marrow mesenchymal cells (cf. reference 61). Transgenic pigs expressing human TFPI have been generated to overcome this incompatibility (cf. references 62, 63).

CD39 rapidly hydrolyses ATP and ADP to AMP. AMP is hydrolysed by ecto-5'-nucleotidase (CD73) to adenosine, an anti-thrombotic and cardiovascular protective mediator. Transgenic pigs expressing human CD39 under the control of the murine H-2Kb promoter showed reduced infarct size after myocardial ischaemia/reperfusion injury (cf. reference 64). Subsequently, kidneys (cf. referenced 65, 66) and islets (cf. reference 67) of genetically multi-modified pigs (including expression of human CD39) were tested in xenotransplantation experiments in non-human primates. However specific effects of human CD39 could not be distinguished among the other genetic modifications.

Reduced expression of pro-coagulatory TF has been achieved by siRNA mediated knockdown (cf. reference 68). Aberrant phagocytosis of human platelets during perfusion of porcine livers could be partially overcome by deleting the porcine asialoglycoprotein receptor (ASGR) (cf. reference 69). In addition to modifications targeting coagulation disorders in xenotransplantation, transgenic pigs have been produced that express anti-apoptotic and anti-inflammatory proteins, such as human tumour necrosis factor-alpha-induced protein 3 (A20) (cf. reference 70) and human haeme oxygenase-1 (HO-1) (cf. reference 71).

(4) Genetic modifications to decrease the risk for zoonoses.

Xenotransplantation of porcine cells, tissues or organs carries the risk of transmitting porcine microorganisms to the human recipient and induction of zoonotic disease (cf. references 72, 73). Allotransplantation is in rare cases associated with transmission of pathogenic microorganisms such as human immunodeficiency and rabies viruses. Xenotransplants are however strictly regulated as advanced therapy medicinal products (ATMP) and subject to the highest level of safety requirements and thus may actually be safer than allografts in the future (cf. reference 74). Although a wide range of bacteria, viruses, parasites and fungi pose theoretical risks, comprehensive investigations of different pig colonies have shown that the actual number of microorganisms found in pigs is limited, e.g. (cf. references 26, 75). A complete picture of the microorganisms in individual pigs is not yet available and will strongly depend on the test methods used, but it is unlikely that many new pathogens will be identified. One reason is the thousand years of co-existence of humans and pigs, and second is that most pig donors are produced under SPF or near-SPF conditions, preventing infections from third species. Nevertheless, screening for putative zoonotic microorganisms and sterility testing is mandatory.

Porcine endogenous retroviruses (PERVs) cannot easily be eliminated, since they are integrated into the porcine genome, and thus pose the most refractory source of risk (reviewed in reference 39). PERV-A and PERV-B are able to infect some human tumour cells and to a lesser extent human primary cells. PERV-C infects only pig cells, but recombinants with PERV-A can infect human cells and are characterised by a high replication rate. Since PERV-C is not present in all pigs, it is possible to identify and select PERV-C-free animals as potential donors. Thus far, in vivo transmission of PERVs has not been documented, despite many studies, including the first clinical trials of porcine islets with more than 200 patients. Neither has PERV transmission been documented in numerous preclinical trials of pig cells and organs transplanted into non-human primates (reviewed in reference 39). However, in almost all clinical xenotransplantation trials, no immunosuppression was applied and only a small number of cells was transplanted. Animal xenotransplantation and PERV infection experiments involve recipient species that do not have a fully functional PERV receptor, e.g. non-human primates or rats. Data on PERV infectivity in vivo are therefore not considered to be complete.

Strategies to prevent PERV transmission include (i) selection of PERV-C-free animals (cf. reference 23) with low expression of PERV-A and -B; (ii) vaccines based on neutralising antibodies (for review, see reference 76); and (iii) long-term reduction of PERV expression by PERV-specific siRNA (e.g. references 77, 78). A paper reported simultaneous inactivation of up to 62 proviruses in an immortal pig kidney cell line (PK15) using the CRISPR/Cas9 system (cf. reference 79). This approach could be reproduced with primary pig cells, generating healthy PERV-free animals (cf. reference 80; commented in references 81, 82).

*Donor organism for xenogeneic organ transplants and methods of producing same*

Organ overgrowth in the recipient organism is a major problem when a genetically modified organ from a xenogeneic donor organism (e.g., from a pig) is transplanted into a recipient organism (e.g., into a baboon). In accordance with the present invention, organ overgrowth is counteracted by a combination of treatments: (i) decreasing the blood pressure of the recipient (e.g., of the baboon) to match the lower levels of the donor (e.g., of the pig); (ii) tapering cortisone at an early stage; and/or (iii) using the sirolimus prodrug temsirolimus to mitigate myocardial hypertrophy (cf. Examples 1 to 4). However, the present inventors have found that the growth of pigs (and thus of their organs) can also be counteracted genetically, by knocking out the growth hormone receptor (GHR) gene (cf. Example 7). The present inventors have found that the body weight of GHR-KO (GHR-knock out) pigs was reduced by 60% compared with controls, and most organs weights were reduced proportionally. Moreover, GHR-KO pigs had markedly reduced serum insulin-like growth factor 1 (IGF1) levels. Strikingly, by treating GHR-KO pigs with IGF1, it will be possible to promote their growth (shown in references 88, 108, 109 and 110 for patients with Laron syndrome). Hence, by treating GHR-KO pigs with IGF1, the size of the pigs and their organs can be adjusted at will.

In view of the above, the present inventors have knocked out the GHR gene in GT-KO/hCD46/hTM pigs that can be used as donor organisms for xeno-transplantation into primates (cf. Example 7). Hence, the present invention provides GHR-KO mammals (e.g., pigs) that are suitable donors for xeno-transplantation, and whose size/organ size can be adjusted by treating them with IGF1. Using such donor mammals in the xeno-transplantation in accordance with the present invention may cooperate with the above-mentioned drug treatments of the present invention to counteract organ overgrowth. Additionally, using GHR-KO mammals as donors could avoid long-term treatment of the recipient with an mTOR inhibitor. Finally, being able to adjust the size of the donor organs to the needs of the recipient may be particularly useful for recipients of relatively small size, who require relatively small donor organs.

Accordingly, the present invention provides a donor organism for xenogeneic organ transplants, wherein the donor organism has a homozygous knock-out of the growth hormone receptor gene, and wherein the donor organism is a mammal. Such donor organism can be obtained as described in Example 7.

In one embodiment of the donor organism for xenogeneic organ transplants in accordance with the present invention, the donor organism additionally has a homozygous knock-out of the alpha-1,3-galactosyltransferase gene, an insertion of a sequence encoding human CD46, and an insertion of a sequence encoding human thrombomodulin. In an alternative embodiment, the donor organism additionally has homozygous knock-outs of the alpha-1,3-galactosyltransferase, CMAH and B4GALNT2 genes, and insertions of a sequence encoding human CD46, a sequence encoding human CD55, a sequence encoding human CD59, a sequence encoding hemeoxygenase 1 (preferably human hemeoxygenase 1) and a sequence encoding A20 (preferably human A20). In the latter embodiment, the donor organism may additionally have insertions of a sequence encoding LEA29Y or PD-L1, preferably human PD-L1. Details relating to the above-mentioned sequences that may be inserted in the donor organism for xenogeneic organ transplants in accordance with the present invention are found in the section relating to the method of prolonging the survival of a primate following heart/kidney/lung/liver transplantation in accordance with the present invention, and in the section relating to the primate with a transplanted heart/kidney/lung/liver in accordance with the present invention. In particular, the transcripts that are preferably expressed when inserting the genes which are described in those sections are also the transcripts that are preferably expressed when inserting the corresponding sequences into the donor organism for xenogeneic organ transplants in accordance with the present invention.

In another embodiment of the donor organism for xenogeneic organ transplants in accordance with the present invention, the donor organism may have one or more additional genetic modification(s). Details relating to these one or more additional genetic modification(s) are found in the section "Genetically modified mammal and method of producing same" above. One of these one or more additional genetic modification(s) may be insertion of a sequence encoding human a1,2-fucosyltransferase. Preferably, the human $\alpha$1,2-fucosyltransferase is the protein expressed from the following gene: Official Symbol: FUT1; Official Full Name: fucosyltransferase 1 (H blood group); Primary source: HGNC:HGNC:4012; See related: Ensembl:ENSG00000174951, MIM:211100.

In another embodiment of the donor organism for xenogeneic organ transplants in accordance with the present invention, the donor organism belongs to the family *Suidae,* preferably to the genus *Sus*, more preferably to the species *S. scrofa,* most preferably to the subspecies *S. s. domesticus.* In a preferred embodiment, the donor organism belongs to the species *S. scrofa.* In a further embodiment, the donor organism is a pig of German Landrace/Large White cross-bred genetic background, a Duroc pig, a Swabian-Hall (Schwäbisch-Hällische) pig, a black mini pig, or an Auckland Island pig; preferably a pig of German Landrace/Large White cross-bred genetic background.

The donor organism for xenogeneic organ transplants in accordance with the present invention may also be a genetically modified organism in accordance with the present invention, as described above.

The present invention also provides a method of making a donor organism for xenogeneic organ transplants, comprising the step of adjusting the size of the donor organism by treating the donor organism with IGF1; wherein the donor organism has a homozygous knock-out of the growth hormone receptor gene, and wherein the donor organism is a non-human mammal. Preferably, the treatment with IGF1 is initiated after birth of the donor organism. For humans, a starting dose of rhIGF-1 0.04 mg/kg twice daily SC is recommended. The dose should be increased at regular increments to reach the maintenance dose of 0.12 mg/kg twice daily by ~2-3 months as tolerated. Long-term treatment with doses less than 0.12 mg/kg/day may lead to suboptimal growth responses. For the non-human donor mammals in accordance with the present invention, a similar dosing regimen may be appropriate.

The embodiments described for the organism for xenogeneic organ transplants in accordance with the present invention also apply to the method of making a donor organism for xenogeneic organ transplants in accordance with the present invention, *mutatis mutandis.*

As will be clear to a person skilled in the art, for all embodiments of the present invention, when reference is made to the insertion of a sequence encoding a protein, such sequence is preferably inserted such that the protein is expressed, e.g. by inserting the sequence as part of an expression cassette that additionally comprises regulatory sequences. Insertion of such sequence in accordance with the present invention may also be referred to as insertion of a transgene.

As will be clear to the skilled person, when reference is made herein to insertions of genes or sequences in a donor organ or donor organism in accordance with the present invention, it has to be ensured that the proteins encoded by these genes or sequences are compatible with the respective recipient organism. Hence, in one embodiment that can be combined with all other embodiments of the present invention, the inserted genes or sequences may be derived from the same species as the recipient organism. High sequence homology of the inserted gene or sequence with the respective gene of the recipient organism would then ensure compatibility. According to the present invention, the donor organism is a mammal and the recipient organism is a primate. Since it is considered that donor organs with insertions of human genes or sequences are compatible even with non-human primate recipients (cf. Examples 1 to 4), in one embodiment the inserted genes or sequences in accordance with the present invention are human genes or human sequences, respectively. In this embodiment, also the recipient organism may be human.

In the following, the present invention will be illustrated by examples, without being limited thereto.

## EXAMPLES

### Example 1: Materials and Methods

The following materials and methods were used in the subsequent experiments of the present invention.

#### Animals

Seven juvenile pigs of cross-bred genetic backround (German Landrace and Large White, blood group 0) served as donors for heart xenotransplantation. All organs were homozygous for alpha 1,3-galactosyltransferase knock-out (GT-KO), and transgeneic for hCD46/thrombomodulin (hTM) (Revivicor, Blacksburg, VA, USA and Institute for Molecular Animal Breeding and Biotechnology, Gene Center, Faculty of Veterinary Medicine, Ludwig-Maximilians-University, Munich, Germany). Localisation and stability of hCD46/hTM expression was verified post-mortem by immunohistochemistry (Fig. 15). Donor heart function and absence of valvular defects were evaluated 7 days prior to transplantation by echocardiographies. Seven male captive-bred baboons (Papio Anubis, blood groups B and AB) were used as recipients.

The study was approved by the local authorities and the Government of Upper Bavaria. All animals were treated in compliance with the Guide for the Care and Use of Laboratory Animals (US National Institutes of Health and National Law).

#### Anesthesia and Analgesia

Premedication of the baboons consisted of an intramuscular injection of ketamine hydrochloride 6-10 mg/kg (Ketavet® 100 mg/mL; Pfizer Deutschland GmbH, Berlin, Germany) and 0.3-0.5 mg/kg midazolam (Midazolam-ratiopharm®; ratiopharm GmbH, Ulm, Germany). General anesthesia was induced with an intravenous bolus of 2.0-2.5 mg/kg propofol (Propofol®-Lipuro 2%; B. Braun Melsungen AG, Melsungen, Germany) and 0.05 mg fentanyl (Fentanyl-Janssen 0.5 mg; Janssen-Cilag GmbH, Neuss, Germany), and maintained with a propofol ($0.16 \pm 0.06$ mg/kg/min) or sevoflurane (1-2 Vol% endexpiratory; Sevorane, AbbVie Germany GmbH & Co. KG, Wiesbaden, Germany) and bolus administrations of fentanyl (5-10 $\mu$g/kg, repeated every 45 min) as described elsewhere (cf. reference 11). Postoperatively, a continuous infusion of fentanyl, ketamine hydrochloride and metamizole (Novaminsulfon-ratiopharm® 1g/2mL; ratiopharm GmbH, Ulm, Germany) was applied to ensure analgesia.

#### Donor explantation and heart preservation

After median sternotomy and heparinization (500 IU/kg), a small canula was inserted into the ascending aorta which was then cross-clamped distal of the canula. In group I the heart was cardiopleged with a single dose of 20ml/kg crystalloid cardioplegic solution at 4°C: experiments #56, 58 and 59 received Custodiol HTK solution (Dr. F. Kohler Chemie GmbH, Bernsheim, Germany), experiments #55 and 57 Belzer's UW solution (Preservation Solutions Inc., Elkhorn, WI, USA). The appendices of the right and left atrium were opened for decompression. The heart was then excised, submersed in cardioplegic solution and stored on ice.

In groups II and III hearts were preserved as described by Steen at al. (cf. reference 10), using 3.5 L of an oxygenated albumin-containing hyperoncotic cardioplegic nutrition solution with hormones and erythrocytes at a temperature of 8°C in a portable extracorporal heart preservation system consisting of a pressure- and flow-controlled roller pump, an $O_2$/$CO_2$ exchanger, a leukocyte filter, an arterial filter and a cooler- heater unit.

After aortic crossclamping, the heart was perfused with 600 ml of preservation medium, then excised and moved into the cardiac preservation system. A large cannula was introduced into the ascending aorta and the mitral valve made temporarily incompetent to prevent left ventricular dilation; the superior caval vein was occluded, inferior caval vein, pulmonary artery and pulmonary veins were however left open for free outlet of perfusate. The heart was submersed in a reservoir filled with cold perfusion medium and antegrade coronary perfusion commenced via the already placed aortic canula. The perfusion pressure remained regulated at precisely 20 mmHg. During implantation, the heart was intermittently perfused for 2 min every 20 min.

Implantation technique

The recipient's thorax was opened at midline. Unfractionated heparin (500 IU/kg; Heparin-Natrium-25000-ratiopharm, ratiopharm GmbH, Ulm, Germany) was given and the heart-lung machine connected, using both caval veins and the ascending aorta. Cardiopulmonary bypass commenced and the recipient cooled (30°C in group I, 34°C in groups II and III). After crossclamping the ascending aorta, the recipient's heart was excised at the atrial levels, both large vessels were cut. The porcine donor heart was transplanted applying Shumway's and Lower's technique (cf. reference 8).

A wireless telemetric transmitter (Data Sciences International, St. Paul, MN, USA) was implanted in a subcutaneous pouch in the right medioclavicular line between the $5^{th}$ and $6^{th}$ rib. Pressure probes were inserted into the aorta and the apex of the left ventricle, AN ECG lead was placed in the right ventricular wall.

Immunosuppressive regimen, anti-inflammatory and additive therapy

Immunosuppression was based on Mohiuddin's regimen (cf. reference 5; there were changes as indicated in the following), with C1 esterase inhibitor instead of cobra venom factor for complement inhibition. Induction consisted of anti-CD20 antibody (Mabthera; Roche Pharma AG, Grenzach-Wyhlen, Germany), ATG (Thymoglobuline, Sanofi-Aventis Germany GmbH, Frankfurt, Germany), and either anti-CD40 antibody (mouse/rhesus chimeric IgG4 clone 2C10R4, NIH Nonhuman Primate Reagent Resource, Mass Biologicals, Boston, MA, USA; courtesy of Keith Reimann) or humanised anti-CD40L pasylated Fab (production: Wacker-Chemie, Burghausen, Germany; purification: XL-protein GmbH, Freising, Germany; courtesy of Arne Skerra, cf. reference 9). Maintenance immunosuppression consisted of MMF (CellCept, Roche, Basle, Switzerland; trough level 2-3 μg/ml), either anti-CD40 antibody or anti-CD40L pasylated Fab, methylprednisolone (Urbasone solubile, Sanofi-Aventis Germany GmbH, Frankfurt, Germany). Anti-inflammatory therapy included Il-6-receptor antibody (RoActemra, Roche Pharma AG, Grenzach-Wyhlen, Germany), TNFa inhibitor (Enbrel, Pfizer Pharma GmbH, Berlin, Germany) and Il-1-receptor antagonist (Kineret, Swedish Orphan Biovitrum GmbH, Martinsried, Germany). Additive therapy: acetylsalicylic acid (Aspirin, Bayer Vital GmbH, Leverkusen, Germany), unfractionated heparin (Heparin-Natrium-25000-ratiopharm, ratiopharm GmbH, Ulm, Germany), C1 esterase inhibitor (Berinert, CSL Behring GmbH, Hattersheim, Germany), ganciclovir (Cymevene, Roche Pharma AG, Grenzach-Wyhlen, Germany), cefuroxime (Cefuroxim Hikma, Hikma Pharma GmbH, Martinsried, Germany) and epoetin beta (NeoRecormon 5000IU, Roche Pharma AG, Grenzach-Wyhlen, Germany).

Starting from 10 mg/kg/d, methylprednisolone was tapered down 1 mg/kg every 10 days in group I and II; in group III, methylprednisolone was tapered down to 0.1 mg/kg within 19 days. Also in group III, temsirolimus (Torisel, Pfizer Pharma GmbH, Berlin, Germany) was added to the maintenance immunosuppression, administered as daily i.v. short infusions aiming at rapamycin trough levels of 5-10 ng/ml. Group III also received continuous i.v. antihypertensive medication with enalapril (Enahexal, Hexal AG, Holzkirchen, Germany) and metoprolol tartrate (Beloc i.v., AstraZeneca GmbH, Wedel, Germany), aiming at mean arterial pressures of 80 mmHg and a heart rate of 100 bpm.

Hemodynamic measurements

After induction of general anesthesia, a central venous catheter (Arrow International, Reading, PA, USA) was inserted in the left jugular vein and an arterial catheter (Thermodilution Pulsiocath; Pulsion Medical Systems, Munich, Germany) in the right femoral artery. Cardiac output and stroke volume were assessed by transpulmonary thermodilution and indexed to the body surface area of the recipient using the formula $0.083*kg^{0.639}$. Measurements were taken after induction of anesthesia and 60 minutes after termination of CPB in steady states and recorded with PiCCOWin software (Pulsion Medical Systems, Munich, Germany). All data were processed with Excel (Microsoft, Redmond, Washington, USA) and analyzed with GraphPad Prism 7.0 (GraphPad Software Inc., San Diego, California, USA).

Quantification of LV mass, LV mass increase and FS

Transthoracic echocardiographies were done in analgosedation at regular intervals using an HP Sonos 7500 (HP Inc., Palo Alto, CA, USA); midpapillary short axis views were recorded. At end-diastole and end-systole, LV diameters (LVEDD, LVESD), diastolic and systolic interventricular (IVSd, IVSs), posterior wall thicknesses (PWd, PWs) were measured; the mean of three measurements was used for further calculations and visualization (Excel and PowerPoint, Microsoft, Redmond, Washington, USA).

LV mass was calculated using formula (1), relative LV mass increase and LV FS using formulas (2) and (3) (cf. references 11, 12).

$$(1)\ \text{LV mass(g)} = 0.8*(1.04([\text{LVEDD+IVSd+PWd}]^3-[\text{LVEDD}]^3))+0.6$$

$$(2)\ \text{LV mass increase (\%)} = ([\text{LV mass}_{end}/\text{LV mass}_{start}] - 1)*100$$

$$(3)\ \text{FS (\%)} = ([\text{LVEDD} - \text{LVESD}]/\text{LVEDD})*100$$

Necropsy and histology

Necropsies and histology were performed at the Institute of Veterinary Pathology, and the Institute of Pathology, both LMU Munich. Specimens were fixed in formalin, embedded in paraffin and plastic, sectioned and haematoxylin-eosin (HE) stained.

Histochemical analysis

Cryosections (8 μm) were generated using standard histological techniques. Myocyte size was quantified as cross-sectional area. 8 μm thick cardiac sections of the left ventricle were stained with Alexafluor647-conjugated wheat germ agglutinin (Life Technologies) and the nuclear dye DAPI (Life Technologies). Images were acquired with a 10x and a 63x objective using a Leica TCS SP8 confocal microscope; ImageJ software was applied to determine the average cross-sectional area of cardiomyocytes in one section (200-300 cells per section and 5-7 sections per heart).

Immunofluorescence staining

Myocardial tissue biopsies were embedded in Tissue-Tek (Sakura Finetek, Zoeterwoude, The Netherlands) and stored frozen at -80°C. For immunofluorescence staining, 5μm thick cryosections were cut, air dried for 30 to 60 min and stored at -20°C until further analysis. The cryosections were fixed with ice cold acetone, hydrated and stained using either one-step direct or two-step indirect immunofluorescence techniques. The following antibodies were used: rabbit anti-human C3b/c (Dako, Glostrup, Denmark), rabbit anti-humanC4b/c-FITC (Dako), goat anti-pig IgM (AbD Serotec, Hercules CA, USA), goat anti-pig IgG-FITC (Southern Biotech, Birmingham, USA). Secondary antibodies were donkey anti-goat IgG-Alexa 488 (Thermo Fischer Scientific, MA, USA), sheep anti-rabbit Cy3 (Sigma-Aldrich, St. Louis, MO, USA). Nuclear staining was performed using 4',6-diamidino-2-phenylindole (DAPI; Boehringer, Roche Diagnostics, Indianapolis, IN, USA). The slides were analyzed using a fluorescence microscope (DM14000B; Leica, Wetzlar, Germany). Quantification of fluorescence intensity was performed using Image J software, version 1.50i (https://imagej.nih.gov/ij/), on unmanipulated TIFF (tagged image file format) images. All pictures were taken under the same conditions in order to allow correct quantification and comparison of fluorescence intensities.

Assessment of anti-non-Gal antibody levels

Plasma levels of anti-non-Gal baboon IgM and IgG antibodies were measured by Flow Cytometry following the consensus protocol published by Azimzadeh et al. (cf. reference 14). Briefly, GT-KO/hCD46/hTM porcine aortic endothelial cells (PAEC) were harvested and suspended at 2x106 cells/ml in staining buffer (PBS+1%BSA). Plasma samples were heat inactivated at 56°C for 30 min and diluted 1/20 in staining buffer. PAEC were incubated with diluted baboon plasma for 45 minutes at 4°C. Cells were then washed with cold staining buffer and incubated with goat anti-human IgM-RPE (Southern Biotech) or goat anti-human IgG-FITC (Thermo Fischer) for 30 minutes at 4°C. Cells were then washed with cold staining buffer, resuspended in phosphate-buffered saline (PBS), acquired on FACS LSRII and analyzed using FlowJo analysis software for detection of mean fluorescence intensity (MFI) in the FITC channel. Data were then plotted using Prism 7 (Graphpad software, Inc.). FITC, fluorescein isothiocyanate; RPE, ribulose-phosphate epimerase.

Western blot analysis

For protein extraction, heart samples were homogenized in Laemmli-extraction-buffer, and the protein content was estimated by the bicinchoninic acid (BCA, Merck, Darmstadt, Germany) protein assay. 20 μg of total protein was separated by 10% SDS-PAGE and transferred to polyvinylidene difluoride (PDVF) membranes (Millipore, Billerica, USA) by electroblotting. Membranes were washed in Tris-buffered saline solution with 0.1% Tween-20 (Merck) (TBS-T) and blocked in 5% w/v fat-free milk powder (Roth, Karlsruhe, Germany) for 1 h at room temperature. Then membranes were washed again in TBS-T and incubated in 5% w/v BSA (Roth) of the appropriate primary antibody overnight at 4°C. The following antibodies were used: rabbit anti-human pmTOR (#5536; Cell Signaling, Frankfurt, Germany) and mouse anti-human PCNA (#2586; Cell Signalling). After washing, membranes were incubated in 5% w/v fat-free milk powder with a horseradish peroxidase labelled secondary antibody for 1 h at room temperature. Bound antibodies were detected using an enhanced chemiluminescence detection reagent (ECL Advance Western Blotting Detection Kit, GE Healthcare, Munich, Germany) and appropriate x-ray films (GE Healthcare). After detection, membranes were stripped (Elution buffer: 2% SDS, 62.5 mM Tris/HCI, pH 6.7 and 100mM β-mercaptoethanoi for 30 min at 70°C) and incubated with an appropriate second antibody. SDS-PAGE, sodium dodecyl sulfate polyacrylamide gel electrophoresis.

Statistical analysis

For survival data, Kaplan-Meier curves were plotted and the Mantel-Cox log-rank test was used to determine significant differences between the groups. For hemodynamic data, statistical significance was determined using unpaired and paired Student's t-test as indicated; data presented as single measurements with bars as group medians. For histochemical analysis, one-way ANOVA with Holm-Sidak's multiple comparisons was used to determine statistical significance; data presented as mean±s.d. $P < 0.05$ was considered significant. s.d., standard deviation.

**Example 2: Results**

gm (GTKO/hCD46/hTM) pig-to-baboon cardiac transplant experiments were conducted applying clinically approved "Shumway's" orthotopic technique (cf. reference 8). All recipients of the study received IS as described more or less by Mohiuddin (cf. reference 5): Induction therapy included anti-CD20, anti-thymocyte-globuline (ATG) and anti-CD40 or our own anti-CD40L pasylated Fab (cf. reference 9). Maintenance therapy consisted of anti-CD40 or anti-CD40L pasylated Fab, MMF and methylprednisolone (MP) tapered down (see Table 1; additive medical treatment is also shown; C1 esterase inhibitor replaced cobra venom factor).

| **Agent Induction** | **Dose** | **Timing** |
|---|---|---|
| anti-CD20 AB | 19 mg $kg^{-1}$, i.v. short infusion | days -7, 0, 7 and 14 |
| ATG | 50 mg $kg^{-1}$, i.v. short infusion | days -2 and -1 |
| anti-CD40 AB or PASylated anti- CD40L Fab | 50 mg $kg^{-1}$ or 20 mg $kg^{-1}$; i.v. short infusion | days -1 and 0 |
| **Maintenance** | | |
| MMF | 40 mg $kg^{-1}$, continuously i.v. | daily, started on day -2 |
| anti-CD40 AB or PASylated anti- CD40L Fab | 50 mg $kg^{-1}$ or 20 mg $kg^{-1}$ i.v. short infusion | days 1, 3, 7, 10 , 14, 19, then weekly |
| methylprednisolone | 10 mg $kg^{-1}$, bolus i.v. | BID, tapered down daily |
| **Anti-inflammatory therapy** | | |
| Il-6-receptor AB | 8 mg $kg^{-1}$, short infusion i.v. | monthly |
| TNFa inhibitor | 0.7 mg $kg^{-1}$, bolus s.c. | weekly |
| Il-1-receptor antagonist | 1.3 mg $kg^{-1}$, bolus s.c. or i.v. | daily |
| **Additive therapy** | | |
| acetylsalicylic acid | 2 mg $kg^{-1}$, bolus i.v. | daily |

(continued)

| | | |
|---|---|---|
| **Additive therapy** | | |
| unfractionated heparin | 20-40 U $kg^{-1}h^{-1}$, continuously i.v. | daily, started on day 5 |
| C1 esterase inhibitor | 17.5 U $kg^{-1}$, i.v. short infusion | days 0, 7 and 14 |
| ganciclovir | 5 mg $kg^{-1}$, continuously i.v. | daily |
| cefuroxim | 50 mg $kg^{-1}$, continuously i.v. | daily, prophylaxis from day 0 to 5 |
| epoetin beta | 2000 U, bolus s.c. or i.v. | days -7, 0 and if necessary |

**Table 1**: Immunosuppressive regimen, anti-inflammatory and additive therapy with corresponding doses and timing intervals. Immunosuppression was based on Mohiuddin's regimen (cf. reference 5), with C1 esterase inhibitor instead of cobra venom factor for complement inhibition. Starting from 10 mg/kg/d, methylprednisolone was tapered down 1 mg/kg every 10 days in group I and II; in group III, methylprednisolone was tapered down to 0.1 mg/kg within 19 days. Also in group III, temsirolimus was added to the maintenance immunosuppression, administered as daily i.v. short infusions aiming at rapamycin trough levels of 5-10 ng/ml. Group III also received continuous i.v. antihypertensive medication with enalapril and metoprolol tartrate, aiming at a mean arterial pressure of 80 mmHg and a heart rate of 100 bpm. AB, antibody; ATG, anti-thymocyte globulin; BID, twice daily; CMV, Cytomegalovirus; Fab, fragment antigen binding; IgG4, immunoglobulin G4; Il or IL, interleukin; i.v., intravenous; MMF, mycophenolate mofetil; PAS, PASylated; s.c., subcutaneous; TNFa, tumor necrosis factor a.

The 11 experiments were subdivided into 3 groups (see Table 2); group I (n=5) had donor organ preservation with 2 clinically approved crystalloid solutions (4°C Custodial HTK, Belzer's UW, each given once; the porcine hearts (blood group 0) were then kept in plastic bags filled with solution, and the bags in ice cubes; static preservation); in groups II (n=4) and III (n=2), the excised hearts were immediately perfused at 8°C with a pressure-controlled roller pump via a cannula fixed in the ascending aorta. The deoxygenated blood which left the donor organ was collected in a reservoir and then passed an oxygenator (non-ischemic preservation). During the left and right atrial anastomoses, the pulmonary artery suture line, perfusion was continued intermittently; it was stopped only when both aortic stumps were connected.

| experiment | donor TBW [kg] | donor HW [g] | recipient TBW [kg] | recipient HW [g] |
|---|---|---|---|---|
| group I | | | | |
| # 55 | 18.4 | 86 | 19.5 | 80 |
| # 56 | 19.8 | 95 | 21.0 | 86 |
| # 57 | 15.8 | 87 | 16.0 | 61 |
| # 58 | 17.0 | 100 | 17.0 | 69 |
| # 59 | 11.7 | 62 | 14.0 | 67 |
| group II | | | | |
| # 60 | 19.2 | 100 | 17.0 | 63 |
| # 62 | 8.0 | 48 | 10.6 | 60 |
| # 63 | 8.0 | 52 | 10.5 | 50 |
| # 64 | 11.3 | 77 | 13.9 | 59 |
| group III | | | | |
| # 66 | 18.7 | 101 | 22.0 | 93 |
| # 67 | 12.7 | 61 | 13.7 | 48 |

**Table 2**: Total body (TBW) and heart weights (HW) of donor pigs and recipient baboons. All donor pigs were genetically modified (GT-KO/hCD46/hTM). According to major procedural changes, the experiments were subdivided into three groups as follows: group I (experiments # 55-59), static crystalloid cardioplegia (4° C) with Custodiol HTK or Belzer's UW solution; group II (experiments # 60, # 62-64), cold (8°C) pressure controlled (20 mmHg) perfusion with oxygenated hyperoncotic cardioplegic perfusion medium in an extracorporeal heart preservation system (courtesy of Stig Steen, Lund University, Lund, Sweden (cf. reference 10), non-ischemic heart preservation); group III (experiments # 66 and # 67), same as in group II but with addition of intensified antihypertensive treatment, faster corticoid tapering and temsirolimus.GT-KO, α1,3-galactosyltransferase 1-knockout; hCD46, human complement regulatory protein CD46; hTM, human thrombomodulin; HTK, histidin-tryptophan-ketoglutarate; UW, University of Wisconsin.

Eleven captive-bred baboons (Papio Anubis, blood groups B and AB) served as recipients. The postoperative treatment of the recipients is described elsewhere (cf. reference 11).

The results of group I (static cold preservation with crystalloid solutions) were as follows: although the ischemic times were kept as short as possible (123 ± 7 min), survivals were three times one day, 3 days and 28 days once. In the latter case (cardiac preservation with Belzer's UW-solution), echocardiography revealed limiting diastolic pump function (thickened LV myocardium, decreased LV cavity and hence diastolic filling; Figure 6A; Figure 3; Figure 1E,F) combined with terminal liver failure (Table 3, Figure 1B,H); in contradistinction, the 4 short-term survivors were always in severe systolic left heart failure after discontinuing cardiopulmonary bypass (CPB), necessitating high dose intravenous catecholamines (Figure 3, Figure 1A-C). All 4 were however weaned from CPB, 3 of them extubated.

| Experiment | Group I # 57 | Group II # 60 | # 63 | # 64 | Group III # 66 | # 67 | Reference |
|---|---|---|---|---|---|---|---|
| Bilirubin (mg/dl) | 1.2 | 0.9 | 2.7 | 4.5 | 0.3 | 0.2 | ≤ 1.2 |
| AST (U/l) | 646 | 896 | 792 | 354 | 101 | 27 | ≤ 49 |
| PR (%) | 30 | 6 | 6 | 6 | 101 | 96 | 70 - 130 |
| CHE (kU/l) | 1.6 | 1.6 | 1.4 | 1.1 | 2.1 | 9.4 | 4.6 - 11.5 |
| Trop T (hs) (ng/ml) | 0.233 | 0.660 | 1.460 | 1.470 | 0.218 | 0.037 | ≤ 0.014 |
| CK total (U/l) | 654 | 636 | 1017 | 953 | 3053 | 143 | ≤ 189 |
| LDH (U/l) | 3252 | 6853 | 2842 | 1627 | 436 | 311 | ≤ 249 |
| Platelets (G/L) | 99 | 101 | 65 | 29 | 216 | 202 | 150 - 300 |
| Survival (d) | 28 | 18 | 27 | 40 | 51 | 90 | |
| Causes of death | heart and liver failure | heart and liver failure | heart and liver failure | heart and liver failure | SCV thrombosis, thoracic duct occlusion | elective euthanasia | |

**Table 3**: Serum levels of liver and heart enzymes, platelet counts and prothrombin ratio at the end of experiments lasting longer than three weeks (right column: normal reference values). Group I and II animals exhibited pathologic biochemical findings corresponding to heart and liver failure; platelet counts were low and LDH was elevated. By contrast, most parameters remained close to or within normal ranges in animals of group III. Experiment 66 had to be euthanized because of vast pleural effusions due to SCV thrombosis and thoracic duct occlusion. Experiment 67 was electively terminated after reaching the study endpoint of 90 day, showing no signs of cardiac or liver dysfunction. AST, aspartate aminotransferase; CHE, cholinesterase; CK, creatine kinase; LDH, lactate dehydrogenase; PR, prothrombine ratio; SCV, superior caval vein; Trop T (hs), troponin T (high sensitive).

In the following groups II and III, organ perfusion (non-ischemic heart preservation; perfusion times 196 ± 44 respectively 224 ± 45 min) as described in Example 1 commenced. Subsequently, all baboons of the two groups were easily weaned from CPB, with low amounts of catecholamines (Figure 3). However, all 3 long-term survivors of group II (18, 27 and 40 days, the 4 day experiment was a technique failure) succumbed to diastolic pump failure combined with terminal liver disease, similar findings as described for the single 28 days survivor of group I (Figure 6A; Table 3; Figure 1A,B,H).

Just before termination of each experiment, the data of the most important biochemical serum measurements of group II animals are shown in Table 3: Bilirubin was elevated, also liver enzymes like AST; prothrombin-ratio and cholinesterase were decreased; troponin T was above normal range. (Figure 1A,B depict the sharp increases of troponin and bilirubin levels just before the experiments had to be terminated). Lactate dehydrogenase levels were elevated and platelet counts low. At autopsy, donor hearts were enlarged - when compared to their weight at the time of implantation, the overgrowth in group I and II had been on average 259%. As a matter of fact, the transplants appeared squeezed by the surrounding mediastinal structures. The diameters of the left and right ventricular walls were enlarged, the left ventricular cavities minute (Figure 4A; cf. references 12, 13). Of note is Figure 4C, which depicts the size of a sibling's heart of the donor of transplant 64: euthanized at the same time, its external cardiac diameters were almost identical.

Myocardial histology of the group II transplants revealed hypertrophic cells (Figure 1E,F,J,K). There were abundant liver cell necroses around the central veins (Figure 1H).

In contradistinction, all the just described pathologic findings were absent in group III recipients, which had antihypertensive treatment (pigs have lower systolic pressures when compared to baboons, on average 80 versus 120 mmHg), early glucocorticoid weaning and additional temsirolimus medication. Of note was the myocardial appearance at autopsy: At the end of the 90 days, echocardiography was normal (Figure 6B). At autopsy, the ventricular walls were nearly normal and the cavities unimpaired in size; the histologic findings of the donor heart and the recipient's liver were also normal (Table 3; Figure 1G,I,J,K,L; Figure 4B).

Figure 2 summarizes immunologic responses of all long-term survivors. Grafts of group II showed no gross signs of humoral rejections and the slight changes may rather be attributed to the described preterminal limitations of the heart function; there were no pathologic immuno-histochemical findings in group III. In both groups, plasma measurements of non-Gal antibodies (cf. reference 14) exhibited no increases above already existing low levels.

In addition to the experiments described above, another baboon was transplanted a heart as described for the baboons of group III, and in accordance with the Materials and Methods of Example 1. The baboon survived for 90 days and the postoperative findings and measurements were the same as in Experiment # 67 described above.

## Example 3: Conclusions

Taken together, the herewith presented conduct of experiments proves at the end (group III), that life supporting (orthotopic) xeno-heart-transplantations are possible applying (pre-) clinical standards and fulfilling in part the requests of the ISHLT guideline (cf. reference 6).

The following contributed to success:

- The adoption of Mohiuddin's basic IS (cf. reference 5), which omits effectively cellular and most importantly humoral rejections; apparent signs of thrombotic-microangiopathy (decreases of thrombocytes, LDH increases) seen in group II may rather be explained with the exorbitant growth of the organs and the increased energy demands of cellular hypertrophy which obviously did not match its supply due to inadequate vascular neogenesis (as has previously been described for other organs (cf. reference 15), Figure 5). However, several important changes were made: Anti-CD40L pasylated Fab seemed to be as effective as anti-CD40 (Figure 2). Anti-inflammatory therapy has been added. Glucocorticoid is tapered down quicker, temsirolimus added and finally anti-hypertensive treatment given.
- The introduction of non-ischemic porcine heart preservation (cf. reference 10). In contradistinction to groups II and III, group I donor organs had static myocardial preservation, as used for clinical allogeneic procedures. With static myocardial preservation, cardiac outputs in the immediate postoperative course were low (Figure 3A), necessitating large amounts of catecholamines (Figure 3B). The resulting high early mortality is in accordance with the findings in the literature over the past 25 years, which describe ranges of 40 to 60 % (cf. reference 7). This phenomenon has been termed "perioperative cardiac xenograft dysfunction" (cf. reference 16) and is obviously similar to cardiac stunning known from the earlier days of cardiac surgery; it does not resemble hyperacute rejection.

  Non-ischemic preservation technique in groups II and III achieved even clinically acceptable results: All 7 recipients came off CPB easily since the cardiac outputs remained unchanged, when compared to baseline.

  Limiting graft overgrowth has also been reported after xenogeneic renal and lung transplantations (cf. reference 15). The authors explained this phenomenon with intrinsic factors (which means, the "overgrowth" of the porcine organs is predetermined), rather than due to extrinsic stimuli (by the various growth hormones of the recipient). A statement, which is supported with the present observation of the near identical size increase of a non-transplanted sibling's heart till the endpoint of experiment 64 (Figure 4C).
- Overgrowth was for the first time successfully counteracted by decreasing the baboons' blood pressure in order to match the lower porcine levels. The glucocorticoid was tapered early, since the drug has been described to cause hypertrophic cardiomyopathy in early life (cf. reference 17). Finally, Sirolimus compounds are known to control the complex network of all cell growth by inhibing the two mTOR kinases (cf. reference 18). There is clinical evidence, that myocardial hypertrophy is attenuated, diastolic pump function improved (cf. references 19, 20); rare genetic overgrowth syndromes are treated in humans (cf. reference 21). In our case, Temsirolimus was chosen and myocardial hypertrophy mitigated, obliteration of LV cavity omitted.

Taken together, the present invention shows for the first time that orthotopic xeno-heart-transplantation is feasible in the difficult gm pig-to-baboon model. Realizing the full range of requests of the ISHLT guidelines (cf. reference 6) seems possible. Safety issues (cf. reference 22) can be addressed.

## Example 4: Extended study protocol

The study protocol for group III was extended aiming at a graft survival of six months (cf. reference 115). The consolidated results are shown in the following Table 4, as well as in Figures 11 to 15. The results presented in Table 4 include the results of the experiments described in Example 2 above, wherein Experiments # 57, 60, 63, 64, 66 and 67 of Table 3 correspond to Experiments 3, 6, 8, 9, 10 and 11 of Table 4, respectively. Experiment 12 of Table 4 corresponds to the additional experiment mentioned in the last paragraph of Example 2 above, and Experiments 13 and 14 of Table 4 are additional experiments for which the study protocol for group III was extended aiming at a graft survival of six months. The data presented in Figures 11 to 15 are based on the results of all experiments that are indicated in

Table 4.

| | Group I | Group II | | | Group III | | | | | |
|---|---|---|---|---|---|---|---|---|---|---|
| **Experiment** | **3** | **6** | **8** | **9** | **10** | **11** | **12** | **13** | **14** | **Reference** |
| **Bilirubin (mg dl$^{-1}$)** | 1.2 | 0.9 | 2.7 | 4.5 | 0.3 | 0.2 | 0.2 | 0.2 | 0.2 | ≤1.2 |
| **AST (U l$^{-1}$)** | 646 | 896 | 792 | 354 | 101 | 27 | 23 | 63 | 28 | ≤49 |
| **PR (%)** | 30 | 6 | 6 | 6 | 101 | 96 | 117 | 26 | 99 | 70–130 |
| **CHE (kU l$^{-1}$)** | 1.6 | 1.6 | 1.4 | 1.1 | 2.1 | 9.4 | 14.4 | 7.3 | 7.2 | 4.6–11.5 |
| **Troponin T (ng ml$^{-1}$)** | 0.233 | 0.660 | 1.460 | 1.470 | 0.218 | 0.037 | 0.018 | 0.556 | 0.140 | ≤0.014 |
| **CK total (U l$^{-1}$)** | 654 | 636 | 1017 | 953 | 3053 | 143 | 66 | 461 | 96 | ≤189 |
| **LDH (U l$^{-1}$)** | 3252 | 6853 | 2842 | 1627 | 436 | 311 | 511 | 962 | 497 | ≤249 |
| **Platelets (billion particles per litre)** | 99 | 101 | 65 | 29 | 216 | 202 | 128 | 271 | 303 | 150–300 |
| **Survival (days)** | 30 | 18 | 27 | 40 | 51 | 90 | 90 | 195 | 182 | |
| **Causes of death** | Heart and liver failure | Heart and liver failure | Heart and liver failure | Heart and liver failure | SVC thrombosis, thoracic duct occlusion | Euthanasia | Euthanasia | Euthanasia | Euthanasia | |

**Table 4**: Serum levels of liver and heart enzymes, platelet counts and prothrombin ratio at the end of experiments that lasted longer than two weeks. Normal reference values are given in the right-most column. Animals from groups I and II exhibited pathological biochemical alterations that correspond to heart and liver failure; platelet counts were low and LDH was elevated. By contrast, most parameters remained close to, or within, normal ranges in animals of group III. The baboon in experiment 10 had to be euthanized because of severe pleural effusions due to superior vena cava (SVC) thrombosis and occlusion of the thoracic lymph duct. The animals in experiments 11 and 12 were euthanized after reaching the study end point of 90 days, although they did not show any signs of cardiac or liver dysfunction. Animals in experiments 13 and 14 were euthanized after six months; recipient 13 showed the signs of beginning heart and liver dysfunction. CHE, cholinesterase; CK, creatine kinase; PR, prothrombin ratio.

The consolidated results of the extended study confirmed the results of Example 2: In summary, after heart perfusion times of 219 ± 30 min, all five animals of group III were easily taken off CPB, comparable to group II (Fig. 13). None of the recipients in group III showed PCXD; all reached a steady state with good heart function after four weeks. One recipient (experiment 10) developed recalcitrant pleural effusions that were caused by occlusion of the thoracic lymph duct and was therefore euthanized after 51 days. Two recipients (experiments 11 and 12) lived in good health for three months until euthanasia, according to the study protocol (Fig. 11A). In these three recipients, echocardiography revealed no increase in left ventricular mass (Fig. 11D); graft function remained normal with no signs of diastolic dysfunction (Fig. 4B). Biochemical parameters of heart and liver functions as well as LDH levels and platelet counts were normal or only slightly altered throughout the experiments (Table 4, Fig. 11B, C and Fig. 14A, B), consistent with normal histology (Fig. 11). Histology of left ventricular myocardium showed no signs of hypertrophy (Fig. 1J and Fig. 11E), and western blot analysis of the myocardium revealed phosphorylation levels of mTOR that were lower than non-transplanted age-matched control hearts (Fig. 11F). Similar to group II, there were no signs of humoral graft rejection in group III (Fig. 12 and Fig. 2A-D).

The last two recipients in group III (experiments 13 and 14) were allowed to survive in good general condition for 195 and 182 days, with no major changes to platelet counts or serum LDH and bilirubin levels (Fig. 11A, C and Fig. 14A, B). Intravenous temsirolimus treatment was discontinued on day 175 and on day 161. Up to this point, systolic and diastolic heart function was normal. Thereafter, increased growth of the cardiac graft was observed in both recipients (Fig. 11D), emphasizing the importance of mTOR inhibition in the orthotopic xenogeneic heart xenotransplantation model. Similar to the changes observed in group II, the smaller recipient 13 developed signs of diastolic dysfunction, which was associated with elevated serum levels of troponin T and the start of congestive liver damage (increased serum ALT and AST levels, decreased prothrombin ratio and cholinesterase); platelet counts remained within normal ranges (Table 4, Fig. 11B, C and Fig. 14A, B). Histology confirmed hepatic congestion and revealed multifocal myocardial necroses without immune cell infiltrations or signs of thrombotic microangiopathy. In the larger recipient 14, who had to be euthanized simultaneously with animal 13, the consequences of cardiac overgrowth were minimal.

The above results confirm the results of Example 2 and the conclusions described in Example 3. Consistent survival of life-supporting pig hearts in non-human primates for at least three months was achieved, which meets the preclinical efficacy requirements for the initiation of clinical xenotransplantation trials as suggested by an advisory report of the International Society for Heart and Lung Transplantation (cf. reference 6). Two steps contributed to success. First, non-ischaemic porcine heart preservation was found to be important for the survival of the xenografted hearts. Xenografted hearts from group I that underwent ischaemic static myocardial preservation with crystalloid solutions (as used for clinical allogeneic procedures) showed PCXD in four out of five cases, necessitating higher amounts of catecholamines. This phenomenon is clearly similar to 'cardiac stunning', the occurrence of which has been known since the early days of cardiac surgery and does not represent hyperacute rejection (cf. reference 7). By contrast, in groups II and III (non-ischaemic porcine heart preservation by perfusion) (cf. reference 10), all nine recipients came off CPB easily since their cardiac outputs remained unchanged compared to baseline. The short-term results achieved in these groups were excellent even by clinical standards. The second step was the prevention of detrimental xenograft overgrowth. Previous pig-to-baboon kidney and lung transplantation experiments have suggested that growth of the graft depends more on intrinsic factors than on stimuli from the recipient such as growth hormones (cf. reference 15). The massive cardiac hypertrophy in our group-II recipients indicates a more complex situation. Notably, a transplanted heart in this group had a 62% greater weight gain than the non-transplanted heart of a sibling in the same time span (Fig. 4C). In group III, cardiac overgrowth was successfully counteracted by a combination of treatments: (i) decreasing the blood pressure of the baboons to match the lower porcine levels; (ii) tapering cortisone at an early stage-cortisone can cause hypertrophic cardiomyopathy in early life in humans (cf. reference 83); and (iii) using the sirolimus prodrug temsirolimus to mitigate myocardial hypertrophy. Sirolimus compounds are known to control the complex network of cell growth by inhibiting both mTOR kinases (cf. reference 84). There is clinical evidence that sirolimus treatment can attenuate myocardial hypertrophy and improve diastolic pump function (cf. references 19, 20), as well as ameliorate rare genetic overgrowth syndromes in humans (cf. reference 21). In addition to the effects of human thrombomodulin expression in the graft (cf. references 5, 37), temsirolimus treatment may prevent the formation of thrombotic microangiopathic lesions even further by reducing collagen-in-duced platelet aggregation and by destabilizing platelet aggregates formed under shear stress conditions (cf. reference 85). In summary, this study demonstrates that consistent long-term life-supporting orthotopic xenogeneic heart transplantation in the most relevant preclinical model is feasible, facilitating clinical translation of xenogeneic heart transplantation.

<u>Consolidated materials and methods (relating to all of Examples 2 to 4)</u>

*Animals*

Experiments were carried out between February 2015 and August 2018. Fourteen juvenile pigs of cross-bred genetic background (German Landrace and Large White, blood group 0) served as donors for heart xenotransplantation.

All organs were homozygous for a1,3-galactosyltransferase knockout (GTKO), and heterozygous transgenic for human CD46 (hCD46) and human thrombomodulin (hTM) (cf. reference 37) (Revivicor and Institute of Molecular Animal Breeding and Biotechnology). Localization and stability of hCD46 and hTM expression were verified post mortem by immunohistochemistry (Fig. 15). Donor heart function and absence of valvular defects were evaluated seven days before transplantation by echocardiography. Fourteen male captive-bred baboons (P. anubis, blood groups B and AB) were used as recipients (German Primate Centre).

The study was approved by the local authorities and the Government of Upper Bavaria. All animals were treated in compliance with the Guide for the Care and Use of Laboratory Animals (US National Institutes of Health and German Legislation).

*Anaesthesia and analgesia*

Baboons were premedicated by intramuscular injection of ketamine hydrochloride 6-8 mg $kg^{-1}$ (ketavet 100 mg ml-1; Pfizer) and 0.3-0.5 mg $kg^{-1}$ midazolam (midazolam-ratiopharm; Ratiopharm). General anaesthesia was induced with an intravenous bolus of 2.0-2.5 mg $kg^{-1}$ propofol (propofol-lipuro 2%; B. Braun Melsungen) and 0.05 mg fentanyl (fentanyl-janssen 0.5 mg; Janssen-Cilag), and maintained with propofol ($0.16 \pm 0.06$ mg $kg^{-1}$ $min^{-1}$) or sevoflurane (1-2 vol% endexpiratory; sevorane, AbbVie) and bolus administrations of fentanyl (6-8 $\mu$g $kg^{-1}$, repeated every 45 min) as described elsewhere (cf. reference 11). Continuous infusion of fentanyl, ketamine hydrochloride and metamizole (novaminsulfon-ratiopharm 1 g per 2 ml; Ratiopharm) was applied postoperatively to ensure analgesia.

*Explantation and preservation of donor hearts*

Pigs were premedicated by intramuscular injection of ketamine hydrochloride 10-20 mg $kg^{-1}$, azaperone 10 mg $kg^{-1}$ (stresnil 40 mg $ml^{-1}$; Lilly Deutschland) and atropine sulfate (atropin-sulfat B. Braun 0.5 mg; B. Braun Melsungen). General anaesthesia was induced with an intravenous bolus of 20 mg propofol and 0.05 mg fentanyl and maintained with propofol (0.12 mg $kg^{-1}$ $min^{-1}$) and bolus administrations of fentanyl (2.5 $\mu$g $kg^{-1}$, repeated every 30 min).

After median sternotomy and heparinization (500 IU $kg^{-1}$), a small cannula was inserted into the ascending aorta, which was then cross-clamped distal of the cannula. In group I, the heart was perfused with a single dose of 20 ml $kg^{-1}$ crystalloid cardioplegic solution at 4 °C: custodiol HTK solution (Dr. Franz Kohler Chemie) was used for the hearts for animals 2, 4 and 5, Belzer's UW solution (Preservation Solutions) was used for the hearts for animals 1 and 3. The appendices of the right and left atrium were opened for decompression. The heart was then excised, submersed in cardioplegic solution and stored on ice.

In groups II and III, hearts were preserved as described previously (cf. reference 10), using 3.51 of an oxygenated albumin-containing hyperoncotic cardioplegic nutrition solution with hormones and erythrocytes at a temperature of 8 °C in a portable extracorporeal heart preservation system consisting of a pressure- and flow-controlled roller pump, an $O_2/CO_2$ exchanger, a leukocyte filter, an arterial filter and a cooler/heater unit.

After aortic cross-clamping, the heart was perfused with 600 ml preservation medium, excised and moved into the cardiac preservation system. A large cannula was introduced into the ascending aorta and the mitral valve was made temporarily incompetent to prevent left ventricular dilation; the superior vena cava was ligated; however, the inferior vena cava, pulmonary artery and pulmonary veins were left open for free outlet of perfusate. The heart was submersed in a reservoir filled with cold perfusion medium and antegrade coronary perfusion commenced through the already placed aortic cannula. The perfusion pressure was regulated at exactly 20 mm Hg. During implantation, the heart was intermittently perfused for 2 min every 15 min.

*Implantation technique*

The recipient's thorax was opened at the midline. Unfractionated heparin (500 IU $kg^{-1}$; heparin-natrium-25000-ratiopharm, Ratiopharm) was given and the heart-lung machine connected, using both venae cavae and the ascending aorta. CBP commenced and the recipient was cooled to 30°C in group I, and 34 °C in groups II and III. After cross-clamping the ascending aorta, the recipient's heart was excised at the atrial levels, both large vessels were cut. The porcine donor heart was transplanted using Shumway's and Lower's technique (cf. reference 8).

A wireless telemetric transmitter (Data Sciences International) was implanted in a subcutaneous pouch in the right medioclavicular line between the fifth and sixth rib. Pressure probes were inserted into the ascending aorta and the apex of the left ventricle, an electrocardiogram lead was placed in the right ventricular wall.

*Immunosuppressive regimen, anti-inflammatory and additive therapy.*

Immunosuppression was based on the previously published regimen (cf. reference 5), with C1 esterase inhibitor instead of cobra venom factor for complement inhibition (Table 5). Induction consisted of anti-CD20 antibody (mabthera; Roche Pharma), ATG (thymoglobuline, Sanofi-Aventis), and either an anti-CD40 monoclonal antibody (mouse/rhesus chimeric IgG4 clone 2C10R4, NIH Non-human Primate Reagent Resource, Mass Biologicals; courtesy of K. Reimann; animals 1-3, 5, 7, 8, 11-14) or humanized anti-CD40L PASylated Fab (XL-Protein and Wacker-Chemie; animals 4, 6, 9, 10). Maintenance immunosuppression consisted of mycophenolate mofetil (CellCept, Roche; trough level 2-3 μg $ml^{-1}$), either the anti-CD40 monoclonal antibody (animals 1-3, 5, 7, 8, 11-14) or anti-CD40L PASylated Fab (animals 4, 6, 9, 10), and methylprednisolone (urbasone soluble, Sanofi-Aventis). Anti-inflammatory therapy included an IL-6-receptor antagonist (RoActemra, Roche), TNF inhibitor (enbrel, Pfizer) and an IL-1-receptor antagonist (Kineret, Swedish Orphan Biovitrum). Additive therapy consisted of acetylsalicylic acid (aspirin, Bayer Vital), unfractionated heparin (heparin-natrium-25000-ratiopharm, Ratiopharm), C1 esterase inhibitor (berinert, CSL Behring), ganciclovir (cymevene, Roche), cefuroxime (cefuroxim, Hikma) and epoetin beta (neorecormon 5000IU, Roche P).

Starting from 10 mg $kg^{-1}$ per day, methylprednisolone was slowly reduced by 1 mg $kg^{-1}$ every 10 days in group I and II; in group III, methylprednisolone was tapered down to 0.1 mg $kg^{-1}$ within 19 days. Also in group III, temsirolimus (torisel, Pfizer) was added to the maintenance immunosuppression, administered as daily short intravenous infusions aiming at rapamycin trough levels of 5-10 ng $ml^{-1}$. Group III also received continuous intravenous antihypertensive medication with enalapril (Enahexal, Hexal AG, Holzkirchen, Germany) and metoprolol tartrate (Beloc, AstraZeneca), aiming at mean arterial pressures of 80 mm Hg and a heart rate of 100 b.p.m.

*Haemodynamic measurements*

After induction of general anaesthesia, a central venous catheter (Arrow International) was inserted in the left jugular vein and an arterial catheter (Thermodilution Pulsiocath; Pulsion Medical Systems) in the right femoral artery. Cardiac output and stroke volume were assessed by transpulmonary thermodilution and indexed to the body surface area of the recipient using the formula $0.083 \times B^{0.639}$ where B is body weight in kg. Measurements were taken after induction of anaesthesia and 60 min after termination of CPB in steady state and recorded with PiCCOWin software (Pulsion Medical Systems). All data were processed with Excel (Microsoft) and analysed with GraphPad Prism 7.0 (GraphPad Software).

*Quantification of left ventricular mass, left ventricular mass increase and fractional shortening*

Transthoracic echocardiographic examinations were carried out under analgosedation at regular intervals using an HP Sonos 7500 (HP) and a Siemens Acuson X300 (Siemens); midpapillary short axis views were recorded. Left ventricular end diastolic diameter (LVEDD) and left ventricular end systolic diameter (LVESD), interventricular septum thickness at end diastole (IVSd) and posterior wall thickness at end diastole (PWd) were measured; the mean of three measurements was used for further calculations and visualization (Excel and PowerPoint, Microsoft).

Left ventricular mass was calculated using equation (1), relative left ventricular mass increase and left ventricular (LV) fractional shortening (FS) was calculated using equations (2) and (3) according to previously published methods (cf. references 13, 12).

$$\text{LV mass (g)} = 0.8(1.04((\text{LVEDD} + \text{IVSd} + \text{PWd})^3 - \text{LVEDD})) + 0.6 \quad (1)$$

$$\text{LV mass increase (\%)} = ((\text{LV mass}_{end}/\text{LV mass}_{start}) - 1) \times 100 \quad (2)$$

$$\text{FS (\%)} = ((\text{LVEDD} - \text{LVESD})/\text{LVEDD}) \times 100 \quad (3)$$

*Necropsy and histology*

Necropsies and histology were performed at the Institute of Veterinary Pathology and the Institute of Pathology. Specimens were fixed in formalin, embedded in paraffin and plastic, sectioned and stained with haematoxylin and eosin.

*Histochemical analysis*

Cryosections (8 μm) were generated using standard histological techniques. Cardiomyocyte size was quantified as the cross-sectional area. In brief, 8-μm thick cardiac sections of the left ventricle were stained with Alexa Fluor 647-conjugated wheat germ agglutinin (Life Technologies) and the nuclear dye 4',6-diamidino-2-phenylindole (DAPI, Life Technologies). Images were acquired with a 63x objective using a Leica TCS SP8 confocal microscope; SMASH software (MATLAB, https://de.mathworks.com/products/matlab.html) was used to determine the average cross-sectional area of cardiomyocytes in one section (200-300 cells per section and 5- 8 sections per heart).

*Immunofluorescence staining*

Myocardial tissue biopsies were embedded in Tissue-Tek (Sakura Finetek) and stored frozen at -80 °C. For immunofluorescence staining, 5-μm cryosections were cut, air-dried for 30 to 60 min and stored at -20 °C until further analysis. The cryosections were fixed with ice-cold acetone, hydrated and stained using either one-step direct or two-step indirect immunofluorescence techniques. The following antibodies were used: rabbit anti-human C3b/c (DAKO), rabbit anti-human C4b/c-FITC (DAKO), goat anti-pig IgM (AbD Serotec), goat anti-human IgG-FITC (Sigma-Aldrich) and rabbit anti-human fibrinogen-FITC (DAKO). Secondary antibodies were donkey anti-goat IgG-Alexa Fluor 488 (Thermo Fisher Scientific), sheep anti-rabbit Cy3 (Sigma-Aldrich). Nuclear staining was performed using DAPI (Boehringer, Roche Diagnostics). The slides were analysed using a fluorescence microscope (DM14000B; Leica). Five to ten immunofluorescence pictures per marker were acquired randomly and the fluorescence intensity was quantified using ImageJ software, version 1.50i (https://imagej.nih.gov/ij/) on unmanipulated TIFF images. All pictures were taken under the same conditions to allow correct quantification and comparison of fluorescence intensities.

*Assessment of non-galactose-α1,3-galactose antibody levels*

Plasma levels of non-galactose-α1,3-galactose baboon IgM and IgG antibodies were measured by flow cytometry following the consensus protocol published previously (cf. reference 14). In brief, GTKO/hCD46/hTM porcine aortic endothelial cells were collected and suspended at 2 × 106 cells per ml in staining buffer (PBS containing 1% BSA). Plasma samples were heat-inactivated at 56 °C for 30 min and diluted 1:20 in staining buffer. Porcine aortic endothelial cells were incubated with diluted baboon plasma for 45 min at 4 °C. Cells were then washed with cold staining buffer and incubated with goat anti-human IgM-RPE (Southern Biotech) or goat anti-human IgG-FITC (Thermo Fisher) for 30 min at 4 °C. After rewashing with cold staining buffer, cells were resuspended in PBS, fluorescence was acquired on FACS LSRII (BD Biosciences) and data were analysed using FlowJo analysis software for detection of mean fluorescence intensity (MFI) in the FITC channel or in the RPE channel. Data were then plotted using Prism 7 (GraphPad Software).

*Western blot analysis*

For protein extraction, heart samples were homogenized in Laemmli sample buffer and the protein content was estimated using the bicinchoninic acid (BCA, Merck) protein assay. Then, 20 μg total protein was separated by 10% SDS-PAGE and transferred to PDVF membranes (Millipore) by electroblotting. Membranes were washed in Tris-buffered saline solution with 0.1% Tween-20 (Merck) (TBS-T) and blocked in 5% w/v fat-free milk powder (Roth) for 1 h at room temperature. Membranes were then washed again in TBS-T and incubated in 5% w/v BSA (Roth) of the appropriate primary antibody overnight at 4 °C. The following antibodies were used: rabbit anti-human p-mTOR (5536; Cell Signaling), rabbit anti-human mTOR (2983; Cell Signaling) and rabbit anti-human GAPDH (2118; Cell Signaling). After washing, membranes were incubated in 5% w/v fat-free milk powder with a horseradish peroxidase-labelled secondary antibody (goat anti-rabbit IgG; 7074; Cell Signaling) for 1 h at room temperature. Bound antibodies were detected using an enhanced chemiluminescence detection reagent (ECL Advance Western Blotting Detection Kit, GE Healthcare) and appropriate X-ray films (GE Healthcare). After detection, membranes were stripped (2% SDS, 62.5 mM Tris-HCl, pH 6.7, 100 mM β-mercaptoethanol) for 30 min at 70 °C and incubated with the appropriate second antibody.

*Immunohistochemical staining*

Myocardial tissue was fixed with 4% formalin overnight, paraffin-embedded and 3-μm sections were cut and dried. Heat-induced antigen retrieval was performed in Target Retrieval solution (S1699, DAKO) in a boiling water bath for 20 min for hCD46 and in citrate buffer, pH 6.0, in a steamer for 45 min for hTM, respectively. Immunohistochemistry was performed using the following primary antibodies: mouse anti-human CD46 monoclonal antibody (HM2103, Hycult Biotech) and mouse anti-human thrombomodulin monoclonal antibody (sc-13164, Santa Cruz). The secondary antibody was a biotinylated AffiniPure goat anti-mouse IgG (115-065-146, Jackson ImmunoResearch). Immunoreactivity was visualized using 3,3-diaminobenzidine tetrahydrochloride dihydrate (DAB) (brown colour). Nuclear counterstaining was done with haemalum (blue colour).

*Statistical analysis*

For survival data, Kaplan-Meier curves were plotted and the Mantel-Cox log-rank test was used to determine significant differences between groups. For haemodynamic data, statistical significance was determined using unpaired and paired two-sided Student's t-tests as indicated; data presented as single measurements with bars as group means ± s.d. For histochemical analysis, a one-way ANOVA with Holm-Sidak's multiple comparisons was used to determine statistical significance; data are mean ± s.d.; $P < 0.05$ was considered significant. No statistical methods were used to predetermine sample size. The experiments were not randomized and the investigators were not blinded to allocation during experiments and outcome assessment.

*Consolidated overview of immunosuppressive regimen, anti-inflammatory and additive therapy with corresponding doses and timing intervals*

| Agent | Dose | Timing |
|---|---|---|
| Induction | | |
| anti-CD20 Ab | 19 mg $kg^{-1}$, i.v. short infusion | days -7, 0, 7 and 14 |
| ATG | 5 mg $kg^{-1}$, continuously i.v. | days -2 and -1 |
| anti-CD40 mAb or anti-CD40L PASylated Fab* | 50 mg $kg^{-1}$ or 20 mg $kg^{-1}$; i.v. short infusion | days -1 and 0 |
| Maintenance | | |
| MMF | 40 mg $kg^{-1}$, continuously i.v. | daily, started on day -2 |
| anti-CD40 mAb or anti-CD40L PASylated Fab* | 50 mg $kg^{-1}$ or 20 mg $kg^{-1}$ i.v. short infusion | days 3, 7, 10, 14, 19, then weekly |
| methylprednisolone | 10 mg $kg^{-1}$, bolus i.v. | daily, tapered down |
| Anti-inflammatory therapy | | |
| IL6-receptor antagonist | 8 mg $kg^{-1}$, short infusion i.v. | monthly |
| TNFα inhibitor | 0.7 mg $kg^{-1}$, bolus s.c. | weekly |
| IL1-receptor antagonist | 1.3 mg $kg^{-1}$, bolus s.c. or i.v. | daily |
| Additive therapy | | |
| acetylsalicylic acid | 2 mg $kg^{-1}$, bolus i.v. | daily |
| unfractionated heparin | 20-40 U $kg^{-1}h^{-1}$, continuously i.v. | daily, started on day 5 |
| C1 esterase inhibitor | 17.5 U $kg^{-1}$, i.v. short infusion | days 0, 1, 7 and 14 |
| ganciclovir | 5 mg $kg^{-1}$, continuously i.v. | daily |
| cefuroxim | 50 mg $kg^{-1}$, continuously i.v. | daily, prophylaxis from day 0 to 5 |
| epoetin beta | 2,000 U, bolus s.c. or i.v. | days -7, 0 and if necessary |

**Table 5**: Immunosuppression was based on the previously published regimen (cf. reference 5), with C1 esterase inhibitor instead of cobra venom factor for complement inhibition. Starting from 10 mg $kg^{-1}$ per day, methylprednisolone was slowly reduced by 1 mg $kg^{-1}$ every 10 days in group I and II; in group III, methylprednisolone was reduced to 0.1 mg $kg^{-1}$ within 19 days. In group III, temsirolimus was added to the maintenance immunosuppression, administered as daily infusions (rapamycin trough levels: 5–10 ng $ml^{-1}$). Group III animals also received continuous antihypertensive medication (enalapril and metoprolol tartrate). Ab, antibody; mAb, monoclonal antibody; ATG, anti-thymocyte globulin; CMV, cytomegalovirus; IgG4, immunoglobulin G4; IL, interleukin; i.v., intravenous; MMF, mycophenolate mofetil; PASylated, conjugated with a long structurally disordered Pro-Ala-Ser amino acid chain; s.c., subcutaneous; TNFα, tumour necrosis factor α.

*Anti-CD40 monoclonal antibody: animals 1–3, 5, 7, 8, 11–14; anti-CD40L PASylated Fab: animals 4, 6, 9, 10.

**Example 5: Identification of human α1,2-fucosyltransferase gene in genetically modified pigs**

As mentioned above, the donor pigs used in Examples 1 to 4 were homozygous for alpha 1,3-galactosyltransferase knockout (GT-KO), and transgenic for hCD46/thrombomodulin (hTM) (Revivicor, Blacksburg, VA, USA and Institute for Molecular Animal Breeding and Biotechnology, Gene Center, Faculty of Veterinary Medicine, Ludwig-Maximilians-University, Munich, Germany). When nanopore-sequencing the genome of the transgenic pigs, it was unexpectedly found that the genome contains two copies of an additional transgene, i.e. of the human a1,2-fucosyltransferase gene, within an array of multiple hCD46 minigene copies (Figure 16). Expression of human a1,2-fucosyltransferase in the genetically modified heart/kidney/lung/liver of the xenogeneic donor organism in of the present invention may contribute to avoiding xenograft rejection after transplantation into primates (see also reference 112).

**Example 6: Generation of genetically modified pigs for cardiac xenotransplantation**

Heart transplantation is still the treatment of choice for patients with terminal cardiac failure, but the need for donated human organs far exceeds supply and alternatives are urgently required. Remarkable progress has been made in heterotopic abdominal (cf. reference 5) and life-supporting orthotopic cardiac xenotransplantation (cf. Examples 1 to 4) in baboons with triple-modified donor hearts (GTKO/hCD46/hTM). The GTKO/hCD46 background was supplied by Revivicor Inc., Blacksburg, VA; the hTM transgene controlled by the porcine THBD gene promoter was added (cf. reference 37)). However, this genetic background is not free of PERV-C, the structure of the hCD46 transgene locus has not been clarified, and the hTM transgene is at a different integration site, complicating the propagation of the animals by breeding. Therefore, GTKO/hCD46/hTM pigs will be established de novo on a PERV-C free background, and additionally the CMAH and B4GALNT2 genes will be knocked out.

In order to establish GTKO/hCD46/hTM pigs de novo, the following strategy will be pursued: The expression of transgenes randomly inserted into the genome is largely dependent on the context of neighboring chromatin. These so-called position effects result in variable transgene expression between different transgenic lines and often in inconsistent expression within one line. A strategy avoiding these disadvantages of random transgene insertion is the targeted placement of expression cassettes into so-called "safe harbor" loci that have an open configuration and ensure stable transgene expression.

Thus, first an advantageous safe harbor was identified. It was found that in pig endothelial wild-type cells the transcriptional level of CMAH is similar to that of B4GALNT2, whereas the transcriptional level of GGTA1 is approximately 5-fold higher. Moreover, it was found that in the porcine heart, expression of GGTA1 is 20- to 30-fold higher than expression of CMAH or B4GALNT2 (Figure 17). Also for porcine liver and adipose tissue, it was found that expression of GGTA1 is higher than expression of CMAH or B4GALNT2 (Figure 17). Thus, it was found that GGTA1 is highly expressed in multiple tissues and cell types, most importantly in endothelial cells, rendering it an advantageous safe harbor for transgene placement. Targeted integration of several xenoprotective transgenes into this safe harbor also prevents their segregation during breeding.

Accordingly, in order to establish GTKO/hCD46/hTM pigs de novo, the GGTA1, CMAH, and B4GALNT2 genes will be inactivated using CRISPR/Cas technology in male and female cells of PERV-C free piglets. An hCD46 minigene will be placed under the control of the endogenous porcine GGTA1 promoter followed by a selectable marker gene flanked by loxP sites and an hTM expression cassette with the porcine THBD promoter. Male and female primary kidney cells will be established with this basic set of genetic modifications and piglets will be generated by nuclear transfer and propagated by breeding. Further modifications can be added to this locus by Cre-driven cassette exchange with a bacterial artificial chromosome (BAC) vector engineered by recombineering to carry one or more additional expression cassettes (Fig. 18). The presence of the desired genetic modifications will be assessed by PCR. Expression studies on single-cell clones will be done by RT-PCR and immunofluorescence analyses.

**Example 7: Knockout of the growth hormone receptor (GHR) gene in pigs to adjust the size of organs for xenotransplantation**

Organ overgrowth in the recipient organism is a major problem when a genetically modified organ from a xenogeneic donor organism (e.g., a pig) is transplanted into a recipient organism (e.g., a baboon). Since growth control of xenotransplanted porcine organs is not only important in preclinical pig-to-baboon transplantation experiments but also for clinical transplantation studies, a genetic approach was sought to reduce the size of pigs in one step. This avoids long-term treatment of the recipient with an mTOR inhibitor, e.g. with rapamycin.

Loss-of-function mutations of the growth hormone receptor (GHR) gene in humans cause Laron syndrome, which is characterized by growth retardation and obesity, but reduced incidences of cancer and diabetes, and increased longevity (cf. reference 89). Thus, first the effects of a GHR knockout on the background of wild-type pigs were tested (cf. reference 86). A CRISPR/Cas9 system was designed and injected into porcine zygotes to mutate exon 3 of the GHR gene. Two heterozygous founder sows (GHR+/-) with a 1-bp or 7-bp insertion in GHR exon 3 (causing a reading frame shift; Fig. 19) were mated with wild-type boars (German Landrace) to obtain heterozygous F1 pigs, which were intercrossed to produce GHR-/- (KO) pigs. GHR-KO pigs lacked GHR (Fig. 20) and had markedly reduced serum insulin-like growth factor 1 (IGF1) levels (Fig. 21A) and reduced IGF-binding protein 3 (IGFBP3) activity, but increased IGFBP2 levels (Fig. 21B). Serum GH concentrations were significantly elevated compared with control pigs (Fig. 21C). GHR-KO pigs had a normal birth weight. Growth retardation became significant at the age of five weeks. At the age of six months, the body weight of GHR-KO pigs was reduced by 60 % compared with controls (Fig. 22A,B). Most organ weights of GHR-KO pigs were reduced proportionally to body weight (Fig. 23A,B). However, the weights of liver, kidneys, and heart were disproportionately reduced, while the relative brain weight was almost doubled (Fig. 23A,B). GHR-KO pigs had a markedly increased percentage of total body fat relative to body weight (Fig. 24A) and displayed transient juvenile hypoglycemia (Fig. 24B) along with decreased serum triglyceride and cholesterol levels (Fig. 24C,D). Analysis of insulin receptor related signaling in the liver of adult fasted pigs revealed increased phosphorylation of IRS1 and PI3K. In agreement with the loss of GHR, phosphorylation of STAT5 was significantly reduced (cf. reference 86).

Mating of a GHR-/- boar and a GHR-/- sow resulted in a litter of six healthy GHR-/- piglets, which had a smaller ($p<0.001$) birth weight (808±103 g) than GHR-/- offspring from heterozygous parents (1331±298 g). The former managed to catch up in body weight with the latter at an age of about 16 weeks (8.2±1.6 kg vs. 7.3±1.9 kg; $p=0.36$).

Since GHR-deficiency had no adverse effects on the background of wild-type pigs, the GHR-KO mutation was introduced also into the GGTA1-KO, hCD46/hTBM transgenic background that was successful for cardiac xenotransplantation (Figure 25). The first piglets were born and showed the the same degree of growth retardation as observed in GHR-KO pigs on a wild-type background. Specifically the body weight of two GHR-KO, GGTA1-KO, hCD46/hTBM transgenic pigs at age 9 months was 70.3 and 73.4 kg. Wild-type pigs have this body weight three months earlier. Circulating IGF1 levels were 23.5 ± 0.7 ng/mL compared with 33.8 ± 1.5 ng/mL in GHR-KO only pigs (Mean ± SD) and 215.5 ± 141.2 ng/mL in wild-type controls.

The above may have important implications for clinical xenotransplantation, since

i) the organs of farm pigs are too large for many patients;
ii) minipigs, which have a more appropriate organ size, have a high load of porcine endogenous retroviruses (PERVs) (cf. reference 87);
iii) the size of GHR-KO pigs and their organs can be adjusted by treatment with recombinant insulin-like growth factor 1 (IGF1) (reviewed in reference 88) to the needs of the recipient; and
iv) GHR deficiency has been shown to be beneficial in terms stress resistance of cells and tissues (cf. reference 89), eventually resulting in an increased life span of the xenograft.

With regard to aspect iii) above, it is pointed out that when the size of GHR-KO pigs and their organs is adjusted to the needs of the recipient organism, the beneficial size effect will not be negated when the GHR-deficient organ is transplanted into an IGF1 intact xeno-recipient. This is because GHR deficient donor pigs can be used at an older age (beyond the steepest phase of their growth curve). Moreover, locally produced IGF1 is more important for organ growth than liver-derived circulating IGF1 (cf. reference 114). Thus, in accordance with the present invention, the size of GHR deficient organs can be adjusted to the recipient's needs by treatment of the donor pig with IGF1.

## Materials and Methods

*Generation of GHR mutant pigs in wild-type background using CRISPR/Cas*

All animal procedures in this study were approved by the responsible animal welfare authority (Regierung von Oberbayern; permission 55.2-1-54-2532-70-12) and performed according to the German Animal Welfare Act and Directive 2010/63/EU on the protection of animals used for scientific purposes.

For CRISPR/Cas-assisted GHR gene disruption using a single guide RNA (sgRNA) specific for exon 3 sequence 5'-TTCATGCCACTGGACAGATG-3' (SEQ ID NO: 8), a corresponding oligonucleotide was cloned into the pEX-A-U6-gRNA vector as described previously (cf. reference 90). Cas9 mRNA and sgRNA were in vitro-transcribed using the Ambion Maxiscript SP6 kit (Thermo Fisher Scientific).

Porcine zygotes (German landrace background) were produced in vitro as described previously (cf. reference 91), and Cas9 mRNA (50 ng/μL) and sgRNA (100 ng/μL) was injected into their cytoplasm 8.5-9.5 h after in vitro fertilization. Recipient gilts were synchronized in the estrous cycle by oral administration of 4 mL Altrenogest (Regumate®; MSD Animal Health) for 15 days, followed by intramuscular injection of 750 IU ECG (Intergonan®; MSD Animal Health) and 750 IU HCG (Ovogest®; MSD Animal Health) after an additional 24 and 104 h, respectively. Embryo transfer was performed laparoscopically into one oviduct (cf. references 92, 93). Pregnancy was confirmed by ultrasonographic examination first on day 21 and again 4-6 weeks later.

Genomic DNA was isolated from tail tips of piglets using the Wizard DNA Extraction Kit (Promega). GHR mutations were detected by sequencing a GHR exon 3 PCR product obtained using primers GHR_Fw 5'-acc gct ctg aag ctg tga cc-3' (SEQ ID NO: 9) and GHR_Rv 5'-cac cct cag ata ctc tca tgc-3' (SEQ ID NO: 10). Based on the detected mutations, an XcmI restriction fragment length polymorphism assay was established, yielding fragments of 203 bp and 441 bp for wild-type GHR and a single fragment of 644 bp for the mutated GHR sequence.

Two female founder animals with different frameshift mutations were mated with wild-type boars to generate heterozygous F1 offspring. Heterozygous offspring of the same founder were intercrossed to obtain homozygous animals (GHR-KO) with the respective GHR mutations.

*Generation of GHR mutant pigs in GGTA1-KO, hCD46/hTBM transgenic background*

All animal procedures in this study were approved by the responsible animal welfare authority (Regierung von Oberbayern; permission 55.2-1-54-2532-70-12) and performed according to the German Animal Welfare Act and Directive 2010/63/EU on the protection of animals used for scientific purposes.

Primary cells were isolated from a female GGTA1-KO, hCD46/hTBM transgenic pig. Cells were co-transfected using a Cas9 expression plasmid (CMV-Cas9; cf. reference 111) and a plasmid transcribing the ttcatgccactggacaGAtgGGG (SEQ ID NO: 11) gRNA (PAM underlined), specific for the porcine GHR exon 3. Single cell clones were produced; PCR products from the porcine GHR gene were amplified with the primer pair 5'-gcacacttcagatgctacctaa (SEQ ID NO: 12) and 5'-cacatgctcaccctcagatac (SEQ ID NO: 13); Sanger sequencing of the PCR product was performed and analyzed for frame shift mutations by bi-directional Sanger sequencing, using the primers 5'-accgctctgaagctgtgacc (SEQ ID NO: 14) and 5'-caggagaccagagaccttatct (SEQ ID NO: 15). A cell clone carrying a bi-allelic constellation of deleterious mutations (ctggacagatggggt (SEQ ID NO: 16) / ctggacagatggggt (SEQ ID NO: 16) → ctgTCC-Atggggt (SEQ ID NO: 17) / ctggacag---gggt (SEQ ID NO: 18)) was used for somatic cell nuclear transfer.

Recipient gilts were synchronized in the estrous cycle by oral administration of 4 mL Altrenogest (Regumate®; MSD Animal Health) for 15 days, followed by intramuscular injection of 750 IU ECG (Intergonan®; MSD Animal Health) and 750 IU HCG (Ovogest®; MSD Animal Health) after an additional 24 and 104 h, respectively. Embryo transfer was performed laparoscopically into one oviduct (cf. references 92, 93). Pregnancy was confirmed by ultrasonographic examination first on day 21 and again 4-6 weeks later.

Genomic DNA was isolated from tail tips of piglets using the Wizard DNA Extraction Kit (Promega). GHR mutations were detected by sequencing a GHR exon 3 PCR product obtained using primers GHR_Fw 5'-acc gct ctg aag ctg tga cc-3' (SEQ ID NO: 9) and GHR_Rv 5'-cac cct cag ata ctc tca tgc-3' (SEQ ID NO: 10). Based on the detected mutations, an XcmI restriction fragment length polymorphism assay was established, yielding fragments of 203 bp and 441 bp for wild-type GHR and a single fragment of 644 bp for the mutated GHR sequence.

Four female GTKO.hCD46.hTBM.GHRKO were born and confirmed to carry the respective mutation constellation by Sanger sequencing. Two of the four piglets died on the first postnatal day, the other two pigs were raised to adulthood without clinical problems.

*Ligand immunostaining of porcine GHR (wild-type background only)*

Liver and kidney tissue of GHR-KO and control pigs was fixed overnight in 4% formaldehyde and routinely embedded in paraffin. Paraffin sections were dewaxed, and endogenous peroxidase and biotin were blocked with 1% $H_2O_2$ in Tris-buffered saline (TBS) for 15 min and by using the avidin/biotin blocking kit (no. SP-2001; Vector Laboratories), respectively. After blocking with 0.5% fish gelatin for 30 min, the slides were incubated in 0.5 μg/mL recombinant rat GH (no. 16343667, ImmunoTools) in 0.2% fish gelatin solution overnight at 4 °C. They were then washed 3 times for 5 min in TBS and incubated in goat anti-rat GH polyclonal antibody solution (dilution 1:2,400, no. AF1566, R&D Systems) for 6 h at room temperature. After 3 washing steps in TBS (10 min each), the slides were incubated in biotinylated rabbit anti-goat IgG solution (dilution 1:100, no. BA-5000, Vector Laboratories) for 1 h at room temperature, washed 3 times for 10 min in TBS, and finally incubated with horseradish peroxidase-labelled avidin biotin complex for 30 min (no. PK-6100, VECTASTAIN Elite ABC-Peroxidase kit, Vector Laboratories). Immunoreactivity was visualized using 3,3'-diaminobenzidine tetrahydrochloride dihydrate (DAB) (brown color). Nuclear counterstaining was performed with Mayer's hemalum (blue color). As specificity controls for the ligand immunohistochemistry assay, rat GH as well as rat GH plus primary antibody were omitted.

*Blood collection*

Animals were fasted overnight (16 h) before blood collection from the jugular vein. After clotting for 30 min at room temperature, serum was separated by centrifugation (1200 x g) for 20 min at 6 °C and stored at -80 °C until analysis. For repeated blood sampling required to analyze GH secretion profiles, central venous catheters (Argon Careflow™; Merit Medical) were surgically inserted through the external ear vein. Blood samples were collected every 30 min for 9 h, starting at 11 a.m., and processed for serum collection as described above. During the test, the animals were fed regularly and had free access to water.

*Clinical chemistry, hormone assays, IGFBP ligand blot analysis (wild-type background only)*

Clinical-chemical parameters in serum were determined using a Cobas 311 system (Hitachi) or an AU480 autoanalyzer (Beckman-Coulter) and adapted reagents from Roche Diagnostics or Beckman-Coulter, respectively. Serum GH concentrations were measured by an ELISA for rat/mouse GH (EZRMGH-45K; Merck) that cross-reacts with porcine GH. To calculate the area under the GH curve, values below the quantification limit of the assay (<0.07 ng/mL) were arbitrarily set to 0.07 ng/mL. IGF1 levels in serum were determined by RIA after dissociation of IGF1 from IGFBPs by acidification and blocking the IGF1 binding sites with an excess of IGF2 (cf. reference 94). IGFBP ligand blot analysis of serum samples was performed as described previously (cf. reference 95) using serial dilutions of recombinant human IGFBP3 (41/38 kDa), IGFBP2 (32 kDa), IGFBP5 (29 kDa) and IGFBP4 (24 kDa) for quantification. Plasma insulin was determined using a species-specific RIA (Merck Millipore) as previously described (cf. reference 96). Blood glucose levels were determined immediately using a Precision Xceed® glucometer and Precision XtraPlus® test strips (Abbott) (cf. reference 97). Serum leptin levels were measured using a multi-species leptin radioimmunoassay (Cat. # XL-85K; EMD Millipore Corporation) that has been validated for porcine samples (cf. reference 98).

*Growth parameters and body composition*

Body weight and body length (distance between tip of the snout and tail root in straightened animals) of GHR-KO and control pigs were determined at weekly intervals. Relative body length was calculated by dividing the body length by the cube root of the body weight to retain the same dimensions. Since not all animals could be weighed/measured at exactly the same ages, raw data were adjusted by linear interpolation to defined ages/time points.

The percentage of total body fat was determined in 6-month-old GHR-KO and control pigs using dual-energy X-ray absorptiometry (DXA; Lunar iDXA, GE Healthcare) as previously described (cf. reference 99). In addition, magnetic resonance imaging (MRI; Magnetom Open, Siemens) (cf. reference 100) was performed to visualize and determine the muscle to fat ratio as the area of longissimus dorsi muscle divided by the area of its overlying back fat at the last rib.

*Necropsy (wild-type background only)*

GHR-KO and control pigs were euthanized at 6 months of age under anesthesia by intravenous injection of T61® (Intervet) and immediately subjected to necropsy. Organs were dissected and weighed to the nearest mg. Tissue samples were collected as described previously (cf. reference 101) and routinely fixed in neutral buffered formalin solution (4%) for 24 h or frozen immediately on dry ice and stored at -80 °C for molecular profiling. Formalin-fixed tissue specimens were embedded in paraffin. Muscle sections were stained with hematoxylin and eosin (H&E).

*Immunoblot analysis of signaling cascades (wild-type background only)*

The concentrations and phosphorylation status of GHR-related signaling molecules in the liver were evaluated by Western blot analyses as described previously (cf. reference 102). Briefly, liver tissue samples were homogenized in Laemmli extraction buffer, and the protein content was determined by the bicinchoninic acid protein assay. Forty micrograms of total protein was separated by SDS-PAGE and transferred to PVDF membranes (Millipore) by electro-blotting. Membranes were washed in TBS with 0.1% Tween-20 and blocked in 5% w/v fat-free milk powder (Roth) for 1 h. The membranes were then washed again and incubated in 5% w/v BSA (Roth) solution with the appropriate primary antibodies overnight at 4 °C. The antibodies and concentrations used are listed in Suppl. Table 1. After washing, the membranes were incubated in 5% w/v fat-free milk powder solution with the secondary antibody (donkey anti-rabbit; 1:2000; GE Healthcare) for 1 h. Bound antibodies were detected using the ECL Advance Western Blotting Detection Kit (GE Healthcare) and appropriate films from the same supplier. Band intensities were quantified using the ImageQuant software package (GE Healthcare).

*Statistical analyses (wild-type background only)*

Longitudinal data for body weight and body length or relative body length respectively were analyzed using PROC MIXED (SAS 8.2), taking the effects of pig line (#2529; #2533), group (GHR-KO; control), sex, age, and interaction group*age into account. Least squares means (LSMs) and standards errors (SEs) of LSMs were calculated for group*age and compared using Student's t-test. Data for glucose homeostasis and serum lipid concentrations were analyzed using PROC GLM (SAS 8.2), taking the effects of group, sex, age and the interaction group*age into account. LSMs and SEs were calculated for group*age and compared using Student's t-test. Body composition, organ weight and clinical-chemical data were analyzed using PROC GLM taking the effects of group and sex into account. LSMs and SEs were calculated for groups and compared using Student's t-test. IGFBP ligand blot and Western immunoblot data were evaluated for significant differences between GHR-KO and control pigs using the Mann-Whitney U test.

## INDUSTRIAL APPLICABILITY

The methods, processes and products of the invention are commercially useful. For example, the compositions in accordance with the invention can be sold to prolong the survival of a primate that has been transplanted a heart, kidney, lung or liver from a xenogeneic mammal. The living primate in accordance with the invention can be used to find novel compositions and/or treatment regimens for further prolonging the survival of a primate that has been transplanted a xenogeneic heart, kidney, lung or liver. The donor organisms for xenogeneic organ transplants can be used to obtain organs for xeno-transplantation. Thus, the invention is industrially applicable.

## REFERENCES

1. Eurotransplant. Annual Report 2016. 104 (Eurotransplant International Foundation, 2017).
2. Lund, L. H. et al. The Registry of the International Society for Heart and Lung Transplantation: Thirty-fourth Adult Heart Transplantation Report-2017; Focus Theme: Allograft ischemic time. J. Heart Lung Transplant. 36, 1037-1046 (2017).
3. Rossano, J. W. et al. The Registry of the International Society for Heart and Lung Transplantation: Twentieth Pediatric Heart Transplantation Report-2017; Focus Theme: Allograft ischemic time. J. Heart Lung Transplant. 36, 1060-1069 (2017).
4. Kirklin, J. K. et al. Eighth annual INTERMACS report: Special focus on framing the impact of adverse events. J. Heart Lung Transplant. 36, 1080-1086 (2017).
5. Mohiuddin, M. M. et al. Chimeric 2C10R4 anti-CD40 antibody therapy is critical for long-term survival of GTKO.hCD46.hTBM pig-to-primate cardiac xenograft. Nat Commun 7, 11138 (2016).
6. Cooper, D. K. et al. Report of the Xenotransplantation Advisory Committee of the International Society for Heart and Lung Transplantation: the present status of xenotransplantation and its potential role in the treatment of end-stage cardiac and pulmonary diseases. J. Heart Lung Transplant. 19, 1125-1165 (2000).
7. Mohiuddin, M. M., Reichart, B., Byrne, G. W. & McGregor, C. G. A. Current status of pig heart xenotransplantation. International Journal of Surgery 23, 234-239 (2015).
8. LOWER, R. R. & SHUMWAY, N. E. Studies on orthotopic homotransplantation of the canine heart. Surg Forum 11, 18-19 (1960).
9. Binder, U. & Skerra, A. PASylation®: A versatile technology to extend drug delivery. Current Opinion in Colloid & Interface Science 31, 10-17 (2017).
10. Steen, S., Paskevicius, A., Liao, Q. & Sjöberg, T. Safe orthotopic transplantation of hearts harvested 24 hours after brain death and preserved for 24 hours. Scand. Cardiovasc. J. 50, 193-200 (2016).
11. Mayr, T. et al. Hemodynamic and perioperative management in two different preclinical pig-to-baboon cardiac xenotransplantation models. Xenotransplantation 24, (2017).
12. Lang, R. M. et al. Recommendations for chamber quantification: a report from the American Society of Echocardiography's Guidelines and Standards Committee and the Chamber Quantification Writing Group, developed in conjunction with the European Association of Echocardiography, a branch of the European Society of Cardiology. Journal of the American Society of Echocardiography : official publication of the American Society of Echocardiography 18, 1440-1463 (2005).
13. Devereux, R. B. et al. Echocardiographic assessment of left ventricular hypertrophy: comparison to necropsy findings. The American Journal of Cardiology 57, 450-458 (1986).
14. Azimzadeh, A. M. et al. Development of a consensus protocol to quantify primate anti-non-Gal xenoreactive antibodies using pig aortic endothelial cells. Xenotransplantation 21, 555-566 (2014).
15. Tanabe, T. et al. Role of Intrinsic (Graft) Versus Extrinsic (Host) Factors in the Growth of Transplanted Organs Following Allogeneic and Xenogeneic Transplantation. Am. J. Transplant. 17, 1778-1790 (2017).
16. Byrne, G. W. & McGregor, C. G. A. Cardiac xenotransplantation: progress and challenges. Curr Opin Organ Transplant 17, 148-154 (2012).
17. Kumar, R. S. & Vimala, J. Steroid-Induced Hypertrophic Cardiomyopathy. APJOC 1-5 (2010).
18. Saxton, R. A. & Sabatini, D. M. mTOR Signaling in Growth, Metabolism, and Disease. Cell 168, 960-976 (2017).
19. Imamura, T. et al. Everolimus Attenuates Myocardial Hypertrophy and Improves Diastolic Function in Heart Transplant Recipients. Int Heart J 57, 204-210 (2016).
20. Paoletti, E. mTOR Inhibition and Cardiovascular Diseases: Cardiac Hypertrophy. Transplantation (2017). doi:10.1097/TP.0000000000001691
21. Manning, B. D. Game of TOR - The Target of Rapamycin Rules Four Kingdoms. N. Engl. J. Med. 377,1297-1299 (2017).
22. Schuurman, H.-J. Microbiological safety of clinical xenotransplantation products: monitoring strategies and regulatory aspects. A commentary. Xenotransplantation 23, 440-443 (2016).
23. Fischer, K. et al. Efficient production of multi-modified pigs for xenotransplantation by 'combineering', gene stacking and gene editing. Sci Rep 6:29081 (2016). doi: 10.1038/srep29081
24. Cooper, D.K., Novitzky, D., Becerra, E., and Reichart, B.. Are there indications for heterotopic heart transplantation in 1986? A 2- to 11-year follow-up of 49 consecutive patients undergoing heterotopic heart transplantation. The Thoracic and Cardiovascular Surgeon 34, 300-304 (1986).
25. Abicht, J., Mayr, T., Reichart, B., Buchholz, S., Werner, F., Lutzmann, I., Schmoeckel, M., Bauer, A., Thormann, M., Langenmayer, M.C., et al. Preclinical heterotopic intrathoracic heart Xenotransplantation: a possibly useful clinical technique. Xenotransplantation, doi: 10.1111/xen.12213 (2015). [Epub ahead of print].
26. Wynyard S., Nathu D., Garkavenko O., Denner J. Elliot R.. Microbiologic safety of the first clinical pig islet xenotransplantation trial in New Zealand. Xenotransplantation 21, 309-323 (2014)
27. Li S, et ol. (2014) Dual fluorescent reporter pig for Cre recombination: transgene placement at the ROSA26 locus. PLoS one 9(7):el02455.
28. Petersen B, et al. (2016) Efficient production of biallelic GGTA1 knockout pigs by cytoplasmic microinjection of CRISPR/Cas9 into zygotes. Xenotransplantation 23(5):338-346.
29. Cooper DK, Ekser B, Ramsoondar J, Phelps C, & Ayares D (2016) The role of genetically engineered pigs in xenotransplantation research. The Journal of pathology 238(2):288-299.
30. Rieblinger B, et al. (2018) Strong xenoprotective function by single-copy transgenes placed sequentially at a permissive locus. Xenotransplantation.
31. Kourtzelis I, Magnusson PU, Kotlabova K, Lambris JD, & Chavakis T (2015) Regulation of instant blood mediated inflammatory reaction (IBMIR) in pancreatic islet xenotransplantation: points for therapeutic inventions. Advances in experimental medicine and biology 865:171-188.
32. Bulato C, Radu C, & Simoni P (2012) Studies on coagulation incompatibilities for xenotransplantation. Methods of molecular biology (Clifton, N.J.) 885:71-89.
33. Cowan PJ & Robson SC (2015) Progress towards overcoming coagulopathy and hemostatic dysfunction associates with xenotransplantation. Int J Surg 23(Pt B):296-300.
34. Roussel JC, et al. (2008) Pig thrombomodulin binds human thrombin but is a poor cofactor for activation of human protein C and TAFI. American journal of transplantation: official journal of the American Society of Transplantation and the American Society of Transplant Surgeons 8(6):1101-1112.
35. Petersen B, et al. (2009) Pigs transgenic for human thrombomodulin have elevated production of activated protein C. Xenotransplantation 16(6):486-495.
36. Yazaki S, et al. (2012) Production of cloned pigs expressing human thrombomodulin in endothelial cells. Xenotransplantation 19(2):82-91,
37. Wuensch A, et al. (2014) Regulatory sequences of the porcine THBD gene facilitate endothelial-specific expression of bioactive human thrombomodulin in single- and multitransgenic pigs. Transplantation 97(2):138-147.
38. Byrne GW, McGregor CG, & Breimer ME (2015) Recent investigations into pig antigen and anti-pig antibody expression. Int J Surg 23(Pt B):223-228.
39. Denner J & Tonjes RR (2012) Infection barriers to successful xenotransplantation focusing on porcine endogenous retroviruses. Clin Microbiol Rev 25(2):318-343.
40. Phelps CJ, et al. (2003) Production of alpha 1,3-galactosyltransferase-deficient pigs. Sciene 299(5605):411-414.
41. Klymiuk N, Aigner B, Brem G, & Wolf E (2010) Genetic modification of pigs as organ donors for xenotransplantation. Molecular reproduction and development 77(3):209-221.
42. Hauschild J, et al. (2011) Efficient generation of a biallelic knockout in pigs using zinc-finger nucleases. Proceedings of the National Academy of Sciences of the United States of America 108(29):12013-12017.
43. Kwon DN, et al. (2013) Production of biallelic CMP-Neu5Ac hydroxylase knock-out pigs. Scientific reports 3:1981.
44. Lutz AJ, et al. (2013) Double knockout pigs deficient in N-glycolylneuraminic acid and galactose alpha-1,3-galactose reduce the humoral barrier to xenotransplantation. Xenotransplantation 20(1):27-35.
45. Burlak C, et al. (2014) Reduced binding of human antibodies to cells from GGTA1/CMAH KO pigs. American journal of transplantation: official journal of the American Society of Transplantation and the American Society of Transplant Surgeons 14(8):1895-1900.
46. Miyagawa S, et al. (2015) Generation of alpha1,3-galactosyltransferase and cytidine monophospho-N-acetylneuraminic acid hydroxylase gene double-knockout pigs. J Reprod Dev 61(5):449-457.
47. Estrada JL, et al. (2015) Evaluation of human and non-human primate antibody binding to pig cells lacking GGTA1/CMAH/beta4GalNT2 genes. Xenotransplantation 22(3):194-202.
48. Griesemer A, Yamada K, _& Sykes M (2014) Xenotransplantation: immunological hurdles and progress towards tolerance. Immunol Rev 258(1)241-258.
49. Vadori M & Cozzi E (2015) The immunological barriers to xenotransplantation. Tissue antigens 86(4):239-253.
50. Dawson JR, Vidal AC, & Malyguine AM (2000) Natural killer cell-endothelial cell interactions in xenotransplantation. Immunological research 22(2-3):165-176.
51. Weiss EH, et al. (2009) HLA-E/human beta2-microglobulin transgenic pigs: protein against xenogeneic human anti-pig natural killer cell cytotoxicity. Transplantation 87(1).35-43.
52. Bartlett ST, et al. (2016) Report from IPITA-TTS Opinion Leaders Meeting on the Future of beta-Cell Replacement. Transplantation 100 Suppl 2:S1-44.
53. Aikin RA (2012) How to kill two birds with one transgenic pig. Diabetes 61(6):1348-1349.
54. Klymiuk N, et al. (2012) Xenogafted islet cell cluster from INSLEA29Y transgenic pig rescue diabetes and prevent immune rejection in humanized mice. Diabetes 61(6):1527-1532.
55. Wolf-van Buerck L, et al. (2017) LEA29Y expression in transgenic neonatal porcine islet-like cluster promotes long-lasting xenograft survival in humanized mice without immunosuppressive therapy. Scientific reports 7(1):3572.
56. Klose R, et al. (2005) Expression of biologically active human TRAIL in transgenic pigs. Transplantation 80(2):222-230.
57. Kemter E, et al. (2012) Human TNF-related apoptosis-inducing ligand-expressing dendritic cells from transgenic pigs attenuate human xenogeneic T cell responses. Xenotransplantation 19(1):40-51.
58. Seol JG, et al. (2010) Production of transgenic cloned miniature pigs with membrane-bound human Fas ligand (FasL) by somatic call nuclear transfer. Nature Precedings <http://hdl.handle.net/10101/npre.2010.4539.1>
59. Taylor FB, Jr., Peer GT, Lockhart MS, Ferrell G, & Esmon CT (2001) Endothelial cell protein C receptor plays an important role in protein C activation in vivo. Blood 97(6):1685-1688.
60. Iwase H, et al. (2014) Regulation of human platelet aggregation by genetically modified pig endothelial cells and thrombin inhibition. Xenotransplantation 21(1):72-83.
61. Ji H, et al. (2015) Pig BMSCs Transfected with Human TFPI Combat Species Incompatibility and Regulate the Human TF Pathway in Vitro and in a Rodent Model. Cellular physiology and biochemistry: international journal of experimental cellular physiology, biochemistry, and pharmacology 36(1):233-249.
62. Lee HJ, Lee BC, Kim YH, Paik NW, & Rho HM (2011) Characterization of transgenic pigs that express human decay accelerating factor and cell membrane-tethered human tissue factor pathway inhibitor. Reproduction in domestic animals = Zuchthygiene 46(2):325-332.
63. Wijkstrom M, et al. (2015) Glucose metabolism in pigs expressing human genes under an insulin promoter. Xenotransplantation 22(1):70-79.
64. Wheeler DG, et al. (2012) Transgenic swine: expression of human CD39 protects against myocardial injury. Journal of molecular and cellular cardiology 52(2):958-961.
65. Le Bas-Bernardet S, et al. (2015) Bortezomib, C1-inhibitor and plasma exchange do not prolong the survival of multi-transgenic GalT-KO pig kidney xenografts in baboons. American journal of transplantation: official journal of the American Society of Transplantation and the American Society of Transplant Surgeons 15(2):358-370.
66. Pintore L, et al. (2013) Clinicopathological findings in non-human primate recipients of porcine renal xenografts: quantitative and qualitative evaluation of proteinuria. Xenotransplantation 20(6):449-457.
67. Bottino R, et al. (2014) Pig-to-monkey islet xenotransplantation using multi-transgenic pigs. American journal of transplantation: official journal of the American Society of Transplantation and the American Society of Transplant Surgeons 14(10):2275-2287.
68. Ahrens HE, et al. (2015) siRNA mediated knockdown of tissue factor expression in pigs for xenotransplantation. American journal of transplantation: official journal of the American Society of Transplantation and the American Society of Transplant Surgeons 15(5):1407-1414.
69. Paris LL, et al. (2015) Reduced human platelet uptake by pig livers deficient in the asialoglycoprotein receptor 1 protein. Xenotransplantation 2(3):203-210.
70. Oropeza M, et al. (2009) Transgenic expression of the human A20 gene in cloned pigs provides protection against apoptotic and inflammatory stimuli. Xenotransplantation 16(6):522-534.
71. Pertersen B, et al. (2011) Transgenic expression of human heme oxygenase-1 in pigs confers resistance against xenograft during ex vivo perfusion of porcine kidneys. Xenotransplantation 18(6) :355-368.
72. Fishman JA, Scobie L, & Takeuchi Y (2012) Xenotransplantation-associates infectious risk: a WHO consultation. Xenotransplantation 19(2):72-81.
73. Mueller NJ, Takeuchi Y, Mattiuzzo G, & Scobie L (2011) Microbial safety in xenotransplantation. Current opinion in organ transplantation 16(2):201-206.
74. Fishman JA (2014) Assessment of infectious risk in clinical xenotransplantation: the lesson for clinical allotransplantation. Xenotransplantation 21(4):307-308.
75. Morozov VA, et al. (2015) Extended microbiological characterization of Göttingen minipigs in the context of xenotransplantation: detection and vertical transmission of hepatitis E virus. PloS one 10(10):e0139893.
76. Denner J (2013) Immunising with the transmembrane envelope proteins of different retroviruses including HIV-1: a comparative study. Hum Vaccin Immunother 9(3):462-470.
77. Dieckhoff B, et al. (2008) Knockdown of porcine endogenous retrovirus (PERV) expression by PERV-specific shRNA in transgenic pigs. Xenotransplantation 15(1):36-45.
78. Ramsoondar J, et al. (2009) Production of transgenic pigs that express porcine endogenous retrovirus small interfering RNAs. Xenotransplantation 16(3):164-180.

79. Yang L, et al. (2015) Genome-wide inactivation of porcine endogenous retroviruses (PERVs). Science 350(6264):1101-1104.

80. Niu D, et al. (2017) Inactivation of porcine endogenous retroviruses in pigs using CRISPR-Cas9. Science 357(6357):1303-1307.

81. Denner J (2017) Paving the Path toward Porcine Organs for Transplantation. The New England journal of medicine 377(19):1891-1893.

82. Denner J (2017) Advances in organ transplant from pigs. Science 357(6357):1238-1239.

83. Lesnik, J.J., Singh, G. K., Balfour, I.C. & Wall, D.A. Steroid-induced hypertrophic cardiomyopathy following stem cell transplantation in a neonate: a case report. Bone Marrow Transplant. 27, 1105-1108 (2001).

84. Saxon, R. A. / Sabatini, D. M. mTOR signaling in growth, metabolism, and disease. Cell 168, 960-976 (2017).

85. Asian, J.E., Tormoen, G.W., Loren, C.P., Pang, J. & McCarty, O.J. S6K1 and mTOR regulate Rac1-driven platelet activation and aggregation. Blood 118, 3129-3136 (2011).

86. Hinrichs, A., B. Kessler, M. Kurome, A. Blutke, E. Kemter, M. Bernau, A.M. Scholz, B. Rathkolb, S. Renner, S. Bultmann, H. Leonhardt, M.H. de Angelis, H. Nagashima, A. Hoeflich, W.F. Blum, M. Bidlingmaier, R. Wanke, M. Dahlhoff, and E. Wolf, Growth hormone receptor-deficient pigs resemble the pathophysiology of human Laron syndrome and reveal altered activation of signaling cascades in the liver. Mol Metab, 2018. 11: p. 113-128.

87. Bittmann, I., D. Mihica, R. Plesker, and J. Denner, Expression of porcine endogenous retroviruses (PERV) in different organs of a pig. Virology, 2012. 433(2): p. 329-36.

88. Cohen, J., S. Blethen, J. Kuntze, S.L. Smith, K.G. Lomax, and P.M. Mathew, Managing the child with severe primary insulin-like growth factor-1 deficiency (IGFD): IGFD diagnosis and management. Drugs R D, 2014.14(1): p. 25-9.

89. Guevara-Aguirre, J., P. Balasubramanian, M. Guevara-Aguirre, M. Wei, F. Madia, C.W. Cheng, D. Hwang, A. Martin-Montalvo, J. Saavedra, S. Ingles, R. de Cabo, P. Cohen, and V.D. Longo, Growth hormone receptor deficiency is associated with a major reduction in pro-aging signaling, cancer, and diabetes in humans. Sci Transl Med, 2011. 3(70): p. 70ra13.

90. Dahlhoff, M., Gaborit, N., Bultmann, S., Leonhardt, H., Yarden, Y., Schneider, M.R., 2017. CRISPR-assisted receptor deletion reveals distinct roles for ERBB2 and ERBB3 in skin keratinocytes. FEBS Journal 284:3339-3349.

91. Umeyama, K., Honda, K., Matsunari, H., Nakano, K., Hidaka, T., Sekiguchi, K., et al., 2013. Production of diabetic offspring using cryopreserved epididymal sperm by in vitro fertilization and intrafallopian insemination techniques in transgenic pigs. Journal of Reproduction and Development 59:599-603.

92. Kurome, M., Kessler, B., Wuensch, A., Nagashima, H., Wolf, E., 2015. Nuclear transfer and transgenesis in the pig. Methods in Molecular Biology (Clifton, NJ) 1222:37-59.

93. Besenfelder, U., Modl, J., Muller, M., Brem, G., 1997. Endoscopic embryo collection and embryo transfer into the oviduct and the uterus of pigs. Theriogenology 47:1051-1060.

94. Blum, W.F., Breier, B.H., 1994. Radioimmunoassays for IGFs and IGFBPs. Growth Regulation 4(Suppl 1):11-19.

95. Wirthgen, E., Hoflich, C., Spitschak, M., Helmer, C., Brand, B., Langbein, J., et al., 2016. Quantitative Western ligand blotting reveals common patterns and differential features of IGFBP-fingerprints in domestic ruminant breeds and species. Growth Hormone & IGF Research 26:42-49.

96. Renner, S., Fehlings, C., Herbach, N., Hofmann, A., von Waldthausen, D.C., Kessler, B., et al., 2010.Glucose intolerance and reduced proliferation of pancreatic beta-cells in transgenic pigs with impaired glucose-dependent insulinotropic polypeptide function. Diabetes 59:1228-1238.

97. Blutke, A., Renner, S., Flenkenthaler, F., Backman, M., Haesner, S., Kemter, E., et al., 2017. The Munich MIDY Pig Biobank - a unique resource for studying organ crosstalk in diabetes. Molecular Metabolism 6:931-940.

98. Papadopoulos, G.A., Erkens, T., Maes, D.G., Peelman, L.J., van Kempen, T.A., Buyse, J., et al., 2009. Peripartal feeding strategy with different n-6:n-3 ratios in sows: effect on gene expression in backfat white adipose tissue postpartum. British Journal of Nutrition 101:197-205.

99. Kremer, P.V., Fernandez-Figares, I., Forster, M., Scholz, A.M., 2012. In vivo body composition in autochthonous and conventional pig breeding groups by dual-energy X-ray absorptiometry and magnetic resonance imaging under special consideration of Cerdo Iberico. Animal: An International Journal of Animal Bioscience 6:2041-2047.

100. Kremer, P.V., Forster, M., Scholz, A.M., 2013. Use of magnetic resonance imaging to predict the body composition of pigs in vivo. Animal: An International Journal of Animal Bioscience 7:879-884.

101. Albl, B., Haesner, S., Braun-Reichhart, C., Streckel, E., Renner, S., Seeliger, F., et al., 2016. Tissue sampling guides for porcine biomedical models. Toxicologic Pathology 44:414-420.

102. Streckel, E., Braun-Reichhart, C., Herbach, N., Dahlhoff, M., Kessler, B., Blutke, A., et al., 2015. Effects of the glucagon-like peptide-1 receptor agonist liraglutide in juvenile transgenic pigs modeling a pre-diabetic condition. Journal of Translational Medicine 13:73.

103. Yang H, Wu Z. Genome Editing of Pigs for Agriculture and Biomedicine. Front Genet. 2018 Sep 4;9:360. doi:

10.3389/fgene.2018.00360. eCollection 2018. Review. PubMed PMID: 30233645; PubMed Central PMCID: PMC6131568.

104. Fischer K, Kind A, Schnieke A. Assembling multiple xenoprotective transgenes in pigs. Xenotransplantation. 2018 Nov;25(6):e12431. doi: 10.1111/xen.12431. Epub 2018 Jul 28. Review. PubMed PMID: 30055014.

105. Ryu J, Prather RS, Lee K. Use of gene-editing technology to introduce targeted modifications in pigs. J Anim Sci Biotechnol. 2018 Jan 29;9:5. doi: 10.1186/s40104-017-0228-7. eCollection 2018. Review. PubMed PMID: 29423214; PubMed Central PMCID: PMC5787920.

106. Li, B., Ruotti, V., Stewart, R. M., Thomson, J. A. & Dewey, C. N. RNA-Seq gene expression estimation with read mapping uncertainty. Bioinformatics 26, 493-500, doi:10.1093/bioinformatics/btp692 (2010).

107. Klymiuk N, Seeliger F, Bohlooly-Y M, Blutke A, Rudmann DG, Wolf E. Tailored Pig Models for Preclinical Efficacy and Safety Testing of Targeted Therapies. Toxicol Pathol. 2016 Apr;44(3):346-57. doi: 10.1177/0192623315609688. Epub 2015 Oct 27. Review. PubMed PMID: 26511847.

108. Chatterjee S, Shapiro L, Rose SJ, Mushtaq T, Clayton PE, Ten SB, Bhangoo A, Kumbattae U, Dias R, Savage MO, Metherell LA, Storr HL. Phenotypic spectrum and responses to recombinant human IGF1 (rhIGF1) therapy in patients with homozygous intronic pseudoexon growth hormone receptor mutation. Eur J Endocrinol. 2018 May;178(5):481-489. doi: 10.1530/EJE-18-0042. Epub 2018 Mar 2. PubMed PMID: 29500309.

109. Kemp SF. Insulin-like growth factor-I deficiency in children with growth hormone insensitivity: current and future treatment options. BioDrugs. 2009;23(3):155-63. doi: 10.2165/00063030-200923030-00002. Review. PubMed PMID: 19627167.

110. Carel JC, Chaussain JL, Chatelain P, Savage MO. Growth hormone insensitivity syndrome (Laron syndrome): main characteristics and effects of IGF1 treatment. Diabetes Metab. 1996 Jul;22(4):251-6. Review. PubMed PMID: 8767171.

111. Mali P, Yang L, Esvelt KM, Aach J, Guell M, DiCarlo JE, Norville JE, Church GM. RNA-guided human genome engineering via Cas9. Science. 2013 Feb 15;339(6121):823-6.

112. Osman N, McKenzie IF, Ostenried K, Ioannou YA, Desnick RJ, Sandrin MS. Combined transgenic expression of alpha-galactosidase and alpha1,2-fucosyltransferase leads to optimal reduction in the major xenoepitope Galalpha(1,3)Gal. Proc Natl Acad Sci U S A. 1997 Dec 23;94(26):14677-82.

113. Loveland BE, Milland J, Kyriakou P, Thorley BR, Christiansen D, Lanteri MB, van Regensburg M, Duffield M, French AJ, Williams L, Baker L, Brandon MR, Xing P-X, Kahn D, McKenzie IFC. Characterization of a CD46 transgenic pig and protection of transgenic kidneys against hyperacute rejection in non-immunosuppressed baboons. Xenotransplantation 2004; 11: 171-183

114. Yakar S, Liu JL, Stannard B, Butler A, Accili D, Sauer B, LeRoith D. Normal growth and development in the absence of hepatic insulin-like growth factor I. Proc Natl Acad Sci U S A. 1999 Jun 22;96(13):7324-9.

115. Längin M, Mayr T, Reichart B, Michel S, Buchholz S, Guethoff S, Dashkevich A, Baehr A, Egerer S, Bauer A, et al. Consistent success in life-supporting porcine cardiac xenotransplantation. Nature. 2018 Dec;564(7736):430-433. doi: 10.1038/s41586-018-0765-z. Epub 2018 Dec 5.

116. Konus OL, Ozdemir A, Akkaya A, Erbas G, Celik H, Isik S. Normal liver, spleen, and kidney dimensions in neonates, infants, and children: evaluation with sonography. AJR Am J Roentgenol. 1998 Dec;171(6):1693-8. PubMed PMID: 9843315.

117. Rao UV, Wagner HN Jr. Normal weights of human organs. Radiology. 1972 Feb;102(2):337-9. PubMed PMID: 5009936.

The present invention furthermore relates to the following items:

1. Composition comprising an mTOR inhibitor for use in a method of prolonging the survival of a recipient organism that has been transplanted a genetically modified heart, kidney, lung or liver from a xenogeneic donor organism so that it functionally substitutes the heart, kidney, lung or liver, respectively, of the recipient organism,

wherein the recipient organism receives antihypertensive treatment according to a dosing regimen,

wherein the donor organism is a mammal and wherein the recipient organism is a primate, preferably wherein the donor organism belongs to the family *Suidae.*

2. Composition comprising an mTOR inhibitor for use according to item 1, wherein the donor organism belongs to the species *S. scrofa.*

3. Composition comprising an mTOR inhibitor for use according to item 1 or 2, wherein the composition is for use in a method of preventing heart, kidney, lung or liver overgrowth.

4. Composition comprising an mTOR inhibitor for use according to any of items 1 to 3, wherein the functional substitution is an orthotopic substitution.

5. Composition comprising an mTOR inhibitor for use according to any of items 1 to 4, wherein the recipient organism belongs to the family *Cercopithecidae*, preferably wherein the recipient organism belongs to the genus *Papio.*

6. Composition comprising an mTOR inhibitor for use according to any of items 1 to 4, wherein the recipient organism is a human.

7. Composition comprising an mTOR inhibitor for use according to any of items 1 to 6, wherein the genetically modified heart, kidney, lung or liver has the following genetic modifications:

a) homozygous knock-out of the alpha-1,3-galactosyltransferase gene;

b) insertion of the human CD46 gene; and

c) insertion of the human thrombomodulin gene.

8. Composition comprising an mTOR inhibitor for use according to any of items 1 to 7, wherein the mTOR inhibitor is selected from the following list: dactolisib (BEZ235, NVP-BEZ235), rapamycin (sirolimus), everolimus (RAD001), AZD8055, temsirolimus (CCI-779, NSC 683864), SF2523, 3BDO, PI-103, KU-0063794 I, torkinib (PP242), ridaforolimus (deforolimus, MK-8669), sapanisertib (INK 128, MLN0128), voxtalisib (SAR245409, XL765) analogue, torin 1, omipalisib (GSK2126458, GSK458), OSI-027, PF-04691502, apitolisib (GDC-0980, RG7422), GSK1059615, gedatolisib (PF-05212384, PKI-587), WYE-354, vistusertib (AZD2014), torin 2, WYE-125132 (WYE-132), BGT226 (NVP-BGT226), palomid 529 (P529), PP121, WYE-687, WAY-600, ETP-46464, GDC-0349, XL388, zotarolimus (ABT-578), CZ415, MHY1485, voxtalisib (XL765, SAR245409), chrysophanic acid and CC-223, LY3023414; preferably wherein the mTOR inhibitor is temsirolimus.

9. Composition comprising an mTOR inhibitor for use according to any of items 1 to 8, wherein the recipient organism receives an immunosuppressive maintenance therapy according to a dosing regimen, wherein the dosing regimen comprises the administration of one or more immunosuppressive agent(s) after transplantation, wherein the dosing regimen comprises a first administration of a glucocorticoid that has occurred within 1 day after transplantation, and wherein the dosage (mg/kg/day) of the glucocorticoid administered to the recipient organism according to the dosing regimen has been decreased by a factor of at least 5, preferably by a factor of at least 10, within 5 days after transplantation.

10. Composition comprising an mTOR inhibitor for use according to any of items 1 to 9, wherein before transplantation the heart, kidney, lung or liver from the xenogeneic donor organism had been preserved by non-ischemic preservation, preferably by non-ischemic heart, kidney, lung or liver perfusion.

11. Composition comprising an mTOR inhibitor for use according to any of items 1 to 10, wherein the genetically modified heart, kidney, lung or liver is a genetically modified heart.

12. Composition comprising an mTOR inhibitor for use according to any of items 1 to 10, wherein the genetically modified heart, kidney, lung or liver is a genetically modified kidney.

13. Composition comprising an mTOR inhibitor for use according to any of items 1 to 10, wherein the genetically modified heart, kidney, lung or liver is a genetically modified lung.

14. Composition comprising an mTOR inhibitor for use according to any of items 1 to 10, wherein the genetically modified heart, kidney, lung or liver is a genetically modified liver.

15. Composition comprising one or more antihypertensive agent(s) for use in a method of prolonging the survival of a recipient organism that has been transplanted a genetically modified heart, kidney, lung or liver from a xenogeneic donor organism so that it functionally substitutes the heart, kidney, lung or liver, respectively, of the recipient organism,

wherein the recipient organism is administered an mTOR inhibitor according to a dosing regimen,

wherein the donor organism is a mammal and wherein the recipient organism is a primate, preferably wherein the donor organism belongs to the family *Suidae.*

16. Composition comprising one or more antihypertensive agent(s) for use according to item 15, wherein the donor organism belongs to the species *S. scrofa.*

17. Composition comprising one or more antihypertensive agent(s) for use according to item 15 or 16, wherein the composition is for use in a method of preventing heart, kidney, lung or liver overgrowth.

18. Composition comprising one or more antihypertensive agent(s) for use according to any of items 15 to 17, wherein the functional substitution is an orthotopic substitution.

19. Composition comprising one or more antihypertensive agent(s) for use according to any of items 15 to 18, wherein the recipient organism belongs to the family *Cercopithecidae*, preferably wherein the recipient organism belongs to the genus *Papio.*

20. Composition comprising one or more antihypertensive agent(s) for use according to any of items 15 to 18, wherein the recipient organism is a human.

21. Composition comprising one or more antihypertensive agent(s) for use according to any of items 15 to 20, wherein the genetically modified heart, kidney, lung or liver has the following genetic modifications:

a) homozygous knock-out of the alpha-1,3-galactosyltransferase gene;

b) insertion of the human CD46 gene; and

c) insertion of the human thrombomodulin gene.

22. Composition comprising one or more antihypertensive agent(s) for use according to any of items 15 to 21, wherein the antihypertensive agent(s) are one or more agent(s) selected from the following list: diuretics, calcium channel blockers, angiotensin-converting enzyme (ACE) inhibitors, angiotensin II receptor antagonists, adrenergic receptor antagonists, vasodilators, renin inhibitors, aldosterone receptor antagonists, alpha-2 adrenergic receptor agonists and endothelin receptor blockers, preferably wherein the antihypertensive agent(s) are selected from angiotensin-converting enzyme (ACE) inhibitors and adrenergic receptor antagonists, more preferably wherein the antihypertensive agent(s) is/are enalapril and/or metoprolol tartrate, most preferably wherein the antihypertensive agents are enalapril and metoprolol tartrate.

23. Composition comprising one or more antihypertensive agent(s) for use according to any of items 15 to 22, wherein the recipient organism receives an immunosuppressive maintenance therapy according to a dosing regimen, wherein the dosing regimen comprises the administration of one or more immunosuppressive agent(s) after transplantation, wherein the dosing regimen comprises a first administration of a glucocorticoid that has occurred within 1 day after transplantation, and wherein the dosage (mg/kg/day) of the glucocorticoid administered to the recipient organism according to the dosing regimen has been decreased by a factor of at least 5, preferably by a factor of at least 10, within 5 days after transplantation.

24. Composition comprising one or more antihypertensive agent(s) for use according to any of items 15 to 23, wherein before transplantation the heart, kidney, lung or liver from the xenogeneic donor organism had been preserved by non-ischemic preservation, preferably by non-ischemic heart, kidney, lung or liver perfusion.

25. Composition comprising one or more antihypertensive agent(s) for use according to any of items 15 to 24, wherein the genetically modified heart, kidney, lung or liver is a genetically modified heart.

26. Composition comprising one or more antihypertensive agent(s) for use according to any of items 15 to 24, wherein the genetically modified heart, kidney, lung or liver is a genetically modified kidney.

27. Composition comprising one or more antihypertensive agent(s) for use according to any of items 15 to 24, wherein the genetically modified heart, kidney, lung or liver is a genetically modified lung.

28. Composition comprising one or more antihypertensive agent(s) for use according to any of items 15 to 24, wherein the genetically modified heart, kidney, lung or liver is a genetically modified liver.

29. Non-human living primate whose heart, kidney, lung or liver is functionally substituted by a transplanted, genetically modified heart, kidney, lung or liver, respectively, from a xenogeneic mammal, wherein the heart, kidney, lung or liver of said living primate has been transplanted more than 40 days ago, preferably more than 60 days ago, and most preferably more than 90 days ago, and wherein the xenogeneic mammal belongs to the family *Suidae.*

30. Non-human living primate according to item 29, wherein the xenogeneic mammal belongs to the species *S. scrofa.*

31. Non-human living primate according to item 29 or 30, wherein the functional substitution is an orthotopic substitution.

32. Method of prolonging the survival of a recipient organism that is transplanted a genetically modified heart, kidney, lung or liver from a xenogeneic donor organism, wherein the method comprises the following steps:

(vi) administering one or more antihypertensive agent(s) to the recipient organism according to a dosing regimen, wherein the dosing regimen comprises the administration of one or more antihypertensive agent(s) after transplantation; and

(vii) administering an mTOR inhibitor to the recipient organism according to a dosing regimen, wherein the dosing regimen comprises the administration of an mTOR inhibitor after transplantation;

wherein the donor organism is a mammal, preferably wherein the donor organism belongs to the family Suidae; and

wherein the recipient organism is a primate.

33. The method of item 32, wherein the method additionally comprises the following steps:

(ii) surgically extracting the heart, kidney, lung or liver from the donor organism and preserving the surgically extracted heart, kidney, lung or liver, respectively, by non-ischemic preservation until transplantation;

(iii) transplanting the surgically extracted, preserved heart, kidney, lung or liver of the donor organism into the recipient organism, so that it functionally substitutes the heart, kidney, lung or liver, respectively, of the recipient organism;

(iv) administering an immunosuppressive maintenance therapy to the recipient organism according to a dosing regimen, wherein the dosing regimen comprises the administration of one or more immunosuppressive agent(s) after transplantation, and wherein the dosing regimen comprises a first administration of a glucocorticoid within 1 day after transplantation; and

(viii) decreasing the dosage (mg/kg/day) of the glucocorticoid administered to the recipient organism according to the dosing regimen of step (iv) by a factor of at least 5, preferably by a factor of at least 10, within 5 days after transplantation.

34. The method of item 32 or 33, wherein the method additionally comprises the following steps:

(i) administering an immunosuppressive induction therapy to the recipient organism according to a dosing regimen, wherein the dosing regimen comprises the administration of one or more immunosuppressive agent(s) before transplantation; and

(v) administering an anti-inflammatory therapy to the recipient organism according to a dosing regimen, wherein the dosing regimen comprises the administration of one or more anti-inflammatory agent(s) after transplantation.

35. The method of any of items 32 to 34, wherein the recipient organism belongs to the family Cercopithecidae, preferably wherein the recipient organism belongs to the genus Papio,

36. The method of any of items 32 to 34, wherein the recipient organism is a human.

37. The method of any of items 32 to 36, wherein the dosing regimen of step (vi) comprises administering one or more antihypertensive agent(s) selected from the following list: diuretics, calcium channel blockers, angiotensin-converting enzyme (ACE) inhibitors, angiotensin II receptor antagonists, adrenergic receptor antagonists, vasodilators, renin inhibitors, aldosterone receptor antagonists, alpha-2 adrenergic receptor agonists and endothelin receptor blockers, preferably wherein the dosing regimen comprises administering one or more antihypertensive agent(s) selected from angiotensin-converting enzyme (ACE) inhibitors and adrenergic receptor antagonists, more preferably wherein the dosing regimen comprises administering enalapril and/or metoprolol tartrate, most preferably wherein the dosing regimen comprises administering enalapril and metoprolol tartrate.

38. The method of any of items 32 to 37, wherein the dosing regimen of step (vii) comprises administering an mTOR inhibitor selected from the following list: dactolisib (BEZ235, NVP-BEZ235), rapamycin (sirolimus), everolimus (RAD001), AZD8055, temsirolimus (CCI-779, NSC 683864), SF2523, 3BDO, PI-103, KU-0063794 I, torkinib (PP242), ridaforolimus (deforolimus, MK-8669), sapanisertib (INK 128, MLN0128), voxtalisib (SAR245409, XL765) analogue, torin 1, omipalisib (GSK2126458, GSK458), OSI-027, PF-04691502, apitolisib (GDC-0980, RG7422), GSK1059615, gedatolisib (PF-05212384, PKI-587), WYE-354, vistusertib (AZD2014), torin 2, WYE-125132 (WYE-132), BGT226 (NVP-BGT226), palomid 529 (P529), PP121, WYE-687, WAY-600, ETP-46464, GDC-0349, XL388, zotarolimus (ABT-578), CZ415, MHY1485, voxtalisib (XL765, SAR245409), chrysophanic acid and CC-223, LY3023414;preferably wherein the dosing regimen comprises administering temsirolimus.

39. The method of any of items 32 to 38, wherein the genetically modified heart, kidney, lung or liver is a genetically modified heart.

40. The method of any of items 32 to 38, wherein the genetically modified heart, kidney, lung or liver is a genetically modified kidney.

41. The method of any of items 32 to 38, wherein the genetically modified heart, kidney, lung or liver is a genetically modified lung.

42. The method of any of items 32 to 38, wherein the genetically modified heart, kidney, lung or liver is a genetically modified liver.

43. A genetically modified mammal that has the following genetic modifications:

a) insertion of a sequence encoding a human complement-regulatory protein, and

b) insertion of a sequence encoding human thrombomodulin;

wherein the sequence encoding a human complement-regulatory protein and the sequence encoding human thrombomodulin are inserted at the alpha-1,3-galactosyltransferase gene locus, wherein at least the sequence encoding a human complement-regulatory protein is expressed under the control of the endogenous promoter of the alpha-1,3-galactosyltransferase gene; and

wherein the genetically modified mammal is non-human.

44. The genetically modified mammal of item 43, wherein the genetically modified mammal lacks expression of alpha-1,3-galactosyltransferase.

45. The genetically modified mammal of item 43 or 44, wherein the sequence encoding a human complement-regulatory protein is a sequence encoding human CD46.

46. The genetically modified mammal of any of items 43 to 45, wherein genetically modified mammal additionally has the following genetic modifications:

c) homozygous knock-out of the CMAH gene, and

d) homozygous knock-out of the B4GALNT2 gene.

47. The genetically modified mammal of any of items 43 to 46, wherein genetically modified mammal has one or more additional genetic modification(s).

48. The genetically modified mammal of item 47, wherein the one or more additional genetic modification(s) is/are insertion(s), and wherein the one or more insertion(s) is/are at the alpha-1,3-galactosyltransferase gene locus.

49. The genetically modified mammal of any of items 43 to 48, wherein the genetically modified mammal belongs to the species S. scrofa.

50. The genetically modified mammal of any of items 43 to 49, wherein the genetically modified mammal is free of porcine endogenous retrovirus C (PERV-C).

51. The genetically modified mammal of any of items 43 to 50, wherein the genetically modified mammal is suitable to serve as donor organism for xenogeneic organ transplants.

52. A method of making a genetically modified mammal comprising the following steps:

(a) inserting a sequence encoding a human complement-regulatory protein and a sequence encoding human thrombomodulin at the alpha-1,3-galactosyltransferase gene locus of a mammalian genome to provide a genetically modified genome;

(b) introducing the genetically modified genome into a cell; and

(c) permitting the cell comprising the genetically modified genome to mature into a genetically modified mammal;

wherein the genetically modified mammal is non-human.

53. The method of item 52, wherein the sequence encoding a human complement-regulatory protein is a sequence encoding human CD46.

54. The method of item 52 or 53, wherein the sequence encoding a human complement-regulatory protein and the sequence encoding human thrombomodulin are located on a single expression vector that is inserted into the alpha-1,3-galactosyltransferase gene locus.

55. The method of any of items 52 to 54, wherein the sequence encoding a human complement-regulatory protein and the sequence encoding human thrombomodulin are inserted at the alpha-1,3-galactosyltransferase gene locus such that the genetically modified mammal lacks expression of alpha-1,3-galactosyltransferase.

56. The method of any of items 52 to 55, wherein the sequence encoding a human complement-regulatory protein and the sequence encoding human thrombomodulin are inserted at the alpha-1,3-galactosyltransferase gene locus such that at least the sequence encoding a human complement-regulatory protein is expressed under the control of the endogenous promoter of the alpha-1,3-galactosyltransferase gene.

57. The method of any of items 52 to 56, wherein the method further comprises knocking-out the CMAH and B4GALNT2 genes of the mammalian genome before step (b).

58. The method of any of items 52 to 57, wherein the method further comprises introducing one or more additional genetic modification(s) into the mammalian genome before step (b).

59. The method of item 58, wherein the one or more additional genetic modification(s) is/are genetic insertion(s), and wherein the sequence encoding a human complement-regulatory protein, the sequence encoding human thrombomodulin and the one or more additional insertion(s) is/are located on a single expression vector that is inserted into the alpha-1,3-galactosyltransferase gene locus.

60. The method of any of items 52 to 59, wherein the genetic modifications are introduced using CRISPR/Cas9.

61. The method of any of items 52 to 59, wherein the mammal belongs to the species S. scrofa.

62. The method of any of items 52 to 61, wherein the mammal is free of porcine endogenous retrovirus C (PERV-C).

63. The method of any of items 52 to 62, wherein the genetically modified mammal is suitable to serve as donor organism for xenogeneic organ transplants.

64. A genetically modified mammal obtained by the method of any of items 52 to 63.

65. A donor organism for xenogeneic organ transplants, wherein the donor organism has the following genetic modification:

a) homozygous knock-out of the growth hormone receptor gene;
and wherein the donor organism is a non-human mammal.

66. The donor organism of item 65, wherein the donor organism additionally has the following genetic modifications:

b) homozygous knock-out of the alpha-1,3-galactosyltransferase gene;

c) insertion of a sequence encoding human CD46; and

d) insertion of a sequence encoding human thrombomodulin.

67. The donor organism of item 65, wherein the donor organism additionally has the following genetic modifications:

b) homozygous knock-outs of the alpha-1,3-galactosyltransferase gene, CMAH gene and B4GALNT2 gene; and

c) insertions of a sequence encoding human CD46, a sequence encoding human CD55, a sequence encoding human CD59, a sequence encoding hemeoxygenase 1 and a sequence encoding A20.

68. The donor organism of item 67, wherein the donor organism additionally has the following genetic modification: d) insertion of a sequence encoding LEA29Y or PD-L1.

69. The donor organism of any of items 65 to 68, wherein the donor organism belongs to the species S. scrofa.

70. The donor organism of any of items 65 to 69, wherein the donor organism is free of porcine endogenous retrovirus C (PERV-C).

71. A method of making a donor organism for xenogeneic organ transplants comprising the following step (i):

(i) adjusting the size of the donor organism by treating the donor organism with IGF1;

wherein the donor organism has the following genetic modification:

a) homozygous knock-out of the growth hormone receptor gene;

and wherein the donor organism is a non-human mammal.

72. The method of item 71, wherein the donor organism additionally has the following genetic modifications:

b) homozygous knock-out of the alpha-1,3-galactosyltransferase gene;

c) insertion of a sequence encoding human CD46; and

d) insertion of a sequence encoding human thrombomodulin.

73. The method of item 71, wherein the donor organism additionally has the following genetic modifications:

b) homozygous knock-outs of the alpha-1,3-galactosyltransferase gene, CMAH gene and B4GALNT2 gene; and

c) insertions of a sequence encoding human CD46, a sequence encoding human CD55, a sequence encoding human CD59, a sequence encoding hemeoxygenase 1 and a sequence encoding A20.

74. The method of item 73, wherein the donor organism additionally has the following genetic modification:
d) insertion of a sequence encoding LEA29Y or PD-L1.

75. The method of any of items 71 to 74, wherein the donor organism belongs to the species S. scrofa.

76. The method of any of items any of items 71 to 75, wherein the donor organism is free of porcine endogenous retrovirus C (PERV-C).

77. A donor organism for xenogeneic organ transplants, wherein the donor organism is obtained according to the method of any of items 71 to 76.

**Claims**

1. Composition comprising an oxygenated nutrition solution with hormones for use in a method of prolonging the survival of a recipient primate,

   wherein the recipient primate is transplanted a genetically modified heart from a xenogeneic donor mammal,
   wherein the genetically modified heart is preserved by non-ischemic perfusion using the composition until transplantation, and
   wherein the recipient primate is cooled to 34°C before transplantation.

2. Composition for use according to claim 1, wherein the duration of the non-ischemic perfusion until transplantation is shorter than 15 hours, preferably shorter than 10 hours, more preferably shorter than 5 hours, and most preferably shorter than 4 hours.

3. Composition for use according to claim 1 or 2, wherein during transplantation the heart is intermittently perfused for 2 min every 20 min using the composition.

4. Composition for use according to any of claims 1 to 3, wherein the composition comprises an oxygenated albumin-containing hyperoncotic cardioplegic nutrition solution with hormones and erythrocytes.

5. Composition for use according to any of claims 1 to 4, wherein the composition is used at a temperature of about 8°C.

6. Composition for use according to any of claims 1 to 5, wherein the perfusion pressure remains regulated at 20 mmHg.

7. Composition for use according to any of claims 1 to 6, wherein the heart of the donor mammal is transplanted so that it functionally substitutes the heart of the recipient primate.

8. Composition for use according to claim 7, wherein the functional substitution is an orthotopic substitution.

9. Composition for use according to any of claims 1 to 8, wherein the genetically modified heart has the following genetic modifications:

   a) homozygous knock-out of the alpha-1,3-galactosyltransferase gene;
   b) insertion of a primate CD46 gene, preferably of the human CD46 gene; and
   c) insertion of a primate thrombomodulin gene, preferably of the human thrombomodulin gene.

**10.** Composition for use according to any of claims 1 to 9, wherein the donor mammal is a non-human mammal and has the following genetic modification:

a) homozygous knock-out of the growth hormone receptor gene.

**11.** Composition for use according to any of claims 1 to 10, wherein the donor mammal belongs to the family *Suidae,* preferably wherein the donor mammal belongs to the genus *Sus*, more preferably wherein the donor mammal belongs to the species *S. scrofa*, most preferably wherein the donor mammal belongs to the subspecies *S. s. domesticus.*

**12.** Composition for use according to any of claims 1 to 11, wherein the donor mammal is a pig of German Landrace/Large White cross-bred genetic background, a Duroc pig, a Schwäbisch-Hällische pig, a black mini pig, or an Auckland Island pig; preferably wherein the donor mammal is a pig of German Landrace/Large White cross-bred genetic background.

**13.** Composition for use according to any of claims 1 to 12, wherein the recipient primate belongs to the family *Cercopithecidae*, preferably wherein the recipient primate belongs to the genus *Papio*, more preferably wherein the recipient primate belongs to the species *Papio Anubis* or *Papio Hamadryas*, most preferably wherein the recipient primate belongs to the species *Papio Anubis.*

**14.** Composition for use according to any of claims 1 to 12, wherein the recipient primate belongs to the family *Homonidae*, preferably wherein the recipient primate belongs to the genus *Homo*, more preferably wherein the recipient primate is a human.

FIG. 1

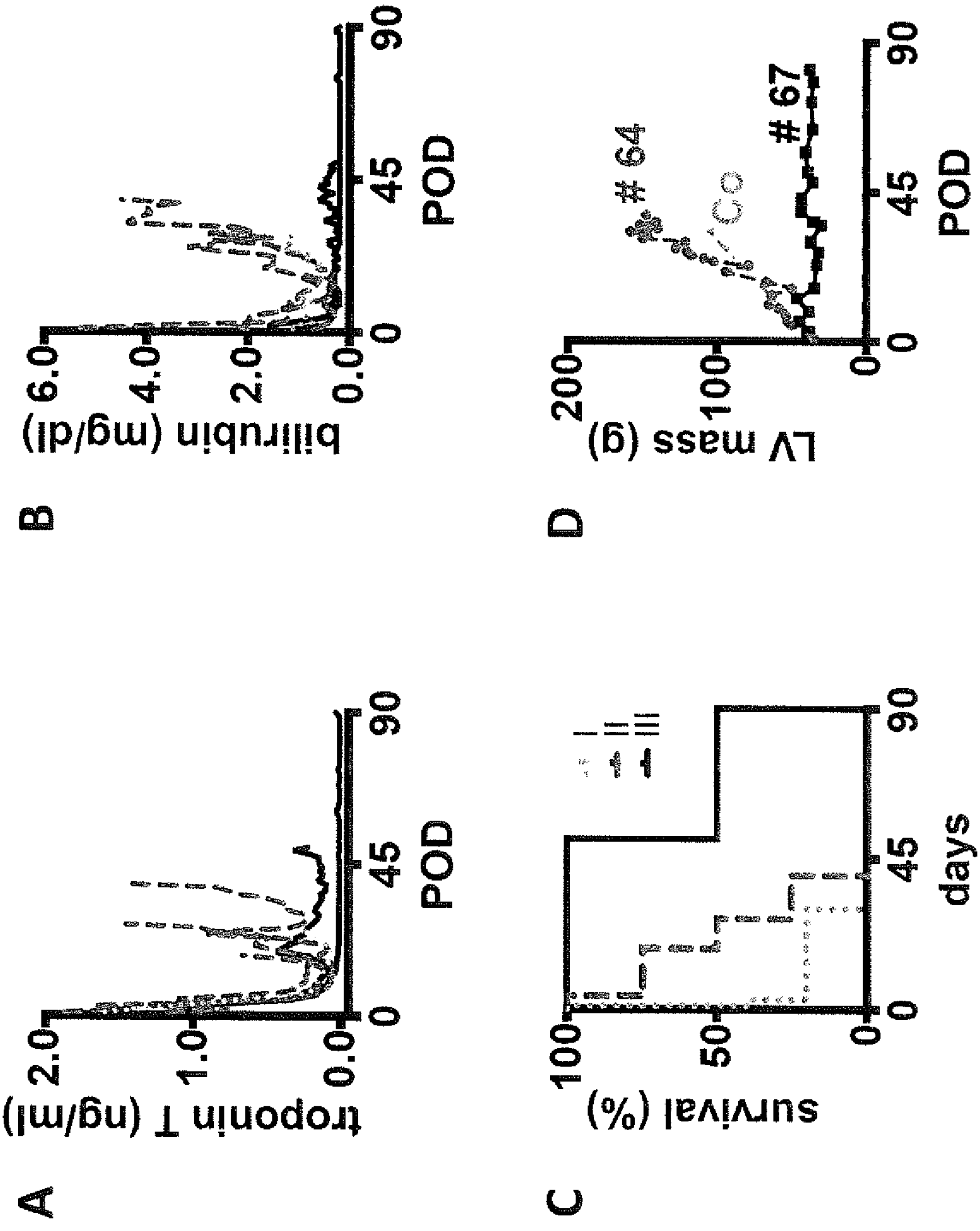
A
troponin T (ng/ml)
2.0
1.0
0.0
0
45
90
POD
B
bilirubin (mg/dl)
6.0
4.0
2.0
0.0
0
45
90
POD
C
survival (%)
100
50
0
0
45
90
days
I
II
III
D
LV mass (g)
200
100
0
0
45
90
POD
# 64
Co
# 67

FIG. 1

E

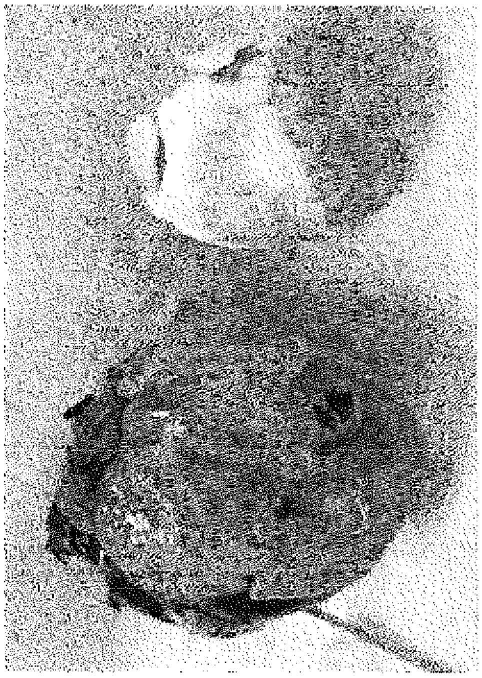

F

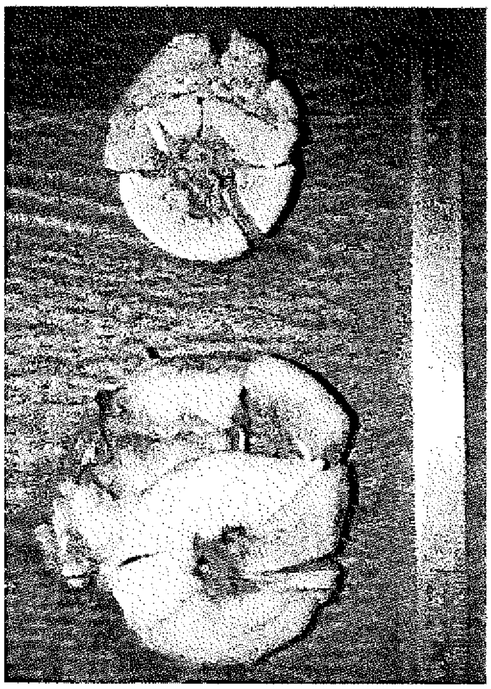

G

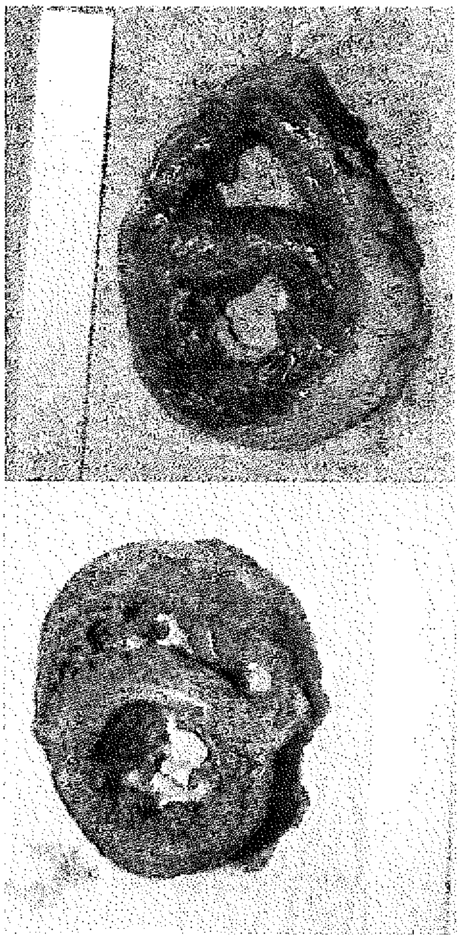

FIG. 1

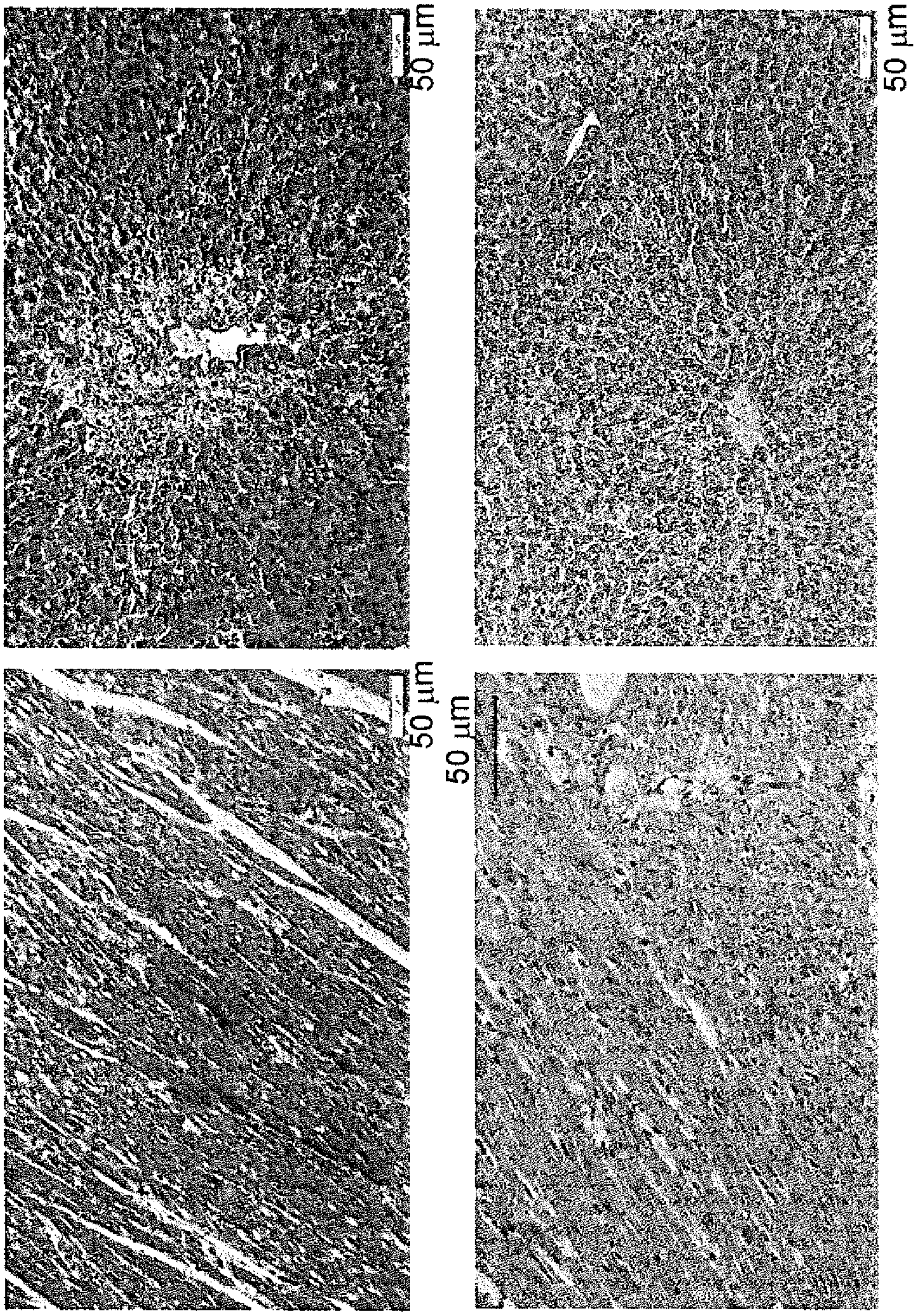


H

I

FIG. 1

J

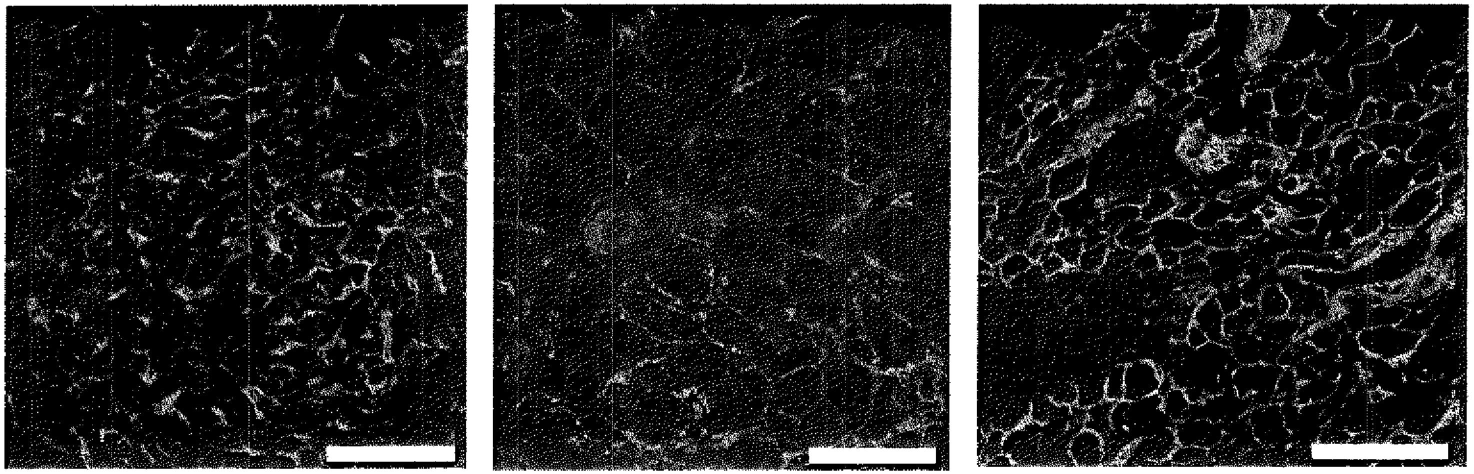

K

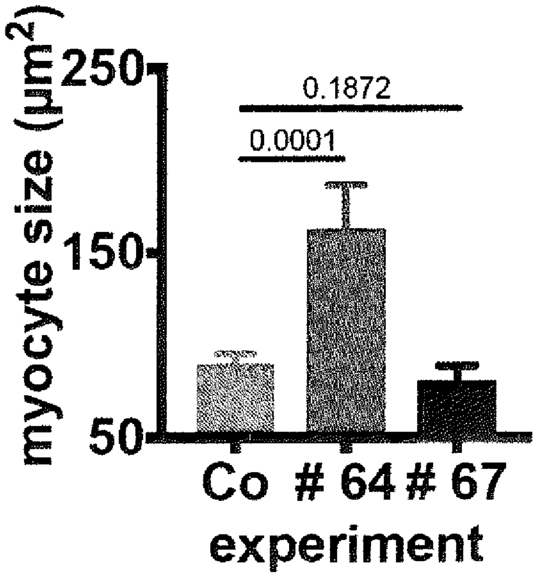
myocyte size (μm²)
250
150
50
0.1872
0.0001
Co
# 64
# 67
experiment

L

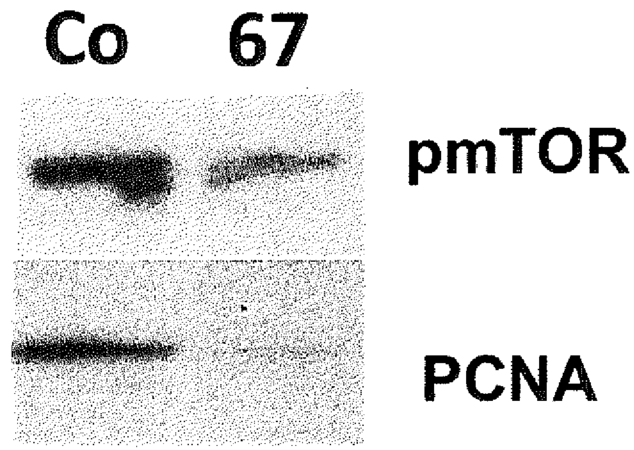
Co
67
pmTOR
PCNA

FIG. 2

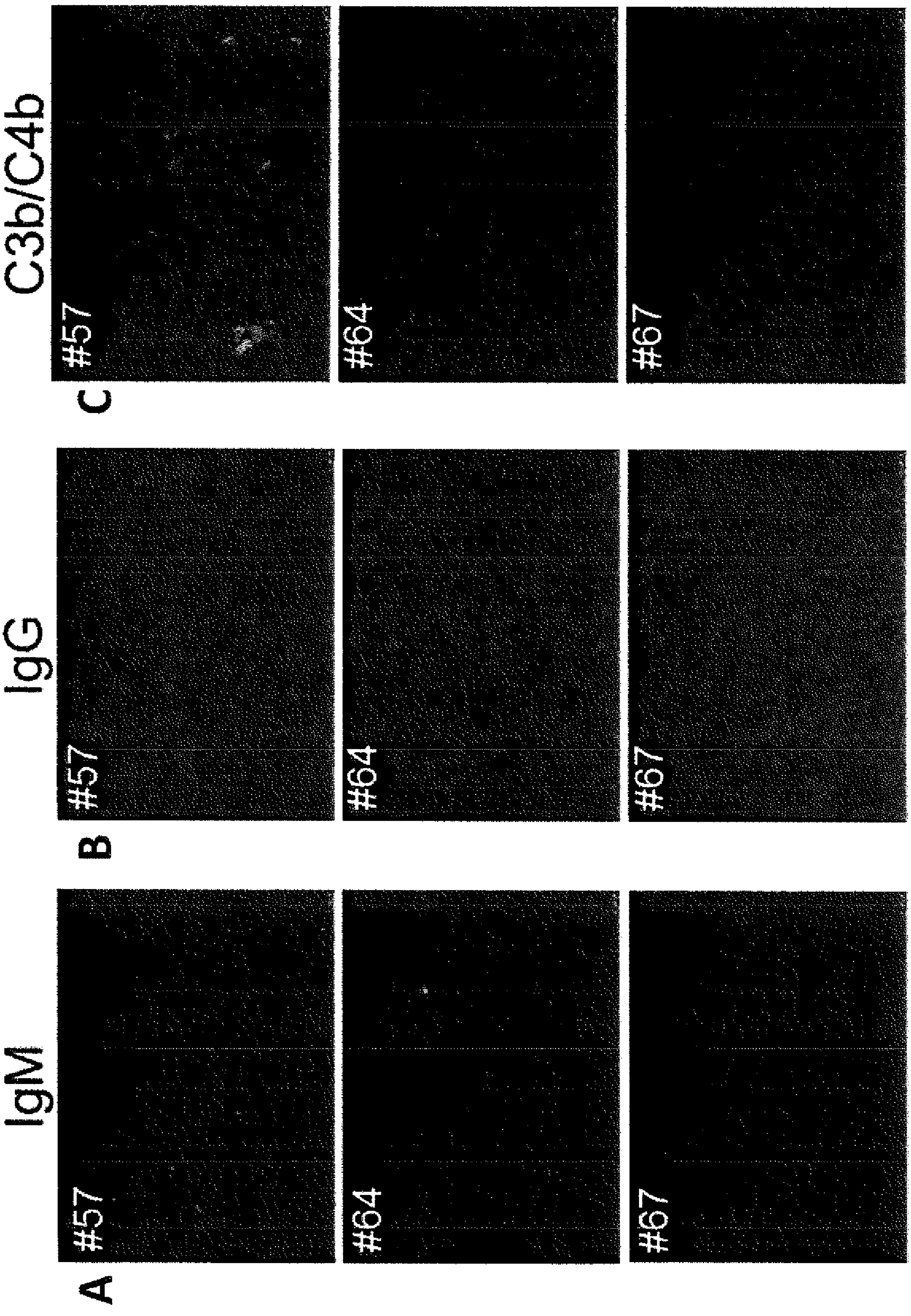

FIG. 2

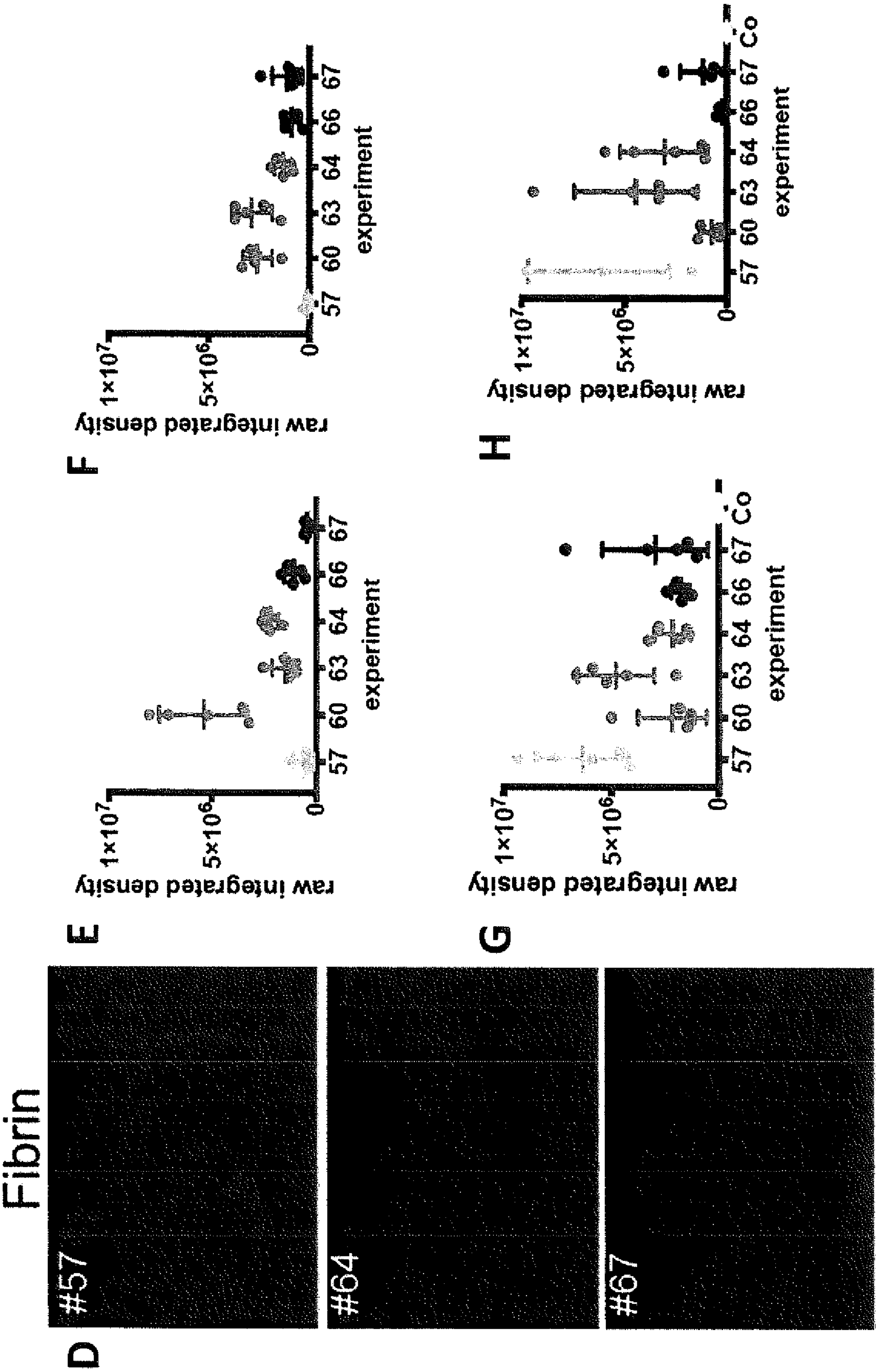
Fibrin
D
#57
#64
#67
E
F
G
H
raw integrated density
1×10^7
5×10^6
0
57 60 63 64 66 67
57 60 63 64 66 67 Co
experiment

FIG. 2

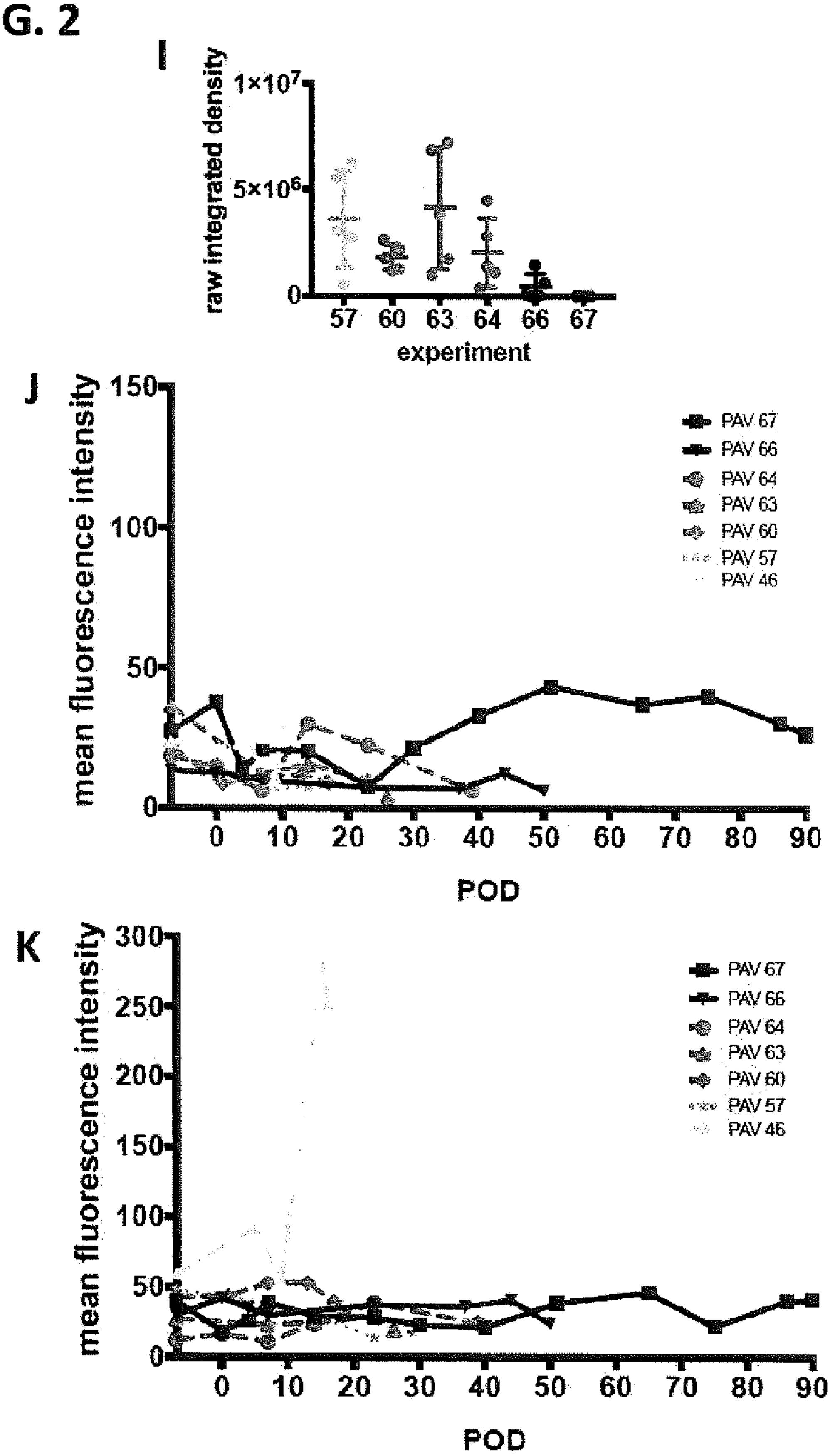
I
raw integrated density
1×10^7
5×10^6
0
57 60 63 64 66 67
experiment
J
mean fluorescence intensity
150
100
50
0
0 10 20 30 40 50 60 70 80 90
POD
PAV 67
PAV 66
PAV 64
PAV 63
PAV 60
PAV 57
PAV 46
K
mean fluorescence intensity
300
250
200
150
100
50
0
0 10 20 30 40 50 60 70 80 90
POD
PAV 67
PAV 66
PAV 64
PAV 63
PAV 60
PAV 57
PAV 46

FIG. 3

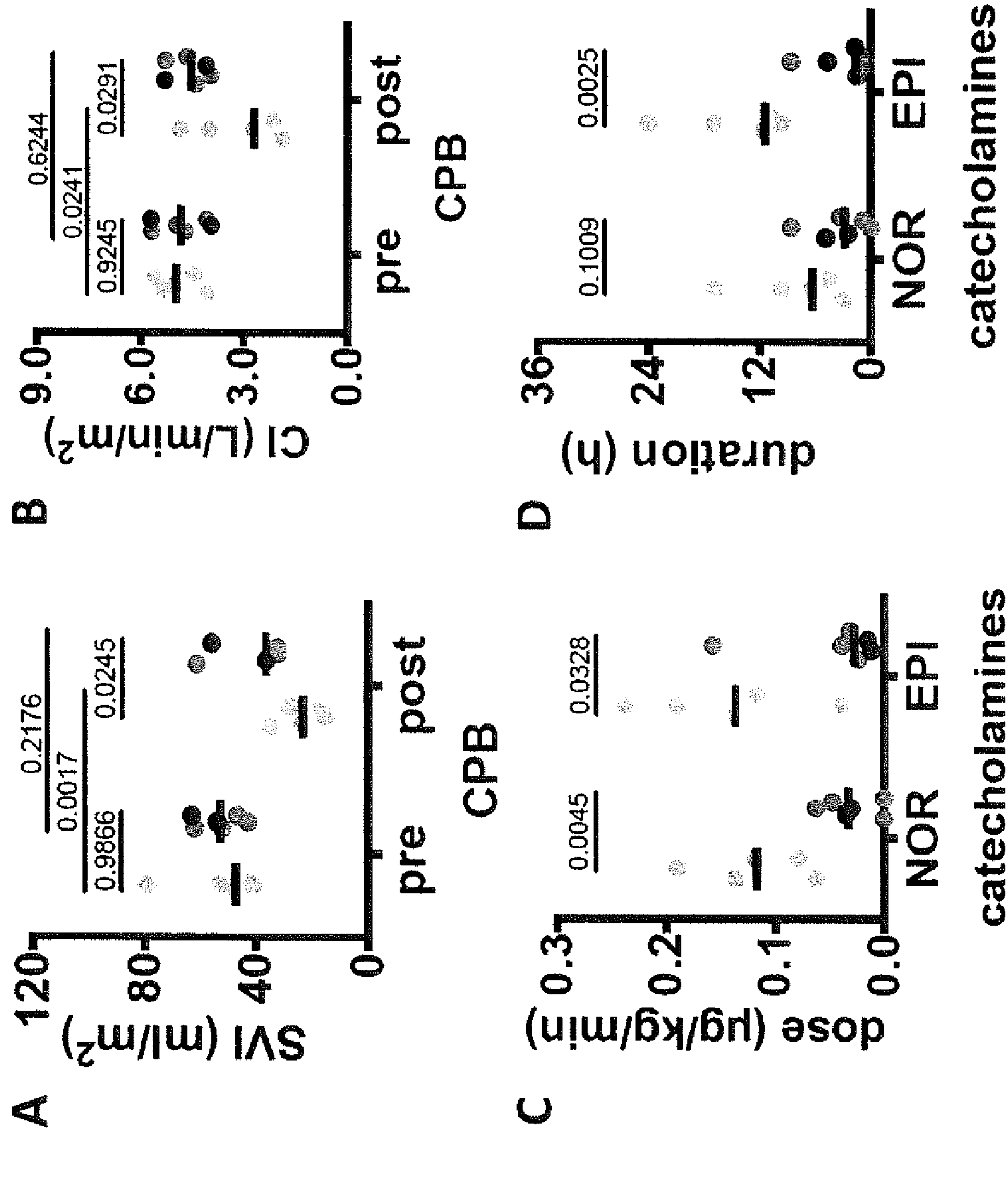
A
SVI (ml/m²)
120
80
40
0
0.9866
0.0017
0.2176
0.0245
pre
post
CPB
B
CI (L/min/m²)
9.0
6.0
3.0
0.0
0.9245
0.0241
0.6244
0.0291
pre
post
CPB
C
dose (µg/kg/min)
0.3
0.2
0.1
0.0
0.0045
0.0328
NOR
EPI
catecholamines
D
duration (h)
36
24
12
0
0.1009
0.0025
NOR
EPI
catecholamines

FIG. 4

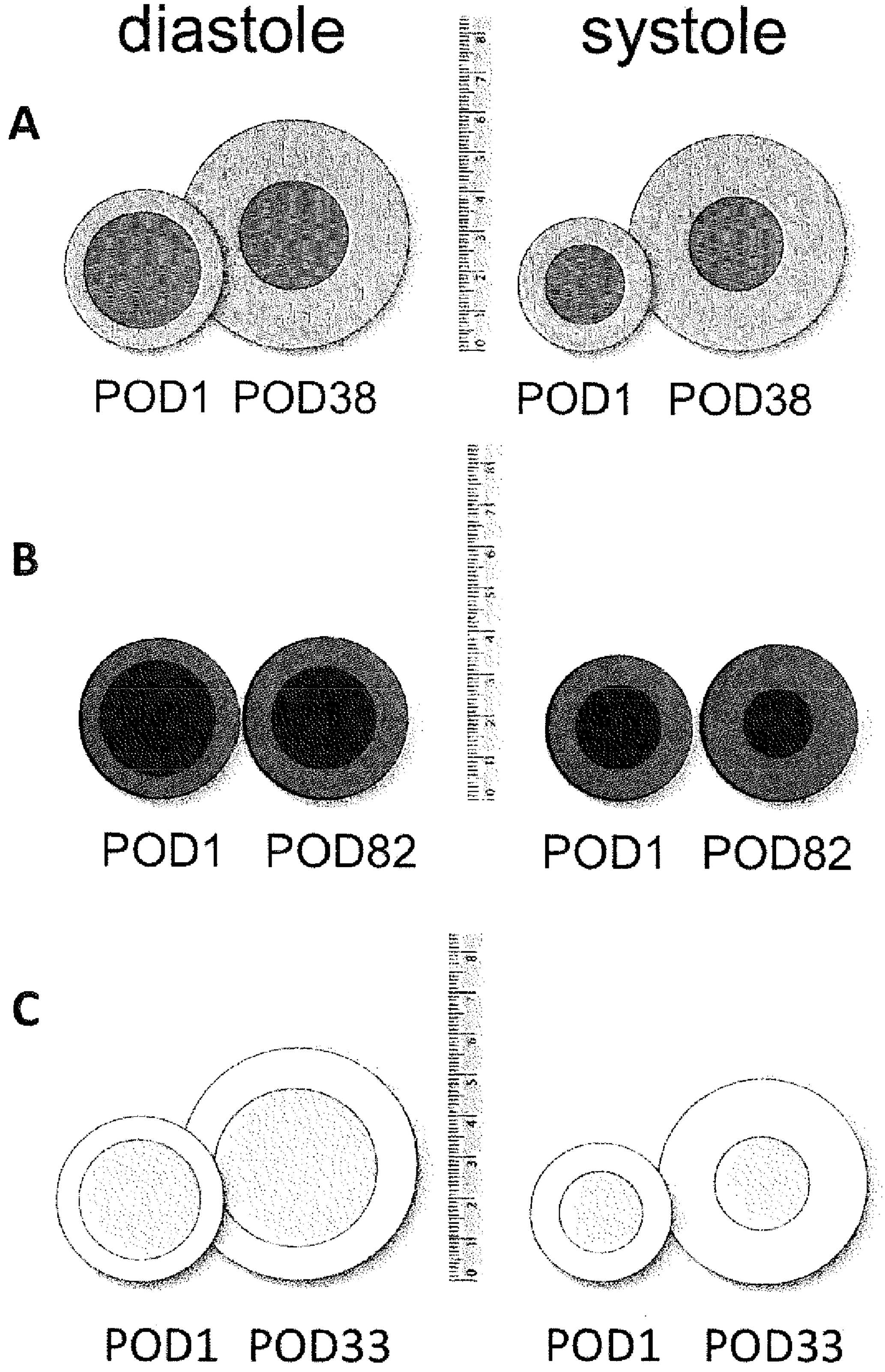
diastole
systole
A
POD1 POD38
POD1 POD38
B
POD1 POD82
POD1 POD82
C
POD1 POD33
POD1 POD33

FIG. 5

A

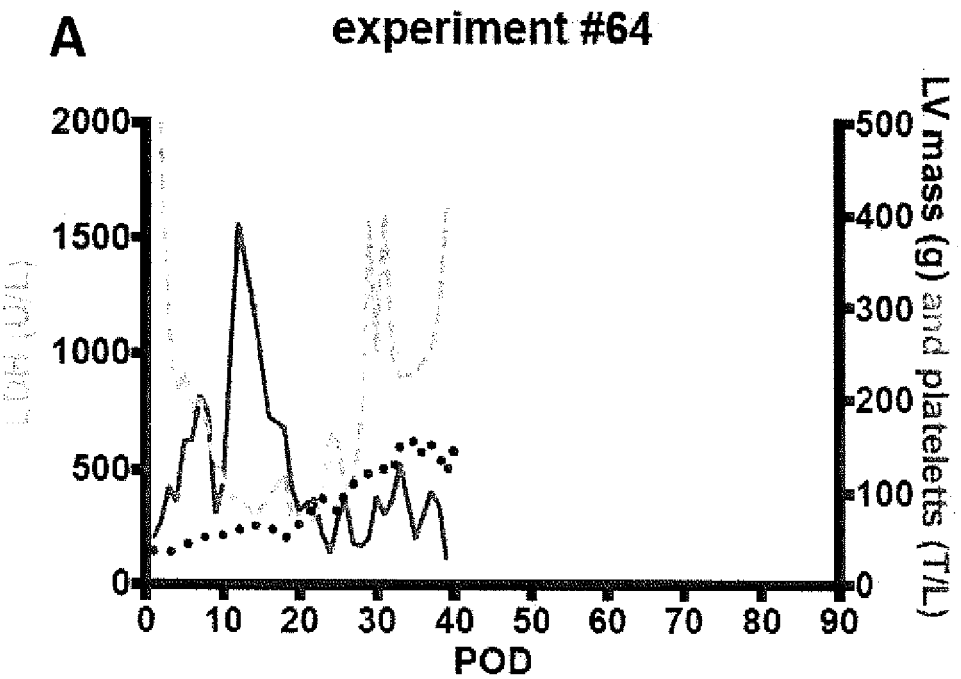
experiment #64
2000
1500
1000
500
0
LDH (U/L)
500
400
300
200
100
0
LV mass (g) and platelets (T/L)
0 10 20 30 40 50 60 70 80 90
POD

B

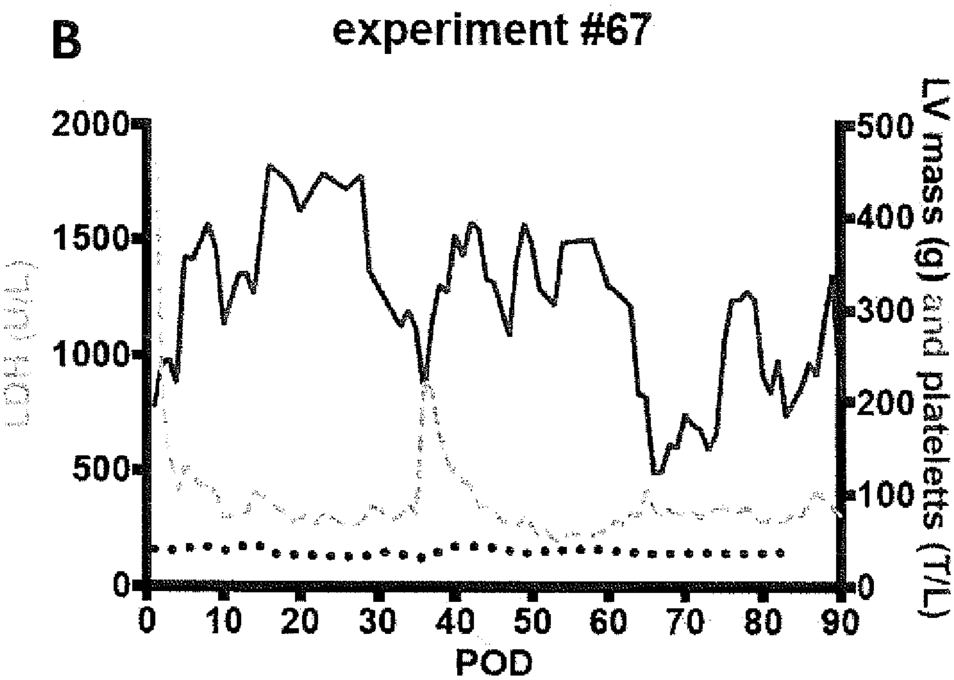
experiment #67
2000
1500
1000
500
0
LDH (U/L)
500
400
300
200
100
0
LV mass (g) and platelets (T/L)
0 10 20 30 40 50 60 70 80 90
POD

FIG. 6

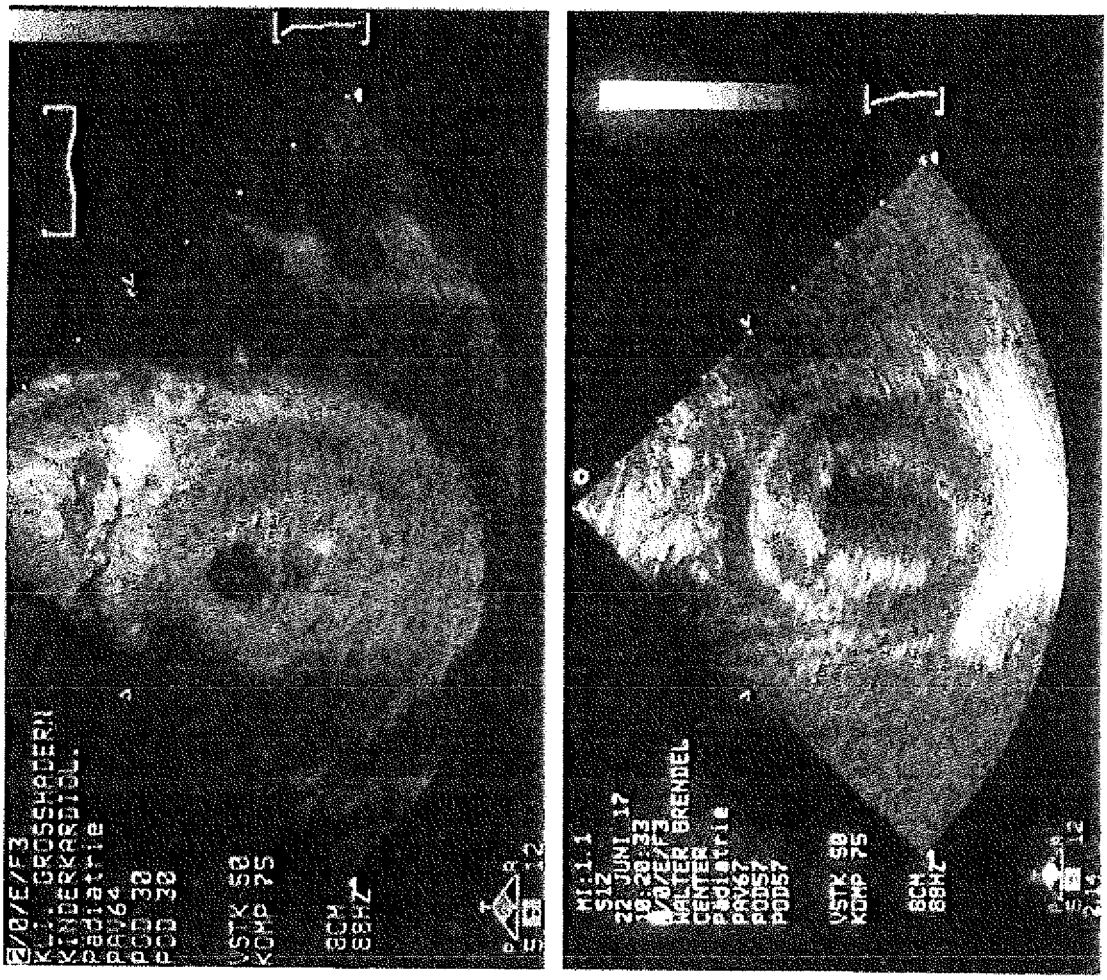

A

B

FIG. 7

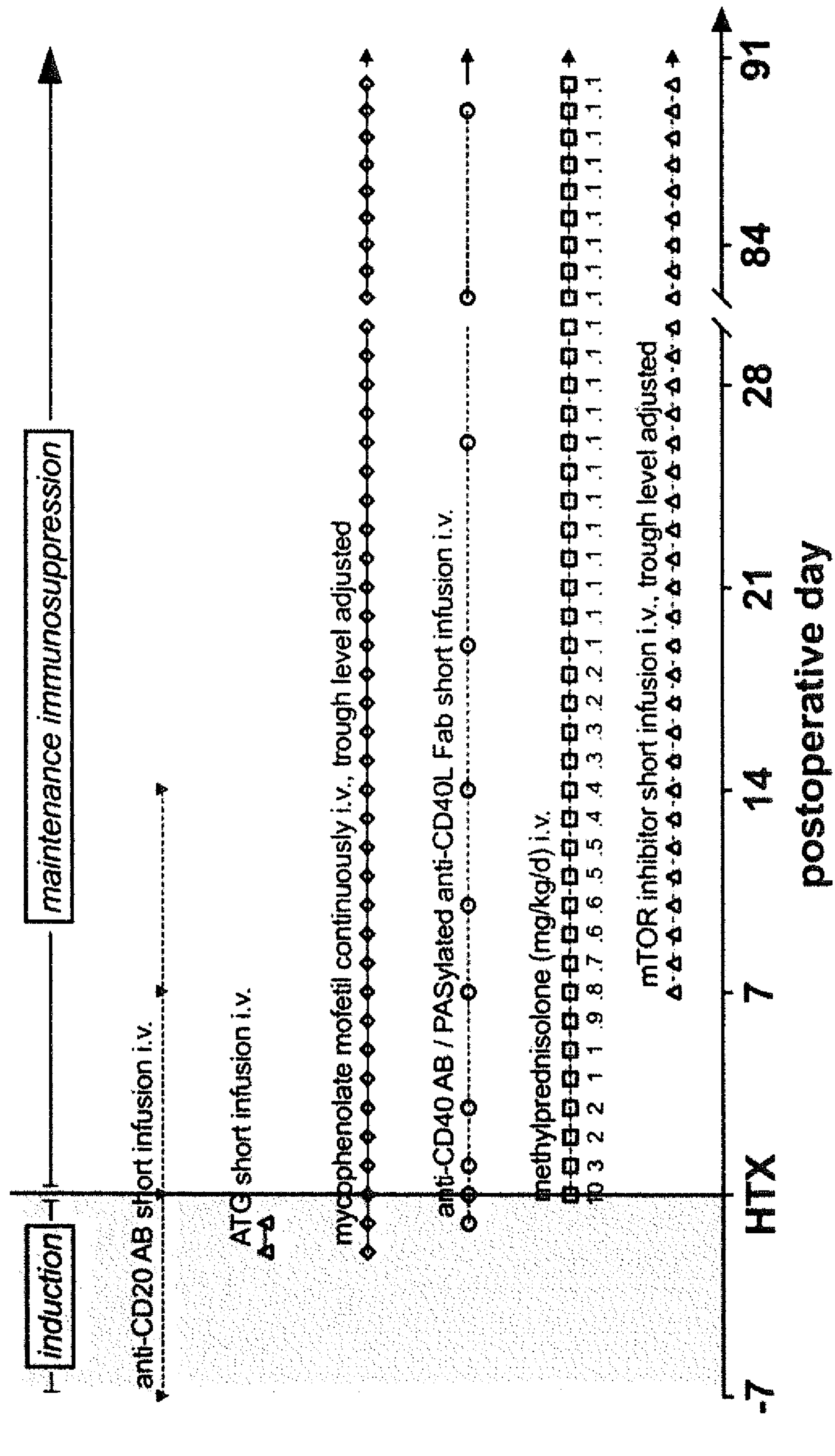
induction
maintenance immunosuppression
anti-CD20 AB short infusion i.v.
ATG short infusion i.v.
mycophenolate mofetil continuously i.v., trough level adjusted
anti-CD40 AB / PASylated anti-CD40L Fab short infusion i.v.
methylprednisolone (mg/kg/d) i.v.
mTOR inhibitor short infusion i.v., trough level adjusted
-7
HTX
7
14
21
28
84
91
postoperative day

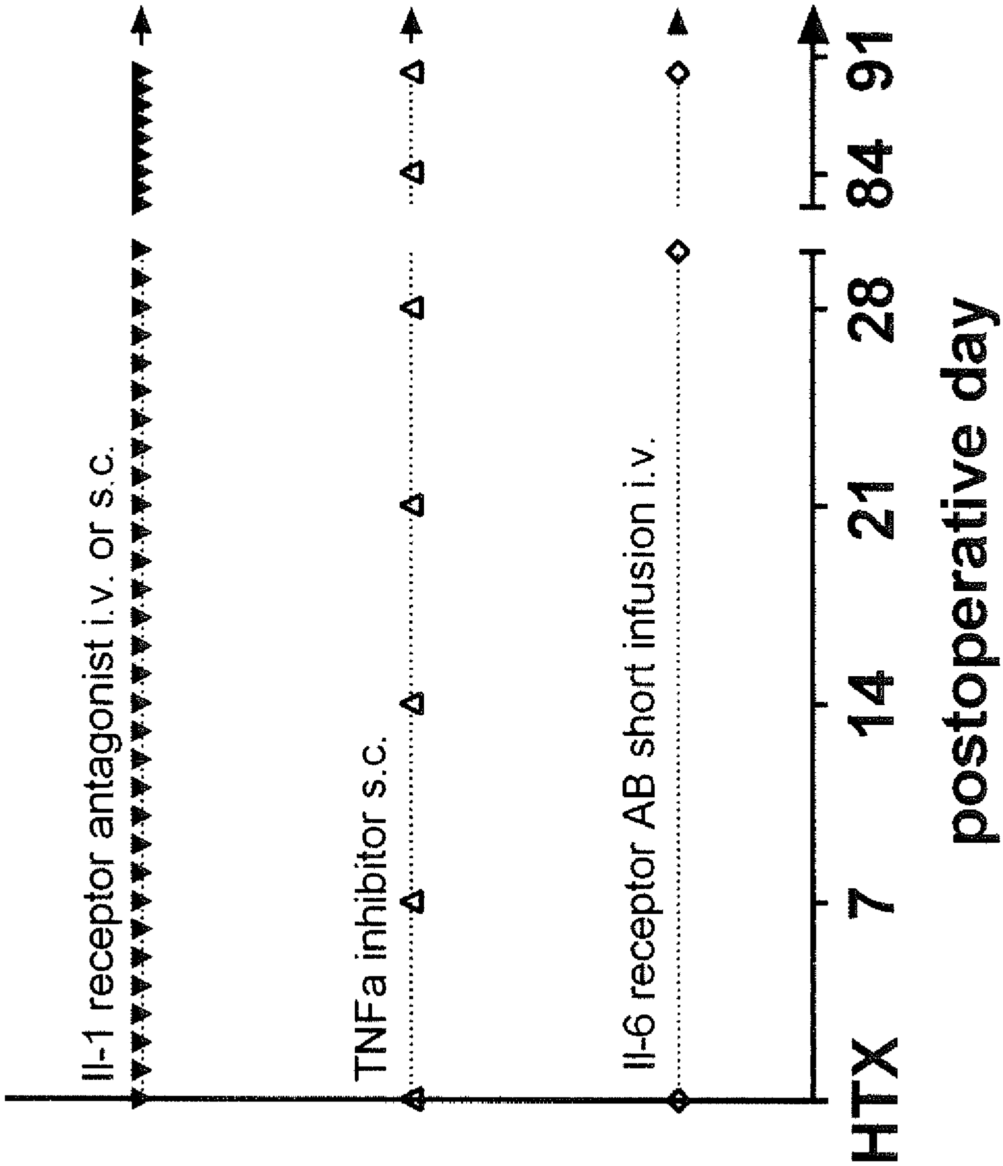

Il-1 receptor antagonist i.v. or s.c.
TNFa inhibitor s.c.
Il-6 receptor AB short infusion i.v.
HTX
7
14
21
28
84
91
postoperative day


FIG. 8

FIG. 9

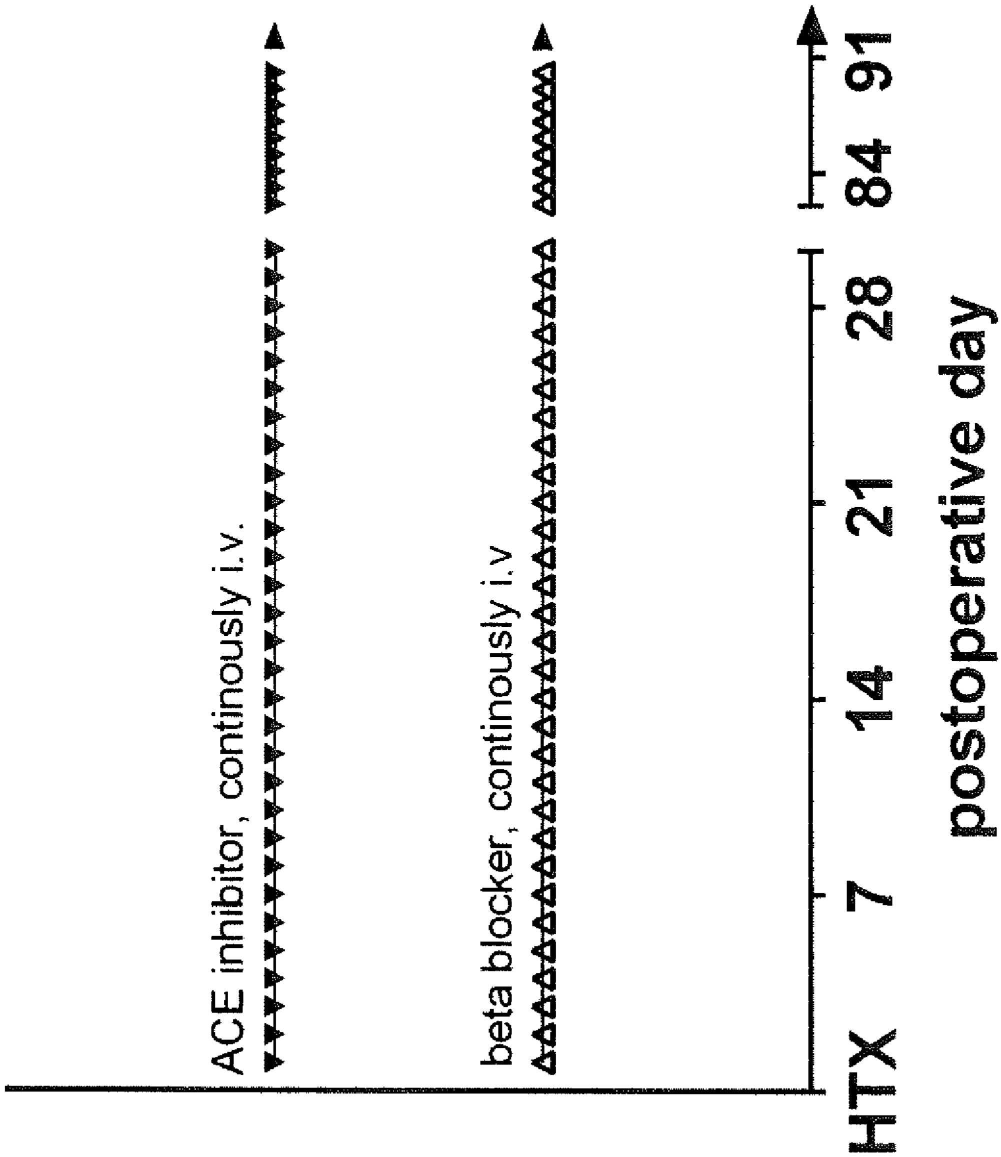
ACE inhibitor, continously i.v.
beta blocker, continously i.v
HTX
7
14
21
28
84
91
postoperative day

FIG. 10

A

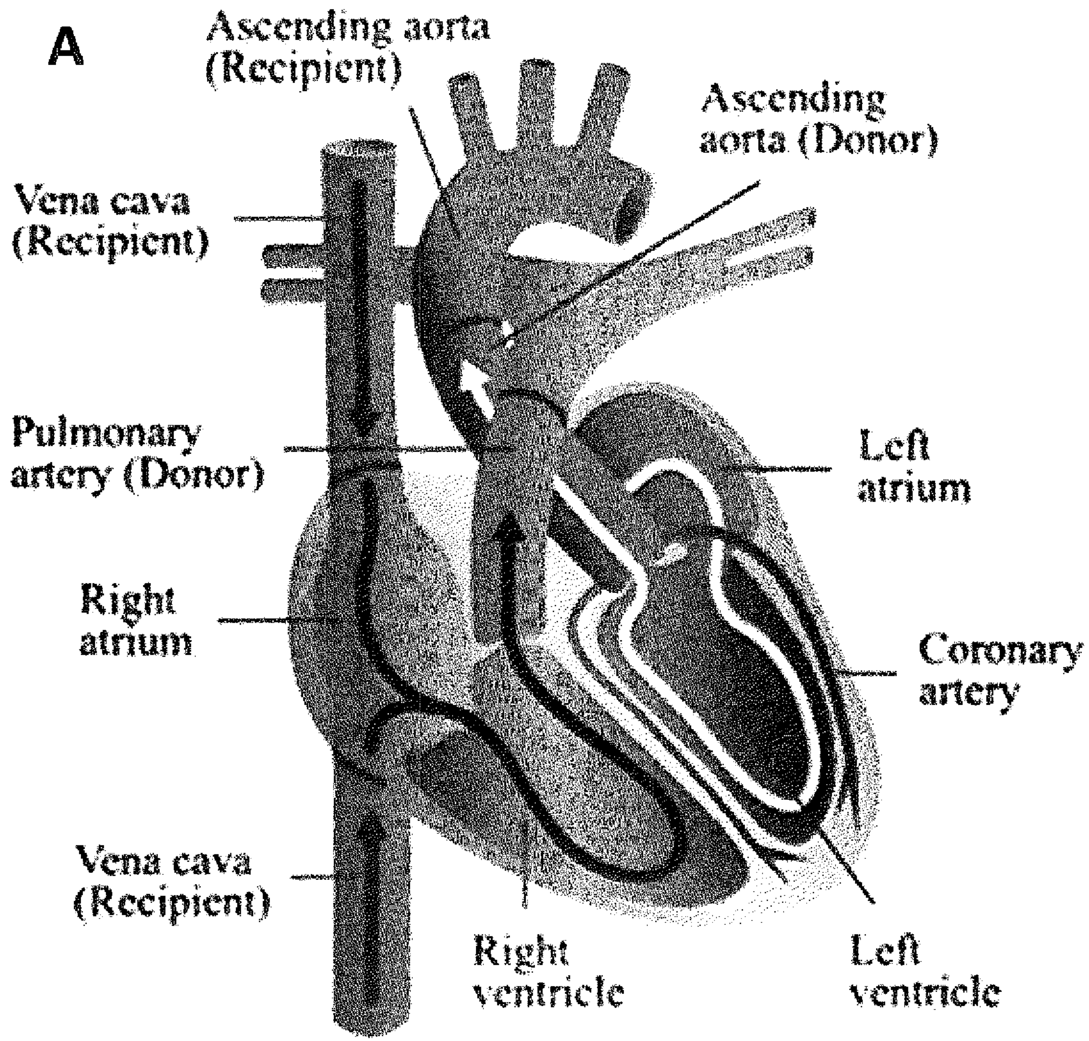
Ascending aorta
(Recipient)
Ascending
aorta (Donor)
Vena cava
(Recipient)
Pulmonary
artery (Donor)
Left
atrium
Right
atrium
Coronary
artery
Vena cava
(Recipient)
Right
ventricle
Left
ventricle

FIG. 10

B

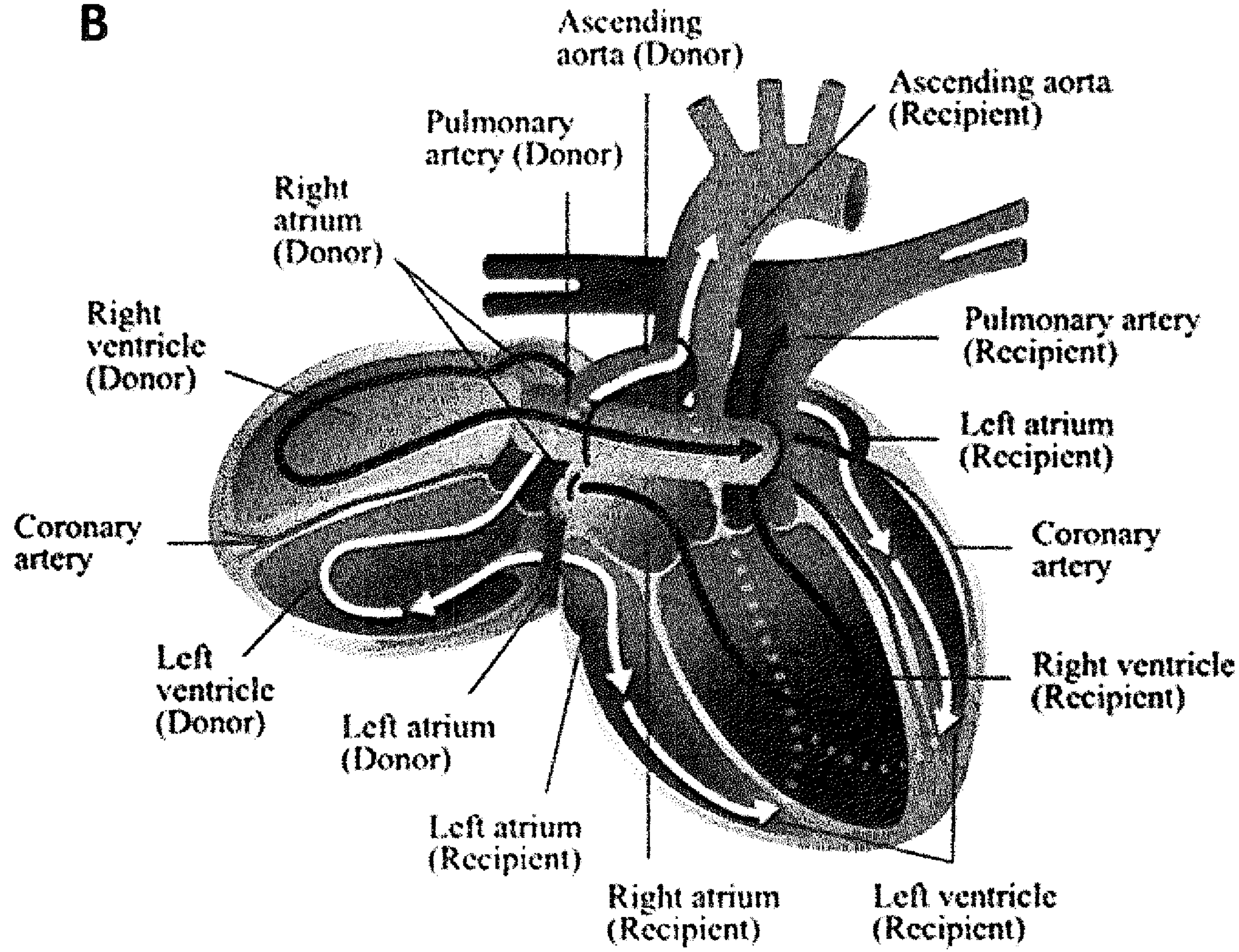
Ascending
aorta (Donor)
Ascending aorta
(Recipient)
Pulmonary
artery (Donor)
Right
atrium
(Donor)
Right
ventricle
(Donor)
Pulmonary artery
(Recipient)
Left atrium
(Recipient)
Coronary
artery
Coronary
artery
Right ventricle
(Recipient)
Left
ventricle
(Donor)
Left atrium
(Donor)
Left atrium
(Recipient)
Right atrium
(Recipient)
Left ventricle
(Recipient)

FIG. 10

C

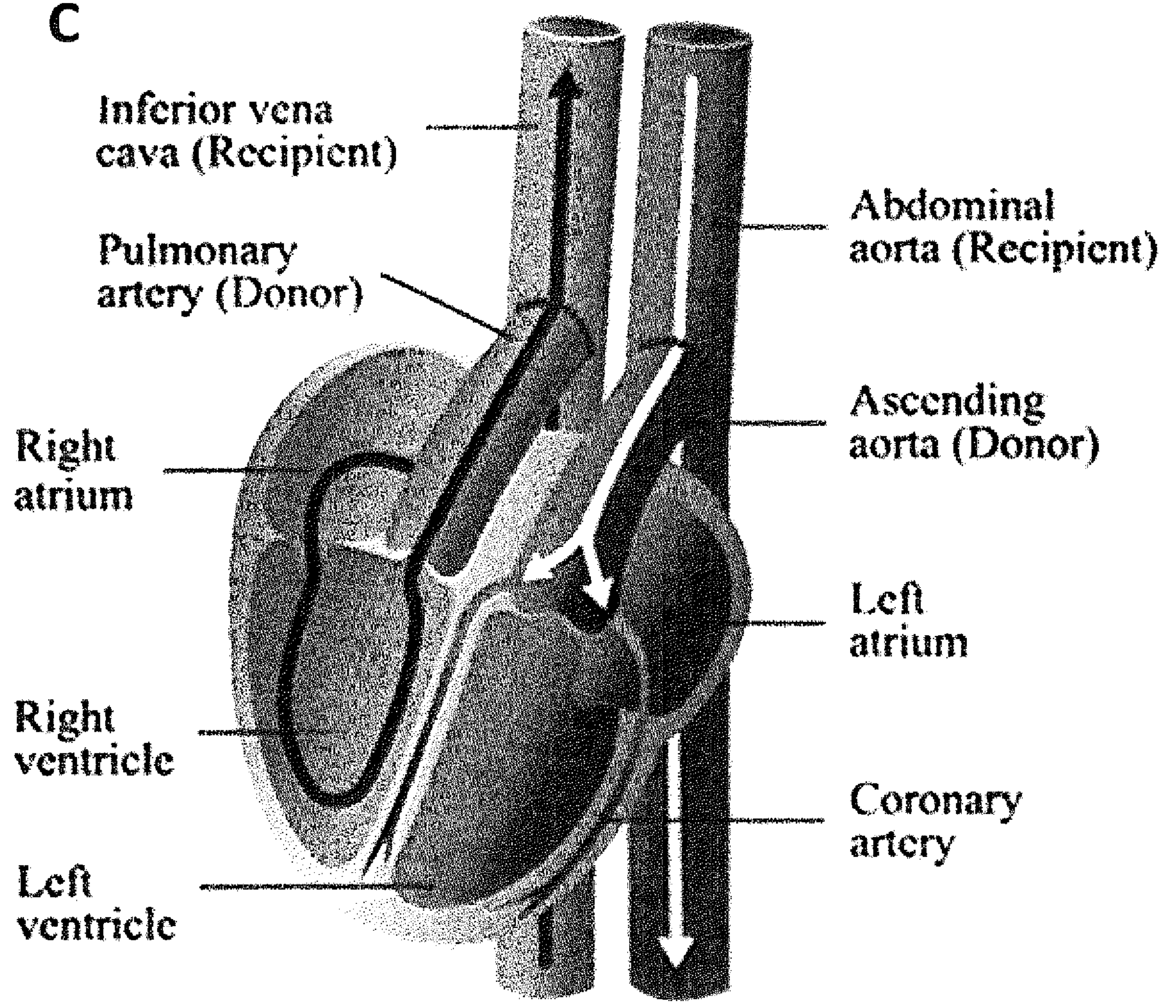
Inferior vena cava (Recipient)
Pulmonary artery (Donor)
Right atrium
Right ventricle
Left ventricle
Abdominal aorta (Recipient)
Ascending aorta (Donor)
Left atrium
Coronary artery

FIG. 11

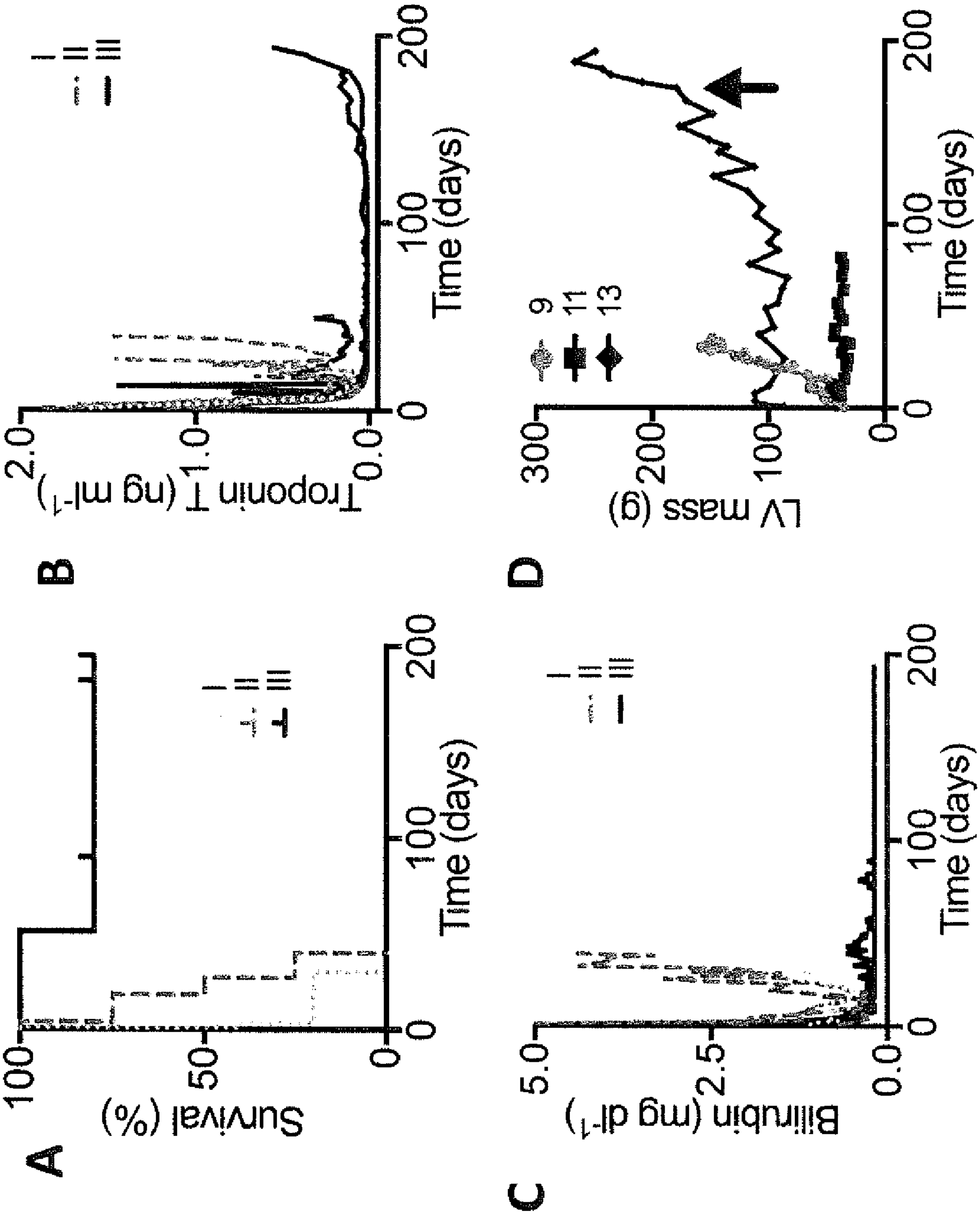
A
Survival (%)
100
50
0
0
100
200
Time (days)
I
II
III
B
Troponin T (ng ml-1)
2.0
1.0
0.0
0
100
200
Time (days)
I
II
III
C
Bilirubin (mg dl-1)
5.0
2.5
0.0
0
100
200
Time (days)
I
II
III
D
LV mass (g)
300
200
100
0
0
100
200
Time (days)
9
11
13

FIG. 11

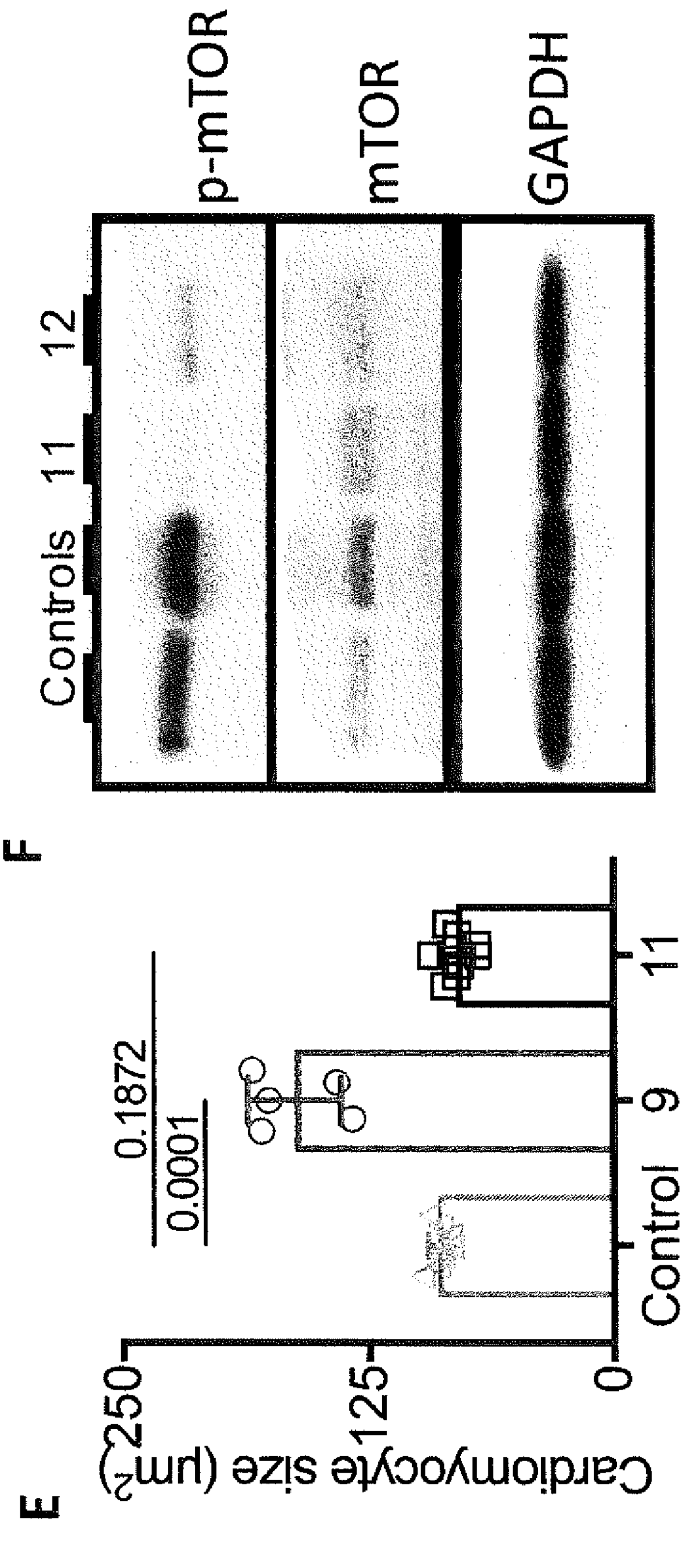
E
Cardiomyocyte size (µm²)
250
125
0
Control
9
11
0.1872
0.0001
F
Controls
11
12
p-mTOR
mTOR
GAPDH

FIG. 12

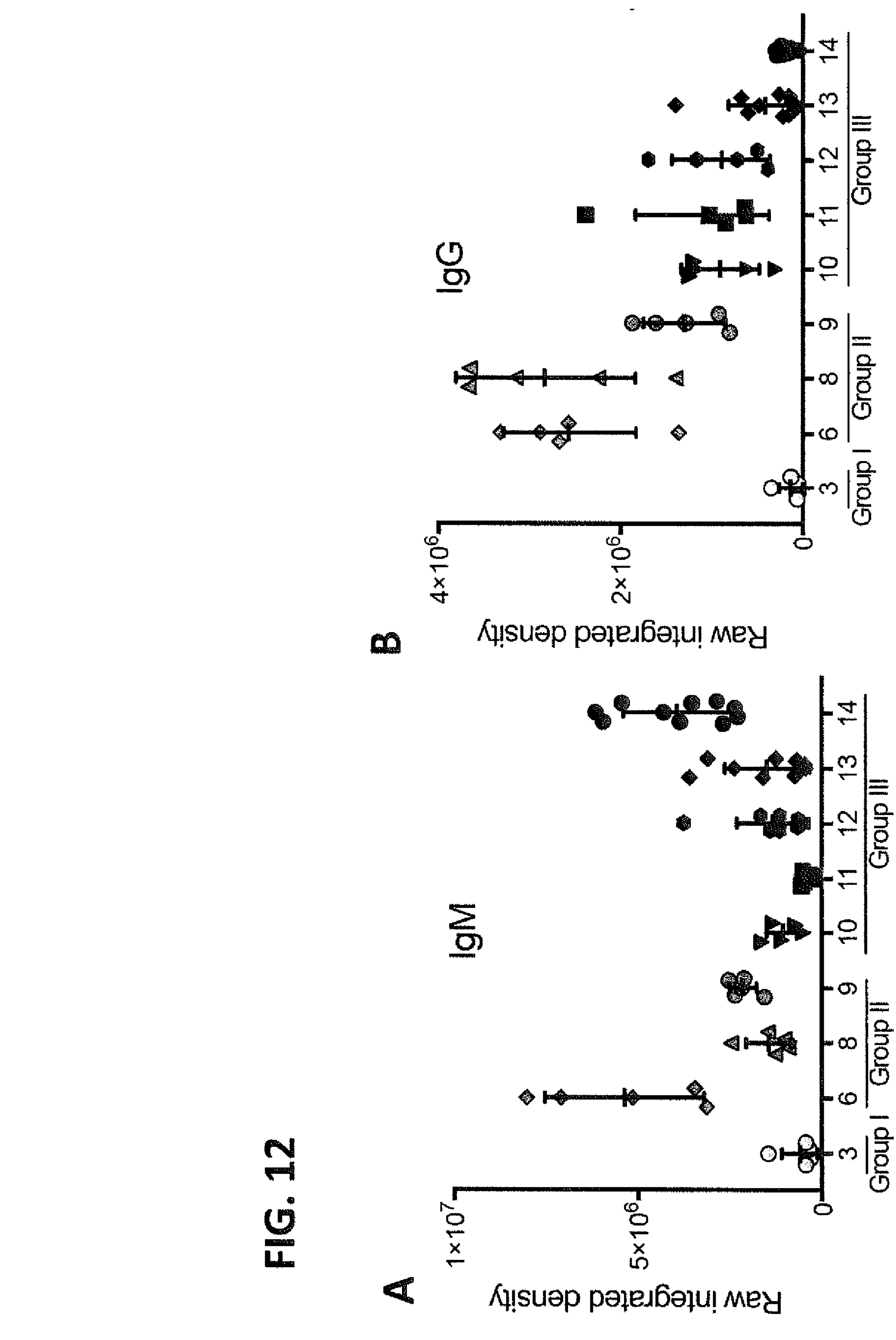
A
IgM
Raw integrated density
1×10^7
5×10^6
0
3
6
8
9
10
11
12
13
14
Group I
Group II
Group III
B
IgG
Raw integrated density
4×10^6
2×10^6
0
3
6
8
9
10
11
12
13
14
Group I
Group II
Group III

FIG. 12

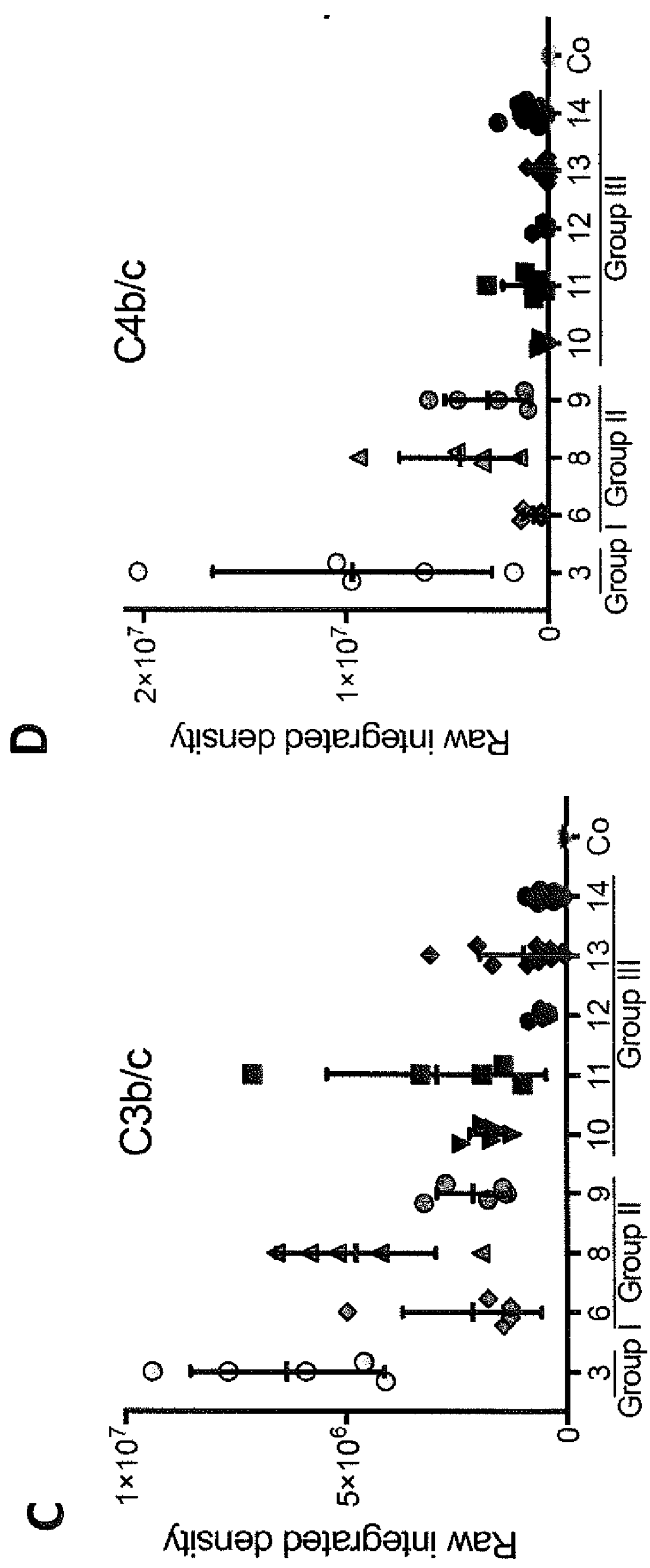
C
C3b/c
Raw integrated density
1×10^7
5×10^6
0
3
6
8
9
10
11
12
13
14
Co
Group I
Group II
Group III
D
C4b/c
Raw integrated density
2×10^7
1×10^7
0
3
6
8
9
10
11
12
13
14
Co
Group I
Group II
Group III

# FIG. 12

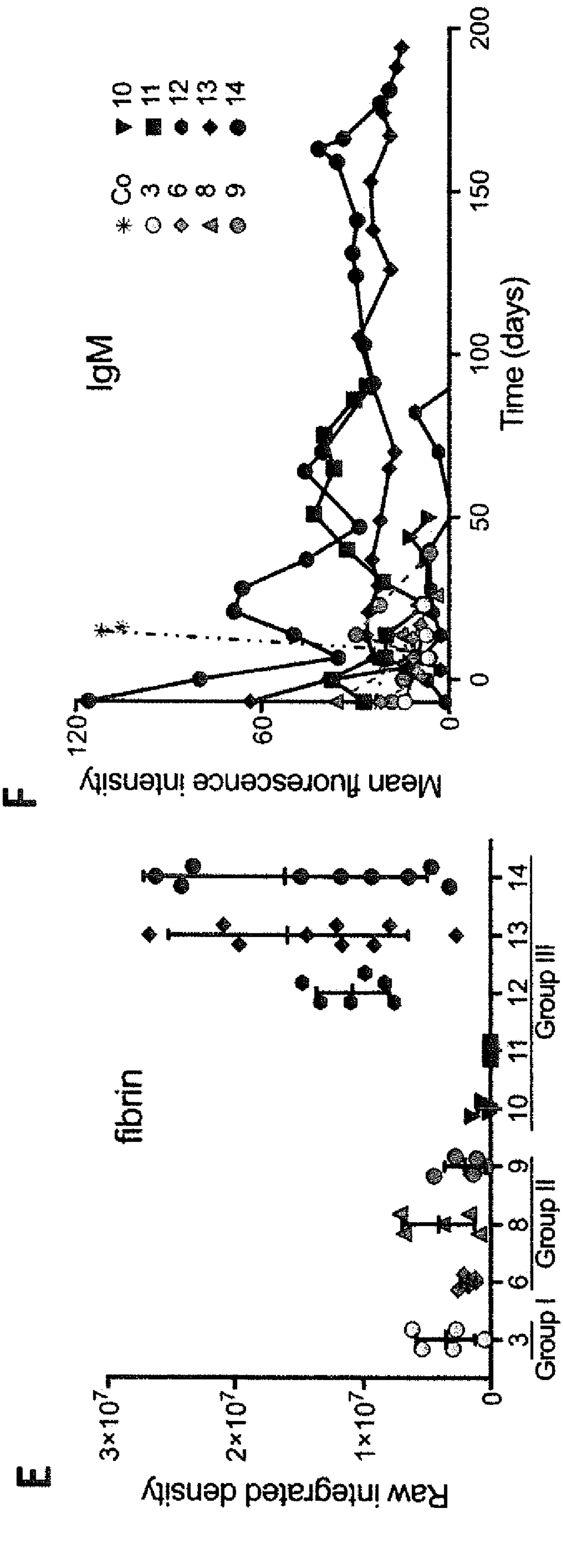
E
fibrin
Raw integrated density
3×10^7
2×10^7
1×10^7
0
3
6
8
9
10
11
12
13
14
Group I
Group II
Group III
F
IgM
Mean fluorescence intensity
120
60
0
50
100
150
200
Time (days)
Co
3
6
8
9
10
11
12
13
14

FIG. 12

G

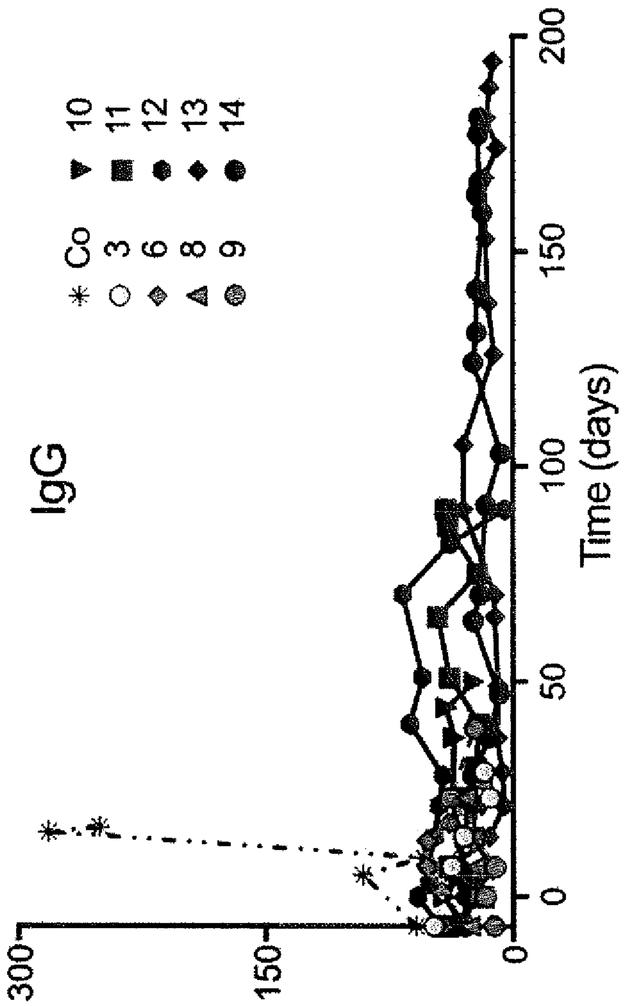
IgG
Mean fluorescence intensity
300
150
0
0
50
100
150
200
Time (days)
Co
3
6
8
9
10
11
12
13
14

FIG. 13

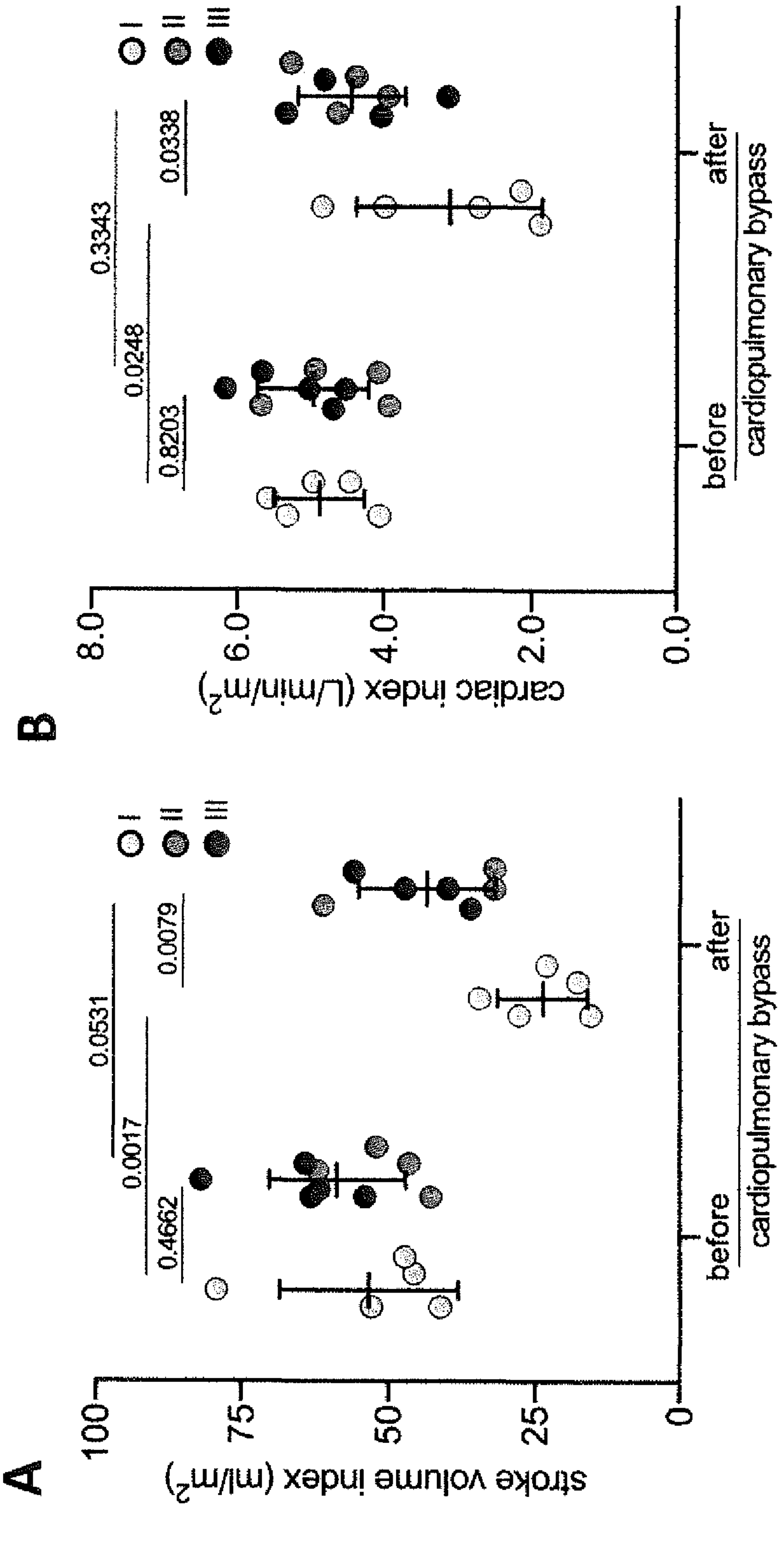
A
stroke volume index (ml/m2)
100
75
50
25
0
0.4662
0.0017
0.0531
0.0079
I
II
III
before
after
cardiopulmonary bypass
B
cardiac index (L/min/m2)
8.0
6.0
4.0
2.0
0.0
0.8203
0.0248
0.3343
0.0338
I
II
III
before
after
cardiopulmonary bypass

FIG. 13

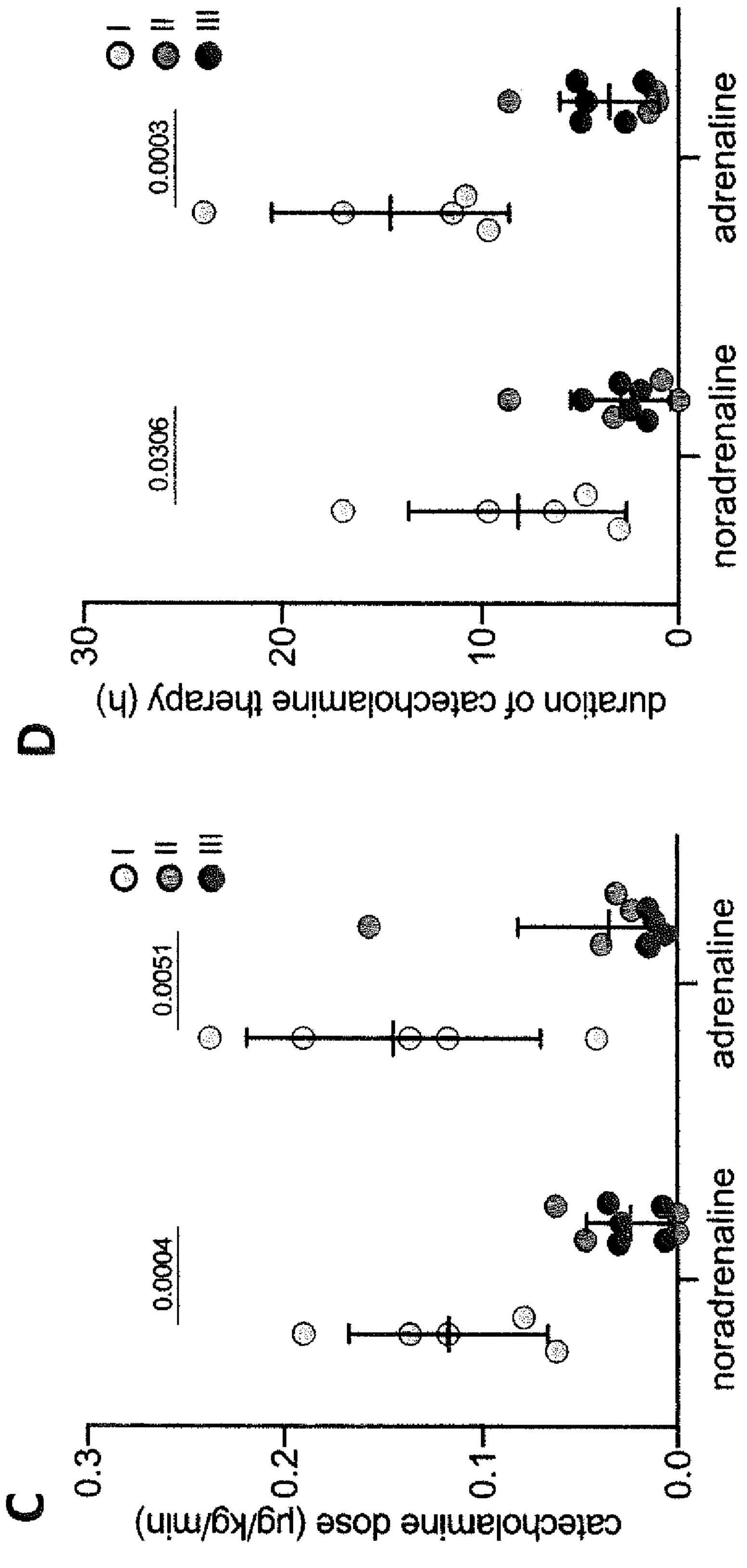
C
catecholamine dose (µg/kg/min)
0.0
0.1
0.2
0.3
noradrenaline
adrenaline
0.0004
0.0051
I
II
III
D
duration of catecholamine therapy (h)
0
10
20
30
noradrenaline
adrenaline
0.0306
0.0003
I
II
III

FIG. 14

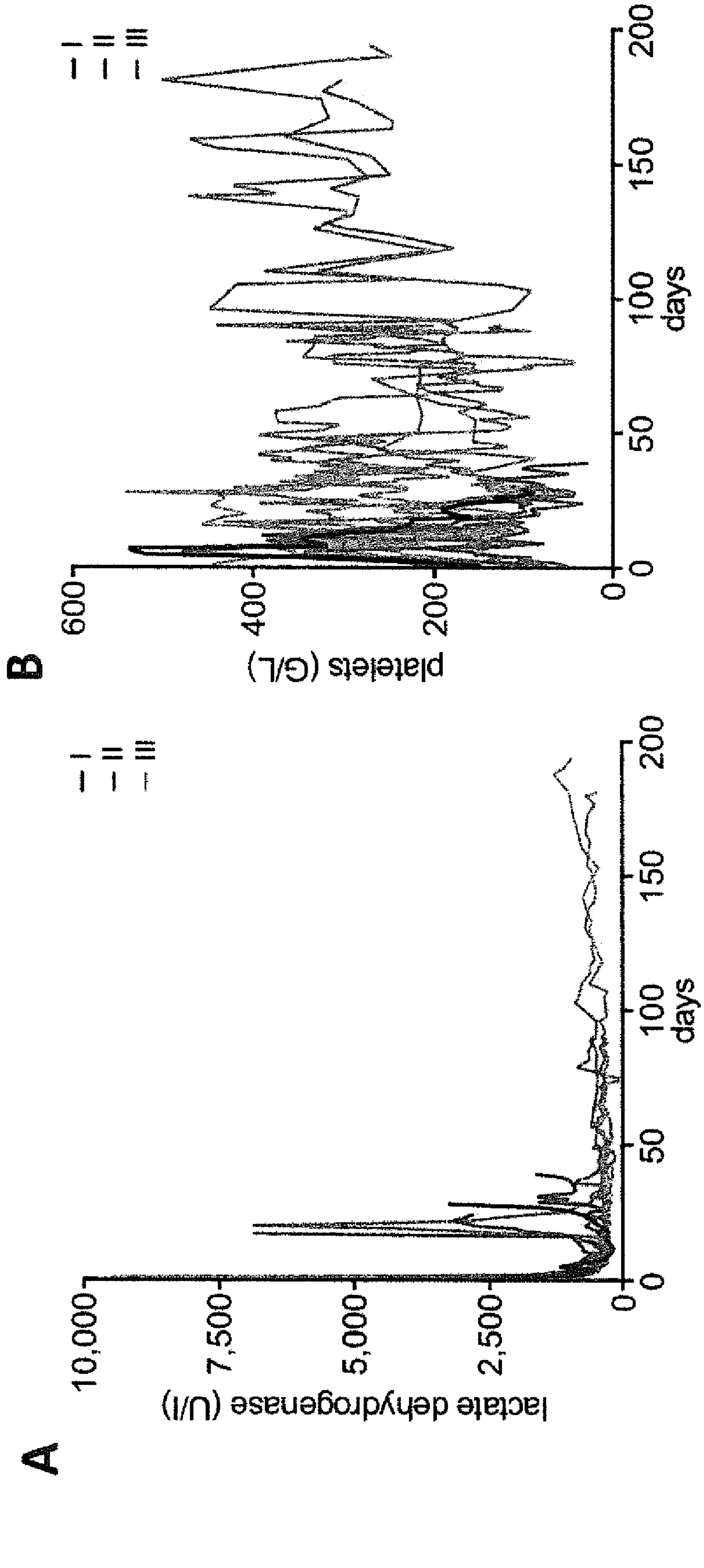
A
lactate dehydrogenase (U/l)
10,000
7,500
5,000
2,500
0
0
50
100
150
200
days
I
II
III
B
platelets (G/L)
600
400
200
0
0
50
100
150
200
days
I
II
III

FIG. 15

A

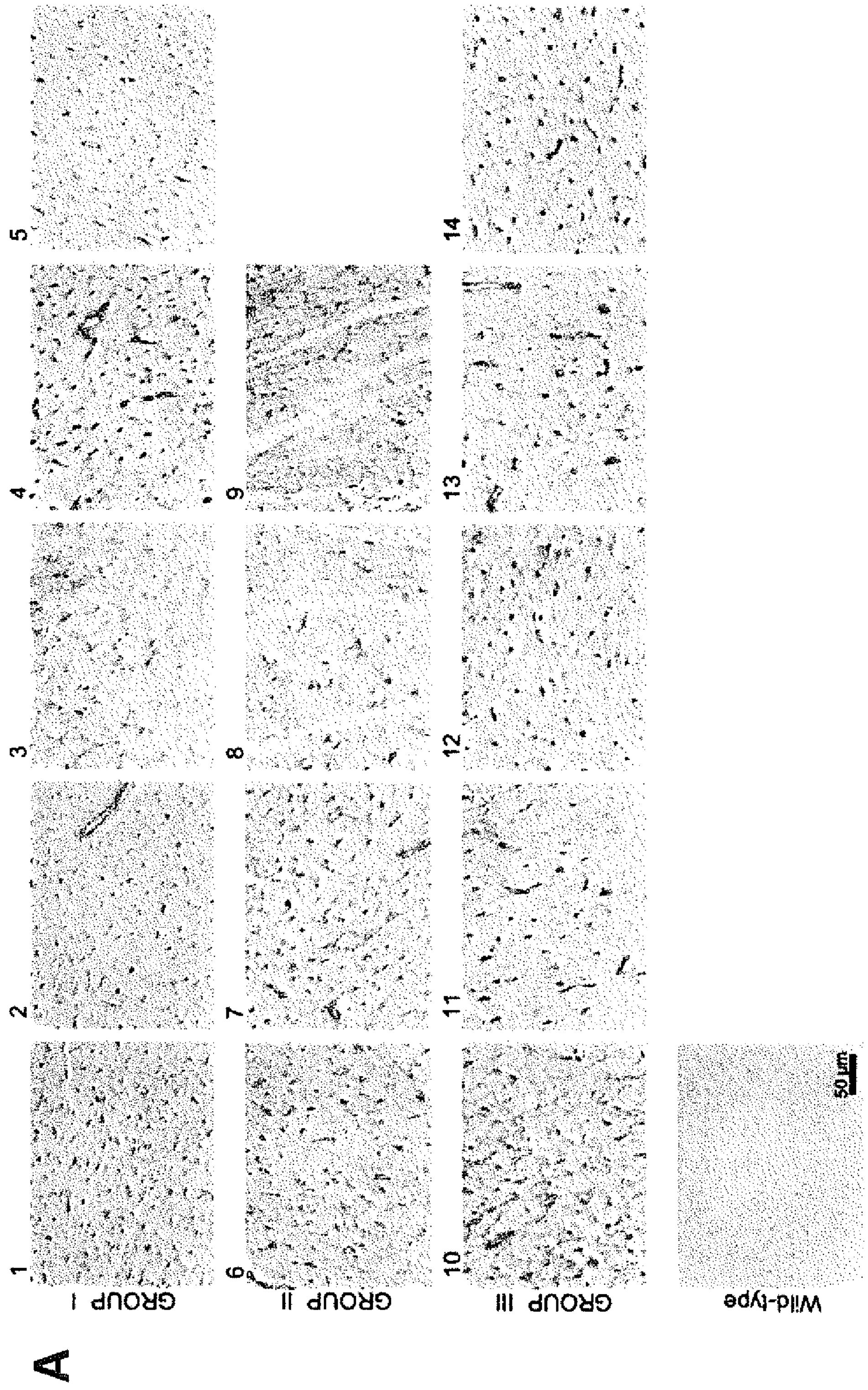
hCD46
GROUP I
GROUP II
GROUP III
Wild-type
1
2
3
4
5
6
7
8
9
10
11
12
13
14
50 µm

FIG. 15

B

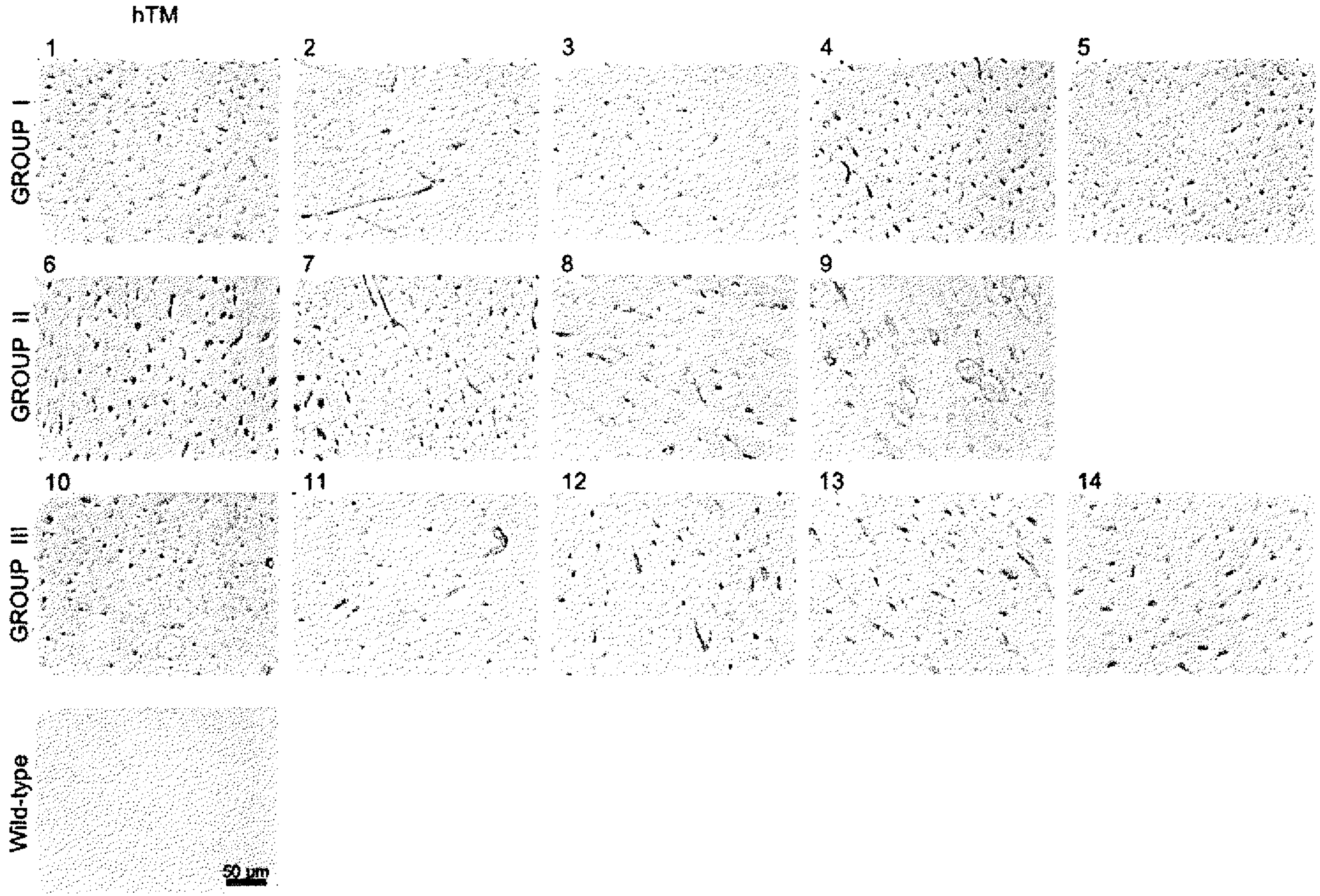
hTM
1
2
3
4
5
6
7
8
9
10
11
12
13
14
GROUP I
GROUP II
GROUP III
Wild-type
50 μm

FIG. 16

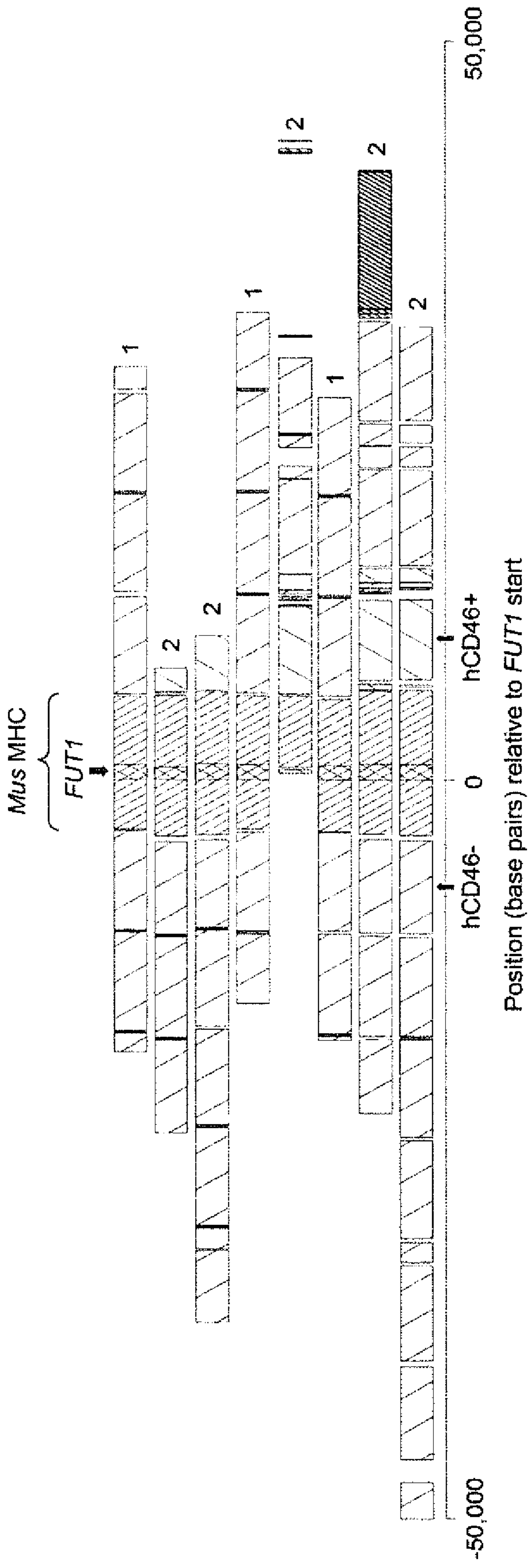
Mus MHC
FUT1
1
2
hCD46-
0
hCD46+
-50,000
50,000
Position (base pairs) relative to FUT1 start

FIG. 17

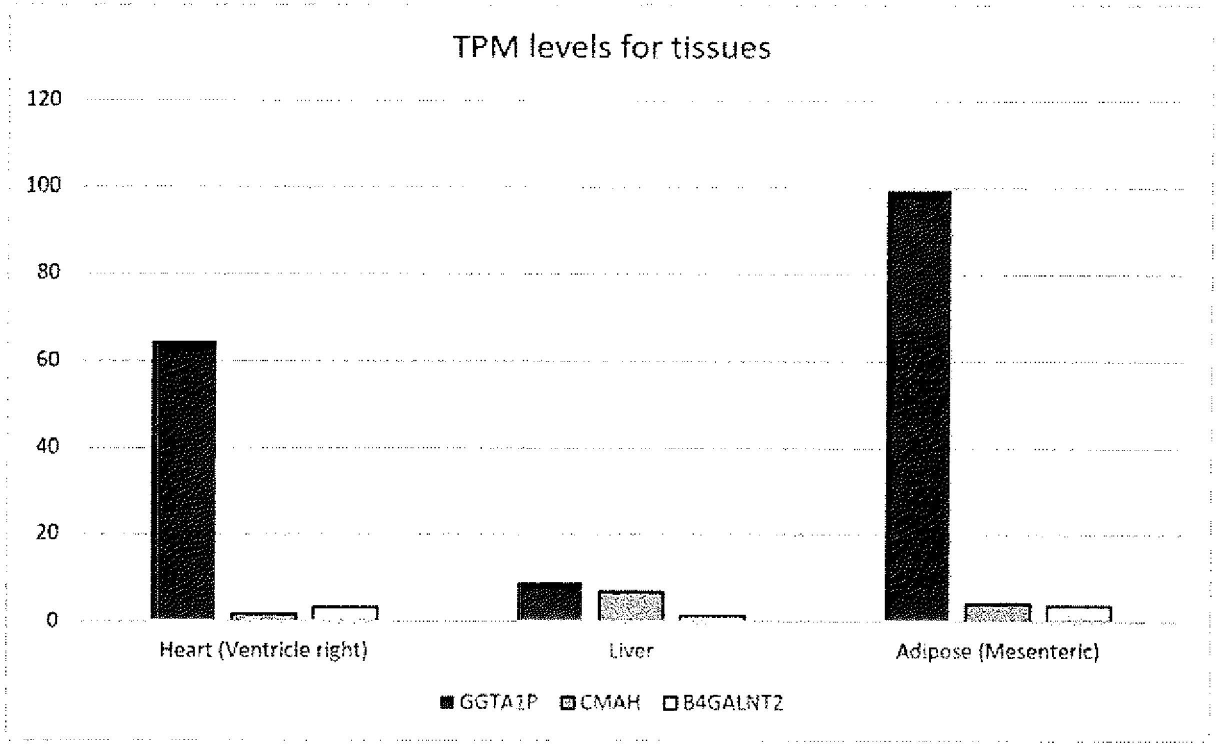


| | Heart (Ventricle right) | Liver | Adipose (Mesenteric) |
|---|---|---|---|
| GGTA1P | 64.3 | 8.8 | 99.2 |
| CMAH | 1.7 | 6.8 | 4.1 |
| B4GALNT2 | 3.2 | 1.2 | 3.6 |

# FIG. 18

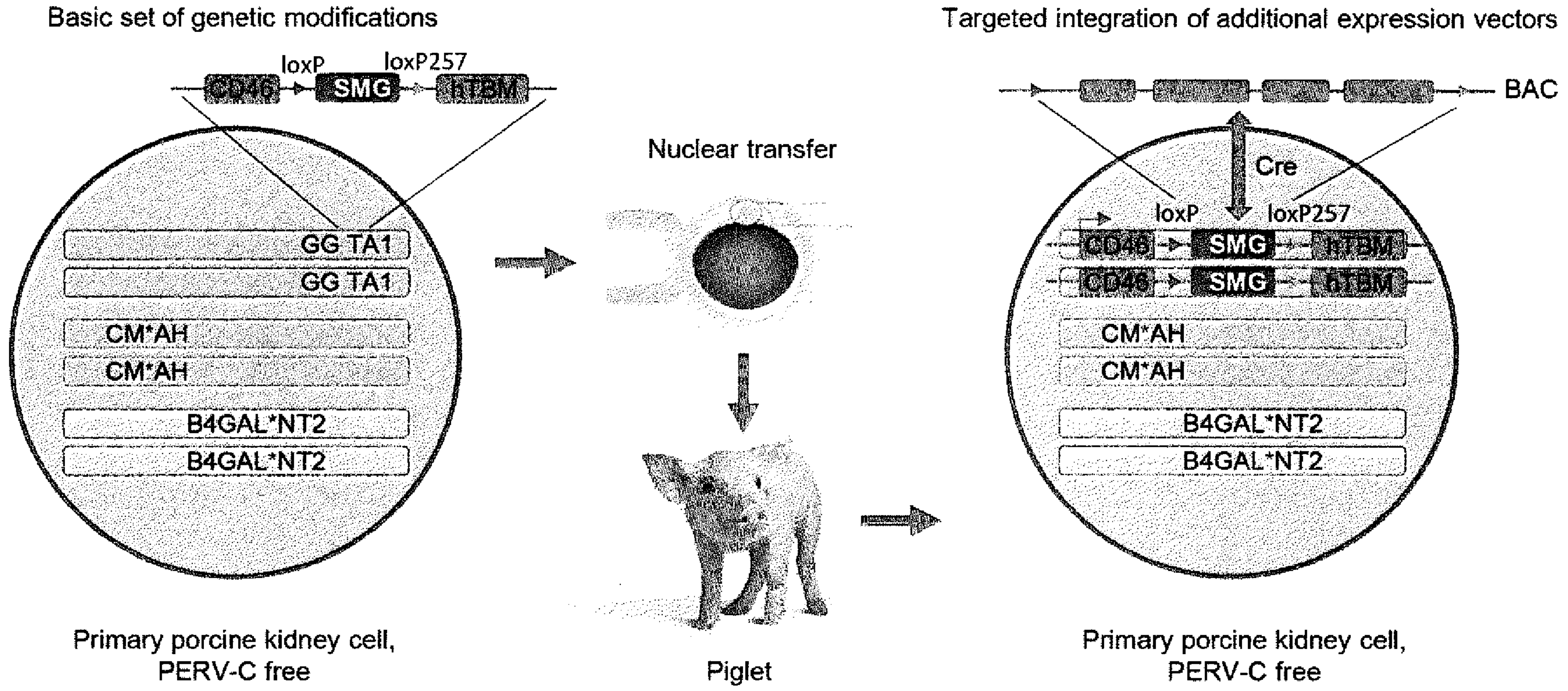
Basic set of genetic modifications
loxP
loxP257
CD46
SMG
hTBM
GG TA1
GG TA1
CM*AH
CM*AH
B4GAL*NT2
B4GAL*NT2
Primary porcine kidney cell,
PERV-C free
Nuclear transfer
Piglet
Targeted integration of additional expression vectors
BAC
Cre
loxP
loxP257
CD46
SMG
hTBM
CD46
SMG
hTBM
CM*AH
CM*AH
B4GAL*NT2
B4GAL*NT2
Primary porcine kidney cell,
PERV-C free

FIG. 19

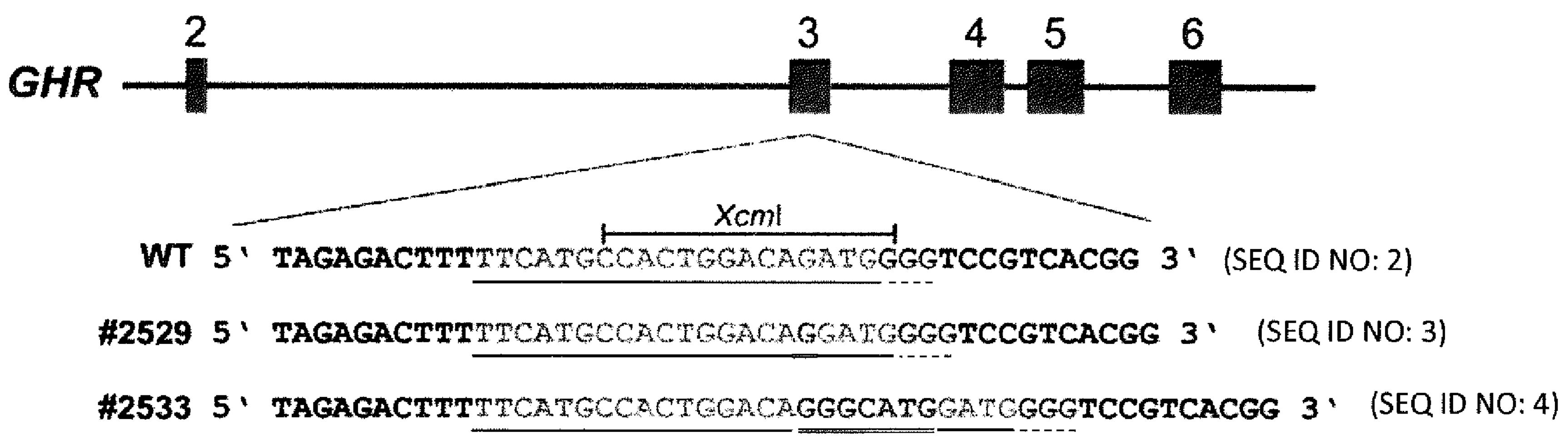
GHR
2
3
4
5
6
XcmI
WT 5' TAGAGACTTTTTCATGCCACTGGACAGATGGGGTCCGTCACGG 3' (SEQ ID NO: 2)
#2529 5' TAGAGACTTTTTCATGCCACTGGACAGGATGGGGTCCGTCACGG 3' (SEQ ID NO: 3)
#2533 5' TAGAGACTTTTTCATGCCACTGGACAGGGCATGGATGGGGTCCGTCACGG 3' (SEQ ID NO: 4)

FIG. 20

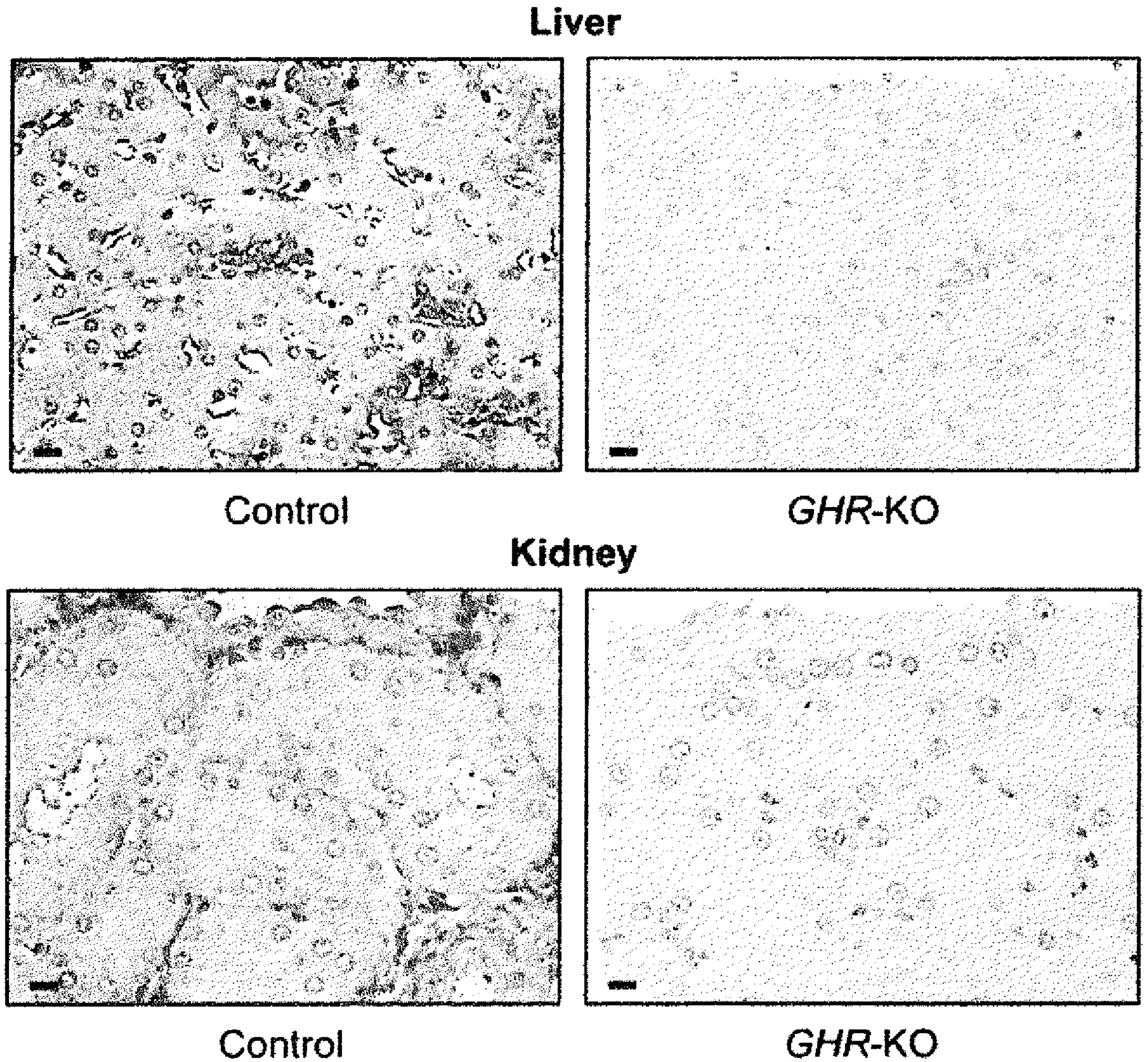

FIG. 21

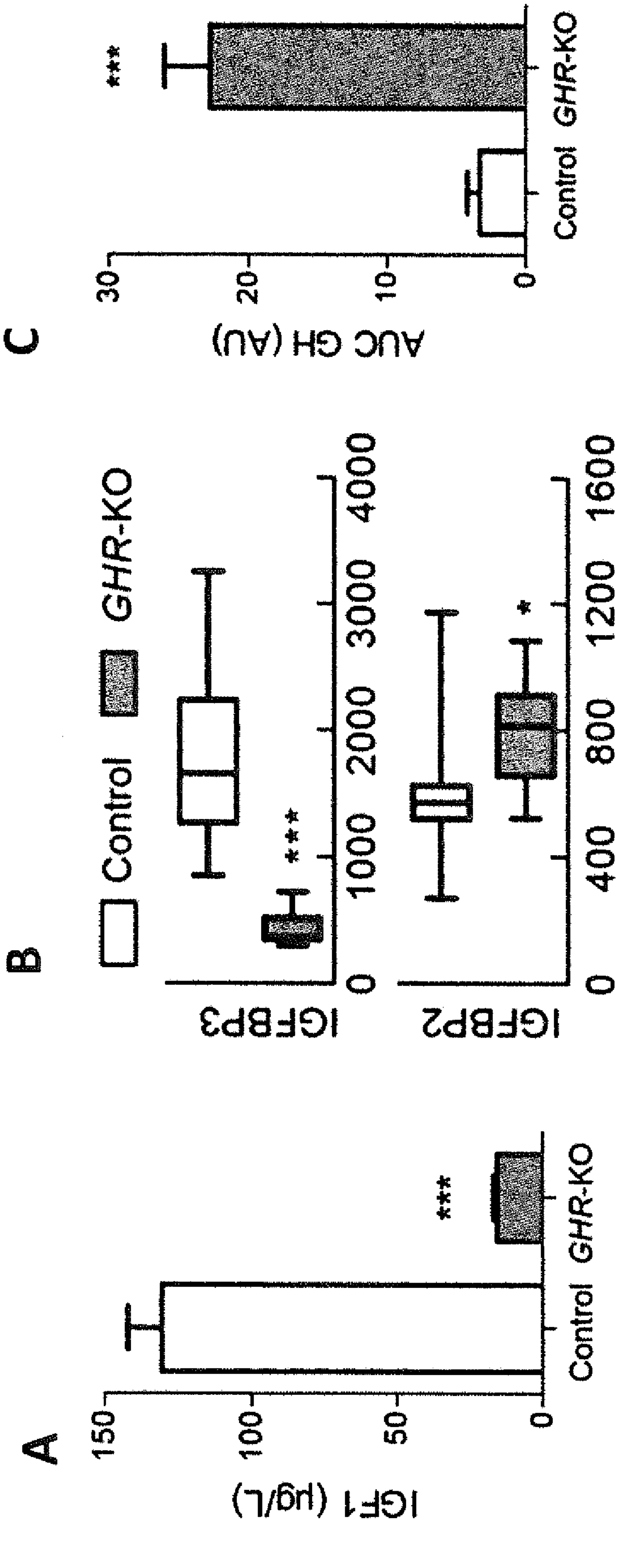
A
IGF1 (μg/L)
150
100
50
0
Control
GHR-KO
***
B
Control
GHR-KO
IGFBP3
***
0
1000
2000
3000
4000
IGFBP2
*
0
400
800
1200
1600
μg/L
C
AUC GH (AU)
30
20
10
0
Control
GHR-KO
***

FIG. 22

A

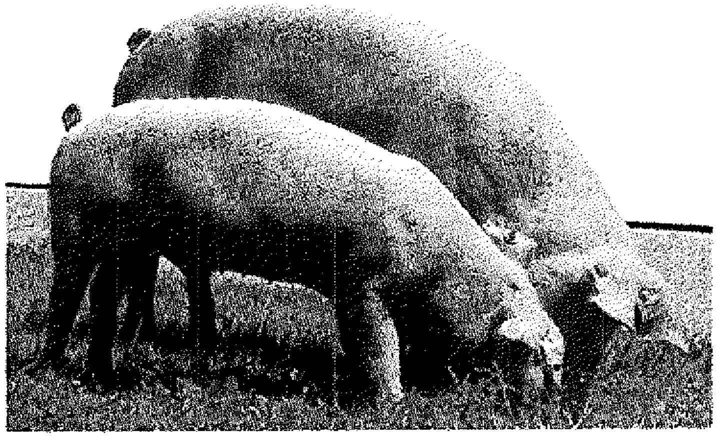

B

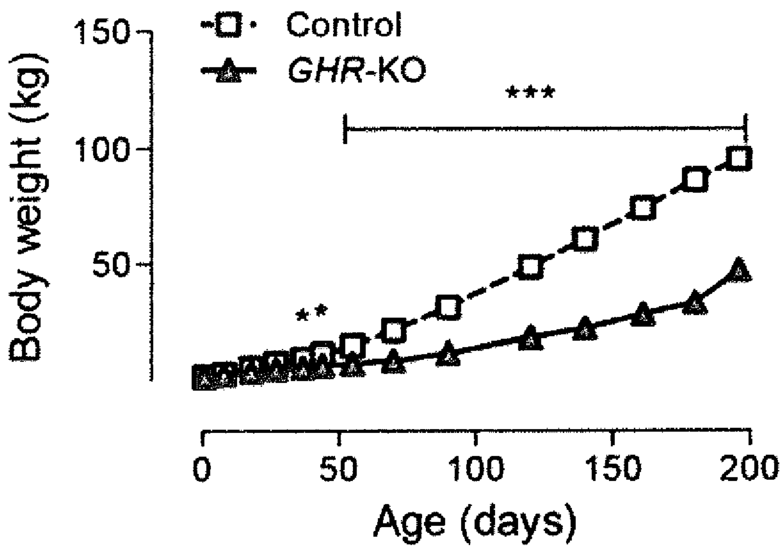
Control
GHR-KO
***
**
150
100
50
Body weight (kg)
0
50
100
150
200
Age (days)

FIG. 23

A

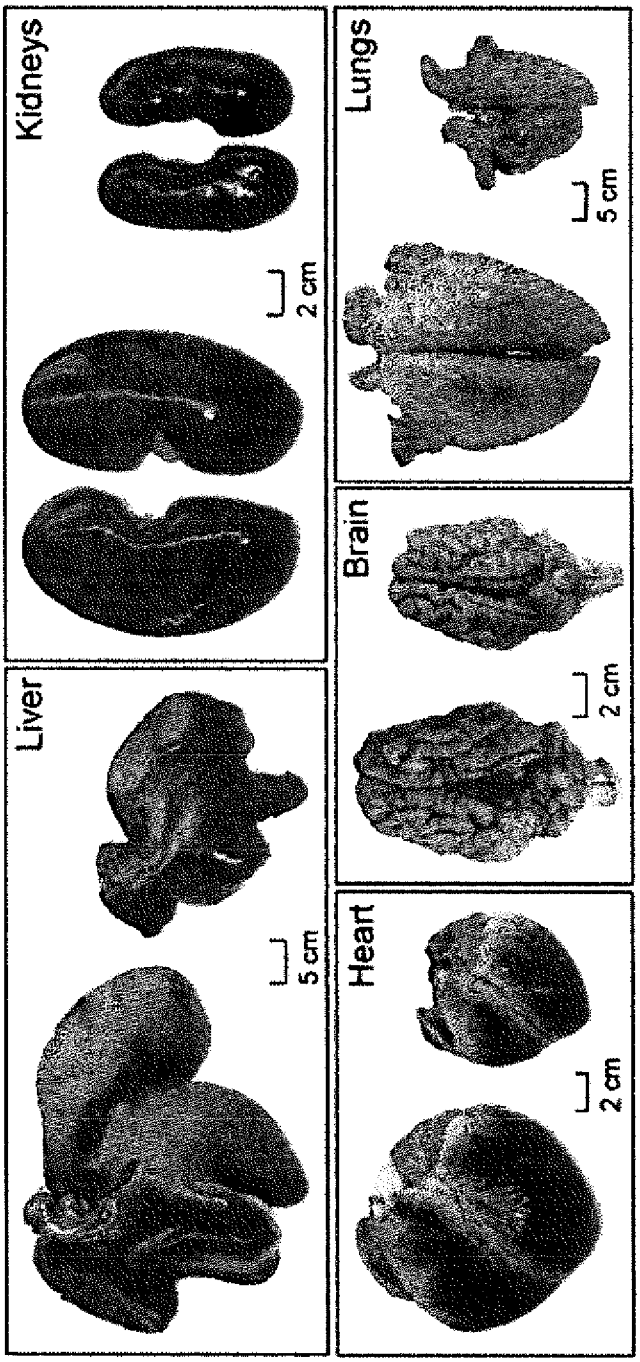

FIG. 23

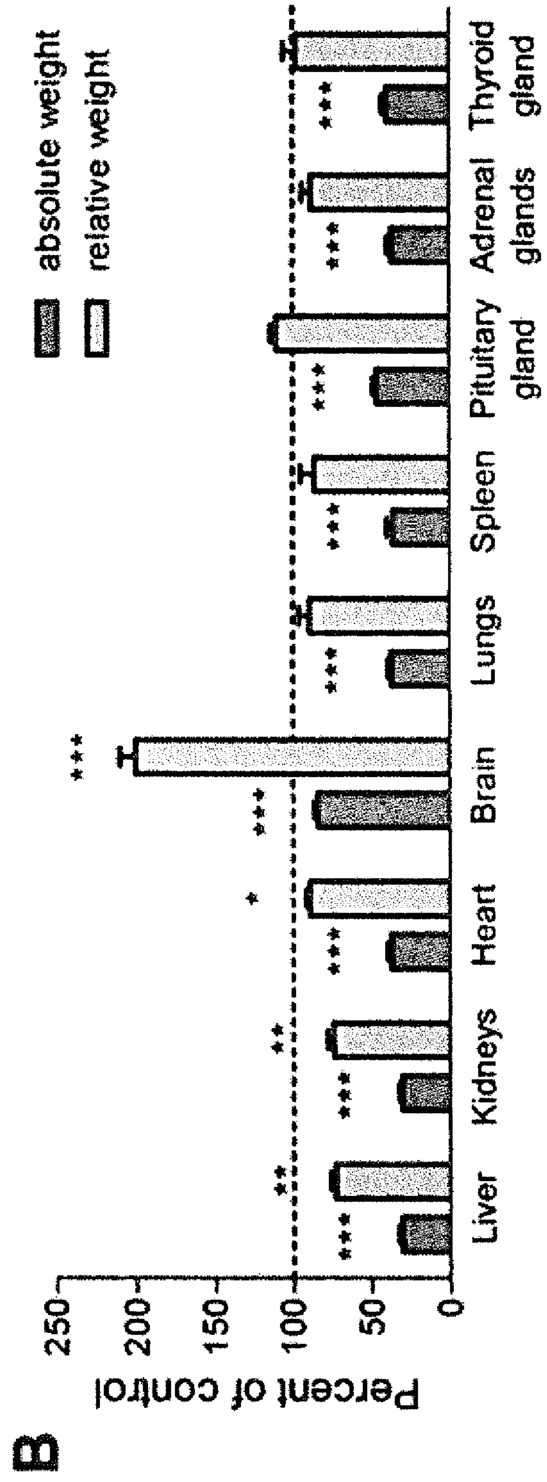
B
absolute weight
relative weight
Percent of control
250
200
150
100
50
0
Liver
Kidneys
Heart
Brain
Lungs
Spleen
Pituitary gland
Adrenal glands
Thyroid gland

FIG. 24

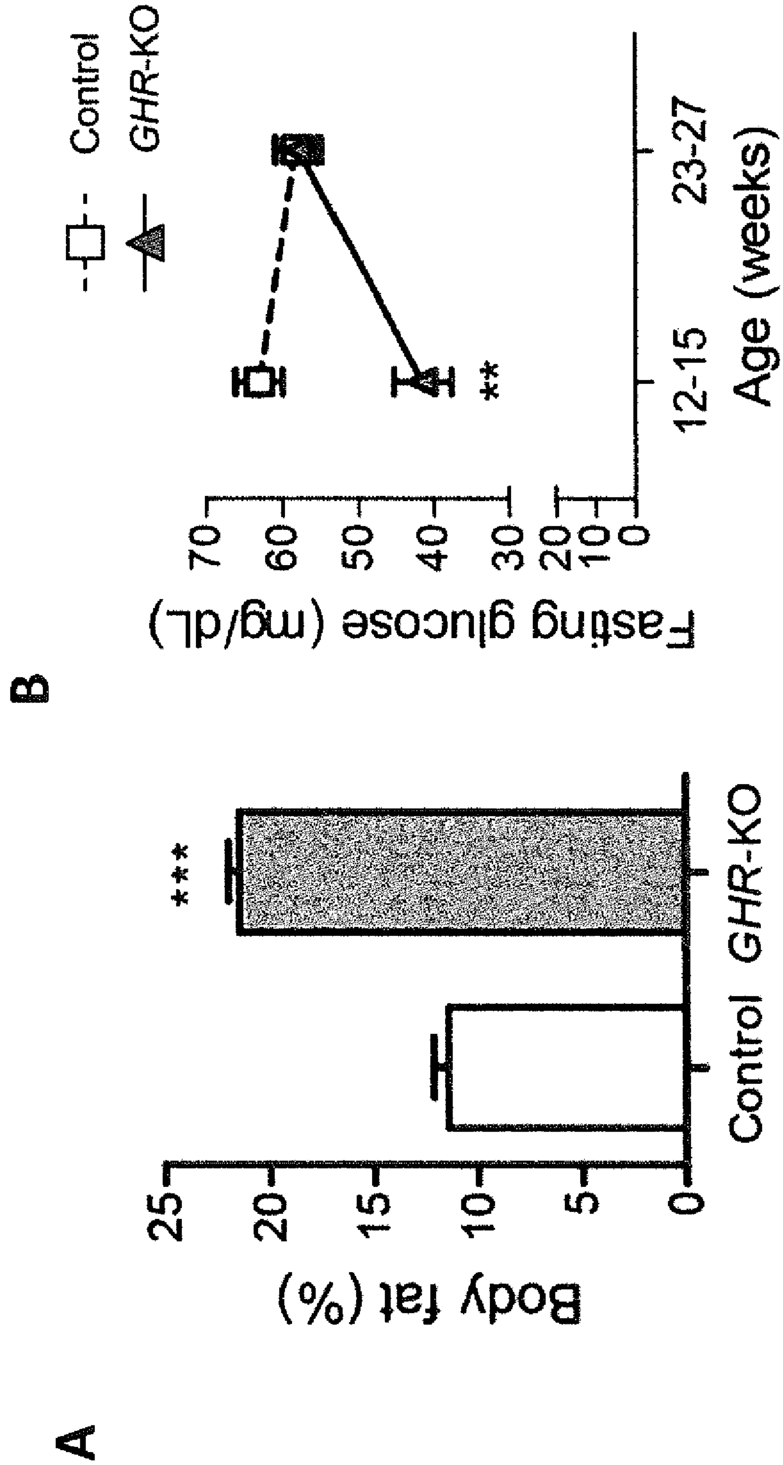
A
Body fat (%)
25
20
15
10
5
0
Control
GHR-KO
***
B
Fasting glucose (mg/dL)
70
60
50
40
30
20
10
0
12-15
23-27
Age (weeks)
Control
GHR-KO
**

FIG. 24

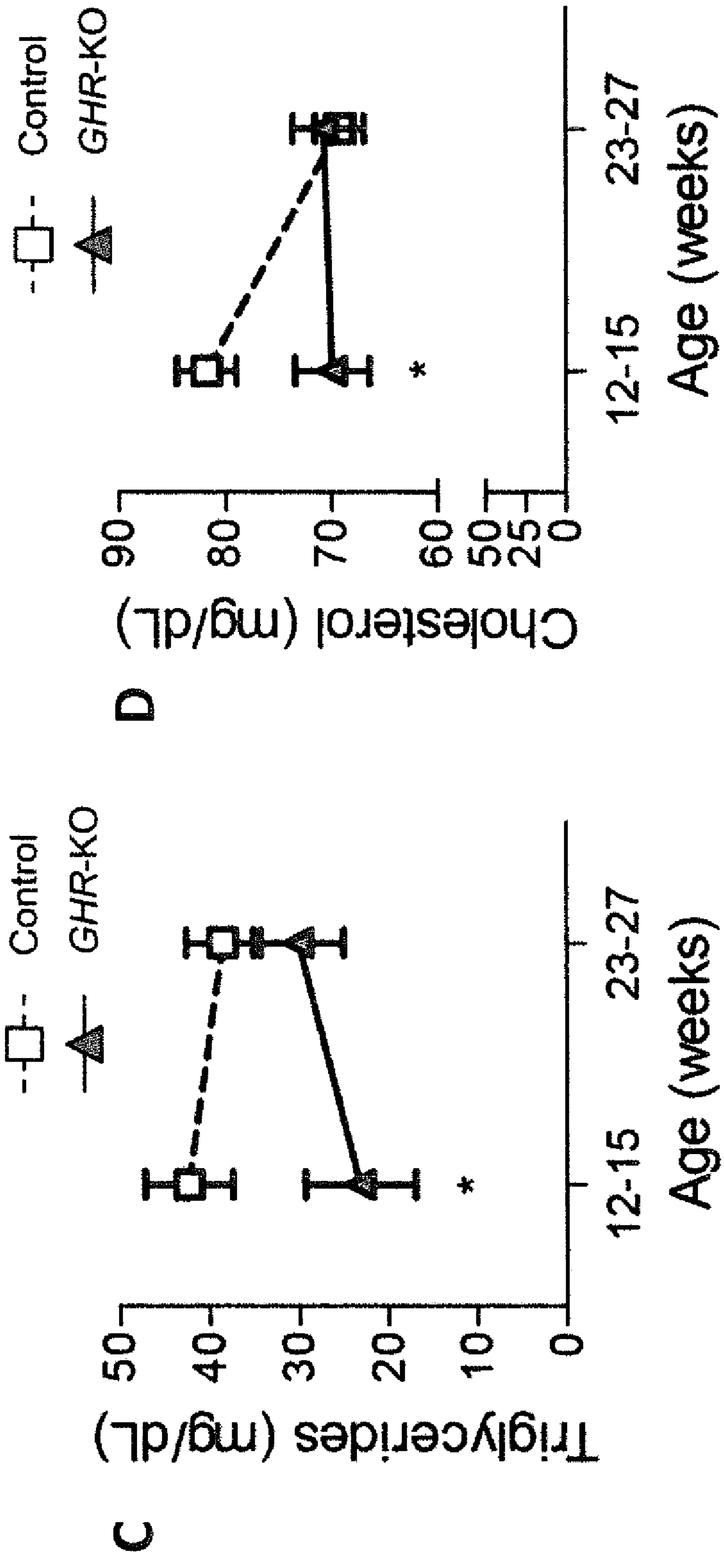
C
Triglycerides (mg/dL)
50
40
30
20
10
0
Control
GHR-KO
*
12-15
23-27
Age (weeks)
D
Cholesterol (mg/dL)
90
80
70
60
50
25
0
Control
GHR-KO
*
12-15
23-27
Age (weeks)

FIG. 25

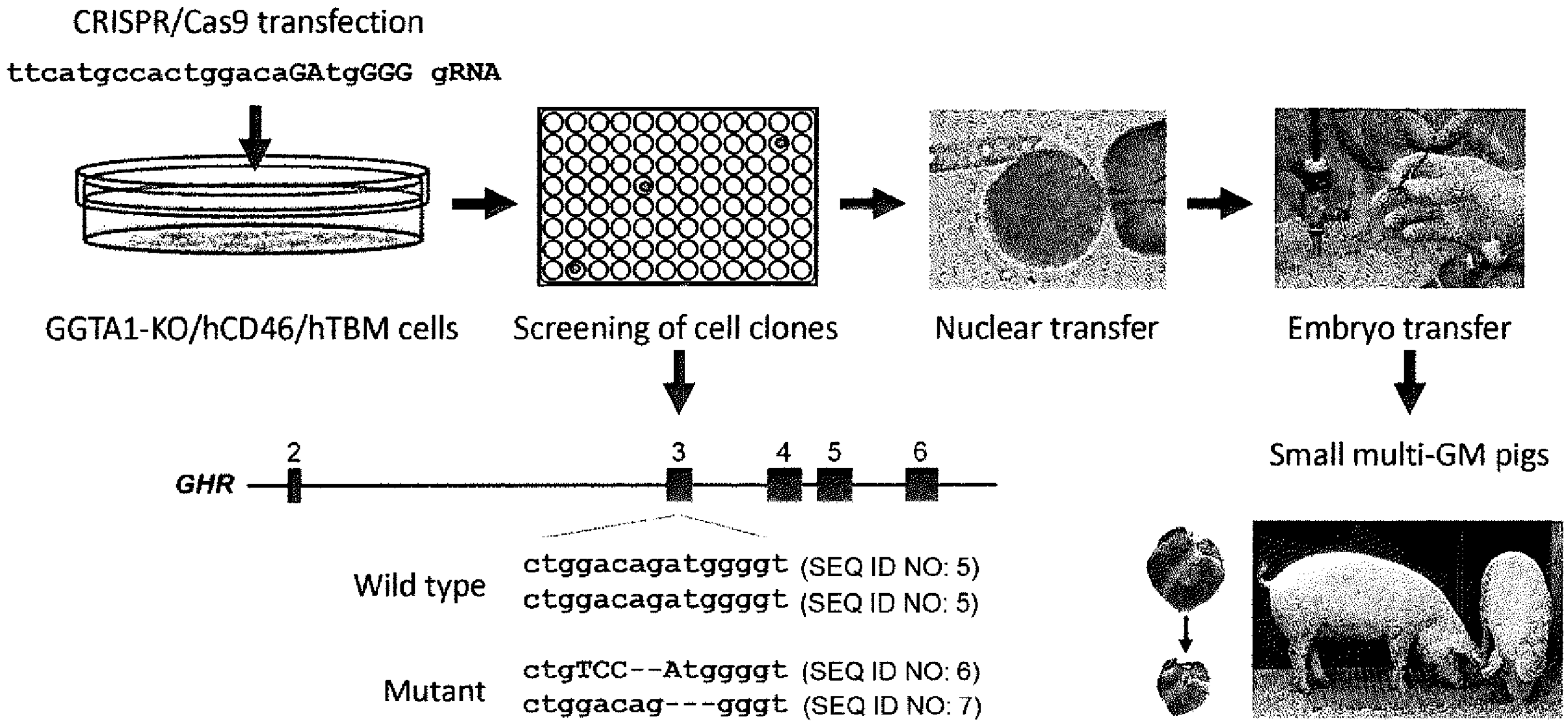
CRISPR/Cas9 transfection
ttcatgccactggacaGAtgGGG gRNA
GGTA1-KO/hCD46/hTBM cells
Screening of cell clones
Nuclear transfer
Embryo transfer
GHR
2
3
4
5
6
Wild type
ctggacagatggggt (SEQ ID NO: 5)
ctggacagatggggt (SEQ ID NO: 5)
Mutant
ctgTCC--Atggggt (SEQ ID NO: 6)
ctggacag---gggt (SEQ ID NO: 7)
Small multi-GM pigs

Europäisches Patentamt
European Patent Office
Office européen des brevets

# EUROPEAN SEARCH REPORT

Application Number
EP 22 19 2363

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | STIG STEEN ET AL: "Safe orthotopic transplantation of hearts harvested 24 hours after brain death and preserved for 24 hours", SCANDINAVIN CARDIOVASCULAR JOURNAL, vol. 50, no. 3, 3 May 2016 (2016-05-03), pages 193-200, XP055404794, NO ISSN: 1401-7431, DOI: 10.3109/14017431.2016.1154598 * the whole document * ----- | 1-14 | INV. A61K45/06 A61K31/436 A61K31/138 A61K31/401 A61P37/06 |
| X | PAOLO BRENNER ET AL: "40 Days Survival after Orthotopic Cardiac Xenotransplantation of Multi-Transgenic Pig Hearts in a Pig-to-Baboon Model with CD40mAb or CD40L Costimulation Blockade and Xenograft Preservation using "Steens" Cold Blood Cardioplegia Perfusion", TRANSPLANTATION, vol. 101, no. 5S-3, 24 May 2017 (2017-05-24), page S65, XP055506519, * the whole document * ----- -/-- | 1-14 | TECHNICAL FIELDS SEARCHED (IPC): A61K A61P |

The present search report has been drawn up for all claims

1

| Place of search | Date of completion of the search | Examiner |
|---|---|---|
| The Hague | 8 June 2023 | Galli, Ivo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 22 19 2363

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BELWAY D ET AL: "Temperature management and monitoring practices during adult cardiac surgery under cardiopulmonary bypass: results of a Canadian national survey", PERFUSION, vol. 26, no. 5, 1 September 2011 (2011-09-01), pages 395-400, XP093052869, ISSN: 0267-6591, DOI: 10.1177/0267659111409095 Retrieved from the Internet: URL:http://journals.sagepub.com/doi/full-xml/10.1177/0267659111409095> * the whole document * ----- | 1-14 | |
| A | ENGELMAN RICHARD: "The Society of Thoracic Surgeons, The Society of Cardiovascular Anesthesiologists, and The American Society of ExtraCorporeal Technology: Clinical Practice Guidelines for Cardiopulmonary Bypass--Temperature Management during Cardiopulmonary Bypass", J. EXTRA-CORPOR TECHNOL. 47(3), 1 September 2015 (2015-09-01), pages 145-154, XP093052863, Retrieved from the Internet: URL:http://www.ncbi.nlm.nih.gov/pubmed/26543248> * the whole document * ----- | 1-14 | TECHNICAL FIELDS SEARCHED (IPC) |

The present search report has been drawn up for all claims

1

| Place of search | Date of completion of the search | Examiner |
|---|---|---|
| The Hague | 8 June 2023 | Galli, Ivo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Eurotransplant. Annual Report 2016. Eurotransplant International Foundation, 2017, 104
- **LUND, L. H. et al.** The Registry of the International Society for Heart and Lung Transplantation: Thirty-fourth Adult Heart Transplantation Report-2017; Focus Theme: Allograft ischemic time. *J. Heart Lung Transplant.,* 2017, vol. 36, 1037-1046
- **ROSSANO, J. W. et al.** The Registry of the International Society for Heart and Lung Transplantation: Twentieth Pediatric Heart Transplantation Report-2017; Focus Theme: Allograft ischemic time. *J. Heart Lung Transplant.,* 2017, vol. 36, 1060-1069
- **KIRKLIN, J. K. et al.** Eighth annual INTERMACS report: Special focus on framing the impact of adverse events. *J. Heart Lung Transplant.,* 2017, vol. 36, 1080-1086
- **MOHIUDDIN, M. M. et al.** Chimeric 2C10R4 anti-CD40 antibody therapy is critical for long-term survival of GTKO.hCD46.hTBM pig-to-primate cardiac xenograft. *Nat Commun,* 2016, vol. 7, 11138
- **COOPER, D. K. et al.** Report of the Xenotransplantation Advisory Committee of the International Society for Heart and Lung Transplantation: the present status of xenotransplantation and its potential role in the treatment of end-stage cardiac and pulmonary diseases. *J. Heart Lung Transplant.,* 2000, vol. 19, 1125-1165
- **MOHIUDDIN, M. M. ; REICHART, B. ; BYRNE, G. W. ; MCGREGOR, C. G. A.** Current status of pig heart xenotransplantation. *International Journal of Surgery,* 2015, vol. 23, 234-239
- **LOWER, R. R. ; SHUMWAY, N. E.** Studies on orthotopic homotransplantation of the canine heart. *Surg Forum,* 1960, vol. 11, 18-19
- **BINDER, U. ; SKERRA, A.** PASylation®: A versatile technology to extend drug delivery. *Current Opinion in Colloid & Interface Science,* 2017, vol. 31, 10-17
- **STEEN, S. ; PASKEVICIUS, A. ; LIAO, Q. ; SJÖBERG, T.** Safe orthotopic transplantation of hearts harvested 24 hours after brain death and preserved for 24 hours. *Scand. Cardiovasc. J.,* 2016, vol. 50, 193-200
- **MAYR, T. et al.** Hemodynamic and perioperative management in two different preclinical pig-to-baboon cardiac xenotransplantation models. *Xenotransplantation,* 2017, 24
- **LANG, R. M. et al.** Recommendations for chamber quantification: a report from the American Society of Echocardiography's Guidelines and Standards Committee and the Chamber Quantification Writing Group, developed in conjunction with the European Association of Echocardiography, a branch of the European Society of Cardiology. *Journal of the American Society of Echocardiography : official publication of the American Society of Echocardiography,* 2005, vol. 18, 1440-1463
- **DEVEREUX, R. B. et al.** Echocardiographic assessment of left ventricular hypertrophy: comparison to necropsy findings. *The American Journal of Cardiology,* 1986, vol. 57, 450-458
- **AZIMZADEH, A. M. et al.** Development of a consensus protocol to quantify primate anti-non-Gal xenoreactive antibodies using pig aortic endothelial cells. *Xenotransplantation,* 2014, vol. 21, 555-566
- **TANABE, T. et al.** Role of Intrinsic (Graft) Versus Extrinsic (Host) Factors in the Growth of Transplanted Organs Following Allogeneic and Xenogeneic Transplantation. *Am. J. Transplant.,* 2017, vol. 17, 1778-1790
- **BYRNE, G. W. ; MCGREGOR, C. G. A.** Cardiac xenotransplantation: progress and challenges. *Curr Opin Organ Transplant,* 2012, vol. 17, 148-154
- **KUMAR, R. S. ; VIMALA, J.** Steroid-Induced Hypertrophic Cardiomyopathy. *APJOC,* 2010, 1-5
- **SAXTON, R. A. ; SABATINI, D. M.** mTOR Signaling in Growth, Metabolism, and Disease. *Cell,* 2017, vol. 168, 960-976
- **IMAMURA, T. et al.** Everolimus Attenuates Myocardial Hypertrophy and Improves Diastolic Function in Heart Transplant Recipients. *Int Heart J,* 2016, vol. 57, 204-210
- **PAOLETTI, E.** mTOR Inhibition and Cardiovascular Diseases: Cardiac Hypertrophy. *Transplantation,* 2017
- **MANNING, B. D.** Game of TOR - The Target of Rapamycin Rules Four Kingdoms. *N. Engl. J. Med.,* 2017, vol. 377, 1297-1299
- **SCHUURMAN, H.-J.** Microbiological safety of clinical xenotransplantation products: monitoring strategies and regulatory aspects. A commentary. *Xenotransplantation,* 2016, vol. 23, 440-443

- **FISCHER, K. et al.** Efficient production of multi-modified pigs for xenotransplantation by 'combineering', gene stacking and gene editing. *Sci Rep,* 2016, vol. 6, 29081
- **COOPER, D.K. ; NOVITZKY, D. ; BECERRA, E. ; REICHART, B..** Are there indications for heterotopic heart transplantation in 1986? A 2- to 11-year follow-up of 49 consecutive patients undergoing heterotopic heart transplantation. *The Thoracic and Cardiovascular Surgeon,* 1986, vol. 34, 300-304
- **ABICHT, J. ; MAYR, T. ; REICHART, B. ; BUCHHOLZ, S. ; WERNER, F. ; LUTZMANN, I. ; SCHMOECKEL, M. ; BAUER, A. ; THORMANN, M. ; LANGENMAYER, M.C. et al.** Preclinical heterotopic intrathoracic heart Xenotransplantation: a possibly useful clinical technique. *Xenotransplantation,* 2015
- **WYNYARD S. ; NATHU D. ; GARKAVENKO O. ; DENNER J. ; ELLIOT R..** Microbiologic safety of the first clinical pig islet xenotransplantation trial in New Zealand. *Xenotransplantation,* 2014, vol. 21, 309-323
- **LI S.** Dual fluorescent reporter pig for Cre recombination: transgene placement at the ROSA26 locus. *PLoS one,* 2014, vol. 9 (7), eI02455
- **PETERSEN B et al.** Efficient production of biallelic GGTA1 knockout pigs by cytoplasmic microinjection of CRISPR/Cas9 into zygotes. *Xenotransplantation,* 2016, vol. 23 (5), 338-346
- **COOPER DK ; EKSER B ; RAMSOONDAR J ; PHELPS C ; AYARES D.** The role of genetically engineered pigs in xenotransplantation research. *The Journal of pathology,* 2016, vol. 238 (2), 288-299
- **RIEBLINGER B et al.** Strong xenoprotective function by single-copy transgenes placed sequentially at a permissive locus. *Xenotransplantation,* 2018
- **KOURTZELIS I ; MAGNUSSON PU ; KOTLABOVA K ; LAMBRIS JD ; CHAVAKIS T.** Regulation of instant blood mediated inflammatory reaction (IBMIR) in pancreatic islet xenotransplantation: points for therapeutic inventions. *Advances in experimental medicine and biology,* 2015, vol. 865, 171-188
- **BULATO C ; RADU C ; SIMONI P.** Studies on coagulation incompatibilities for xenotransplantation. *Methods of molecular biology (Clifton, N.J.),* 2012, vol. 885, 71-89
- **COWAN PJ ; ROBSON SC.** Progress towards overcoming coagulopathy and hemostatic dysfunction associates with xenotransplantation. *Int J Surg,* 2015, vol. 23, 296-300
- **ROUSSEL JC et al.** Pig thrombomodulin binds human thrombin but is a poor cofactor for activation of human protein C and TAFI. *American journal of transplantation: official journal of the American Society of Transplantation and the American Society of Transplant Surgeons,* 2008, vol. 8 (6), 1101-1112
- **PETERSEN B et al.** Pigs transgenic for human thrombomodulin have elevated production of activated protein C. *Xenotransplantation,* 2009, vol. 16 (6), 486-495
- **YAZAKI S et al.** Production of cloned pigs expressing human thrombomodulin in endothelial cells. *Xenotransplantation,* 2012, vol. 19 (2), 82-91
- **WUENSCH A et al.** Regulatory sequences of the porcine THBD gene facilitate endothelial-specific expression of bioactive human thrombomodulin in single- and multitransgenic pigs. *Transplantation,* 2014, vol. 97 (2), 138-147
- **BYRNE GW ; MCGREGOR CG ; BREIMER ME.** Recent investigations into pig antigen and anti-pig antibody expression. *Int J Surg,* 2015, vol. 23, 223-228
- **DENNER J ; TONJES RR.** Infection barriers to successful xenotransplantation focusing on porcine endogenous retroviruses. *Clin Microbiol Rev,* 2012, vol. 25 (2), 318-343
- **PHELPS CJ et al.** Production of alpha 1,3-galactosyltransferase-deficient pigs. *Sciene,* 2003, vol. 299 (5605), 411-414
- **KLYMIUK N ; AIGNER B ; BREM G ; WOLF E.** Genetic modification of pigs as organ donors for xenotransplantation. *Molecular reproduction and development,* 2010, vol. 77 (3), 209-221
- **HAUSCHILD J et al.** Efficient generation of a biallelic knockout in pigs using zinc-finger nucleases. *Proceedings of the National Academy of Sciences of the United States of America,* 2011, vol. 108 (29), 12013-12017
- **KWON DN et al.** Production of biallelic CMP-Neu5Ac hydroxylase knock-out pigs. *Scientific reports,* 2013, vol. 3, 1981
- **LUTZ AJ et al.** Double knockout pigs deficient in N-glycolylneuraminic acid and galactose alpha-1,3-galactose reduce the humoral barrier to xenotransplantation. *Xenotransplantation,* 2013, vol. 20 (1), 27-35
- **BURLAK C et al.** Reduced binding of human antibodies to cells from GGTA1/CMAH KO pigs. *American journal of transplantation: official journal of the American Society of Transplantation and the American Society of Transplant Surgeons,* 2014, vol. 14 (8), 1895-1900
- **MIYAGAWA S et al.** Generation of alpha1,3-galactosyltransferase and cytidine monophospho-N-acetylneuraminic acid hydroxylase gene double-knockout pigs. *J Reprod Dev,* 2015, vol. 61 (5), 449-457
- **ESTRADA JL et al.** Evaluation of human and non-human primate antibody binding to pig cells lacking GGTA1/CMAH/beta4GalNT2 genes. *Xenotransplantation,* 2015, vol. 22 (3), 194-202

- **GRIESEMER A ; YAMADA K ; SYKES M.** Xenotransplantation: immunological hurdles and progress towards tolerance. *Immunol Rev,* 2014, vol. 258 (1), 241-258
- **VADORI M ; COZZI E.** The immunological barriers to xenotransplantation. *Tissue antigens,* 2015, vol. 86 (4), 239-253
- **DAWSON JR ; VIDAL AC ; MALYGUINE AM.** Natural killer cell-endothelial cell interactions in xenotransplantation. *Immunological research,* 2000, vol. 22 (2-3), 165-176
- **WEISS EH et al.** HLA-E/human beta2-microglobulin transgenic pigs: protein against xenogeneic human anti-pig natural killer cell cytotoxicity. *Transplantation,* 2009, vol. 87 (1), 35-43
- **BARTLETT ST et al.** Report from IPITA-TTS Opinion Leaders Meeting on the Future of beta-Cell Replacement. *Transplantation,* 2016, vol. 100 (2), 1-44
- **AIKIN RA.** How to kill two birds with one transgenic pig. *Diabetes,* 2012, vol. 61 (6), 1348-1349
- **KLYMIUK N et al.** Xenogafted islet cell cluster from INSLEA29Y transgenic pig rescue diabetes and prevent immune rejection in humanized mice. *Diabetes,* 2012, vol. 61 (6), 1527-1532
- **WOLF-VAN BUERCK L et al.** LEA29Y expression in transgenic neonatal porcine islet-like cluster promotes long-lasting xenograft survival in humanized mice without immunosuppressive therapy. *Scientific reports,* 2017, vol. 7 (1), 3572
- **KLOSE R et al.** Expression of biologically active human TRAIL in transgenic pigs. *Transplantation,* 2005, vol. 80 (2), 222-230
- **KEMTER E et al.** Human TNF-related apoptosis-inducing ligand-expressing dendritic cells from transgenic pigs attenuate human xenogeneic T cell responses. *Xenotransplantation,* 2012, vol. 19 (1), 40-51
- **SEOL JG et al.** Production of transgenic cloned miniature pigs with membrane-bound human Fas ligand (FasL) by somatic call nuclear transfer. *Nature Precedings,* 2010, http://hdl.handle.net/10101/npre.2010.4539.1
- **TAYLOR FB, JR. ; PEER GT ; LOCKHART MS ; FERRELL G ; ESMON CT.** Endothelial cell protein C receptor plays an important role in protein C activation in vivo. *Blood,* 2001, vol. 97 (6), 1685-1688
- **IWASE H et al.** Regulation of human platelet aggregation by genetically modified pig endothelial cells and thrombin inhibition. *Xenotransplantation,* 2014, vol. 21 (1), 72-83
- **JI H et al.** Pig BMSCs Transfected with Human TFPI Combat Species Incompatibility and Regulate the Human TF Pathway in Vitro and in a Rodent Model. *Cellular physiology and biochemistry: international journal of experimental cellular physiology, biochemistry, and pharmacology,* 2015, vol. 36 (1), 233-249
- **LEE HJ ; LEE BC ; KIM YH ; PAIK NW ; RHO HM.** Characterization of transgenic pigs that express human decay accelerating factor and cell membrane-tethered human tissue factor pathway inhibitor. *Reproduction in domestic animals = Zuchthygiene,* 2011, vol. 46 (2), 325-332
- **WIJKSTROM M et al.** Glucose metabolism in pigs expressing human genes under an insulin promoter. *Xenotransplantation,* 2015, vol. 22 (1), 70-79
- **WHEELER DG et al.** Transgenic swine: expression of human CD39 protects against myocardial injury. *Journal of molecular and cellular cardiology,* 2012, vol. 52 (2), 958-961
- **LE BAS-BERNARDET S et al.** Bortezomib, C1-inhibitor and plasma exchange do not prolong the survival of multi-transgenic GalT-KO pig kidney xenografts in baboons. *American journal of transplantation: official journal of the American Society of Transplantation and the American Society of Transplant Surgeons,* 2015, vol. 15 (2), 358-370
- **PINTORE L et al.** Clinicopathological findings in non-human primate recipients of porcine renal xenografts: quantitative and qualitative evaluation of proteinuria. *Xenotransplantation,* 2013, vol. 20 (6), 449-457
- **BOTTINO R et al.** Pig-to-monkey islet xenotransplantation using multi-transgenic pigs. *American journal of transplantation: official journal of the American Society of Transplantation and the American Society of Transplant Surgeons,* 2014, vol. 14 (10), 2275-2287
- **AHRENS HE et al.** siRNA mediated knockdown of tissue factor expression in pigs for xenotransplantation. *American journal of transplantation: official journal of the American Society of Transplantation and the American Society of Transplant Surgeons,* 2015, vol. 15 (5), 1407-1414
- **PARIS LL et al.** Reduced human platelet uptake by pig livers deficient in the asialoglycoprotein receptor 1 protein. *Xenotransplantation,* 2015, vol. 2 (3), 203-210
- **OROPEZA M et al.** Transgenic expression of the human A20 gene in cloned pigs provides protection against apoptotic and inflammatory stimuli. *Xenotransplantation,* 2009, vol. 16 (6), 522-534
- **PERTERSEN B et al.** Transgenic expression of human heme oxygenase-1 in pigs confers resistance against xenograft during ex vivo perfusion of porcine kidneys. *Xenotransplantation,* 2011, vol. 18 (6), 355-368
- **FISHMAN JA ; SCOBIE L ; TAKEUCHI Y.** Xenotransplantation-associates infectious risk: a WHO consultation. *Xenotransplantation,* 2012, vol. 19 (2), 72-81

- **MUELLER NJ ; TAKEUCHI Y ; MATTIUZZO G ; SCOBIE L.** Microbial safety in xenotransplantation. *Current opinion in organ transplantation,* 2011, vol. 16 (2), 201-206
- **FISHMAN JA.** Assessment of infectious risk in clinical xenotransplantation: the lesson for clinical allotransplantation. *Xenotransplantation,* 2014, vol. 21 (4), 307-308
- **MOROZOV VA et al.** Extended microbiological characterization of Göttingen minipigs in the context of xenotransplantation: detection and vertical transmission of hepatitis E virus. *PloS one,* 2015, vol. 10 (10), e0139893
- **DENNER J.** Immunising with the transmembrane envelope proteins of different retroviruses including HIV-1: a comparative study. *Hum Vaccin Immunother,* 2013, vol. 9 (3), 462-470
- **DIECKHOFF B et al.** Knockdown of porcine endogenous retrovirus (PERV) expression by PERV-specific shRNA in transgenic pigs. *Xenotransplantation,* 2008, vol. 15 (1), 36-45
- **RAMSOONDAR J et al.** Production of transgenic pigs that express porcine endogenous retrovirus small interfering RNAs. *Xenotransplantation,* 2009, vol. 16 (3), 164-180
- **YANG L et al.** Genome-wide inactivation of porcine endogenous retroviruses (PERVs). *Science,* 2015, vol. 350 (6264), 1101-1104
- **NIU D et al.** Inactivation of porcine endogenous retroviruses in pigs using CRISPR-Cas9. *Science,* 2017, vol. 357 (6357), 1303-1307
- **DENNER J.** Paving the Path toward Porcine Organs for Transplantation. *The New England journal of medicine,* 2017, vol. 377 (19), 1891-1893
- **DENNER J.** Advances in organ transplant from pigs. *Science,* 2017, vol. 357 (6357), 1238-1239
- **LESNIK, J.J. ; SINGH, G. K. ; BALFOUR, I.C. ; WALL, D.A.** Steroid-induced hypertrophic cardiomyopathy following stem cell transplantation in a neonate: a case report. *Bone Marrow Transplant.,* 2001, vol. 27, 1105-1108
- **SAXON, R. A. ; SABATINI, D. M.** mTOR signaling in growth, metabolism, and disease. *Cell,* 2017, vol. 168, 960-976
- **ASIAN, J.E. ; TORMOEN, G.W. ; LOREN, C.P. ; PANG, J. ; MCCARTY, O.J.** S6K1 and mTOR regulate Rac1-driven platelet activation and aggregation. *Blood,* 2011, vol. 118, 3129-3136
- **HINRICHS, A. ; B. KESSLER ; M. KUROME ; A. BLUTKE ; E. KEMTER ; M. BERNAU ; A.M. SCHOLZ ; B. RATHKOLB ; S. RENNER ; S. BULTMANN.** Growth hormone receptor-deficient pigs resemble the pathophysiology of human Laron syndrome and reveal altered activation of signaling cascades in the liver. *Mol Metab,* 2018, vol. 11, 113-128
- **BITTMANN, I. ; D. MIHICA ; R. PLESKER ; J. DENNER.** Expression of porcine endogenous retroviruses (PERV) in different organs of a pig. *Virology,* 2012, vol. 433 (2), 329-36
- **COHEN, J. ; S. BLETHEN ; J. KUNTZE ; S.L. SMITH ; K.G. LOMAX ; P.M. MATHEW.** Managing the child with severe primary insulin-like growth factor-1 deficiency (IGFD): IGFD diagnosis and management. *Drugs R D,* 2014, vol. 14 (1), 25-9
- **GUEVARA-AGUIRRE, J. ; P. BALASUBRAMANIAN ; M. GUEVARA-AGUIRRE ; M. WEI ; F. MADIA ; C.W. CHENG ; D. HWANG ; A. MARTIN-MONTALVO ; J. SAAVEDRA ; S. INGLES.** Growth hormone receptor deficiency is associated with a major reduction in pro-aging signaling, cancer, and diabetes in humans. *Sci Transl Med,* 2011, vol. 3 (70), 70-13
- **DAHLHOFF, M. ; GABORIT, N. ; BULTMANN, S. ; LEONHARDT, H. ; YARDEN, Y. ; SCHNEIDER, M.R.** CRISPR-assisted receptor deletion reveals distinct roles for ERBB2 and ERBB3 in skin keratinocytes. *FEBS Journal,* 2017, vol. 284, 3339-3349
- **UMEYAMA, K. ; HONDA, K. ; MATSUNARI, H. ; NAKANO, K. ; HIDAKA, T. ; SEKIGUCHI, K. et al.** Production of diabetic offspring using cryopreserved epididymal sperm by in vitro fertilization and intrafallopian insemination techniques in transgenic pigs. *Journal of Reproduction and Development,* 2013, vol. 59, 599-603
- **KUROME, M. ; KESSLER, B. ; WUENSCH, A. ; NAGASHIMA, H. ; WOLF, E.** Nuclear transfer and transgenesis in the pig. *Methods in Molecular Biology (Clifton, NJ),* 2015, vol. 1222, 37-59
- **BESENFELDER, U. ; MODL, J. ; MULLER, M. ; BREM, G.** Endoscopic embryo collection and embryo transfer into the oviduct and the uterus of pigs. *Theriogenology,* 1997, vol. 47, 1051-1060
- **BLUM, W.F. ; BREIER, B.H.** Radioimmunoassays for IGFs and IGFBPs. *Growth Regulation,* 1994, vol. 4 (1), 11-19
- **WIRTHGEN, E. ; HOFLICH, C. ; SPITSCHAK, M. ; HELMER, C. ; BRAND, B. ; LANGBEIN, J. et al.** Quantitative Western ligand blotting reveals common patterns and differential features of IGFBP-fingerprints in domestic ruminant breeds and species. *Growth Hormone & IGF Research,* 2016, vol. 26, 42-49
- **RENNER, S. ; FEHLINGS, C. ; HERBACH, N. ; HOFMANN, A. ; VON WALDTHAUSEN, D.C. ; KESSLER, B. et al.** Glucose intolerance and reduced proliferation of pancreatic beta-cells in transgenic pigs with impaired glucose-dependent insulinotropic polypeptide function. *Diabetes,* 2010, vol. 59, 1228-1238

- **BLUTKE, A. ; RENNER, S. ; FLENKENTHALER, F. ; BACKMAN, M. ; HAESNER, S. ; KEMTER, E. et al.** The Munich MIDY Pig Biobank - a unique resource for studying organ crosstalk in diabetes. *Molecular Metabolism,* 2017, vol. 6, 931-940
- **PAPADOPOULOS, G.A. ; ERKENS, T. ; MAES, D.G. ; PEELMAN, L.J. ; VAN KEMPEN, T.A. ; BUYSE, J. et al.** Peripartal feeding strategy with different n-6:n-3 ratios in sows: effect on gene expression in backfat white adipose tissue postpartum. *British Journal of Nutrition,* 2009, vol. 101, 197-205
- **KREMER, P.V. ; FERNANDEZ-FIGARES, I. ; FORSTER, M. ; SCHOLZ, A.M.** In vivo body composition in autochthonous and conventional pig breeding groups by dual-energy X-ray absorptiometry and magnetic resonance imaging under special consideration of Cerdo Iberico. *Animal: An International Journal of Animal Bioscience,* 2012, vol. 6, 2041-2047
- **KREMER, P.V. ; FORSTER, M. ; SCHOLZ, A.M.** Use of magnetic resonance imaging to predict the body composition of pigs in vivo. *Animal: An International Journal of Animal Bioscience,* 2013, vol. 7, 879-884
- **ALBL, B. ; HAESNER, S. ; BRAUN-REICHHART, C. ; STRECKEL, E. ; RENNER, S. ; SEELIGER, F. et al.** Tissue sampling guides for porcine biomedical models. *Toxicologic Pathology,* 2016, vol. 44, 414-420
- **STRECKEL, E. ; BRAUN-REICHHART, C. ; HERBACH, N. ; DAHLHOFF, M. ; KESSLER, B. ; BLUTKE, A. et al.** Effects of the glucagon-like peptide-1 receptor agonist liraglutide in juvenile transgenic pigs modeling a pre-diabetic condition. *Journal of Translational Medicine,* 2015, vol. 13, 73
- **YANG H ; WU Z.** Genome Editing of Pigs for Agriculture and Biomedicine. *Front Genet.,* 04 September 2018, vol. 9, 360
- **FISCHER K ; KIND A ; SCHNIEKE A.** Assembling multiple xenoprotective transgenes in pigs. *Xenotransplantation,* 28 July 2018, vol. 25 (6), e12431
- **RYU J ; PRATHER RS ; LEE K.** Use of gene-editing technology to introduce targeted modifications in pigs. *J Anim Sci Biotechnol.,* 29 January 2018, vol. 9, 5
- **LI, B. ; RUOTTI, V. ; STEWART, R. M. ; THOMSON, J. A. ; DEWEY, C. N.** RNA-Seq gene expression estimation with read mapping uncertainty. *Bioinformatics,* 2010, vol. 26, 493-500
- **KLYMIUK N ; SEELIGER F ; BOHLOOLY-Y M ; BLUTKE A ; RUDMANN DG ; WOLF E.** Tailored Pig Models for Preclinical Efficacy and Safety Testing of Targeted Therapies. *Toxicol Pathol.,* 27 October 2015, vol. 44 (3), 346-57
- **CHATTERJEE S ; SHAPIRO L ; ROSE SJ ; MUSHTAQ T ; CLAYTON PE ; TEN SB ; BHANGOO A ; KUMBATTAE U ; DIAS R ; SAVAGE MO.** Phenotypic spectrum and responses to recombinant human IGF1 (rhIGF1) therapy in patients with homozygous intronic pseudoexon growth hormone receptor mutation. *Eur J Endocrinol.,* 02 March 2018, vol. 178 (5), 481-489
- **KEMP SF.** Insulin-like growth factor-I deficiency in children with growth hormone insensitivity: current and future treatment options. *BioDrugs,* 2009, vol. 23 (3), 155-63
- **CAREL JC ; CHAUSSAIN JL ; CHATELAIN P ; SAVAGE MO.** Growth hormone insensitivity syndrome (Laron syndrome): main characteristics and effects of IGF1 treatment. *Diabetes Metab.,* July 1996, vol. 22 (4), 251-6
- **MALI P ; YANG L ; ESVELT KM ; AACH J ; GUELL M ; DICARLO JE ; NORVILLE JE ; CHURCH GM.** RNA-guided human genome engineering via Cas9. *Science,* 15 February 2013, vol. 339 (6121), 823-6
- **OSMAN N ; MCKENZIE IF ; OSTENRIED K ; IOANNOU YA ; DESNICK RJ ; SANDRIN MS.** Combined transgenic expression of alpha-galactosidase and alpha1,2-fucosyltransferase leads to optimal reduction in the major xenoepitope Galalpha(1,3)Gal. *Proc Natl Acad Sci U S A.,* 23 December 1997, vol. 94 (26), 14677-82
- **LOVELAND BE ; MILLAND J ; KYRIAKOU P ; THORLEY BR ; CHRISTIANSEN D ; LANTERI MB ; VAN REGENSBURG M ; DUFFIELD M ; FRENCH AJ ; WILLIAMS L.** Characterization of a CD46 transgenic pig and protection of transgenic kidneys against hyperacute rejection in non-immunosuppressed baboons. *Xenotransplantation,* 2004, vol. 11, 171-183
- **YAKAR S ; LIU JL ; STANNARD B ; BUTLER A ; ACCILI D ; SAUER B ; LEROITH D.** Normal growth and development in the absence of hepatic insulin-like growth factor I. *Proc Natl Acad Sci U S A.,* 22 June 1999, vol. 96 (13), 7324-9
- **LÄNGIN M ; MAYR T ; REICHART B ; MICHEL S ; BUCHHOLZ S ; GUETHOFF S ; DASHKEVICH A ; BAEHR A ; EGERER S ; BAUER A et al.** Consistent success in life-supporting porcine cardiac xenotransplantation. *Nature,* 05 December 2018, vol. 564 (7736), 430-433
- **KONUS OL ; OZDEMIR A ; AKKAYA A ; ERBAS G ; CELIK H ; ISIK S.** Normal liver, spleen, and kidney dimensions in neonates, infants, and children: evaluation with sonography. *AJR Am J Roentgenol.,* December 1998, vol. 171 (6), 1693-8
- **RAO UV ; WAGNER HN JR.** Normal weights of human organs. *Radiology,* February 1972, vol. 102 (2), 337-9

<110> REICHART, Bruno

<120> Methods and compositions for prolonging the survival after orthotopic and heterotopic xenogeneic heart, kidney, lung or liver transplantations

<130> 213 592

<150> EP 18 165 107.6
<151> 2018-03-29
<150> US 62/650,085
<151> 2018-03-29

<160> 18

<170> BiSSAP 1.3.6

<210> 1
<211> 1152
<212> DNA
<213> Artificial Sequence

<220>
<223> Codon optimized variant LEA29Y

<400> 1
atgggggtac tgctcacaca gaggacgctg ctcagtctgg tccttgcact cctgtttcca 60

agcatggcga gcatggcgat gcacgtggcc cagcctgctg tggtactggc cagcagccga 120

ggcatcgcca gctttgtgtg tgagtatgca tctccaggca aatatactga ggtccgggtg 180

acagtgcttc ggcaggctga cagccaggtg actgaagtct gtgcggcaac ctacatgatg 240

gggaatgagt tgaccttcct agatgattcc atctgcacgg gcacctccag tggaaatcaa 300

gtgaacctca ctatccaagg actgagggcc atggacacgg gactctacat ctgcaaggtg 360

gagctcatgt acccaccgcc atactacgag ggcataggca acggaaccca gatttatgta 420

attgatccag aaccgtgccc agattctgat caggagccca aatcttctga caaaactcac 480

acatccccac cgtccccagc acctgaactc ctgggggggat cgtcagtctt cctcttcccc 540

ccaaaaccca aggacaccct catgatctcc cggacccctg aggtcacatg cgtggtggtg 600

gacgtgagcc acgaagaccc tgaggtcaag ttcaactggt acgtggacgg cgtggaggtg 660

cataatgcca agacaaagcc gcgggaggag cagtacaaca gcacgtaccg ggtggtcagc 720

gtcctcaccg tcctgcacca ggactggctg aatggcaagg agtacaagtg caaggtctcc 780

aacaaagccc tcccagcccc catcgagaaa accatctcca aagccaaagg gcagccccga 840

gaaccacagg tgtacaccct gcccccatcc cgggatgagc tgaccaagaa ccaggtcagc 900

ctgacctgcc tggtcaaagg cttctatccc agcgacatcg ccgtggagtg ggagagcaat 960

gggcagccgg agaacaacta caagaccacg cctcccgtgc tggactccga cggctccttc 1020

ttcctctaca gcaagctcac cgtggacaag agcaggtggc agcaggggaa cgtcttctca 1080

tgctccgtga tgcatgaggc tctgcacaac cactacacgc agaagagcct ctccctgtct 1140

ccgggtaaat ga 1152

<210> 2
<211> 43
<212> DNA
<213> Sus

<220>
<223> GHR wild type

<400> 2
tagagacttt ttcatgccac tggacagatg gggtccgtca cgg 43

<210> 3
<211> 44
<212> DNA
<213> Artificial Sequence

<220>
<223> GHR mutant #2529

<400> 3
tagagacttt ttcatgccac tggacaggat ggggtccgtc acgg 44

<210> 4
<211> 50
<212> DNA
<213> Artificial Sequence

<220>
<223> GHR mutant #2533

<400> 4
tagagacttt ttcatgccac tggacagggc atggatgggg tccgtcacgg 50

<210> 5
<211> 15
<212> DNA
<213> Sus

<220>
<223> GHR wild type

<400> 5
ctggacagat ggggt 15

<210> 6
<211> 13
<212> DNA
<213> Artificial Sequence

<220>
<223> GHR mutant

<400> 6
ctgtccatgg ggt 13

<210> 7
<211> 12
<212> DNA
<213> Artificial Sequence

<220>
<223> GHR mutant

<400> 7
ctggacaggg gt 12

<210> 8
<211> 20
<212> DNA
<213> Sus

<220>
<223> GHR

<400> 8
ttcatgccac tggacagatg 20

<210> 9
<211> 20

<212> DNA
<213> Artificial Sequence

<220>
<223> Primer

<400> 9
accgctctga agctgtgacc 20

<210> 10
<211> 21
<212> DNA
<213> Artificial Sequence

<220>
<223> Primer

<400> 10
caccctcaga tactctcatg c 21

<210> 11
<211> 23
<212> RNA
<213> Sus

<220>
<223> GHR

<400> 11
ttcatgccac tggacagatg ggg 23

<210> 12
<211> 22
<212> DNA
<213> Artificial Sequence

<220>
<223> Primer

<400> 12
gcacacttca gatgctacct aa 22

<210> 13
<211> 21
<212> DNA
<213> Artificial Sequence

<220>
<223> Primer

<400> 13
cacatgctca ccctcagata c 21

<210> 14
<211> 20
<212> DNA
<213> Artificial Sequence

<220>
<223> Primer

<400> 14
accgctctga agctgtgacc 20

<210> 15
<211> 22
<212> DNA
<213> Artificial Sequence

<220>
<223> Primer

<400> 15
caggagacca gagaccttat ct 22

<210> 16
<211> 15
<212> DNA
<213> Sus

<220>
<223> GHR

<400> 16
ctggacagat ggggt 15

<210> 17
<211> 13
<212> DNA
<213> Artificial Sequence

<220>
<223> GHR mutant

<400> 17
ctgtccatgg ggt 13

<210> 18
<211> 12
<212> DNA
<213> Artificial Sequence

<220>
<223> GHR mutant

<400> 18
ctggacaggg gt 12